# EUROPEAN PATENT APPLICATION

(11) **EP 4 066 844 A1**
(43) Date of publication of application: **05.10.2022**
(21) Application number: 22154748.2
(22) Date of filing: 03.07.2020
(51) Int. Cl.: A61K 35/74, A23L 33/135, A61K 9/19, C12R 1/01, A61P 25/00, A61P 25/24, A61P 25/28, A61P 35/00, A61K 35/742, C12N 1/20, A61K 9/00

(54) **COMPOSITIONS COMPRISING ANAEROSTIPES HADRUS**

(30) Priority: 05.07.2019 EP 19184768; 13.01.2020 GB 202000437; 11.02.2020 EP 20156697
(62) Divisional of application: 20736337.5
(71) Applicant: 4D Pharma Research Limited, Aberdeen, Aberdeenshire AB25 2ZS (GB)
(72) Inventor: MULDER, Imke Elisabeth, Aberdeen, AB25 2ZS (GB); REICHARDT, Nicole, Aberdeen, AB25 2ZS (GB); SAVIGNAC, Helene, Aberdeen, AB25 2ZS (GB); CHETAL, Sasha, Aberdeen, AB25 2ZS (GB); DINAN, Ted, Cobh (IE); CRYAN, John, Cork (IE); YUILLE, Samantha, Aberdeen, AB25 2ZS (GB)
(74) Representative: Brand Murray Fuller LLP

(57) **Abstract**

The invention provides pharmaceutical compositions comprising bacteria and the use of such compositions in the treatment of disease.

SEQUENCE LISTING

<110>   4D PHARMA RESEARCH LIMITED

<120>   COMPOSITIONS COMPRISING BACTERIAL STRAINS

<130>   P12653WOEPDIV

<141>   2020-07-03

<150>   EP 19184768.0
<151>   2019-07-05

<150>   GB 2000437.0
<151>   2020-01-13

<150>   EP 20156697.3
<151>   2020-02-11

<160>   16

<170>   SeqWin2010, version 1.0

<210>   1
<211>   1521
<212>   DNA
<213>   Anaerostipes hadrus

<400>   1
tttgatcctg gctcaggatg aacgctggcg gcgtgcttaa cacatgcaag tcgaacgaaa   60
caccttattt gattttcttc ggaactgaag atttggtgat tgagtggcgg acgggtgagt   120
aacgcgtggg taacctaccc tgtacagggg gataacagtc agaaatgact gctaataccg   180
cataagacca cagcaccgca tggtgcaggg gtaaaaactc cggtggtaca ggatggaccc   240
gcgtctgatt agctggttgg tgaggtaacg gctcaccaag gcgacgatca gtagccggct   300
tgagagagtg aacggccaca ttgggactga gacacggccc aaactcctac gggaggcagc   360
agtggggaat attgcacaat gggggaaacc ctgatgcagc gacgccgcgt gagtgaagaa   420
gtatctcggt atgtaaagct ctatcagcag ggaagaaaat gacggtacct gactaagaag   480
ccccggctaa ctacgtgcca gcagccgcgg taatacgtag ggggcaagcg ttatccggaa   540
ttactgggtg taaagggtgc gtaggtggta tggcaagtca gaagtgaaaa cccagggctt   600
aactctggga ctgcttttga aactgtcaga ctggagtgca ggagaggtaa gcggaattcc   660
tagtgtagcg gtgaaatgcg tagatattag gaggaacatc agtggcgaag gcggcttact   720
ggactgaaac tgacactgag gcacgaaagc gtggggagca aacaggatta gataccctgg   780
tagtccacgc cgtaaacgat gaatactagg tgtcggggcc gtagaggctt cggtgccgca   840
gccaacgcag taagtattcc acctggggag tacgttcgca agaatgaaac tcaaaggaat   900
tgacggggac ccgcacaagc ggtggagcat gtggtttaat tcgaagcaac gcgaagaacc   960
ttacctggtc ttgacatcct tctgaccggt ccttaaccgg acctttcctt cgggacagga   1020
gagacaggtg gtgcatggtt gtcgtcagct cgtgtcgtga gatgttgggt taagtcccgc   1080
aacgagcgca acccttatct ttagtagcca gcatataagg tgggcactct agagagactg   1140
ccagggataa cctggaggaa ggtggggacg acgtcaaatc atcatgcccc ttatgaccag   1200
ggctacacac gtgctacaat ggcgtaaaca gagggaagca gcctcgtgag agtgagcaaa   1260
tcccaaaaat aacgtctcag ttcggattgt agtctgcaac tcgactacat gaagctggaa   1320
tcgctagtaa tcgcgaatca gaatgtcgcg gtgaatacgt tcccgggcct tgtacacacc   1380
gcccgtcaca ccatgggagt cagtaacgcc cgaagtcagt gacccaaccg taaggaggga   1440
gctgccgaag gcgggaccga taactggggt gaagtcgtaa caaggtagcc gtatcggaag   1500
gtgcggctgg atcacctcct t                                             1521

<210>   2
<211>   1434
<212>   DNA
<213>   Anaerostipes hadrus

<400>   2
gatgaacgct ggcggcgtgc ttaacacatg caagtcgaac gaaacacctt atttgatttt   60

cttcggaact gaagatttgg tgattgagtg gcggacgggt gagtaacgcg tgggtaacct 120
gccctgtaca gggggataac agtcagaaat gactgctaat accgcataag accacagcac 180
cgcatggtgc aggggtaaaa actccggtgg tacaggatgg acccgcgtct gattagctgg 240
ttggtgaggt aacggctcac caaggcgacg atcagtagcc ggcttgagag agtgaacggc 300
cacattggga ctgagacacg gcccaaactc ctacgggagg cagcagtggg gaatattgca 360
caatggggga aaccctgatg cagcgacgcc gcgtgagtga agaagtatct cggtatgtaa 420
agctctatca gcagggaaga aaatgacggt acctgactaa gaagccccgg ctaactacgt 480
gccagcagcc gcggtaatac gtagggggca agcgttatcc ggaattactg ggtgtaaagg 540
gtgcgtaggt ggtatggcaa gtcagaagtg aaaacccagg gcttaactct gggactgctt 600
ttgaaactgt cagactggag tgcaggagag gtaagcggaa ttcctagtgt agcggtgaaa 660
tgcgtagata ttaggaggaa catcagtggc gaaggcggct tactggactg aaactgacac 720
tgaggcacga aagcgtgggg agcaaacagg attagatacc ctggtagtcc acgccgtaaa 780
cgatgaatac taggtgtcgg ggccgtagag gcttcggtgc cgcagccaac gcagtaagta 840
ttccacctgg ggagtacgtt cgcaagaatg aaactcaaag gaattgacgg ggacccgcac 900
aagcggtgga gcatgtggtt taattcgaag caacgcgaag aaccttacct ggtcttgaca 960
tccttctgac cggtccttaa ccggaccttt ccttcgggac aggagagaca ggtggtgcat 1020
ggttgtcgtc agctcgtgtc gtgagatgtt gggttaagtc ccgcaacgag cgcaacccct 1080
atctttagta gccagcatat aaggtgggca ctctagagag actgccaggg ataacctgga 1140
ggaaggtggg gacgacgtca aatcatcatg ccccttatga ccagggctac acacgtgcta 1200
caatggcgta aacagaggga agcagcctcg tgagagtgag caaatcccaa aaataacgtc 1260
tcagttcgga ttgtagtctg caactcgact acatgaagct ggaatcgcta gtaatcgcga 1320
atcagaatgt cgcggtgaat acgttcccgg gtcttgtaca caccgcccgt cacaccatgg 1380
gagtcagtaa cgcccgaagt cagtgaccca accgtaagga gggagctgcc gaag 1434

<210> 3
<211> 1381
<212> DNA
<213> Anaerostipes butyraticus

<400> 3
ggctcaggat gaacgctggc ggcgtgctta acacatgcaa gtcgaacgaa gcttgatccc 60
ggatcccttc ggggtgacgg gtgatatgac tgagtggcgg acgggtgagt aacgcgtggg 120
caacctgcct catacagggg gataacagtt agaaatgact gctaagaccg cataagacca 180
cagcaccgca tggtggagtg gtaaaagctc cggtggtatg agatgggccc gcgtctgatt 240
agctggttgg tgaggtaacg gctctaccaa ggcaacgatc agtagccggc ttgagagagt 300
gaacggccac attgggactg agacacggcc caaactccta cgggaggcag cagtggggaa 360
tattgcacaa tgggggaaac cctgatgcag cgacgccgcg tgagtgaaga agtatttcgg 420
tatgtaaagc tctatcagca gggaagaaaa tggacggtac ctgactaaga agccccggct 480
aactacgtgc cagcagccgc ggtaatacgt agggggcaag cgttatccgg aattactggg 540
tgtaaagggt gcgtaggtgg cagggcaagt cagaagtgaa aacccggggc tcaactccgg 600
gactgctttt gaaactgtcc agctggagtg caggagaggt aagcggaatt cctagtgtag 660
cggtgaaatg cgtagatatt aggaggaaca tcagtggcga aggcggctta ctggactgaa 720
actgacactg aggcacgaaa gcgtggggag caaacaggat tagataccct ggtagtccac 780
gccgtaaacg atgaatacta ggtgtcgggg ccgtagaggc ttcggtgccg cagcaaacgc 840
attaagtatt ccacctgggg agtacgttcg caagaatgaa actcaaagga attgacgggg 900
acccgcacaa gcggtggagc atgtggttta attcgcaagc aacgcgaaga accttacctg 960
gtcttgacat ccccttgacc gctccttaat cggagttttc cttcgggaca agggtgacag 1020
gtggtgcatg gttgtcgtca gctcgtgtcg tgagatgttg ggttaagtcc cgcaacgagc 1080
gcaaccccta tctttagtag ccagcacggg aaaggtgggc actctagaga gactgccagg 1140
gagaacctgg aggaaggtgg gggacgacgt caaatcatca tgccccttat gaccagggct 1200
acacacgtgc tacaatggcg taaacaaagg gaagcgaccc tgtgaaggca agcaaatccc 1260
aaaaataacg tcacagttcg gattgtagtc tgcaactcga ctacatgaag ctggaatcgc 1320
tagtaatcgc gaatcagaat gtcgcggtga atacgttccc gggtcttgta cacaccgccc 1380
g 1381

<210> 4
<211> 1456
<212> DNA
<213> Anaerostipes rhamnosivorans

<400> 4
gcgcttaata catgtcaagt cgaacgaagc atttaggatt gaagttttcg gatggatttc 60
ctatatgact gagtggcgga cgggtgagta acgcgtgggg aacctgccct atacaggggg 120

ataacagctg gaaacggctg ctaataccgc ataagcgcac agaatcgcat gattcagtgt 180
gaaaagccct ggcagtatag gatggtcccg cgtctgatta gctggttggt gaggtaacgg 240
ctcaccaagg cgacgatcag tagccggctt gagagagtga acggccacat tgggactgag 300
acacggccca aactcctacg ggaggcagca gtggggaata ttgcacaatg ggggtaaacc 360
ctgatgcagc gacgccgcgt gagtgaagaa gtatttcggt atgtaaagct ctatcagcag 420
ggaagaaaac agacggtacc tgactaagaa gccccggcta actacgtgcc agcagccgcg 480
gtaatacgta gggggcaagc gttatccgga attactgggt gtaaagggtg cgtaggtggc 540
atggtaagtc agaagtgaaa gcccggggct taaccccggg actgcttttg aaactgtcat 600
gctggagtgc aggagaggta agcggaattc ctagtgtagc ggtgaaatgc gtagatatta 660
ggaggaacac cagtggcgaa ggcggcttac tggactgtca ctgacactga tgcacgaaag 720
cgtggggagc aaacaggatt agataccctg gtagtccacg ccgtaaacga tgaatactag 780
gtgtcggggc cgtagaggct tcggtgccgc agcaaacgca gtaagtattc cacctgggga 840
gtacgttcgc aagaatgaaa ctcaaaggaa ttgacgggga cccgcacaag cggtggagca 900
tgtggtttaa ttcgaagcaa cgcgaagaac cttacctggt cttgacatcc caatgaccga 960
accttaaccg gttttttctt tcgagacatt ggagacaggt ggtgcatggt tgtcgtcagc 1020
tcgtgtcgtg agatgttggg ttaagtcccg caacgagcgc aacccctatc tttagtagcc 1080
agcatttaag gtgggcactc tagagagact gccagggata acctggagga aggtggggac 1140
gacgtcaaat catcatgccc cttatggcca gggctacaca cgtgctacaa tggcgtaaac 1200
aaagggaagc gaagtcgtga ggcgaagcaa atcccagaaa taacgtctca gttcggattg 1260
tagtctgcaa ctcgactaca tgaagctgga atcgctagta atcgtgaatc agaatgtcac 1320
ggtgaatacg ttcccgggtc ttgtacacac cgcccgtcac accatgggag tcagtaacgc 1380
ccgaagtcag tgacccaacc gcaaggaggg agctgccgaa ggtgggaccg ataactgggg 1440
tgaagtcgta acaagg 1456

<210> 5
<211> 1456
<212> DNA
<213> Anaerostipes caccae

<400> 5
gcgcttaata catgtcaagt cgaacgaagc atttaggatt gaagttttcg gatggatttc 60
ctatatgact gagtggcgga cgggtgagta acgcgtgggg aacctgccct atacaggggg 120
ataacagctg gaaacggctg ctaataccgc ataagcgcac agaatcgcat gattcagtgt 180
gaaaagccct ggcagtatag gatggtcccg cgtctgatta gctggttggt gaggtaacgg 240
ctcaccaagg cgacgatcag tagccggctt gagagagtga acggccacat tgggactgag 300
acacggccca aactcctacg ggaggcagca gtggggaata ttgcacaatg ggggtaaacc 360
ctgatgcagc gacgccgcgt gagtgaagaa gtatttcggt atgtaaagct ctatcagcag 420
ggaagaaaac agacggtacc tgactaagaa gccccggcta actacgtgcc agcagccgcg 480
gtaatacgta gggggcaagc gttatccgga attactgggt gtaaagggtg cgtaggtggc 540
atggtaagtc agaagtgaaa gcccggggct taaccccggg actgcttttg aaactgtcat 600
gctggagtgc aggagaggta agcggaattc ctagtgtagc ggtgaaatgc gtagatatta 660
ggaggaacac cagtggcgaa ggcggcttac tggactgtca ctgacactga tgcacgaaag 720
cgtggggagc aaacaggatt agataccctg gtagtccacg ccgtaaacga tgaatactag 780
gtgtcggggc cgtagaggct tcggtgccgc agcaaacgca gtaagtattc cacctgggga 840
gtacgttcgc aagaatgaaa ctcaaaggaa ttgacgggga cccgcacaag cggtggagca 900
tgtggtttaa ttcgaagcaa cgcgaagaac cttacctggt cttgacatcc caatgaccga 960
accttaaccg gttttttctt tcgagacatt ggagacaggt ggtgcatggt tgtcgtcagc 1020
tcgtgtcgtg agatgttggg ttaagtcccg caacgagcgc aacccctatc tttagtagcc 1080
agcatttaag gtgggcactc tagagagact gccagggata acctggagga aggtggggac 1140
gacgtcaaat catcatgccc cttatggcca gggctacaca cgtgctacaa tggcgtaaac 1200
aaagggaagc gaagtcgtga ggcgaagcaa atcccagaaa taacgtctca gttcggattg 1260
tagtctgcaa ctcgactaca tgaagctgga atcgctagta atcgtgaatc agaatgtcac 1320
ggtgaatacg ttcccgggtc ttgtacacac cgcccgtcac accatgggag tcagtaacgc 1380
ccgaagtcag tgacccaacc gcaaggaggg agctgccgaa ggtgggaccg ataactgggg 1440
tgaagtcgta acaagg 1456

<210> 6
<211> 1115
<212> DNA
<213> Anaerostipes hadrus NCIMB 43457

<220>
<221> s

<222>   1027
<223>   a, t, c or g

<220>
<221>   y
<222>   1030
<223>   a, t, c or g

<220>
<221>   s
<222>   1035
<223>   a, t, c or g

<220>
<221>   s
<222>   1058
<223>   a, t, c or g

<220>
<221>   w
<222>   1064
<223>   a, t, c or g

<400>   6
ttacggttgg gtcactgact tcgggcgtta ctgactccca tggtgtgacg ggcggtgtgt   60
acaagacccg ggaacgtatt caccgcgaca ttctgattcg cgattactag cgattccagc   120
ttcatgtagt cgagttgcag actacaatcc gaactgagac gttatttttg ggatttgctc   180
gacctcgcgg ttctgcctcc ctttgtttac gccattgtag cacgtgtgta gccctgctca   240
taaggggcat gatgatttga cgtcatcccc accttcctcc aggttatccc tggcagtctc   300
tctagagtgc ccggccagac cgctggctac taaagatagg ggttgcgctc gttgcgggac   360
ttaacccaac atctcacgac acgagctgac gacaaccatg caccacctgt catccctgtc   420
ccgaaggaaa ggcaacatta cttgccggtc agggagatgt caagagcagg taaggttctt   480
cgcgttgctt cgaattaaac cacatgctcc accgcttgtg cgggtccccg tcaattcctt   540
tgagtttcat tcttgcgaac gtactcccca ggtggactac ttattgcgtt tgctgcggca   600
ccgaacagct ttgctgcccg acacctagta gtcatcgttt acggcgtgga ctaccagggt   660
atctaatcct gtttgctccc cacgctttcg agcctcaacg tcagttaccg tccagtaagc   720
cgccttcgcc actggtgttc ctcctaatat ctacgcattt caccgctaca ctaggaattc   780
cgcttacctc tccggtactc tagattgaca gtttccaatg cagtcccggg gttgagcccc   840
gggttttcac atcagacttg ccactccgtc tacgctccct ttacacccag taaatccgga   900
taacgcttgc accatacgta ttaccgcggc tgctggcacg tatttagccg gtgcttctta   960
gtcaggtacc gtcattttct tccctgctga tagagcttta cataccgaaa tacttcatcg   1020
ctcacgsggy gtcgstgcat cagggtttcc cccattgsac aatwttcccc actgctgcct   1080
cctgtaggag tgggggccgt gtctcagtca caatg   1115

<210>   7
<211>   1406
<212>   DNA
<213>   Anaerostipes hadrus NCIMB 43526

<220>
<221>   k
<222>   333
<223>   a, t, c or g

<220>
<221>   w
<222>   335
<223>   a, t, c or g

<220>
<221>   w
<222>   428
<223>   a, t, c or g

<220>
<221>   y
<222>   817
<223>   a, t, c or g

<400>   7
ttcggcagct ccctccttac ggttgggtca ctgacttcgg gcgttactga ctcccatggt   60
gtgacgggcg gtgtgtacaa gacccgggaa cgtattcacc gcgacattct gattcgcgat   120
tactagcgat tccagcttca tgtagtcgag ttgcagacta caatccgaac tgagacgtta   180
tttttgggat ttgctcactc tcacgaggct gcttccctct gtttacgcca ttgtagcacg   240
tgtgtagccc tggtcataag gggcatgatg atttgacgtc gtccccacct tcctccaggt   300
tatccctggc agtctctcta gagtgcccac ctkawatgct ggctactaaa gataggggtt   360
gcgctcgttg cgggacttaa cccaacatct cacgacacga gctgacgaca accatgcacc   420
acctgtcwct cctgtcccga aggaaaagtc cggttaagga ccggtcagaa ggatgtcaag   480
accaggtaag gttcttcgcg ttgcttcgaa ttaaaccaca tgctccaccg cttgtgcggg   540
tccccgtcaa ttcctttgag tttcattctt gcgaacgtac tccccaggtg gaatacttac   600
tgcgttggct gcggcaccga agcctctacg gccccgacac ctagtattca tcgtttacgg   660
cgtggactac cagggtatct aatcctgttt gctccccacg ctttcgtgcc tcagtgtcag   720
tttcagtcca gtaagccgcc ttcgccactg atgttcctcc taatatctac gcatttcacc   780
gctacactag gaattccgct tacctctcct gcactcyagt ctgacagttt caaaagcagt   840
cccagagtta agccctgggt tttcacttct gacttgccat accacctacg caccctttac   900
acccagtaat tccggataac gcttgccccc tacgtattac cgcggctgct ggcacgtagt   960
tagccggggc ttcttagtca ggtaccgtca ttttcttccc tgctgataga gctttacata   1020
ccgagatact tcttcactca cgcggcgtcg ctgcatcagg gtttccccca ttgtgcaata   1080
ttccccactg ctgcctcccg taggagtttg ggccgtgtct cagtcccaat gtggccgttc   1140
actctctcaa gccggctact gatcgtcgcc ttggtgagcc gttacctcac caaccagcta   1200
atcagacgcg ggtccatcct gtaccaccgg agtttttacc cctgcaccat gcggtgctgt   1260
ggtcttatgc ggtattagca gtcatttctg actgttatcc ccctgtacag ggcaggttac   1320
ccacgcgtta ctcacccgtc cgccactcaa tcaccaaatc ttcagttccg aagaaaatca   1380
aataaggtgt ttcgttcgac ttgcat                                        1406

<210>   8
<211>   1392
<212>   DNA
<213>   consensus 16S ribosomal RNA sequence from the Eubacterium callanderi
strain deposited under accession number NCIMB 43455

<400>   8
ctgctgagtc cttgcggttc tctcacaggc ttcgggtgtt gccaactctc gtggtgtgac   60
gggcggtgtg tacaagaccc gggaacgcat tcaccgcggc attctgatcc gcgattacta   120
gcaactccaa cttcatgcag gcgagttgca gcctgcaatc cgaactggga tctgttttaa   180
gggatttgct tcacctcgcg gcttcgcggc cctctgttca gaccattgta gcacgtgtgt   240
agcccaggtc ataaggggca tgatgatttg acgtcatccc caccttcctc cgtattgtct   300
acggcagtcc ctctagagtg cccaactgaa tgctggcaac taaaggcagg ggttgcgctc   360
gttgcgggac ttaacccaac atctcacgac acgagctgac gacaaccatg caccacctgt   420
ctctctgttc ccgaaggaaa cttcctatct ctaggctcgt cagaggatgt caagacctgg   480
taaggttctt cgcgttgctt cgaattaaac cacatgctcc gctgcttgtg cgggtccccg   540
tcaattcctt tgagtttcaa ccttgcggtc gtactcccca ggcggaatgc ttattgtgtt   600
aactgcggca ctgagtttcc ccaacaccta gcattcatcg tttacggcgt ggactaccag   660
ggtatctaat cctgttcgct ccccacgctt tcgcacctca gcgtcagtat ttgtccagca   720
agccgccttc gccactggtg ttcctcctaa tatctacgca tttcaccgct acactaggaa   780
ttccacttgc ctctccaata ctcaagtcta ccagtttcca atgcacttca ccggttgagc   840
cggtaccttt cacatcagac ttaatagacc gcctacgcgc cctttacgcc cagtcattcc   900
ggacaacgct tgtcccctac gtattaccgc ggctgctggc acgtagttag ccgggacttc   960
ctcattgggt accgtcatgt tttcttcccc aataacagag ctttacgatc cgaaaacctt   1020
cttcactcac gcggtattgc tgcgtcaggg ttgcccccat tgcgcaatat tccccactgc   1080
tgcctcccgt aggagtctgg accgtgtctc agttccagtg tgaccgttcg ccctctcaga   1140
ccggttaccc atcgtcgcct tggtgggctg ttatctcacc aactagctaa tgggacgcgg   1200
gtccatccta tggcaccgga gttttcatga tcttgccatg cgacaaaacc ataatataag   1260
gctttactcc cagtttcccg aggctattcc tttccatagg gcaggttacc cacgcgttac   1320
tcacccgttc gccactttcc agttctaatt tcacccgaag gatcattcaa aaccttctcg   1380
ttcgactgca tg                                                       1392

<210>   9
<211>   1363
<212>   DNA
<213>   consensus 16S ribosomal RNA sequence from Eubacterium eligens strain
E. eligens strain ref. 1

<400>   9
ttcggcggct ccttctttcg ttaggtcacc gacttcgggc attttcgact cccatggtgt   60
gacgggcggt gtgtacaaga cccgggaacg tattcaccgc agcattctga tctgcgatta   120
ctagcgattc cagcttcatg tagtcgagtt gcagactaca atccgaactg agacgttatt   180
tttgtgattt gcttggcctc acgacttcgc ttcactttgt ttacgccatt gtagcacgtg   240
tgtagcccaa gtcataaggg gcatgatgat ttgacgtcat ccccaccttc ctccaggtta   300
tccctggcag tctccctaga gtgcccatct tactgctggc tactaaggat aggggttgcg   360
ctcgttgcgg gacttaaccc aacatctcac gacacgagct gacgacaacc atgcaccacc   420
tgtcaccact gtcccgaagg aaaggacaca ttactgtccg gtcagtggga tgtcaagact   480
tggtaaggtt cttcgcgttg cttcgaatta aaccacatgc tccaccgctt gtgcgggtcc   540
ccgtcaattc ctttgagttt cattcttgcg aacgtactcc ccaggtggaa tacttattgc   600
gtttgctgcg gcaccgaagc ccttatgggc cccgacacct agtattcatc gtttacggcg   660
tggactacca gggtatctaa tcctgtttgc tccccacgct ttcgagcctc agtgtcagtt   720
acagtccagt gagccgcctt cgccactggt gttcctccta atatctacgc atttcaccgc   780
tacactagga attccactca cccctcctgc actctagcct tacagtttca aaagcagttc   840
cggggttgag ccccggattt tcacttctga cttgcatggc cacctacact ccctttacac   900
ccagtaaatc cggataacgc ttgctccata cgtattaccg cggctgctgg cacgtattta   960
gccggagctt cttagtcagg taccgtcact atcttccctg ctgatagagc tttacataac   1020
gaattacttc ttcactcacg cggcgtcgct gcatcagagt ttcctccatt gtgcaatatt   1080
ccccactgct gcctcccgta ggagtctggg ccgtgtctca gtcccaatgt ggccggtcac   1140
cctctcaggt cggctactga tcgtcgcctt ggtgggctgt tatctcacca actagctaat   1200
cagacgcggg tccatcttat accaccggag tttttcacac catgtcatgc aacattgtgc   1260
gcttatgcgg tattaccagc cgtttccagc tgctatcccc cagtacaagg caggttaccc   1320
acgcgttact cacccgtccg ccactcagtc ataagcaact tca   1363

<210>   10
<211>   1396
<212>   DNA
<213>   consensus 16S ribosomal RNA sequence from Eubacterium rectale strain
E. rectale strain ref. 2

<400>   10
gctccttcct ttcggttagg tcactggctt cgggcattcc caactcccat ggtgtgacgg   60
gcggtgtgta caagacccgg gaacgtattc accgcagcat tctgatctgc gattactagc   120
gattccagct tcgtgtagtc gggttgcaga ctacagtccg aactgagacg ttatttttga   180
gatttgctcg gcttcacagc tttgcttccc tttgtttacg ccattgtagc acgtgtgtag   240
cccaagtcat aaggggcatg atgatttgac gtcatccccg ccttcctcca ggttatccct   300
ggcagtctct ctagagtgcc cggccgaacc gctggctact aaagataagg gttgcgctcg   360
ttgcgggact taacccaaca tctcacgaca cgagctgacg acaaccatgc accacctgtc   420
actcctgctc cgaagagaag gtacggttaa gtaccggtca gaaggatgtc aagacttggt   480
aaggttcttc gcgttgcttc gaattaaacc acatgctcca ccgcttgtgc gggtccccgt   540
caattccttt gagtttcatt cttgcgaacg tactccccag gtggaatact tactgcgttt   600
gcgacggcac cgagaagcaa tgcttcccaa cacctagtat tcatcgttta cggcgtggac   660
taccagggta tctaatcctg tttgctcccc acgctttcga gcctcagcgt cagttatcgt   720
ccagtaagcc gccttcgcca ctggtgttcc tcctaatatc tacgcatttc accgctacac   780
taggaattcc gcttacccct ccgacactct agtacgacag tttccaatgc agtaccgggg   840
ttgagccccg ggctttcaca tcagacttgc cgcaccgcct gcgctccctt tacacccagt   900
aaatccggat aacgcttgca ccatacgtat taccgcggct gctggcacgt atttagccgg   960
tgcttcttag tcaggtaccg tcattatctt ccctgctgat agagctttac ataccgaaat   1020
acttcttcgc tcacgcggcg tcgctgcatc aggctttcgc ccattgtgca atattcccca   1080
ctgctgcctc ccgtaggagt ttgggccgtg tctcagtccc aatgtggccg gtcaccctct   1140
caggtcggct atggatcgtc gccttggtgg gccgttacct caccaactag ctaatccaac   1200
gcgggtccat cttataccac cggagttttt cacactgcat catgcgatgc tgtgcgctta   1260
tgcggtatta gcagccgttt ccaactgtta tccccctgta caaggcaggt tacccacgcg   1320
ttactcaccc gtccgccact cagtcacaaa ataatcagtc ccgaaggaaa tcaaataaag   1380
tgcttcgtcg actgca   1396

<210>    11
<211>    1397
<212>    DNA
<213>    consensus 16S ribosomal RNA sequence from Eubacterium rectale strain
E. rectale strain ref. 1

<400>    11
gctccttcct ttcggttagg tcactggctt cgggcattcc caactcccat ggtgtgacgg   60
gcggtgtgta caagacccgg gaacgtattc accgcagcat tctgatctgc gattactagc   120
gattccagct tcgtgtagtc gggttgcaga ctacagtccg aactgagacg ttatttttga   180
gatttgctcg gcttcacagc tttgcttccc tttgtttacg ccattgtagc acgtgtgtag   240
cccaagtcat aaggggcatg atgatttgac gtcatccccg ccttcctcca ggttatccct   300
ggcagtctct ctagagtgcc cggccgaacc gctggctact aaagataagg gttgcgctcg   360
ttgcgggact taacccaaca tctcacgaca cgagctgacg acaaccatgc accacctgtc   420
actcctgctc cgaagagaag gtacggttaa gtaccggtca gaaggatgtc aagacttggt   480
aaggttcttc gcgttgcttc gaattaaacc acatgctcca ccgcttgtgc gggtccccgt   540
caattccttt gagtttcatt cttgcgaacg tactccccag gtggaatact tactgcgttt   600
gcgacggcac cgagaagcaa tgcttcccaa cacctagtat tcatcgttta cggcgtggac   660
taccagggta tctaatcctg tttgctcccc acgctttcga gcctcagcgt cagttatcgt   720
ccagtaagcc gccttcgcca ctggtgttcc tcctaatatc tacgcatttc accgctacac   780
taggaattcc gcttacccct ccgacactct agtacgacag tttccaatgc agtaccgggg   840
ttgagccccg ggctttcaca tcagacttgc cgcaccgcct gcgctccctt tacacccagt   900
aaatccggat aacgcttgca ccatacgtat taccgcggct gctggcacgt atttagccgg   960
tgcttcttag tcaggtaccg tcattatctt ccctgctgat agagctttac ataccgaaat   1020
acttcttcgc tcacgcggcg tcgctgcatc aggctttcgc ccattgtgca atattcccca   1080
ctgctgcctc ccgtaggagt ttgggccgtg tctcagtccc aatgtggccg gtcaccctct   1140
caggtcggct atggatcgtc gccttggtgg gccgttacct caccaactag ctaatccaac   1200
gcgggtccat cttataccac cggagttttt cacactgcat catgcgatgc tgtgcgctta   1260
tgcggtatta gcagccgttt ccaactgtta tccccctgta caaggcaggt tacccacgcg   1320
ttactcaccc gtccgccact cagtcacaaa ataatcagtc ccgaaggaaa tcaaataaag   1380
tgcttcgttc gactgca                                                  1397

<210>    12
<211>    1401
<212>    DNA
<213>    consensus 16S ribosomal RNA sequence from Faecalicatena fissicatena
strain F. fissicatena strain ref. 1

<400>    12
ggcagctccc tccttacggt tgggtcactg acttcgggcg ttaccaactc ccatggtgtg   60
acgggcggtg tgtacaagac ccgggaacgt attcaccgcg acattctgat tcgcgattac   120
tagcgattcc agcttcatgt agtcgagttg cagactacaa tccgaactga gacgttattt   180
ttgggatttg ctcaacctcg cggtattgcc tccctttgtt tacgccattg tagcacgtgt   240
gtagccctgc tcataagggg catgatgatt tgacgtcatc cccaccttcc tccaggttat   300
ccctggcagt ctctctagag tgcccggcca aaccgctggc tactaaagat aagggttgcg   360
ctcgttgcgg gacttaaccc aacatctcac gacacgagct gacgacaacc atgcaccacc   420
tgtctccact gccccgaagg gaaggcgcca ttacacgccg gtcagtggga tgtcaagagc   480
aggtaaggtt cttcgcgttg cttcgaatta aaccacatgc tccaccgctt gtgcgggtcc   540
ccgtcaattc ctttgagttt cattcttgcg aacgtactcc ccaggtggaa tacttattgc   600
gtttgctgcg gcaccgaatg gctttgccac ccgacaccta gtattcatcg tttacggcgt   660
ggactaccag ggtatctaat cctgtttgct ccccacgctt tcgagcctca acgtcagtga   720
tcgtccagta agccgccttc gccactggtg ttcctcctaa tatctacgca tttcaccgct   780
acactaggaa ttccacttac ctctccgaca ctctagttac atagtttcca atgcagtccc   840
ggggttgagc cccgggtttt cacatcagac ttacataacc gtctacgctc cctttacacc   900
cagtaaatcc ggataacgct tgccccctac gtattaccgc ggctgctggc acgtagttag   960
ccggggcttc ttagtcaggt accgtcattt tcttccctgc tgatagaagt ttacataccg   1020
aaatacttca tccttcacgc ggcgtcgctg catcagggtt tcccccattg tgcaatattc   1080
cccactgctg cctcccgtag gagtttgggc cgtgtctcag tcccaatgtg gccggtcacc   1140
ctctcaggtc ggctactgat cgtcgccttg gtaagccgtt accttaccaa ctagctaatc   1200
agacgcgggt ccatctcata ccaccggagt ttttcccact gtaccatgcg gtaccgtggt   1260
cttatgcggt attagcagtc atttctaact gttatccccc tgtatgaggc aggttaccca   1320
cgcgttactc acccgtccgc cgctcagtcg caaaactctt caatccgaag aaatcaaagt   1380

aaagcgctcc gctcgacttg c                                                1401

<210>   13
<211>   1407
<212>   DNA
<213>   consensus 16S ribosomal RNA sequence from Faecalicatena contorta
strain F. contorta strain ref. 1

<400>   13
tgcagtcgag cgaagcagct ttacttagat ttcttcggat tgaaagagtt ttgcgactga   60
gcggcggacg ggtgagtaac gcgtgggtaa cctgcctcat acagggggat aacagttaga   120
aatgactgct aataccgcat aagaccacgg taccgcatgg tacagtggga aaaactccgg   180
tggtatgaga tggacccgcg tctgattagc tggttggtaa ggtaacggct taccaaggcg   240
acgatcagta gccgacctga gagggtgacc ggccacattg ggactgagac acggcccaaa   300
ctcctacggg aggcagcagt ggggaatatt gcacaatggg ggaaaccctg atgcagcgac   360
gccgcgtgaa ggatgaagta tttcggtatg taaacttcta tcagcaggga agaaaatgac   420
ggtacctgac taagaagccc cggctaacta cgtgccagca gccgcggtaa tacgtagggg   480
gcaagcgtta tccggattta ctgggtgtaa agggagcgta gacggttatg taagtctgat   540
gtgaaaaccc ggggctcaac cccgggactg cattggaaac tatgtaacta gagtgtcgga   600
gaggtaagtg gaattcctag tgtagcggtg aaatgcgtag atattaggag gaacaccagt   660
ggcgaaggcg gcttactgga cgatgactga cgttgaggct cgaaagcgtg gggagcaaac   720
aggattagat accctggtag tccacgccgt aaacgatgaa tactaggtgt cgggtggcaa   780
agccattcgg tgccgcagca aacgcaataa gtattccacc tggggagtac gttcgcaaga   840
atgaaactca aaggaattga cggggacccg cacaagcggt ggagcatgtg gtttaattcg   900
aagcaacgcg aagaacctta cctgctcttg acatccccct gaccggcgcg taatggtgcc   960
tttccttcgg gacaggggag acaggtggtg catggttgtc gtcagctcgt gtcgtgagat   1020
gttgggttaa gtcccgcaac gagcgcaacc cttatcttta gtagccagcg gtatggccgg   1080
gcactctaga gagactgcca gggataacct ggaggaaggt ggggatgacg tcaaatcatc   1140
atgcccctta tgagcagggc tacacacgtg ctacaatggc gtaaacaaag ggaggcgaag   1200
ccgcgaggtg gagcaaatcc caaaaataac gtctcagttc ggattgtagt ctgcaactcg   1260
actacatgaa gctggaatcg ctagtaatcg cgaatcagaa tgtcgcggtg aatacgttcc   1320
cgggtcttgt acacaccgcc cgtcacacca tgggagttgg taacgcccga agtcagtgac   1380
ccaaccgcaa ggagggagct gccgaag                                      1407

<210>   14
<211>   1338
<212>   DNA
<213>   Eubacterium hallii

<400>   14
aggttgggtc actggsttcn ggcatttycn antcccatgg tgtgrcsggc ggtgtgkaca   60
anamccggga acgtattyac cgsgacatty ygrwtygnga ttactagcga ttccagcttc   120
gtgtagtcgg gttgcagaat acagtccgaa ctgggacggc ctttttgtgg tttgstcccc   180
ctcgcgggtt cgcctcactc tgtgaccgcc attgtagcac gtgtgtcgcc cagatcataa   240
ggggcatgat gatttgacgt cgtccccacc ttcctccagg ttatccctgg cagtctctcc   300
agagtgccca gccttacctg ctggctactg aagatagggg ttgcgctcgt tgcgggactt   360
aacccaacat ctcacgacac gagctgacga caaccatgca ccacctgtct cttctgtccc   420
gaaggaaagc accgattaag gtgcggtcag aaggatgtca agacctggta aggttcttcg   480
cgttgcttcg aattaaacca catgctccac cgcttgtgcg ggtccccgtc aattcctttg   540
agtttcattc ttgcgaacgt actccccagg tggaatactt actgcgtttg cggcggcacc   600
gaagcctata cggccccgac acctagtatt catcgtttac ggcgtggact accagggtat   660
ctaatcctgt ttgctcccca cgctttcgtg cctcagtgtc agtaacagtc cagcaggccg   720
ccttcgccac tggtgttcct cctaatatct acgcatttca ccgctacact aggaattccg   780
cctgcctctc ctgtactcta gccgagcagt ttcaaatgca gctccggggt tgagccccgg   840
cctttcacat ctgacttgca ctgccaccta cgcacccttt acacccagta aatccggata   900
acgcttgctc catacgtatt accgcggctg ctggcacgta tttagccgga gcttcttagt   960
caggtaccgt cattatcttc cctgctgata gagctttaca taccgaaata cttcttcact   1020
cacgcggcgt tgctgcatca gggtttcccc cattgtgcaa tattccccac tgctgcctcc   1080
cgtaggagtt tggaccgtgt ctcagttcca atgtggccgt tcatcctctc agaccggcta   1140
ctgatcgttg ccttggtagg ccgttaccct gccaaccagc taatcagacg cgggcccatc   1200
ctgtaccacc ggagtttttc acacwaggag atgtctcctc gtgcgcttat gcggtattag   1260
cagccgtttc cagctgttat ccccctgtac agggcaggtt acccacgcgt tactcacccg   1320
tccgccactc agtcacca

<210>   15
<211>   1403
<212>   DNA
<213>   Anaerostipes caccae

<400>   15
cggcagctcc ctccttgcgg ttgggtcact gacttcgggc gttactgact cccatggtgt   60
gacgggcggt gtgtacaaga cccgggaacg tattcaccgt gacattctga ttcacgatta   120
ctagcgattc cagcttcatg tagtcgagtt gcagactaca atccgaactg agacgttatt   180
tctgggattt gcttcgcctc acgacttcgc ttccctttgt ttacgccatt gtagcacgtg   240
tgtagccctg gccataaggg gcatgatgat ttgacgtcgt ccccaccttc ctccaggtta   300
tccctggcag tctctctaga gtgcccatcc gtaatgctgg ctactaaaga taggggttgc   360
gctcgttgcg ggacttaacc caacatctca cgacacgagc tgacgacaac catgcaccac   420
ctgtctccaa tgtctcgaaa gaaaaaaccg gttaaggttc ggtcattggg atgtcaagac   480
caggtaaggt tcttcgcgtt gcttcgaatt aaaccacatg ctccaccgct tgtgcgggtc   540
cccgtcaatt cctttgagtt tcattcttgc gaacgtactc cccaggtgga atacttactg   600
cgtttgctgc ggcaccgaag cctctacggc cccgacacct agtattcatc gtttacggcg   660
tggactacca gggtatctaa tcctgtttgc tccccacgct ttcgtgcatc agtgtcagtg   720
acagtccagt aagccgcctt cgccactggt gttcctccta atatctacgc atttcaccgc   780
tacactagga attccgctta cctctcctgc actccagcat gacagtttca aaagcagtcc   840
cggggttaag ccccgggctt tcacttctga cttaccatgc cacctacgca ccctttacac   900
ccagtaattc cggataacgc ttgcccccta cgtattaccg cggctgctgg cacgtagtta   960
gccggggctt cttagtcagg taccgtctgt tttcttccct gctgatagag ctttacatac   1020
cgaaatactt cttcactcac gcggcgtcgc tgcatcaggg tttcccccat tgtgcaatat   1080
tccccactgc tgcctcccgt aggagtttgg gccgtgtctc agtcccaatg tggccgttca   1140
ctctctcaag ccggctactg atcgtcgcct tggtgagccg ttacctcacc aaccagctaa   1200
tcagacgcgg gaccatccta tactgccagg gcttttcaca ctgaatcatg cgattctgtg   1260
cgcttatgcg gtattagcag ccgtttccag ctgttatccc cctgtatagg gcaggttccc   1320
cacgcgttac tcacccgtcc gccactcagt catataggaa atccatccga aaacttcaat   1380
cctaaatgct tcgttcgact gca   1403

<210>   16
<211>   1382
<212>   DNA
<213>   Anaerostipes rhamnosivorans

<400>   16
tattttggat tgaagttttc ggatggatct ccttaatgac tgagtggcgg acgggtgagt   60
aacgcgtggg gaacctgccc tatacagggg gataacagct ggaaacggct gctaataccg   120
cataagcgca cagaatcgca tgattcagtg tgaaaagccc tggcagtata ggatggtccc   180
gcgtctgatt agctggttgg cggggtaacg gcccaccaag gcgacgatca gtagccggct   240
tgagagagtg gacggccaca ttgggactga gacacggccc aaactcctac gggaggcagc   300
agtggggaat attgcacaat gggggaaacc ctgatgcagc gacgccgcgt gagtgaagaa   360
gtatttcggt atgtaaagct ctatcagcag ggaagaaata agacggtacc tgactaagaa   420
gccccggcta actacgtgcc agcagccgcg gtaatacgta gggggcaagc gttatccgga   480
attactgggt gtaaagggtg cgtaggtggc atgataagtc agaagtgaaa gcccggggct   540
taaccccggg actgcttttg aaactgtaat gctagagtgc aggagaggta agcggaattc   600
ctagtgtagc ggtgaaatgc gtagatatta ggaggaacac cagtggcgaa ggcggcttac   660
tggactgtca ctgacactga ggcacgaaag cgtggggagc aaacaggatt agataccctg   720
gtagtcnacg ccgtaaacga tgaatactag gtgtcggggc cgtagaggct tcggtgccgc   780
agcaaacgca gtaagtattc cacctgggga gtacgttcgc aagaatgaaa ctcaaaggaa   840
ttgacgggga cccgcacaag cggtggagca tgtggtttaa ttcgaagcaa cgcgaagaac   900
cttacctggt cttgacatcc ttctgaccgg ttnnnaaccg aacctttcct tcgggacaga   960
agtgacaggt ggtgcatggt tgtcgtcagc tcgtgtcgtg agatgttggg ttaagtcccg   1020
caacgagcgc aacccctatc tttagtagcc agcatataag gtgggcactc tagagagact   1080
gccagggata acctggagga aggtggggac gacgtcaaat catcatgccc cttatggcca   1140
gggctacaca cgtgctacaa tggcgtaaac aaagggaagc gaccccgcga gggcaagcaa   1200
atcccagaaa taacgtctca gttcggattg tagtctgcaa ctcgactaca tgaagctgga   1260
atcgctagta atcgtgaatc agaatgtcac ggtgaatacg ttcccgggtc ttgtacacac   1320
cgcccgtcac accatgggag tcagtaacgc ccgaagtcag tgacccaacc gcaaggagga   1380
ga   1382

## Description

### TECHNICAL FIELD

This invention is in the field of compositions comprising bacterial strains and the use of such compositions in the treatment of disease.

### BACKGROUND TO THE INVENTION

The human intestine is thought to be sterile *in utero,* but it is exposed to a large variety of maternal and environmental microbes immediately after birth. Thereafter, a dynamic period of microbial colonization and succession occurs, which is influenced by factors such as delivery mode, environment, diet and host genotype, all of which impact upon the composition of the gut microbiota, particularly during early life. Subsequently, the microbiota stabilizes and becomes adult-like [1]. The human gut microbiota contains more than 500-1000 different phylotypes belonging essentially to two major bacterial divisions, the Bacteroidetes and the Firmicutes [2]. The successful symbiotic relationships arising from bacterial colonization of the human gut have yielded a wide variety of metabolic, structural, protective and other beneficial functions. The enhanced metabolic activities of the colonized gut ensure that otherwise indigestible dietary components are degraded with release of by-products providing an important nutrient source for the host. Similarly, the immunological importance of the gut microbiota is well-recognized and is exemplified in germfree animals which have an impaired immune system that is functionally reconstituted following the introduction of commensal bacteria [3-5].

Dramatic changes in microbiota composition have been documented in gastrointestinal disorders such as inflammatory bowel disease (IBD). For example, the levels of *Clostridium* cluster XIVa bacteria are reduced in IBD patients whilst numbers of *E*. *coli* are increased, suggesting a shift in the balance of symbionts and pathobionts within the gut [6-9]. Interestingly, this microbial dysbiosis is also associated with imbalances in T effector cell populations.

In recognition of the potential positive effect that certain bacterial strains may have on the animal gut, various strains have been proposed for use in the treatment of various diseases (see, for example, [10-13]). Also, certain strains, including mostly *Lactobacillus* and *Bifidobacterium* strains, have been proposed for use in treating various inflammatory and autoimmune diseases that are not directly linked to the intestines (see [14] and [15] for reviews). However, the relationship between different diseases and different bacterial strains, and the precise effects of particular bacterial strains on the gut and at a systemic level and on any particular types of diseases, are poorly characterised.

The discovery of the size and complexity of the human microbiome has resulted in an on-going evaluation of many concepts of health and disease. Certainly, dramatic changes in microbiota composition have been documented in gastrointestinal disorders such as inflammatory bowel disease (IBD) [16-19]. More recently, there is increased interest in the art regarding alternations in the gut microbiome that may play a pathophysiological role in human brain diseases [20]. Preclinical and clinical evidence are strongly suggesting a link between brain development and microbiota [21].

A growing body of preclinical literature has demonstrated bidirectional signalling between the brain and the gut microbiome, involving multiple neurocrine and endocrine signalling systems. Indeed, increased levels of *Clostridium* species in the microbiome have been linked to brain disorders [22], and an imbalance of the Bacteroidetes and Firmicutes phyla has also been implicated in brain development disorders [23]. Suggestions that altered levels of gut commensals, including those of *Bifidobacterium, Lactobacillus, Sutterella, Prevotella* and *Ruminococcus* genera and of the Alcaligenaceae family are involved in immune-mediated central nervous system (CNS) disorders, are questioned by studies suggesting a lack of alteration in the microbiota between patients and healthy subjects [24]. This indicates that, at present, the practical effect of the link between the microbiome and human brain diseases is poorly characterised. Accordingly, more direct analytical studies are required to identify the therapeutic impact of altering the microbiome on CNS disorders.

In recognition of the potential positive effect that certain bacterial strains may have on the animal gut, various strains have been proposed for use in the treatment of various diseases (see, for example, [24-27]). Also, certain strains, including mostly *Lactobacillus* and *Bifidobacterium* strains, have been proposed for use in treating various inflammatory and autoimmune diseases that are not directly linked to the intestines (see [28] and [29] for reviews). In addition, a range of probiotics have been investigated in animal models to determine a role of the gut microbiome in modulating emotional behaviour, and *Bifidobacterium* and *Lactobacillus* are the main genera showing beneficial effects, reducing anxiety and repetitive behaviours, and increasing social interaction [30-32]. However, the relationship between different diseases and different bacterial strains, and the precise effects of particular bacterial strains on the gut and at a systemic level and on any particular types of diseases, are poorly characterised, particularly for central nervous system diseases.

There is a growing body of evidence to suggest that the microbiota-gut-brain axis is affected in autism spectrum disorders (ASD) and other neurodevelopmental and neuropsychiatric disorders. Animal models have provided considerable insight into how the microbiota may be involved in ASD. Furthermore, preclinical studies have demonstrated that targeting the gut microbiota through administration of beneficial live biotherapeutics display efficacy in improving autistic-related behaviour in animal models, including the maternal immune activation (MIA) mouse model and the black and tan, brachyuric (BTBR) mouse. The BTBR mouse is a genetically modified, inbred mouse strain that displays a number of behaviours associated with ASD such as impaired sociability, repetitive behaviour and increased anxiety. Moreover, these mice also exhibit gastrointestinal dysfunctions along with alterations to the composition of the gut microbiota. Consequently, it represents an appropriate animal model for investigating the role of the microbiota-gut-brain axis in ASD.

Reference [33] discusses possible methods of treating neurodevelopmental disorders by administering a composition comprising a bacterial species selected from *Bacteroides* and/or *Enterococcus,* but provides data only for *Bacteroides.* Reference [34] discusses a similar use of *Bacteroides* and *Enterococcus,* with data limited to *Bacteroides fragilis, Bacteroides vulgatus* and *Enterococcus faecalis.* Reference [35] discusses butyrate production by *Anaerostipes hadrus.* Similarly, reference [36] discusses butyrate production by a bacterial consortium comprising strains *of Anaerostipes caccae* and *Eubacterium hallii.* Reference [37] discusses consortia comprising both a specific strain of *Anaerostipes hadrus* and a specific strain of *Anaerostipes caccae,* one of which was described as affecting the onset of diabetes in a mouse model, but with no other therapeutic effects being described. Reference [38] discusses the use of synthetic faecal compositions to treat a range of diseases, and suggests a large number of bacterial species to potentially include in such a composition (Table 1). Reference [39] discusses the use of compositions comprising bacteria from the phyla *Firmicutes* and *Bacteroidetes* to reduce the abundance of antibiotic resistance genes in the microbiome. Reference [40] discusses engineering bacteria to produce GABA, for the treatment of mental illnesses or diseases of the central nervous system. However, only data related to the production of GABA is provided, and no therapeutic effect is shown.

There is a requirement in the art for new methods of treating diseases and in particular for new methods of treating central nervous system disorders. There is also a requirement for the potential effects of gut bacteria to be characterised so that new therapies using gut bacteria can be developed.

### SUMMARY OF THE INVENTION

The inventors have developed new therapies using bacterial strains of the order *Clostridiales,* to treat or prevent a variety of indications (including, but not limited to, central nervous system disorders or conditions and cancer).

The invention provides a composition comprising a Gram-positive, rod-shaped and anaerobic bacterial strain of the order *Clostridiales,* wherein the bacterial strain does not belong to the genera *Roseburia* or *Bariatricus,* or the family *Clostridiacae,* for use in a method of treating or preventing a central nervous system disorder or condition. The invention also provides a Gram-positive, rod-shaped and anaerobic bacterial strain of the order *Clostridiales,* wherein the bacterial strain does not belong to the genera *Roseburia* or *Bariatricus,* or the family *Closiridiacae,* for use in a method of treating or preventing a central nervous system disorder or condition. In preferred embodiments, such bacterial strains are of the genus *Anaerostipes, Eubacterium* or *Faecalicatena.*

Further embodiments of the invention are detailed below.

### Use in therapy

The invention also provides a composition comprising a bacterial strain of the genus *Anaerostipes,* for use in therapy.

The inventors have developed new compositions comprising a bacterial strain of the genus *Anaerostipes* that can be used in therapy. In particular, the inventors have developed new compositions comprising a strain of the genus *Anaerostipes* for use in treating and preventing diseases or conditions mediated by histone deacetylase (HDAC) activity. The inventors have identified that bacterial strains from the genus *Anaerostipes* can be effective for reducing histone deacetylase activity. Histone deacetylase activity has been shown to mediate pathological symptoms in an array of diseases and conditions including but not limited to autoimmune or inflammatory diseases and conditions including, but not limited to, Graft-versus-host disease (GVHD), inflammatory bowel diseases, such as Crohn's disease, neurodegenerative diseases, such as Parkinson's disease, brain injury, such as stroke, and a range of cancers. As such, the compositions of the invention may have pleiotropic benefits in the treatment or prevention of multiple diseases mediated at least in part by HDAC activity. In some embodiments, the compositions of the invention are for use in the treatment of prevention of diseases mediated by increased HDAC activity.

As described in the examples, oral administration of compositions comprising *Anaerostipes* may reduce the activity of histone deacetylase in models of disease. Also, as described in the examples, oral administration of compositions comprising *Anaerostipes* may reduce hyperactivity in mice models of disease. In certain embodiments, the compositions of the invention may be for use in the treatment or prevention of a disease or condition associated with hyperactivity. The compositions may be for use in the treatment or prevention of hyperactivity. The compositions may be for use in the treatment or prevention of hyperactivity associated with behavioural disorders, such as attention deficit hyperactive disorder. Therefore, the inventors have identified compositions effective in the prevention or treatment of diseases mediated by HDAC activity and compositions effective in the treatment or prevention of behavioural disorders. Behavioural disorders suitable for treatment with compositions of the invention may or may not be mediated in part by HDAC activity.

In particular embodiments, the invention provides a composition comprising a bacterial strain of the genus *Anaerostipes,* for use in treating or preventing diseases mediated HDAC activity. The inventors have identified that treatment with bacterial strains from this genus can reduce the activity of HDAC, which can provide clinical benefits in the treatment of diseases mediated by HDAC activity. In some embodiments, the compositions of the invention have been found to be particularly beneficial in reducing Class I HDAC activity. Class IHDACs are ubiquitously expressed and most commonly reside in the nucleus. Class I HDACs deacetylate histone lysine residues to restore positive charge to the histone, thereby increasing electrostatic binding between histones and DNA. HDAC activity therefore increases chromatin compaction causing downregulation of the expression of genes at the underlying DNA sequence. HDACs also have additional regulatory effects by modifying non-histone protein targets. The inhibition of the acetylation of non-histone protein targets may be beneficial in the treatment or prevention of other aspects of disease not directly related to the control of gene expression by chromatin expansion. In certain embodiments, the compositions of the invention can therefore be used to regulate target gene expression.

In some embodiments the invention provides a composition comprising a bacterial strain of the genus *Anaerostipes* for use in a method of treating or preventing an inflammatory bowel disease mediated by HDAC activity. Inhibition of HDAC activity has been shown to suppress the production of proinflammatory cytokines in the gastrointestinal tract. Thus, the compositions of the invention may be useful in the treatment of inflammatory diseases. In particular, the compositions of the invention may be useful in the treatment or prevention of conditions associated with increased colonic proinflammatory cytokine pathogenesis. In some embodiments, the compositions of the invention are for use in the treatment or prevention of inflammatory bowel disease. In some embodiments, the compositions of the invention are for use in the treatment or prevention of ulcerative colitis. In some embodiments, the compositions of the invention are for use in the treatment or prevention of Crohn's disease. The inventors have seen particularly good results with a bacteria of the species *Anaerostipes hadrus* and thus the invention provides a composition comprising a bacterial strain of this species for use in the treatment or prevention of inflammatory disease. In preferred embodiments, the invention provides a composition comprising a bacterial strain of the species *Anaerostipes hadrus* for use in the treatment or prevention of colitis.

In certain embodiments, the compositions of the invention are for use in a method of reducing histone deacetylase activity in the treatment or prevention of a disease or condition mediated by histone deacetylase activity.

In certain embodiments, the composition is for use in a patient with elevated histone deacetylase activity. In certain embodiments, the composition is for use in a patient with elevated Class I HDAC activity. The effect on histone deacetylase activity shown for *Anaerostipes* strains may be particularly beneficial for such patients.

Furthermore, the inventors have identified that treatment with a bacterial strain of the genus *Anaerostipes* can reduce the activation of proinflammatory molecules, such as IL-6, TNF-α and IL-1β. Chronic inflammation induced by IL-6 can ultimately lead to cell death. Therefore, the bacterial strains of the invention may be particularly useful in the treatment or prevention of inflammatory or autoimmune disorders. In some embodiments, the bacterial strains are useful in the treatment of inflammatory or autoimmune disorders characterised by the enhanced activation of IL-6.

In particular embodiments, the invention provides a composition comprising a bacterial strain of the genus *Anaerostipes,* for use in a method of treating or preventing a disease or condition selected from the group consisting of: a neurodegenerative disease, such as Alzheimer's disease, Huntington's disease or Parkinson's disease; brain injury, such as stroke; behavioural or psychiatric disorders, such as attention deficit hyperactivity disorder, obsessive compulsive disorder, anxiety disorder, biopolar disorder, or post-traumatic stress disorder; an inflammatory or autoimmune disease, such as asthma, arthritis, psoriasis, multiple sclerosis, diabetes, allograft rejection, graft-versus-host disease, or an inflammatory bowel disease, such as Crohn's disease; or cancer, such as prostate cancer, colorectal cancer, breast cancer, lung cancer, liver cancer or gastric cancer.

In some embodiments, the compositions of the invention are for use in the treatment or prevention of cancer. Dysregulation of acetylation pathways in cancer have been implicated in cancer cell survival and tumour immune evasion. For example, HDAC mediated deacetylation of p53 reduces the stability and half-life of p53. Acetylated p53 binds and regulates the expression of cell cycle regulatory and pro-apoptotic genes with greater efficacy, reducing cancer cell growth and promoting apoptosis. Deacetylation of p53 may therefore inhibit apoptosis in cancer cells, increasing cancer cell survival. In certain embodiments, the compositions of the invention are for use in the treatment or prevention of cancers. In some embodiments, the compositions of the invention are for use in the treatment of cancers with non-mutated p53. In some embodiments, the compositions of the invention are for use in a method of increasing apoptosis in cancer cells. In some embodiments, the compositions of the invention are for use in a method of decreasing tumour immune evasion. In some embodiments, the compositions of the invention are for use in the treatment or prevention of cancers with increased HDAC-activity. In some embodiments, the compositions are for use as pro-apoptotic medicaments, for example for use in the treatment or prevention of cancers. In certain embodiments, the invention provides a composition comprising a bacterial strain of the species *Anaerostipes hadrus* for use in the treatment or prevention of cancer,

In further preferred embodiments, the invention provides a composition comprising a bacterial strain of the genus *Anaerostipes,* for use in a method of treating or preventing cancer, such as breast, lung or liver cancer. In further preferred embodiments, the invention provides a bacterial strain of the genus *Anaerostipes,* for use in a method of treating or preventing cancer, such as breast, lung or liver cancer. In certain embodiments, the composition is for use in a method of reducing tumour size or preventing tumour growth in the treatment of cancer. In certain embodiments, the invention provides a composition comprising a bacterial strain of the species *Anaerostipes hadrus,* for use in the treatment of cancer. In certain embodiments, the invention provides a composition comprising a bacterial strain of the genus *Anaerostipes,* preferably of the species *Anaerostipes hadrus,* for use in the treatment of cancer, wherein the bacterial strain is not the bacterial strain deposited under accession number NCIMB 43457.

### Central nervous system disorders or conditions

The inventors have, in particular, identified and developed new therapies for treating and preventing central nervous system disorders, such as those mediated by the microbiota-gut-brain axis. Such therapies use a composition comprising a Gram-positive, rod-shaped and anaerobic bacterial strain of the order *Clostridiales,* wherein the bacterial strain does not belong to the genera *Roseburia* or *Bariatricus,* or the family *Clostridiacae.*

Specifically, the inventors have identified that bacterial strains of the genus *Anaerostipes* (such as a bacterial strain of the species *Anaerostipes hadrus*) can be effective for treating and preventing diseases and conditions mediated by the microbiota-gut-bram axis. In addition, the inventors have identified that bacterial strains of the genus *Eubacterium* and *Faecalicatena* can be effective for treating and preventing diseases and conditions mediated by the microbiota-gut-brain axis. As described in the examples, oral administration of compositions comprising *Anaerostipes hadrus* can reduce symptoms associated with dysfunction of the microbiota-gut-brain axis in an animal model of autism spectrum disorders. Also, as described in the examples, oral administration of compositions comprising bacterial strain of the genus *Eubacterium* or *Faecalicatena* may reduce symptoms associated with dysfunction of the microbiota gut brain axis in a mouse model of autism spectrum disorders. In addition, as described in the examples, oral administration of compositions comprising a *Eubacterium* or *Faecalicatena* strain may modulate the levels of signalling molecules associated with the function of the microbiota-gut-brain axis, and neurodevelopmental and neuropsychiatric disorders.

In preferred embodiments, the invention provides a composition comprising a bacterial strain of the genus *Anaerostipes,* for use in a method of treating or preventing a central nervous system disorder or condition. For example, the composition may comprise a bacterial strain of the species *Anaerostipes hadrus, Anaerostipes butyraticus, Anaerostipes caccae,* and *Anaerostipes rhamnosivorans. In particular,* the composition may comprise a bacterial strain of the species *Anaerostipes hadrus.* Alternatively, the composition may comprise a bacterial strain which has at least 95%, at least 96%, at least 97%, at least 98%, at least 99%, at least 99.5% or at least 99.95% identity to the sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 15 or SEQ ID NO: 16. The central nervous system disorder or condition may be mediated by the microbiota-gut-brain axis.

The invention also provides the use of a bacterial strain of the genus *Anaerostipes* (e.g. a bacterial strain of the species *Anaerostipes hadrus, Anaerostipes butyraticus, Anaerostipes caccae,* and *Anaerostipes rhamnosivorans*) in the manufacture of a medicament for therapy, for example for treating or preventing a central nervous system disorder or condition, as described herein. The invention also provides a method of treating or preventing a disease in a patient in need thereof, in particular in a method of treating or preventing a central nervous system disorder or condition, as described herein, comprising administering a composition comprising a bacterial strain of the genus *Anaerostipes* (e.g. a bacterial strain of the species *Anaerostipes hadrus, Anaerostipes butyraticus, Anaerostipes caccae,* and *Anaerostipes rhamnosivorans)* to a patient in need thereof.

The invention provides a composition comprising a bacterial strain of the genus *Anaerostipes* (e.g. a bacterial strain of the species *Anaerostipes hadrus*) for use in a method of treating or preventing a disease or condition mediated by dysfunction of the microbiota-gut-brain axis. Also provided is a method of treating or preventing a disease or condition mediated by dysfunction of the microbiota-gut-brain axis, comprising administering a composition comprising a bacterial strain of the *Anaerostipes.*

The composition comprising a bacterial strain of the genus *Anaerostipes* (e.g. a bacterial strain of the species *Anaerostipes hadrus*) may be used in a method of treating or preventing a neurodevelopmental disorder or a neuropsychiatric condition. The inventors have identified that treatment with bacterial strains from the genus *Anaerostipes* can provide clinical benefits in mouse models of central nervous system disorders, in particular those mediated by the microbiota-gut-brain axis.

The inventors have identified that treatment with bacterial strains from this genus may modulate signalling in the central, autonomic and enteric nervous systems; and/or may modulate the activity of the hypothalamus-pituitary-adrenal (HPA) axis pathway; and/or may modulate neuroendocrine and/or neuroimmune pathways; and/or may modulate the levels of commensal metabolites, inflammatory markers and/or gastrointestinal permeability of a subject.

The composition comprising a bacterial strain of the genus *Anaerostipes* (e.g. a bacterial strain of the species *Anaeroslipes hadrus*) may be used in a method of treating or preventing a disease or condition selected from the group consisting of: autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's disease; Parkinson's disease; and/or chronic pain.

The composition comprising a bacterial strain of the genus *Anaerostipes* (e.g. a bacterial strain of the species *Anaerostipes hadrus*) may be useful for treating or preventing motor neuron disease; Huntington's disease; Guillain-Barre syndrome and/or meningitis. The effect shown for the *Anaerostipes* bacterial strains on the microbiota-gut-brain axis and on diseases mediated by the microbiota-gut-brain axis suggests therapeutic benefits for other diseases and conditions mediated by the microbiota-gut-brain axis, such as those disclosed herein.

The composition comprising a bacterial strain of the genus *Anaerostipes* (e.g. a bacterial strain of the species *Anaerostipes hadrus*) may be used in a method of treating comorbidities associated with diseases and conditions mediated by the microbiota-gut-brain axis, such as those disclosed herein. For example, the composition may be used in a method of treating gastrointestinal comorbidities associated with diseases and conditions mediated by the microbiota-gut-brain axis, such as those disclosed herein. The mouse model experiments used in this application for the assessment of the symptoms of autism spectrum disorders are known in the art to be applicable for the assessment of the symptoms other central nervous system disorders including those disclosed herein [41- 42].

The composition comprising a bacterial strain of the genus *Anaerostipes* (e.g. a bacterial strain of the species *Anaerostipes hadrus*) may be used in a method of treating or preventing autism spectrum disorders, such as autism. The inventors have identified that treatment with a *Anaerostipes* strain can reduce symptom severity in a mouse model of autism spectrum disorders and can prevent or reduce stereotyped, repetitive, compulsive and anxious behaviour. Compositions comprising a bacterial strain of the genus *Anaerostipes* may be particularly effective for treating autism spectrum disorders. Thus, the composition comprising a bacterial strain of the genus *Anaerostipes* (such as a bacterial strain of the species *Anaerostipes hadrus)* may be used in the treatment of autism spectrum disorders. The composition may be used to treat the behavioural symptoms (e.g. of autism spectrum disorders). For example, the composition may prevent, reduce or alleviate one or more stereotyped, repetitive, compulsive and/or anxious behaviour. The composition comprising a bacterial strain of the genus *Anaerostipes* (such as a bacterial strain of the species *Anaerostipes hadrus)* may be used in the treatment of the gastrointestinal symptoms of autism spectrum disorders. In preferred embodiments, the composition may be used in the treatment of the behavioural and gastrointestinal symptoms of autism spectrum disorders. Treatment with strains from the genus *Anaerostipes* (such as a bacterial strain of the species *Anaerostipes hadrus*) may modulate signalling in the central, autonomic and enteric nervous systems; may modulate the activity of the HPA axis pathway; may modulate neuroendocrine and/or neuroimmune pathways; and/or may modulate the levels of commensal metabolites, inflammatory markers and/or gastrointestinal permeability of a subject, all of which are implicated in the neuropathology of autism spectrum disorders. In certain embodiments, treatment with strains from the genus *Anaerostipes* (such as a bacterial strain of the species *Anaerostipes hadrus)* strains may modulate the levels of oxytocin and/or vasopressin hormones.

The composition comprising a bacterial strain of the genus *Anaerostipes* (e.g. a bacterial strain of the species *Anaerostipes hadrus)* may be used in a method of treating or preventing obsessive compulsive disorder (OCD). In some embodiments, the composition may be used for preventing, reducing or alleviating one or more of stereotyped, repetitive, compulsive and/or anxious behaviour in the treatment of OCD. Treatment with strains from the genus *Anaerostipes* may modulate signalling in the central, autonomic and enteric nervous systems; may modulate the activity of the HPA axis pathway; may modulate neuroendocrine and/or neuroimmune pathways; and/or may modulate the levels of commensal metabolites and/or gastrointestinal permeability of a subject, all of which are implicated in the neuropathology of OCD.

The composition comprising a bacterial strain of the genus *Anaerostipes* (e.g. a bacterial strain of the species *Anaerostipes hadrus*) may be used in a method of treating or preventing major depressive disorder (MDD). Treatment with strains from the genus *Anaerostipes* may provide clinical benefits in a mouse model of depression. Accordingly, the composition may be used in the treatment of depression. Compositions comprising a bacterial strain of the genus *Anaerostipes* may be particularly effective for treating depression. In some cases, the composition may be used to prevent, reduce or alleviate one or more of stereotyped, repetitive, compulsive and/or anxious behaviour in the treatment of depression. Treatment with strains from the genus *Anaerostipes* (such as a bacterial strain of the species *Anaerostipes hadrus)* may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of MDD. Treatment with strains from the genus *Anaerostipes* (such as a bacterial strain of the species disclosed herein) may modulate the levels of oxytocin and/or vasopressin hormones.

The composition comprising a bacterial strain of the genus *Anaerostipes* (e.g. a bacterial strain of the species *Anaerostipes hadrus*) may be used in a method of treating or preventing anxiety disorders. Treatment with strains from the genus *Anaerostipes* reduces disease incidence and disease severity in a mouse model of anxiety in the examples of this application. The composition may be used in the treatment of anxiety disorder. Compositions comprising a bacterial strain of the genus *Anaerostipes* may be particularly effective for treating anxiety disorder. The composition may be used to prevent, reduce or alleviate one or more of stereotyped, repetitive, compulsive and/or anxious behaviour in the treatment of anxiety.

The composition comprising a bacterial strain of the genus *Anaerostipes* (e.g. a bacterial strain of the species *Anaeroslipes hadrus*) may be used in a method of treating or preventing a stress disorder, such as post-traumatic stress disorder. Compositions comprising a bacterial strain of the genus *Anaerostipes* may reduce stress in mouse models of stress disorders. Treatment with strains from the genus *Anaerostipes* may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of stress disorder. In certain embodiments, treatment with strains from the genus *Anaerostipes* may modulate the levels of oxytocin and/or vasopressin hormones.

The composition comprising a bacterial strain of the genus *Anaerostipes* (e.g. a bacterial strain of the species *Anaerostipes hadrus*) may be used in a method of treating or preventing schizophrenia spectrum and psychotic disorders, such as schizophrenia. Compositions comprising a bacterial strain of the species *Anaerostipes* may improve positive and negative symptoms in mouse models of schizophrenia spectrum and psychotic disorders. Treatment with strains from the genus *Anaerostipes* may modulate signalling in the central, autonomic and enteric nervous systems; may modulate the activity of the HPA axis pathway; may modulate neuroendocrine and/or neuroimmune pathways; and may modulate the levels of commensal metabolites and/or gastrointestinal permeability of a subject, all of which are implicated in the neuropathology of schizophrenia spectrum and psychotic disorders.

The composition comprising a bacterial strain of the genus *Anaerostipes* (e.g. a bacterial strain of the species *Anaerostipes hadrus)* may be used in a method of treating or preventing bipolar disorder, Compositions comprising a bacterial strain of the genus *Anaerostipes* may reduce occasions of mania and/or depression in mouse models of bipolar disorder. Treatment with strains from the genus *Anaerostipes* may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of bipolar disorder. In certain embodiments, treatment with strains from the genus *Anaerostipes* may modulate the levels of oxytocin and/or vasopressin hormones.

The composition comprising a bacterial strain of the genus *Anaerostipes* (e.g. a bacterial strain of the species *Anaerostipes hadrus)* may be used in a method of treating or preventing neurocognitive disorders, such as Alzheimer's disease. Compositions comprising a bacterial strain of the genus *Anaerostipes* may improve cognitive and behavioural functioning in mouse models of neurocognitive disorders. Treatment with strains from the genus *Anaerostipes* may modulate signalling in the central, autonomic and enteric nervous systems; may modulate the activity of the HPA axis pathway; may modulate neuroendocrine and/or neuroimmune pathways; and may modulate the levels of commensal metabolites and/or gastrointestinal permeability of a subject, all of which are implicated in the neuropathology of neurocognitive disorders.

The composition comprising a bacterial strain of the genus *Anaerostipes* (e.g. a bacterial strain of the species *Anaerostipes hadrus*) may be used in a method of treating or preventing Parkinson's disease. Compositions comprising a bacterial strain of the genus *Anaerostipes* may improve motor and cognitive functions in mouse models of Parkinson's disease. Treatment with strains from the genus *Anaerostipes* may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of Parkinson's disease. In certain embodiments, treatment with strains from the genus *Anaerostipes* strains may modulate the levels of oxytocin and/or vasopressin hormones.

The composition comprising a bacterial strain of the genus *Anaerostipes* (e.g. a bacterial strain of the species *Anaerostipes hadrus*) may be used compositions disclosed herein a method of modulating the microbiota-gut-brain axis in the treatment or prevention of a disease or condition mediated by the microbiota-gut-brain axis. In particular, the compositions disclosed herein may be used in modulating the microbiota-gut-brain axis in the treatment or prevention of autism spectrum disorders; obsessive compulsive disorder; major depressive disorder; anxiety disorders; stress disorders; schizophrenia spectrum disorders; bipolar disorders; neurocognitive disorders and Parkinson's disease.

In some embodiments, bacterial strains from the genus *Anaerostipes* may provide therapeutic benefits in the treatment of behavioural disorders selected from the list consisting of: attention deficit hyperactive disorder, oppositional defiant disorder and conduct disorder. The inventors have identified that treatment with *Anaerostipes* strains reduce hyperactivity in mice, which is a symptom of behavioural disorders such has ADHD. The strains of the invention may therefore be useful in the treatment or prevention of behavioural disorders, in particular in the treatment or prevention of behavioural disorders associated with hyperactivity, such as ADHD. In certain embodiments, the compositions of the invention are for use in the treatment or prevention of hyperactivity in a subject. In certain embodiments, the invention provides a composition comprising a bacterial strain of the species *Anaerostipes hadrus* for use in the treatment or prevention of behavioural disorders. In preferred embodiments, the invention provides a composition comprising a bacterial strain of the species *Anaerostipes hadrus* for use in the treatment or prevention of ADHD

In some embodiments, the invention provides a composition comprising a bacterial strain of the genus *Anaerostipes,* for use in a method of treating or preventing a neurodegenerative disease mediated by HDAC activity. In some embodiments, the compositions of the invention may be useful in the treatment or prevention of symptoms of neurodegenerative diseases mediated by HDAC activity. The inventors have identified that the strains of the invention inhibit HDAC activity. Histone acetylation and deacetylation are important epigenetic regulators of gene expression. Histone acetylation imbalance has been implicated in the pathogenesis of neurodegenerative diseases such as Alzheimer's disease, Huntington's disease and Parkinson's disease. In some embodiments, the strains of the invention are for use in the treatment or prevention of age-associated neurodegenerative diseases. In some embodiments, the compositions of the invention are for use in the treatment or prevention of age-onset neurodegenerative diseases, such as age-onset Parkinson's disease or age-onset Alzheimer's disease. In certain embodiments, the invention provides a composition comprising a bacterial strain of the species *Anaerostipes hadrus* for use in the treatment or prevention of neurodegenerative disease. In preferred embodiments, the invention provides a composition comprising a bacterial strain of the species *Anaerostipes hadrus* for use in the treatment or prevention of Alzheimer's disease, Huntington's disease or Parkinson's disease.

In certain embodiments of the invention, the compositions are for use in treating brain injury. The neuroprotective activity of the compositions of the invention and their ability to reduce levels of histone deacetylase activity (HDAC) may make them useful for treating brain injury. In preferred embodiments, the compositions of the invention are for use in treating stroke, such as treating brain injury resulting from a stroke. In certain embodiments, the invention provides a composition comprising a bacterial strain of the species *Anaerostipes hadrus* for use in the treatment or prevention of brain injury, in particular stroke.

The bacterial strain in the composition may be of the species *Anaerostipes hadrus, Anaerostipes butyraticus, Anaerostipes caccae,* and *Anaerostipes rhamnosivorans.* The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes hadrus* (e.g. SEQ ID NO: 1, 2, 6 or 7). The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes butyraticus* (e.g. SEQ ID NO: 3). The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes rhamnosivorans* (e.g. SEQ ID NO: 4 or 16). The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes caccae* (e.g. SEQ ID NO: 5 or 15). Thus, the bacterial strain may have a 16s rRNA sequence that is at least 95%, 96%, 97%, 98%, 99%, 99.5%, 99.9% or 100% identical to SEQ ID NO: 1-7, or 15. In preferred embodiments, the bacterial strain may have a 16s rRNA sequence that is at least 95%, 96%, 97%, 98%, 99%, 99.5%, 99.9% or 100% identical to SEQ ID NO: 6 or 7. For example, the bacterial strain has a 16s rRNA sequence that is at least 95%, 96%, 97%, 98%, 99%, 99.5%, 99.9% or 100% identical to SEQ ID NO: 6. For example, the bacterial strain has a 16s rRNA sequence that is at least 95%, 96%, 97%, 98%, 99%, 99.5%, 99.9% or 100% identical to SEQ ID NO: 7.

The invention also provides a composition comprising a bacterial strain, wherein the bacterial strain has a 16s rRNA sequence that is least 95%, 96%, 97%, 98%, 99%, 99.5% or 99.9% identical to any one of SEQ ID NOs: 1-7, and 15 for use in therapy, for example in a method of treating or preventing a central nervous system disorder or condition as described herein.

In preferred embodiments, the composition comprises a bacterial strain deposited under accession number NCIMB 43457, NCIMB 43526 or a derivative thereof.

In other preferred embodiments, the invention provides a composition comprising a bacterial strain of the genus *Eubacterium* or *Faecalicatena,* for use in a method of treating or preventing a central nervous system disorder or condition, such as a neurodevelopmental disorder or neuropsychiatric condition, such as those disclosed in more detail herein. The invention also provides a bacterial strain of the genus *Eubacterium* or *Faecalicatena,* for use in a method of treating or preventing a central nervous system disorder or condition, such as a neurodevelopmental disorder or neuropsychiatric condition, such as those disclosed in more detail herein.

Preferred bacterial species include *Eubacterium callanderi, Eubacterium limosum, Eubacterium rectale, Eubacterium eligens, Eubacterium hallii, Faecalicatena fissicatena* or *Faecalicatena contorta.* In especially preferred embodiments, compositions of the invention comprise a bacterial strain of the species *Eubacterium callanderi* or *Eubacterium limosum,* more preferably *Eubacterium callanderi.* Preferred bacterial strains also include those having a 16s rRNA gene sequence that is at least 95%, 96%, 97%, 98%, 99%, 99.5% or 99.9% identical to SEQ ID NO: 8, 9, 10, 11, 12, 13 or 14; preferably to SEQ ID NO: 8; more preferably wherein the bacterial strain has the 16s rRNA gene sequence represented by SEQ ID NO: 8.

In some embodiments, the central nervous system disorder or condition is selected from the group consisting of autism spectrum disorders (ASDs); child developmental disorder; obsessive compulsive disorder (OCD); major depressive disorder (MDD); depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; chronic pain; motor neuron disease; Huntington's disease; Guillain-Barre syndrome and meningitis. The mouse model experiments used in this application for the assessment of the symptoms of autism spectrum disorders are known in the art to be applicable for the assessment of the symptoms of other central nervous system disorders including those listed above (see, e.g. [43-45]). The effect shown for the bacterial strains from the *Eubacterium* or *Faecalicatena* genera on the microbiota-gut-brain axis and on diseases mediated by the microbiota-gut-brain axis may provide therapeutic benefits for other diseases and conditions mediated by the microbiota-gut-brain axis (with the above only being exemplary). In some embodiments, compositions of the invention are for use in a method of treating comorbidities (such as gastrointestinal comorbidities) associated with diseases and conditions mediated by the microbiota-gut-brain axis, such as those listed above.

The inventors have identified that treatment with bacterial strains from the *Eubacterium* or *Faecalicatena* genera may modulate signalling in the central, autonomic and enteric nervous systems; may modulate the activity of the hypothalamus-pituitary-adrenal (HPA) axis pathway; may modulate neuroendocrine and/or neuroimmune pathways, and/or may modulate gastrointestinal permeability of a subject. In particular, compositions comprising a bacterial strain of the species *Eubacterium callanderi* may be particularly effective at modulating signalling in the central, autonomic and enteric nervous systems; modulating the activity of the hypothalamus-pituitary-adrenal (HPA) axis pathway; modulating neuroendocrine and/or neuroimmune pathways, and/or modulating gastrointestinal permeability of a subject. The above mechanisms are implicated in the neuropathology of *inter alia,* autism spectrum disorders, obsessive compulsive disorder (OCD), major depressive disorder (MDD), stress disorders, schizophrenia spectrum and psychotic disorders, bipolar disorder, and neurocognitive disorders such as Parkinson's disease.

In a preferred embodiment, the central nervous system disorder or condition is an autism spectrum disorder, such as autism. The inventors have identified that treatment with *Eubacterium* strains (in particular *Eubacterium callanderi*) can reduce symptom severity in a mouse model of autism spectrum disorders and can prevent or reduce stereotyped, repetitive, compulsive and anxious behaviour. The invention may be for use in reducing stereotyped, repetitive, compulsive or anxious behaviour, in particular in the treatment of autism spectrum disorders. In preferred embodiments, the invention provides a composition comprising a bacterial strain of the species *Eubacterium callanderi* for use in a method of treating or preventing an autism spectrum disorder, such as autism.

In another preferred embodiment, the central nervous system disorder or condition is obsessive compulsive disorder (OCD). The invention may be for use in reducing stereotyped, repetitive, compulsive or anxious behaviour in the treatment of OCD. In preferred embodiments, the invention provides a composition comprising a bacterial strain of the species *Eubacterium callanderi* for use in a method of treating or preventing OCD.

In another preferred embodiment, the central nervous system disorder or condition is major depressive disorder (MDD). Treatment with *Eubacterium* or *Faecalicatena* strains may provide clinical benefits in a mouse model of depression. The invention may be for use in modulating oxytocin and/or vasopressin hormones in the treatment or prevention of MDD. In preferred embodiments, the invention provides a composition comprising a bacterial strain of the species *Eubacterium callanderi* for use in a method of treating or preventing MDD.

In another preferred embodiment, the central nervous system disorder or condition is selected from anxiety disorders. The inventors have identified that treatment with *Eubacterium* strains (in particular *Eubacterium callanderi*) reduces disease incidence and disease severity in a mouse model of anxiety in the examples of this application. The invention may be for use in reducing stereotyped, repetitive, compulsive or anxious behaviour in the treatment of anxiety. In preferred embodiments, the invention provides a composition comprising a bacterial strain of the species *Eubacterium callanderi* for use in a method of treating or preventing anxiety disorders.

In another preferred embodiment, the central nervous system disorder or condition is selected from stress disorders, such as post-traumatic stress disorder. Compositions comprising a bacterial strain of the *Eubacterium* or *Faecalicatena* genera may reduce stress in mouse models of stress disorders. The invention may be for use in modulating the levels of oxytocin and/or vasopressin hormones in the treatment or prevention of stress disorders. In preferred embodiments, the invention provides a composition comprising a bacterial strain of the species *Eubacterium callanderi* for use in a method of treating or preventing stress disorders.

In another preferred embodiment, the central nervous system disorder or condition is selected from schizophrenia spectrum and psychotic disorders, such as schizophrenia. Compositions comprising a bacterial strain of the *Eubacterium* or *Faecalicatena* genera may improve positive and negative symptoms in mouse models of schizophrenia spectrum and psychotic disorders. In preferred embodiments, the invention provides a composition comprising a bacterial strain of the species *Eubacterium callanderi* for use in a method of treating or preventing schizophrenia spectrum and psychotic disorders.

In another preferred embodiment, the central nervous system disorder or condition is bipolar disorder. Compositions comprising a bacterial strain of the *Eubacterium* or *Faecalicatena* genera may reduce occasions of mania and/or depression in mouse models of bipolar disorder. The invention may be for use in modulating the levels of oxytocin and/or vasopressin hormones in the treatment or prevention of bipolar disorder. In preferred embodiments, the invention provides a composition comprising a bacterial strain of the species *Eubacterium callanderi* for use in a method of treating or preventing bipolar disorder.

In another preferred embodiment, the central nervous system disorder or condition is selected from neurocognitive disorders, such as Alzheimer's disease. Compositions comprising a bacterial strain of the *Eubacterium* or *Faecalicatena* genera may improve cognitive and behavioural functioning in mouse models of neurocognitive disorders. In preferred embodiments, the invention provides a composition comprising a bacterial strain of the species *Eubacterium callanderi* for use in a method of treating or preventing neurocognitive disorders, such as Alzheimer's disease

In further preferred embodiments, the invention provides a composition comprising a bacterial strain of the *Eubacterium* or *Faecalicatena* genera, for use in a method of treating or preventing Parkinson's disease. Compositions comprising a bacterial strain of the *Eubacterium* or *Faecalicatena* genera may improve motor and cognitive functions in mouse models of Parkinson's disease. The invention may be for use in modulating the levels of oxytocin and/or vasopressin hormones in the treatment or prevention of Parkinson's disease. In preferred embodiments, the invention provides a composition comprising a bacterial strain of the species *Eubacterium callanderi* for use in a method of treating or preventing Parkinson's disease.

In certain embodiments, the compositions of the invention are for use in a method of modulating the microbiota-gut-brain axis in the treatment or prevention of a disease or condition mediated by the microbiota-gut-brain axis. In particular, the compositions of the invention may be used in modulating the microbiota-gut-brain axis in the treatment or prevention of autism spectrum disorders; obsessive compulsive disorder; major depressive disorder; anxiety disorders; stress disorders; schizophrenia spectrum disorders, bipolar disorders, neurocognitive disorders and Parkinson's disease.

### Further embodiments of the invention

In certain embodiments, the composition is for oral administration. Oral administration of an *Anaerostipes, Eubacterium* or *Faecalicatena* strain can be effective for treating central nervous system disorders and conditions, in particular those mediated by the microbiota-gut-brain axis. Also, oral administration is convenient for patients and practitioners and allows delivery to and/or partial or total colonisation of the intestine.

In certain embodiments, the composition of the invention may comprise one or more pharmaceutically acceptable excipients or carriers.

The composition may be lyophilised. For example, the composition can comprise a lyophilised bacteria strain of the species *Anaerostipes hadrus,* or other bacterial strain as used in compositions of the invention (e.g. of the genus *Anaerostipes, Eubacterium* or *Faecalicatena*). Lyophilisation is an effective and convenient technique for preparing stable compositions that allow delivery of bacteria. Lyophilisation of bacterial strains or compositions of the invention is preferred.

The invention also provides a food product comprising the composition comprising a bacterial strain of the genus *Anaerostipes* (e.g. a bacterial strain of the species *Anaerostipes hadrus*) as described herein for use in therapy, such as in a method of treating or preventing a central nervous system disorder or condition as disclosed herein. In certain embodiments, the invention provides a food product comprising the composition as described above. Such food products preferably comprise a bacterial strain of the genus *Anaerostipes, Eubacterium* or *Faecalicatena.*

The invention also provides a vaccine composition comprising a bacterial strain of the genus *Anaerostipes* (e.g. a bacterial strain of the species *Anaerostipes hadrus* as described herein for use in preventing a central nervous system disorder or condition as disclosed herein. In certain embodiments, the invention provides a vaccine composition comprising the composition as described above. Such vaccine compositions preferably comprise a bacterial strain of the genus *Anaerostipes, Eubacterium* or *Faecalicatena.*

In developing the invention, the inventors have identified and characterised a bacterial genus that is particularly useful for therapy. The data disclosed herein suggest that the genus *Anaerostipes* may be useful for therapy, in particular treating or preventing the diseases described herein, such as autism spectrum disorder. Therefore, in another aspect, the invention provides a cell of a *Anaeroslipes* strain deposited under accession number NCIMB 43457 or NCIMB 43526 a derivative thereof, e.g. for a use or method as disclosed herein. In another aspect, the invention provides a cell of the bacterial strain under accession number NCIMB 43455, or a derivative thereof. The invention also provides a composition comprising such cells, or biologically pure cultures of such cells, e.g. for a use or method as disclosed herein. The invention also provides a cell of the bacterial strain deposited under accession number NCIMB 43455, or a derivative thereof, for use in therapy, in particular for the diseases described herein.

In further aspects, the invention provides new bacterial strains (as defined by 16s rRNA gene sequence in the Bacterial strains section below). Preferably, said bacterial strains are for use in therapy, in particular for the diseases described herein.

In preferred embodiments, the composition comprises a strain deposited under accession number NCIMB 43457, for use in therapy, such as a method of treating or preventing a central nervous system disorder or condition. Exemplary uses include: treating or preventing a neurodevelopmental disorder or a neuropsychiatric condition; and/or treating or preventing autism spectrum disorder, preferably autism. In especially preferred embodiments, the composition comprising a strain deposited under accession number NCIMB 43457 may be used in a method of preventing, reducing or alleviating one or more stereotyped, repetitive, compulsive and/or anxious behaviour, especially in the treatment of autism.

In preferred embodiments, the composition comprises a strain deposited under accession number NCIMB 43526, for use in therapy, such as a method of treating or preventing a central nervous system disorder or condition. Exemplary uses include: treating or preventing a neurodevelopmental disorder or a neuropsychiatric condition; and/or treating or preventing autism spectrum disorder, preferably autism. In especially preferred embodiments, the composition comprising a strain deposited under accession number NCIMB 43526 may be used in a method of preventing, reducing or alleviating one or more stereotyped, repetitive, compulsive and/or anxious behaviour, especially in the treatment of autism. The effect shown for the bacterial strains from the genus *Anaerostipes* on HDAC activity may provide therapeutic benefits for diseases and conditions mediated by aberrant HDAC activity, such as those listed above. In certain embodiments, the compositions of the invention may provide therapeutic benefits in the treatment of diseases or conditions with increased HDAC expression. In certain embodiments, the compositions of the invention may provide therapeutic benefits in the treatment of diseases or conditions with increased HDAC activity.

In certain embodiments of the invention, the bacterial strain in the composition is of *Anaerostipes.* In certain embodiments of the invention, the bacterial strain in the composition is of *Anaerostipes hadrus.* Closely related strains may also be used, such as bacterial strains that have a 16s rRNA gene sequence that is at least 95%, 96%, 97%, 98%, 99%, 99.5% or 99.9% identical to SEQ ID NO:1,SEQ ID NO:2 , SEQ ID NO: 3, SEQ ID NO: 4, SEQ ID NO: 5, SEQ ID NO: 6, SEQ ID NO: 7, SEQ ID NO: 15 or SEQ ID NO: 16. Preferably, the bacterial strain for use in the invention has the 16s rRNA gene sequence represented by SEQ ID NO: 1. Preferably, the bacterial strain for use in the invention has the 16s rRNA gene sequence represented by SEQ ID NO: 2. In further preferable embodiments the bacterial strain for use in the invention has the 16s rRNA gene sequence represented by SEQ ID NO: 6 or 7.

In certain embodiments, the composition of the invention is for oral administration. Oral administration of the strains of the invention can be effective for treating diseases and conditions mediated by HDAC activity. In certain embodiments, oral administration of the strains of the invention can be effective for treating diseases and conditions mediated by Class I HDAC activity Also, oral administration is convenient for patients and practitioners and allows delivery to and / or partial or total colonisation of the intestine.

In certain embodiments, the composition of the invention comprises one or more pharmaceutically acceptable excipients or carriers.

In certain embodiments, the composition of the invention comprises a bacterial strain that has been lyophilised. Lyophilisation is an effective and convenient technique for preparing stable compositions that allow delivery of bacteria.

In certain embodiments, the invention provides a food product comprising the composition as described above.

Additionally, the invention provides a method of treating or preventing a disease or condition mediated by HDAC activity, comprising administering a composition comprising a bacterial strain of the genus *Anaerostipes.*

The invention also provides a cell of the *Anaerostipes hadrus* strain deposited under accession number DSM 108065 or DSM 3319, or derivatives thereof, for use in therapy, in particular for the diseases described herein.

Further numbered embodiments of the invention are provided below:
1. A composition comprising a bacterial strain of the genus *Anaerostipes,* for use in therapy.
2. A composition comprising a bacterial strain which has at least 95%,, at least 96%, at least 97%, at least 98%, at least 99%, at least 99.5% or at least 99.95% identity to the sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 15 or SEQ ID NO: 16, for use in therapy.
3. The composition of embodiment 2, wherein the bacterial strain can
   (a) inhibit the activity of HDAC 1 by at least 40% when assayed using a fluoregenic assay; and/or
   (b) inhibit the activity of HDAC 2 by at least 60% when assayed using a fluoregenic assay; and/or
   (c) inhibit the activity of HDAC 3 by at least 50% when assayed using a fluoregenic assay.
4. The composition according to any preceding embodiment, for use in the treatment or prevention of a disease or condition mediated by histone deacetylase (HDAC) activity.
5. The composition according to any preceding embodiment, for use in the treatment or prevention of a disease or condition mediated by Class I HDAC activity.
6. The composition according to any preceding embodiment, for use in a method of inhibiting Class I HDAC activity in a condition mediated by Class I HDAC activity.
7. The composition according to any preceding embodiment, for use in a method of selectively inhibiting Class I HDAC activity in a condition mediated by Class I HDAC activity.
8. The composition according to any preceding embodiment, wherein the composition is for use in selectively inhibiting HDAC1, HDAC2 or HDAC3 activity in a disease or condition mediated by HDAC1, HDAC2 or HDAC3 activity.
9. The composition according to any preceding embodiment, wherein the composition is for use in the treatment or prevention of a disease or condition in which inhibiting HDAC activity is beneficial.
10. The composition according to any preceding embodiment, for use in a patient with elevated HDAC activity.
11. The composition according to any preceding embodiment, for use in the treatment or prevention of a disease or condition selected from the list consisting of: a neurodegenerative disease, such as Alzheimer's disease, Huntington's disease or Parkinson's disease; brain injury, such as stroke; behavioural or psychiatric disorders, such as attention deficit hyperactivity disorder, obsessive compulsive disorder, anxiety disorder, biopolar disorder, or post-traumatic stress disorder; an inflammatory or autoimmune disease, such as asthma, arthritis, psoriasis, multiple sclerosis, diabetes, allograft rejection, graft-versus-host disease, or an inflammatory bowel disease, such as Crohn's disease; or cancer, such as prostate cancer, colorectal cancer, breast cancer, lung cancer, liver cancer or gastric cancer.
12. The composition of embodiment 11, for use in the treatment or prevention of a neurodegenerative disorder, preferably wherein the bacterial strain is of the species *Anaerostipes hadrus.*
13. The composition of embodiment 12, for use in the treatment or prevention of Parkinson's disease.
14. The composition of embodiment 12, for use in the treatment or prevention of Huntington's disease.
15. The composition of embodiment 12, for use in the treatment or prevention of Alzheimer's disease.
16. The composition of embodiment 11, for use in the treatment or prevention of a behavioural disorder, preferably wherein the bacterial strain is of the species *Anaerostipes hadrus.*
17. The composition of embodiment 16, for use in the treatment or prevention of attention deficit hyperactive disorder.
18. The composition according to embodiment 12, for use in the treatment or prevention of a behavioural disorder, preferably wherein the bacterial strain is of the species *Anaerostipes hadrus.*
19. The composition of embodiment 16, for use in the treatment or prevention of attention deficit hyperactive disorder.
20. The composition according to embodiment 11, for use in the treatment or prevention of hyperactivity, preferably wherein the bacterial strain is of the species *Anaerostipes hadrus.*
21. The composition according to embodiment 11, for use in the treatment or prevention of inflammatory bowel disease, preferably wherein the bacterial strain is of the species *Anaerostipes hadrus.*
22. The composition according to embodiment 21, for use in the treatment or prevention of ulcerative colitis.
23. The composition according to embodiment 21, for use in the treatment or prevention of Crohn's disease.
24. The composition according to embodiment 11, for use in the treatment or prevention of cancer.
25. The composition for use according to embodiment 24, wherein the cancer is selected from the list consisting of prostate cancer, colorectal cancer, breast cancer, lung cancer, liver cancer or gastric cancer.
26. The composition according to any preceding embodiment, for use in the treatment or prevention of an inflammatory or autoimmune disease.
27. The composition according to any preceding embodiment, for use in the prevention or treatment of graft-versus-host disease.
28. The composition of any preceding embodiment, wherein the bacterial strain is of the species *Anaerostipes hadrus.*
29. The composition of any preceding embodiment, wherein the bacterial strain has a 16s rRNA gene sequence that is at least 95%, 96%, 97%, 98%, 99%, 99.5% or 99.9% identical to SEQ ID NO:1.
30. The composition of any preceding embodiment, wherein the bacterial strain has a 16s rRNA gene sequence represented by SEQ ID NO:1.
31. The composition of any preceding embodiment, wherein the bacterial strain has a 16s rRNA gene sequence that is at least 95%, 96%, 97%, 98%, 99%, 99.5% or 99.9% identical to SEQ ID NO:2.
32. The composition of any preceding embodiment, wherein the bacterial strain has a 16s rRNA gene sequence represented by SEQ ID NO:2.
33. The composition of any preceding embodiment, wherein the bacterial strain has a 16s rRNA gene sequence that is at least 95%, 96%, 97%, 98%, 99%, 99.5% or 99.9% identical to SEQ ID NO:6.
34. The composition of any preceding embodiment, wherein the bacterial strain has a 16s rRNA gene sequence represented by SEQ ID NO:6.
35. The composition of any preceding embodiment, wherein the bacterial strain has a 16s rRNA gene sequence that is at least 95%, 96%, 97%, 98%, 99%, 99.5% or 99.9% identical to SEQ ID NO:7.
36. The composition of any preceding embodiment, wherein the bacterial strain has a 16s rRNA gene sequence represented by SEQ ID NO:7.
37. The composition of any preceding embodiment, wherein the bacterial strain has a 16s rRNA gene sequence that is at least 95%, 96%, 97%, 98%, 99%, 99.5% or 99.9% identical to SEQ ID NO:15.
38. The composition of any preceding embodiment, wherein the bacterial strain has a 16s rRNA gene sequence represented by SEQ ID NO:15.
39. The composition of any preceding embodiment, wherein the composition is for oral administration.
40. The composition of any preceding embodiment, wherein the composition comprises one or more pharmaceutically acceptable excipients or carriers.
41. The composition of any preceding embodiment, wherein the bacterial strain is lyophilised.
42. The composition according to any preceding embodiment, for use as a histone deacetylase inhibiting medicament.
43. The composition according to any preceding embodiment, for use as a Class I histone deacetylase inhibiting medicament.
44. The composition according to any preceding embodiment, for use as a HDAC2 inhibiting medicament.
45. The composition according to any preceding embodiment, for use as a selective HDAC2 inhibiting medicament.
46. A food product comprising the composition of any preceding embodiment, for the use of any preceding embodiment.
47. A method of treating or preventing a disease or condition mediated by histone deacetylase activity, comprising administering a composition comprising a bacterial strain of the genus *Anaerostipes* to a patient in need thereof.
48. A cell of the *Anaerostipes hadrus* strain deposited under accession number DSM 108065 or DSM 3319, or a derivative thereof, for use in therapy.
49. A cell of the *Anaerostipes hadrus* strain deposited under accession number DSM 108065 or DSM 3319, or a derivative thereof, for use in the treatment or prevention of a disease or condition as defined in one of embodiments 2-27.
50. A pharmaceutical composition comprising a cell of the *Anaerostipes hadrus* strain deposited under accession number DSM 108065 or DSM 3319, or a derivative thereof.
51. A cell of the *Anaerostipes hadrus* strain deposited under accession number NCIMB 43457 or NCIMB 43526, or a derivative thereof, for use in therapy.
52. A cell of the *Anaerostipes hadrus* strain deposited under accession number NCIMB 43457 or NCIMB 43526, or a derivative thereof, for use in the treatment or prevention of a disease or condition as defined in one of embodiments 2-27.
53. A pharmaceutical composition comprising a cell of the *Anaerostipes hadrus* strain deposited under accession number NCIMB 43457 or NCIMB 43526, or a derivative thereof.
54. A composition comprising a bacterial strain which has at least 95%, at least 96%, at least 97%, at least 98%, at least 99%, at least 99.5% or at least 99.95% identity to the sequence of SEQ ID NO: 6 for use in therapy.
55. A composition comprising a bacterial strain which has at least 95%, at least 96%, at least 97%, at least 98%, at least 99%, at least 99.5% or at least 99.95% identity to the sequence of SEQ ID NO: 7 for use in therapy.
56. A composition comprising a bacterial strain of the genus *Anaerostipes,* for use in a method of treating or preventing a central nervous system disorder or condition.
57. A composition comprising a bacterial strain which has at least 95%, at least 96%, at least 97%, at least 98%, at least 99%, at least 99.5% or at least 99.95% identity to the sequence of SEQ ID NO: 1, SEQ ID NO: 2, SEQ ID NO: 3, SEQ ID NO: 4, SEQ ID NO: 5, SEQ ID NO: 6 SEQ ID NO: 7, SEQ ID NO: 15 or SEQ ID NO: 16, for use in a method of treating or preventing a central nervous system disorder or condition.
58. A composition comprising a bacterial strain which has at least 95%, at least 96%, at least 97%, at least 98%, at least 99%, at least 99.5% or at least 99.95% identity to the sequence of SEQ ID NO: 6 or SEQ ID NO: 7 for use in a method of treating or preventing a central nervous system disorder or condition.
59. The composition for use according to any proceeding embodiment, wherein the bacterial strain is of the species *Anaerostipes hadrus, Anaerostipes butyraticus, Anaerostipes caccae* or *Anaerostipes rhamnosivorans.*
60. The composition for use according to any proceeding embodiment, wherein the central nervous system disorder or condition is mediated by the microbiota-gut-brain axis.
61. The composition for use according to embodiments 56-60, wherein the composition is for use in a method of treating or preventing a neurodevelopmental disorder or a neuropsychiatric condition
62. The composition for use according to embodiments 56-60, wherein the composition is for use in a method of treating or preventing a disorder or condition selected from the group consisting of autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's disease; Parkinson's disease; chronic pain; motor neuron disease; Huntington's disease; Guillain-Barre syndrome and meningitis.
63. The composition for use according to embodiments 56-60, wherein the composition is for use in a method of treating or preventing autism spectrum disorder.
64. The composition for use of embodiment 63, wherein the composition is for use in a method of reducing or preventing autism.
65. The composition for use according to embodiments 64, wherein the composition prevents, reduces or alleviates one or more stereotyped, repetitive, compulsive and/or anxious behaviour.
66. The composition for use according to embodiments 56-60, wherein the composition is for use in a method of treating or preventing obsessive compulsive disorder.
67. The composition for use of embodiment 66, wherein the composition prevents, reduces or alleviates one or more repetitive, compulsive and/or anxious behaviour.
68. The composition for use according to embodiments 56-60, wherein the composition is for use in in a method of treating or preventing major depressive disorder.
69. The composition for use of embodiment 68, wherein the composition treats or prevents acute major depressive episodes and/or the prevention of new episodes (recurrence prevention).
70. The composition for use of embodiment 68 or 69, wherein the composition prevents, reduces or alleviates the occurrence of mild, moderate or severe MDD episodes
71. The composition for use according to embodiments 56-60, wherein the composition is for use in a method of treating or preventing anxiety disorders.
72. The composition for use of embodiment 71, wherein the anxiety disorder is generalised anxiety disorder (GAD); specific phobia; social anxiety disorder; separation anxiety disorder; agoraphobia; panic disorder and/or selective mutism.
73. The composition for use according to embodiments 56-60, wherein the composition is for use in a method of treating or preventing neurocognitive disorders.
74. The composition for use of embodiment 73, wherein the neurocognitive disorder is vascular dementia; mixed form Alzheimer's disease and vascular dementia; Lewy body disease; frontotemporal dementia; Parkinson's dementia, Creutzfeldt-Jakob disease; Huntington's disease; and Wernicke-Korsakoff syndrome.
75. The composition for use according to any preceding embodiment, wherein the composition is for use in a method of modulating the microbiota-gut-brain axis.
76. The composition for use according to embodiments 56-60, wherein the composition is for use in treating epilepsy.
77. The composition for use of any preceding embodiment, wherein the bacterial strain has a 16s rRNA sequence that is at least 95%, 96%, 97%, 98%, 99%, 99.5% or 99.9% identical to the 16s rRNA sequence of a bacterial strain of *Anaerostipes hadrus.*
78. The composition for use of any preceding embodiment, wherein the bacterial strain has a 16s rRNA sequence that is at least 95%, 96%, 97%, 98%, 99%, 99.5% or 99.9% identical to SEQ ID NO: 1, 2, 6 or 7.
79. The composition for use of any embodiment 78, wherein the bacterial strain has a 16s rRNA sequence that is at least 95%, 96%, 97%, 98%, 99%, 99.5% or 99.9% identical to SEQ ID NO: 6 or is preferably SEQ ID NO: 6.
80. The composition for use of any embodiment 78, wherein the bacterial strain has a 16s rRNA sequence that is at least 95%, 96%, 97%, 98%, 99%, 99.5% or 99.9% identical to SEQ ID NO: 6 or is preferably SEQ ID NO: 7.
81. The composition for use of any preceding embodiment, wherein the bacterial strain has a 16s rRNA sequence that is at least 95%, 96%, 97%, 98%, 99%, 99.5% or 99.9% identical to the 16s rRNA sequence of a bacterial strain of *Anaerostipes butyraticus.*
82. The composition for use of any preceding embodiment, wherein the bacterial strain has a 16s rRNA sequence that is at least 95%, 96%, 97%, 98%, 99%, 99.5% or 99.9% identical to SEQ ID NO: 3
83. The composition for use of any preceding embodiment, wherein the bacterial strain has a 16s rRNA sequence that is at least 95%, 96%, 97%, 98%, 99%, 99.5%, or 99.9% identical to the 16s rRNA sequence of a bacterial strain of *Anaerostipes rhamnosivorans*
84. The composition for use of any preceding embodiment, wherein the bacterial strain has a 16s rRNA sequence that is at least 95%, 96%, 97%, 98%, 99%, 99.5%, 99.9% or 100% identical to SEQ ID NO: 4 or 16.
85. The composition for use of any preceding embodiment, wherein the bacterial strain has a 16s rRNA sequence that is at least 95%, 96%, 97%, 98%, 99%, 99.5%, 99.9% or 100% identical to the 16s rRNA sequence of a bacterial strain of *Anaerostipes caccae.*
86. The composition for use of any preceding embodiment, wherein the bacterial strain has a 16s rRNA sequence that is at least 95%, 96%, 97%, 98%, 99%, 99.5%, 99 9% or 100% identical to SEQ ID NO: 5.
87. The composition for use of any preceding embodiment, wherein the bacterial strain has a 16s rRNA sequence that is at least 95%, 96%, 97%, 98%, 99%, 99.5%, 99.9% or 100% identical to SEQ ID NO: 15.
88. The composition for use of any preceding embodiment, wherein the bacterial strain is selected from one of the strains deposited under accession number NCIMB 43457, or a derivative thereof.
89. The composition for use of any preceding embodiment, wherein the bacterial strain is selected from one of the strains deposited under accession number NCIMB 43526, or a derivative thereof.
90. The composition for use of any preceding embodiment, wherein the composition is for oral administration.
91. The composition for use of any preceding embodiment, wherein the composition comprises one or more pharmaceutically acceptable excipients or carriers.
92. The composition for use of any preceding embodiment, wherein the bacterial strain is lyophilised.
93. The composition for use of any preceding embodiment, wherein the bacterial strain is viable and capable of partially or totally colonising the intestine.
94. The composition for use of any preceding embodiment, wherein the composition comprises a single strain of the genus *Anaerostipes,* such as a bacterial strain of the species *Anaerostipes hadrus.*
95. The composition for use of any preceding embodiment, which comprises the bacterial strain of the genus *Anaerostipes,* optionally a bacterial strain of the species *Anaerostipes hadrus,* as part of a microbial consortium.
96. A food product comprising the composition of any preceding embodiment, for the use of any preceding embodiment.
97. A vaccine composition comprising the composition of any preceding embodiment, for the use of any preceding embodiment.
98. A method of treating or preventing a central nervous system disorder or condition, comprising administering a composition comprising a bacterial strain of the genus *Anaerostipes* to a patient in need thereof.
99. A method of treating or preventing a central nervous system disorder or condition, comprising administering a composition comprising a bacterial strain that has at least 95%, at least 96%, at least 97%, at least 98%, at least 99%, at least 99.5% or at least 99.95% identity to the sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 15 or SEQ ID NO: 16 to a patient in need thereof.
100. A method of treating or preventing a central nervous system disorder or condition, comprising administering a composition comprising a bacterial strain that has at least 95%, at least 96%, at least 97%, at least 98%, at least 99%, at least 99.5% or at least 99.95% identity to the sequence of SEQ ID NO: 6 or 7, to a patient in need thereof.
101. Use of a bacterial strain of the genus *Anaerostipes* in the manufacture of a medicament for treating or preventing a central nervous system disorder or condition.
102. Use of a bacterial strain in the manufacture of a medicament for treating or preventing a central nervous system disorder or condition, wherein the a bacterial strain has at least 95%, at least 96%, at least 97%, at least 98%, at least 99%, at least 99 5% or at least 99.95% identity to the sequence SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 15 or SEQ ID NO: 16.
103. Use of a bacterial strain in the manufacture of a medicament for treating or preventing a central nervous system disorder or condition, wherein the a bacterial strain has at least 95%, at least 96%, at least 97%, at least 98%, at least 99%, at least 99.5% or at least 99.95% identity to the sequence of SEQ ID NO: 6 or 7.
104. The method of embodiment 98-100, or the use of embodiment 101-103, wherein the bacterial strain is of the species *Anaerostipes hadrus.*
105. A cell of a *Anaerostipes* strain selected from one of the strains deposited under accession number NCIMB 43457, or a derivative thereof, for the use or method of any one of embodiments.
106. A cell of a *Anaerostipes* strain selected from one of the strains deposited under accession number NCIMB 43526, or a derivative thereof, for the use or method of any one of embodiments.
107. A composition comprising the cell of embodiment 105 or 106, for the use of any one of preceding embodiments.
108. The composition for use of embodiment 107, comprising a pharmaceutically acceptable carrier or excipient.
109. A biologically pure culture of a *Anaerostipes* strain selected from one of the strains deposited under accession number NCIMB 43457, or a derivative thereof.
110. A biologically pure culture of a *Anaerostipes* strain selected from one of the strains deposited under accession number NCIMB 43526, or a derivative thereof.
111. A composition comprising a bacterial strain of the genus *Eubacterium* or *Faecalicatena,* for use in a method of treating or preventing a central nervous system disorder or condition.
112. The composition of embodiment 111, wherein the central nervous system disorder or condition is mediated by the microbiota-gut-brain axis.
113. The composition of embodiment 111 or embodiment 112, wherein the composition is for use in a method of treating or preventing a neurodevelopmental disorder or a neuropsychiatric condition.
114. The composition of any one of embodiments 111-113, wherein the composition is for use in a method of treating or preventing a disorder or condition selected from the group consisting of autism spectrum disorders (ASDs); child developmental disorder; obsessive compulsive disorder (OCD); major depressive disorder (MDD); depression; seasonal affective disorder; anxiety disorders; chronic fatigue syndrome (myalgic encephalomyelitis); stress disorder; post-traumatic stress disorder; schizophrenia spectrum disorders; schizophrenia, bipolar disorder; psychosis; mood disorder; dementia; Alzheimer's; Parkinson's disease; chronic pain; motor neuron disease; Huntington's disease; Guillain-Barre syndrome and meningitis.
115. The composition of embodiment 114, wherein the composition is for use in a method of treating or preventing autism spectrum disorder.
116. The composition of embodiment 114 or 115, wherein the composition is for use in a method of reducing or preventing autism.
117. The composition of any one of embodiments 111-116, wherein the composition is for use in preventing, reducing or alleviating stereotyped, repetitive, compulsive or anxious behaviour.
118. The composition of embodiment 114, wherein the composition is for use in a method of treating or preventing obsessive compulsive disorder.
119. The composition of embodiment 118, wherein the composition is for use in preventing, reducing or alleviating repetitive, compulsive and/or anxious behaviour.
120. The composition of embodiment 114, wherein the composition is for use in a method of treating or preventing major depressive disorder.
121. The composition of embodiment 120, wherein the composition is for use in treating or preventing acute major depressive episodes and/or preventing new episodes (recurrence prevention).
122. The composition of embodiment 120 or 121, wherein the composition is for use in preventing, reducing or alleviating the occurrence of mild, moderate or severe MDD episodes.
123. The composition of embodiment 114, wherein the composition is for use in a method of treating or preventing anxiety disorders.
124. The composition of embodiment 123, wherein the anxiety disorder is generalised anxiety disorder (GAD); specific phobia; social anxiety disorder; separation anxiety disorder; agoraphobia; panic disorder and/or selective mutism.
125. The composition of embodiment 111, wherein the composition is for use in a method of treating or preventing neurocognitive disorders.
126. The composition of embodiment 125, wherein the neurocognitive disorder is vascular dementia; Alzheimer's disease; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.
127. The composition of any one of embodiments 111-126, wherein the composition is for use in a method of modulating the microbiota-gut-brain axis.
128. The composition of embodiments 111-127, wherein the bacterial strain has a 16s rRNA gene sequence that is at least 95%, 96%, 97%, 98%, 99%, 99.5% or 99.9% identical to SEQ ID NO: 8, 9, 10, 11, 12, 13 or 14.
129. The composition of embodiments 111-127, wherein the bacterial strain has a 16s rRNA gene sequence that is at least 95%, 96%, 97%, 98%, 99%, 99.5% or 99.9% identical to SEQ ID NO: 8, or wherein the bacterial strain has the 16s rRNA gene sequence represented by SEQ ID NO: 8.
130. The composition of embodiments 111-127, wherein the bacterial strain has a 16s rRNA gene sequence that is at least 95%, 96%, 97%, 98%, 99%, 99.5% or 99.9% identical to SEQ ID NO: 9, or wherein the bacterial strain has the 16s rRNA gene sequence represented by SEQ ID NO: 9.
131. The composition of embodiments 111-127, wherein the bacterial strain has a 16s rRNA gene sequence that is at least 95%, 96%, 97%, 98%, 99%, 99.5% or 99.9% identical to SEQ ID NO: 10 or SEQ ID NO: 11, or wherein the bacterial strain has the 16s rRNA gene sequence represented by SEQ ID NO: 10 or SEQ ID NO: 11.
132. The composition of embodiments 111-127, wherein the bacterial strain has a 16s rRNA gene sequence that is at least 95%, 96%, 97%, 98%, 99%, 99.5% or 99.9% identical to SEQ ID NO: 12 or wherein the bacterial strain has the 16s rRNA gene sequence represented by SEQ ID NO: 12.
133. The composition of embodiments 111-127, wherein the bacterial strain has a 16s rRNA gene sequence that is at least 95%, 96%, 97%, 98%, 99%, 99.5% or 99.9% identical to SEQ ID NO: 13 or wherein the bacterial strain has the 16s rRNA gene sequence represented by SEQ ID NO: 13.
134. The composition of embodiments 111-127, wherein the bacterial strain has a 16s rRNA gene sequence that is at least 95%, 96%, 97%, 98%, 99%, 99.5% or 99.9% identical to SEQ ID NO: 14 or wherein the bacterial strain has the 16s rRNA gene sequence represented by SEQ ID NO: 14.
135. The composition of any of embodiments 111-129, wherein the bacterial strain is of the species *Eubacterium callanderi.*
136. The composition of any of embodiments 111-129, wherein the bacterial strain is of the species *Eubacterium limosum.*
137. The composition of any of embodiments 111-128 or 130, wherein the bacterial strain is of the species *Eubacterium eligens.*
138. The composition of any of embodiments 111-128 or 131, wherein the bacterial strain is of the species *Eubacterium rectale.*
139. The composition of any of embodiments 111-128 or 134, wherein the bacterial strain is of the species *Eubacterium hallii.*
140. The composition of any of embodiments 111-128 or 132, wherein the bacterial strain is of the species *Faecalicatena fissicatena.*
141. The composition of any of embodiments 111-128 or 133, wherein the bacterial strain is of the species *Faecalicatena contorta.*
142. A composition comprising a bacterial strain, wherein the bacterial strain has a 16s rRNA gene sequence that is least 95%, 96%, 97%, 98%, 99%, 99.5% or 99.9% identical to SEQ ID NO: 8, for use in a method of treating or preventing a central nervous system disorder or condition.
143. A composition comprising a bacterial strain, wherein the bacterial strain has a 16s rRNA gene sequence that is least 95%, 96%, 97%, 98%, 99%, 99.5% or 99.9% identical to SEQ ID NO: 9, for use in a method of treating or preventing a central nervous system disorder or condition.
144. A composition comprising a bacterial strain, wherein the bacterial strain has a 16s rRNA gene sequence that is least 95%, 96%, 97%, 98%, 99%, 99.5% or 99.9% identical to SEQ ID NO: 10 or SEQ ID NO: 11, for use in a method of treating or preventing a central nervous system disorder or condition.
145. A composition comprising a bacterial strain, wherein the bacterial strain has a 16s rRNA gene sequence that is least 95%, 96%, 97%, 98%, 99%, 99.5% or 99.9% identical to SEQ ID NO: 12, for use in a method of treating or preventing a central nervous system disorder or condition.
146. A composition comprising a bacterial strain, wherein the bacterial strain has a 16s rRNA gene sequence that is least 95%, 96%, 97%, 98%, 99%, 99.5% or 99.9% identical to SEQ ID NO: 13, for use in a method of treating or preventing a central nervous system disorder or condition.
147. A composition comprising a bacterial strain, wherein the bacterial strain has a 16s rRNA gene sequence that is least 95%, 96%, 97%, 98%, 99%, 99.5% or 99.9% identical to SEQ ID NO: 14, for use in a method of treating or preventing a central nervous system disorder or condition.
148. A composition according to any of embodiments 142-147, for use in a method according to any of embodiments 112-127.
149. The composition of any one of embodiments 111-148, wherein the composition is for oral administration.
150. The composition of any one of embodiments 111-149, wherein the composition comprises one or more pharmaceutically acceptable excipients or carriers.
151. The composition of any one of embodiments 111-150, wherein the bacterial strain is lyophilised.
152. The composition of any one of embodiments 111-151, wherein the bacterial strain is viable.
153. The composition of any one of embodiments 111-152, wherein the bacterial strain is capable of partially or totally colonising the intestine.
154. The composition of any one of embodiments 111-153, wherein the composition comprises a single species of *Eubacterium* or *Faecalicatena.*
155. The composition of any one of embodiments 111-154, wherein the composition comprises a single strain of *Eubacterium* or *Faecalicatena.*
156. The composition of any one of embodiments 111-155, which comprises the bacterial strain of *Eubacterium* or *Faecalicatena* as part of a microbial consortium.
157. A food product comprising the composition of any one of embodiments 111-156, for the use of any one of embodiments 111-156.
158. A vaccine composition comprising the composition of any one of embodiments 111-157, for the use of any one of embodiments 111-157.
159. A method of treating or preventing a central nervous system disorder or condition, comprising administering a composition comprising a bacterial strain of *Eubacterium* or *Faecalicatena* to a patient in need thereof.
160. A bacterial strain having a 16s rRNA gene sequence that is at least 98%, 99%, 99.5% or 99.9% identical to SEQ ID NO: 8, or wherein the bacterial strain has the 16s rRNA gene sequence represented by SEQ ID NO: 8.
161. A bacterial strain having a 16s rRNA gene sequence that is at least 98%, 99%, 99.5% or 99.9% identical to SEQ ID NO: 9, or wherein the bacterial strain has the 16s rRNA gene sequence represented by SEQ ID NO: 9.
162. A bacterial strain having a 16s rRNA gene sequence that is at least 98%, 99%, 99.5% or 99.9% identical to SEQ ID NO: 10, or wherein the bacterial strain has the 16s rRNA gene sequence represented by SEQ ID NO: 10.
163. A bacterial strain having a 16s rRNA gene sequence that is at least 98%, 99%, 99.5% or 99.9% identical to SEQ ID NO: 11, or wherein the bacterial strain has the 16s rRNA gene sequence represented by SEQ ID NO: 11.
164. A bacterial strain having a 16s rRNA gene sequence that is at least 98%, 99%, 99.5% or 99.9% identical to SEQ ID NO: 12, or wherein the bacterial strain has the 16s rRNA gene sequence represented by SEQ ID NO: 12.
165. A bacterial strain having a 16s rRNA gene sequence that is at least 98%, 99%, 99.5% or 99.9% identical to SEQ ID NO: 13, or wherein the bacterial strain has the 16s rRNA gene sequence represented by SEQ ID NO: 13.
166. A bacterial strain having a 16s rRNA gene sequence that is at least 98%, 99%, 99.5% or 99.9% identical to SEQ ID NO: 14, or wherein the bacterial strain has the 16s rRNA gene sequence represented by SEQ ID NO: 14.
167. A cell of the bacterial strain deposited under accession number NCIMB 43455, or a derivative thereof.
168. A cell of the bacterial strain deposited under accession number NCIMB 42689, or a derivative thereof.
169. A bacterial strain or a cell according to any of embodiments 160-168, for use in therapy.
170. A bacterial strain or a cell according to any of embodiments 160-168, for use in a method according to any of embodiments 112-127.
171. A composition comprising the bacterial strain or the cell according to any of embodiments 160-168, further comprising a pharmaceutically acceptable carrier or excipient.
172. A biologically pure culture of the bacterial strain or the cell according to any of embodiments 160-168.

### BRIEF DESCRIPTION OF DRAWINGS

**Figure** 1 shows the concentration of the short-chain fatty acids acetate (A), propionate (B), isobutyrate (C), isovalerate (D) and valerate (E) in the ceacal content.
**Figure** 2 shows the expression of IL-1β (A), IL-6 (B), TNFα (C), and IL-10 (D) upon LPS or ConA stimulation by splenocytes from mice treated with *Anaerostipes hadrus.*
**Figure** 3 shows the expression of vasopressin receptor (A), CRFR2 (B), CD11b (C), 5HTR1a (D), Grin2A (E) and Grin2B (F) in the hippocampus, mineralocorticoid receptor (G), CRFR2 (H), CRFR1 (I) and CD11b (J) in the amygdala, and CRFR2 (K), CD11b (L) and IL-6 (M) in the prefrontal cortex, normalized to β-actin, with and without treatment with *Anaerostipes hadrus.* ^{∗}=p≤0.05, ^{∗∗}=p≤0.01.
**Figure 4** shows changes in histone deacetylase (HDAC) activity.
**Figure** 5: Effect of *Anaerostipes* on intestinal permeability.
**Figure** 6: Effect of *Anaerostipes* on short-chain fatty acid production.
**Figure** 7: Effect of *Anaerostipes* on peripheral immune markers.
**Figure 8****:** Effect of *Anaerostipes* on gene expression in the brain.
**Figure 9****:** Effect of *Anaerostipes* on stereotyped behaviour in BtBR and MIA mice in the marble burying test.
**Figure 10****:** Effect of *Anaerostipes* on stereotyped or repetitive behaviour in BtBR mice in the grooming test.
**Figure 11****:** Effect *Anaerostipes* on anxiety-like behaviour in the elevated plus maze in BtBR mice, as measured by duration in the open arm.
**Figure 12****:** Effect of *Anaerostipes* on the social behaviour of BtBR and MIA mice on social behaviour in the three-chamber test comparing object *vs* mouse.
**Figure 13**: Effect of *Anacrostipes* on the social behaviour of BtBR and MIA mice on social behaviour in the three-chamber test comparing familiar mouse *vs* novel mouse.
**Figure 14****:** Effect of *Anaerostipes* on cognition performance of BtBR and MIA mice in the novel object recognition test.
**Figure 15****:** Effect of *Anaerostipes* on social behaviour immobility time of BtBR and MIA mice in the forced swimming test.
**Figure 16****:** Effect of *Anaerostipes* on intestinal motility in BtBR mice.
**Figure 17****:** Effect of *Anaerostipes* on intestinal permeability in BtBR and MIA mice.
**Figure 18****:** Effect of *Anaerostipes* on serotonin levels in the brainstem.
**Figure 19****:** Effect of *Anaerostipes* on 5-HIAA/5-HT turnover in the brainstem. 5-HIAA: 5-hydroxyindole-acetic acid; 5-HT: 5-hydroxy-tryptamine (serotonin).
**Figure 20****:** Effect of *Anaerostipes* on gene expression in the amygdala in models of autism spectrum disorders.
**Figure 21** shows the results of the marble burying test and grooming test in Btbr and MIA mice. ^{∗}= p≤0.05 and ^{∗∗}= p≤0.01.
**Figure 22** shows the results of the elevated plus maze and open field test in Btbr and MIA mice. ^{∗}= p≤0.05.
**Figure 23** shows the setup of the experiment to measure the time spent with social stimuli compared with non-social stimuli and time spent exploring a novel animal compared with familiar animal, for both Btbr and MIA mice. The duration is expressed in s. ^{∗∗}= p≤0.01 and ^{∗∗∗∗}= p≤0.0001.
**Figure 24** shows the results of the forced swim test for Btbr and MIA mice. ^{∗∗}= p≤0.01.
**Figure 25** shows the results of the gut motility and gut permeability tests for Btbr and MIA mice. ^{∗}= p≤0.05. 5A shows the results of gut motility assays for Btbr and MIA mice. 5B shows the results of colon permeability assays for MIA mice. 5C shows the results of colon permeability assays for Btbr mice. 5D shows the results of ileum permeability assays for MIA mice. 5E shows the results of ileum permeability assays for Btbr mice.
**Figure 26** shows the brainstem levels of noradrenaline, serotonin and 5-HIAA/5-HT turnover for Btbr and MIA mice. ^{∗}= p≤0.05.
**Figure 27** shows amygdalar gene expression levels expression of (A) Btbr and (B) MIA mice, normalized to β-actin. ^{∗}=p≤0.05.
**Figure 28** shows the levels of acetylated histone protein H3 (top panels) and H4 (bottom panels) in HCT116 cells, after treatment with supernatant of the indicated bacterial strain, or a control treatment ^{∗}= 0.01<p≤0.05, ^{∗∗}= 0.001<p≤0.01, ^{∗∗∗∗}= p≤0.0001.
**Figure 29** shows the levels of acetylated histone protein H3 (top panels) and H4 (bottom panels) in HT29 cells, after treatment with supernatant of the indicated bacterial strain, or a control treatment. ^{∗}= 001<p≤0.05, ^{∗∗}= 0.001<p≤0.01.

### DISCLOSURE OF THE INVENTION

### Bacterial strains

The compositions for use according to the invention comprise a Gram-positive, rod-shaped and anaerobic bacterial strain of the order *Clostridiales,* wherein the bacterial strain does not belong to the genera *Roseburia* or *Bariatricus,* or the family *Clostridiacae* (*i.e.* the bacterial strain belongs to neither the genus *Roseburia* nor *Bariatricus,* nor to the family *Clostridiacae*)*.* The examples demonstrate that such bacteria are useful for treating or preventing central nervous system disorders, cancer and other disorders.

The bacterial strain may, for example, be of the family *Lachnospiraceae* or *Eubacteriaceae.* Preferably, the bacterial strain is of the genus *Anaerostipes, Eubacterium* or *Faecalicatena.* As detailed below, preferably, the bacterial strain is of the species *Anaerostipes hadrus, Eubacterium callanderi, Eubacterium limosum, Eubacterium eligens, Eubacterium hallii, Eubacterium rectale, Faecalicatena fissicatena* or *Faecalicatena contorta*; more preferably *Anaerostipes hadrus, Eubacterium callanderi,* or *Eubacterium limosum*; even more preferably *Anaerostipes hadrus* or *Eubacterium callanderi.*

The term "Gram-positive" means giving a positive result in the Gram strain test (*i.e*. retaining the colour of the crystal violet staining reagent). Retention of crystal violet staining by a bacterium is linked to the thickness of the peptidoglycan layer in the bacterial cell wall. Gram-positive bacteria have a thicker peptidoglycan layer. Gram-staining is commonly used to help classify bacterial strains in the field of microbiology.

The term "rod-shaped" means having a cellular shape that approximates to a round-ended cylinder (e.g. when examined under a light microscope). This shape is similar to that of bacterial strains of the genus *Bacillus* (e.g. when examined under a light microscope). As such, "rod-shaped" is also synonymous with the terms *"bacilliform"* and *"bacilli",* as used in the art. The characteristic shape of a bacterial strain (such as "rod-shaped") is a commonly used classification criterion in the field of microbiology.

The term "anaerobic" means not requiring oxygen for growth. Anaerobic bacterial strains comprise bacterial strains that are obligate anaerobes (*i.e*. those that are harmed by the presence of oxygen); aerotolerant anaerobes, (*i.e*. those that cannot use oxygen for growth, but tolerate its presence); and facultative anaerobes *(i.e.* those that can grow without oxygen, but will use oxygen if it is present).

The above criteria are important because they can inform the phylogenetic classification of bacterial strains. For instance, the bacterial species *Faecalicatena contorta* and *Anaerostipes hadrus,* have previously been classified as belonging to the genus *Eubacterium* [46, 53], based on these criteria in particular. The previous classification of these species as belonging to the *Eubacterium* genus is an indication of the phenotypic (and thus also genotypic) similarities between *Faecalicatena contorta, Anaerostipes hadrus* and *Eubacterium callandari.*

The compositions for use according to the invention may comprise a bacterial strain of the genus *Anaerostipes.* The examples demonstrate that bacteria of this genus are useful for treating or preventing diseases and conditions mediated by HDAC activity. The preferred bacterial strains are of the species *Anaerostipes hadrus.* The examples further demonstrate that bacteria of this genus are particularly useful for treating or preventing CNS disorders such as autism spectrum disorders. The mouse model used by the inventors for the assessment of the effects of the compositions of the invention on autism spectrum disorder are known in the art to be applicable for the assessment of the symptoms other central nervous system disorders including those disclosed herein.

An example of an *Anaerostipes* strain for use in the invention is a strain of the species *Anaerostipes hadrus.* The *Anaerostipes* are Gram-reaction-positive, anaerobes. *Anaerostipes hadrus* may be isolated from the human gut.

The genus *Anaerostipes* currently contains four known species; *Anaerostipes butyraticus, Anaerostipes caccae, Anaerostipes hadrus* and *Anaerostipes rhamnosivorans.*

In certain embodiments, the compositions comprise one or more bacterial strain(s) of the genus *Anaerostipes* and do not contain bacteria from any other bacterial species. In certain embodiments, the compositions comprise a single species of the genus *Anaerostipes* (e.g. one or more strain(s) of the species *Anaerostipes butyraticus, Anaerostipes caccae, Anaerostipes hadrus* or *Anaerostipes rhamnosivorans*) and do not contain any other bacterial strains or species. In certain embodiments, the compositions comprise a single strain of the genus *Anaerostipes* (e.g. a single strain of the species *Anaerostipes butyraticus, Anaerostipes caccae, Anaerostipes hadrus* or *Anaerostipes rhamnosivorans*) and do not contain any other bacterial strains or species.

*Anaerostipes* is a genus of bacteria in the class Clostridia. The scientific classification is as follows: bacteria (kingdom); Firmicutes (phylum); Clostridia (class); Clostridiales (order); Lachnospiraceae (family); *Anaerostipes* (genus). *Anaerostipes* are anaerobic, Gram-positive gut microbes.

The *Anaerostipes hadrus* strain deposited under DSM 3319 was tested for HDAC activity in the examples. A 16s rRNA gene sequence for this strain is provided in SEQ ID NO: 1. The strain was deposited with the DSMZ - German Collection of Microorganisms and Cell Cultures GmbH and is publically available. Another *Anaerostipes hadrus* strain that is publically available from the DSMZ is the strain DSM 108065.

An exemplary *Anaerostipes butyraticus* strain that can be used in the compositions of the invention is the one deposited as DSM 22094. This strain has the 16s rRNA sequence shown in SEQ ID NO: 3.

A suitable *Anaerostipes caccae* strain for use in the invention has been deposited as DSM 14662. This strain has the 16s rRNA sequence shown in SEQ ID NO: 4.

An *Anaerostipes rhamnosivorans* suitable for use in the invention has been deposited as DSM 26241. The 16s rRNA sequence of this strain can be found as SEQ ID NO: 16.

A preferred *Anaerostipes hadrus* strain is the strain deposited under accession number NCIMB 43457. The *Anaerostipes hadrus* bacterium deposited under accession number NCIMB 43457 was tested in the examples. A 16S rRNA sequence for NCIMB 43457 that was tested is provided in SEQ ID NO: 6. This strain was deposited with the international depositary authority NCIMB, Ltd. (Ferguson Building, Craibstone Estate, Bucksburn, Aberdeen, AB21 9YA, Scotland) by 4D Pharma Research Ltd. (Life Sciences Innovation Building, Aberdeen, AB25 2ZS, Scotland) on 9th August 2019 as *"Anaerostipes hadrus"* and was assigned accession number NCIMB 43457.

A preferred *Anaerostipes hadrus* strain is the strain deposited under accession number NCIMB 43526. The *Anaerostipes hadrus* bacterium deposited under accession number NCIMB 43526 was tested in the examples. A 16S rRNA sequence for NCIMB 43526 that was tested is provided in SEQ ID NO: 7. This strain was deposited with the international depositary authority NCIMB, Ltd. (Ferguson Building, Craibstone Estate, Bucksburn, Aberdeen, AB21 9YA, Scotland) by 4D Pharma Research Ltd. (Life Sciences Innovation Building, Aberdeen, AB25 2ZS, Scotland) on 3rd December 2019 as *"Anaerostipes hadrus F18-EA-P01"* and was assigned accession number NCIMB 43526.

A further preferred strain of the invention is the strain *A. caccae* strain ref. 1, which was also tested in the Examples. A 16s rRNA gene sequence for *A. caccae* strain ref. 1 is provided in SEQ ID NO: 15.

The invention also provides a bacterial strain having a 16s rRNA gene sequence that is at least 98%, 99%, 99.5% or 99.9% identical to SEQ ID NO: 15, preferably wherein the bacterial strain has the 16s rRNA gene sequence represented by SEQ ID NO: 15. Preferably such a bacterial strain is of the species *Anaerostipes caccae.* The invention also provides such a bacterial strain for use in therapy, in particular for the diseases described herein. The invention also provides a bacterial strain of the species *Anaerostipes caccae* (or a composition comprising a bacterial strain of the species *Anaerostipes caccae),* for use in therapy, in particular for the diseases described herein.

Bacterial strains that are biotypes of the deposited bacterial strains discussed above are also expected to be effective for treating or preventing diseases and conditions mediated HDAC activity. A biotype is a closely related strain that has the same or very similar physiological and biochemical characteristics.

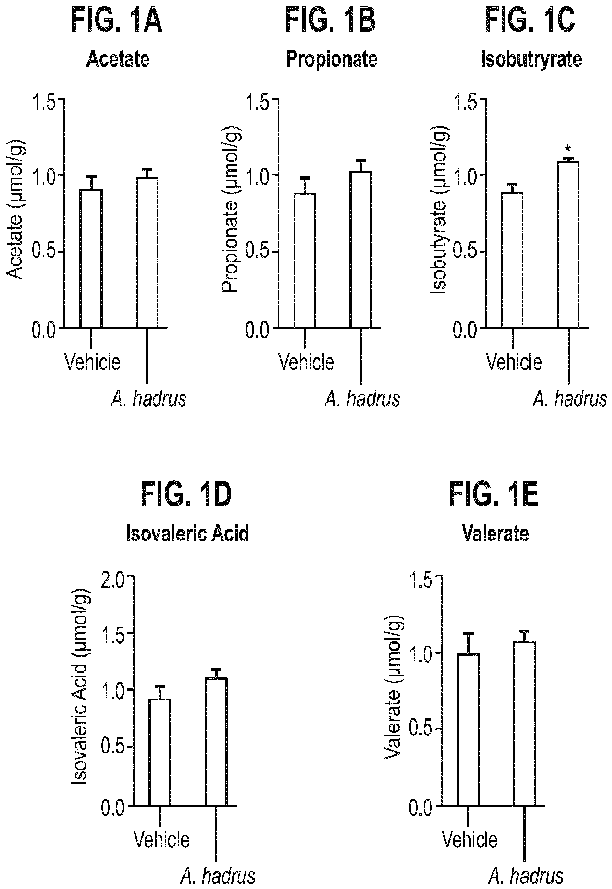

In some embodiments, bacterial strains useful in the invention may be identified by routinely profiling the production and consumption of metabolites by a bacterial strain. The inventors have found that the bacterial strain used in the Examples effect production of acetate, propionate, isobutyrate, isovalerate and valerate (Figure 1). Therefore, in some embodiments, the bacterial strain of the invention induce the production *in vivo* of one or more of the metabolites acetate, propionate, isobutyrate, isovalerate and valerate. Additionally, the bacterial strain of the invention itself produces one or more of acetate and butyrate.

Bacterial strains closely related to the strains tested in the examples are also expected to be effective for treating or preventing autism spectrum disorders and central nervous system disorders and conditions, in particular central nervous system disorders and conditions mediated by the microbiota-gut-brain axis. In certain embodiments, the bacterial strain may have a 16s rRNA sequence that is at least 95%, 96%, 97%, 98%, 99%, 99.5%, 99.9% or 100% identical to the 16s rRNA sequence of a bacterial strain of *Anaerostipes hadrus* (e.g. SEQ ID NO: 1, 2, 6 or 7). The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes butyraticus* (e.g. SEQ ID NO: 3). The bacterial strain may have a 16s rRNA sequence that is at least 95%, 96%, 97%, 98%, 99%, 99.5%, 99.9% or 100% identical to the 16s rRNA sequence of a bacterial strain of *Anaerostipes caccae* (e.g. SEQ ID NO: 4 or 15). In preferred embodiments, the bacterial strain may have a 16s rRNA sequence that is at least 95%, 96%, 97%, 98%, 99%, 99.5%, 99.9% or 100% identical to the 16s rRNA sequence of a bacterial strain of *Anaerostipes hadrus* (e.g. SEQ ID NO: 6).

In some embodiments, the bacterial strain may have a 16s rRNA sequence that is at least 95%, 96%, 97%, 98%, 99%, 99.5%, 99.9% or 100% identical to SEQ ID NO:1; a 16s rRNA sequence that is at least 95%, 96%, 97%, 98%, 99%, 99.5%, 99.9% or 100% identical to SEQ ID NO: 2; a 16s rRNA sequence that is at least 95%, 96%, 97%, 98%, 99%, 99.5%, 99.9% or 100% identical to SEQ ID NO: 3; a 16s rRNA sequence that is at least 95%, 96%, 97%, 98%, 99%, 99.5%, 99.9% or 100% identical to SEQ ID NO: 4 or a 16s rRNA sequence that is at least 95%, 96%, 97%, 98%, 99%, 99.5%, 99.9% or 100% identical to SEQ ID NO: 5 or a 16s rRNA sequence that is at least 95%, 96%, 97%, 98%, 99%, 99.5%, 99.9% or 100% identical to SEQ ID NO: 15 or a 16s rRNA sequence that is at least 95%, 96%, 97%, 98%, 99%, 99.5%, 99.9% or 100% identical to SEQ ID NO: 16. In preferred embodiments the bacterial strain may have a 16s rRNA sequence that is at least 95%, 96%, 97%, 98%, 99%, 99.5%, 99.9% or 100% identical to SEQ ID NO: 6 or 7. For example, the bacterial strain may have a 16s rRNA sequence that is at least 95%, 96%, 97%, 98%, 99%, 99.5%, 99.9% or 100% identical to SEQ ID NO: 6 or the bacterial strain has a 16s rRNA sequence that is SEQ ID NO: 6. The bacterial strain may have a 16s rRNA sequence that is at least 95%, 96%, 97%, 98%, 99%, 99.5%, 99.9% or 100% identical to SEQ ID NO: 7 or the bacterial strain has a 16s rRNA sequence that is SEQ ID NO: 7.

Bacterial strains that are biotypes of the bacteria strain deposited under accession number NCIMB 43457 are also expected to be effective for treating or preventing autism spectrum disorder and central nervous system disorders and conditions, in particular central nervous system disorders and conditions mediated by the microbiota-gut-brain axis. A biotype is a closely related strain that has the same or very similar physiological and biochemical characteristics. For example, a biotype of the bacterial strain deposited under accession number NCIMB 43457 may decrease the expression of TNF-α, decrease the expression of IL-1β and/or increase the expression of IL-6 when tested using the assay discussed in example 9.

Bacterial strains that are biotypes of the bacteria strain deposited under accession number NCIMB 43526 are also expected to be effective for treating or preventing autism spectrum disorder and central nervous system disorders and conditions, in particular central nervous system disorders and conditions mediated by the microbiota-gut-brain axis. A biotype is a closely related strain that has the same or very similar physiological and biochemical characteristics. For example, a biotype of the bacterial strain deposited under accession number NCIMB 43526 may decrease the expression decrease the expression of IL-1β TNFα, IL-6, of TNF-α using the assay discussed in example 3. Alternatively, a biotype of the bacterial strain deposited under accession number NCIMB 43526 may increase the production of acetate, propionate, isobutyrate, isovalerate and valerate using the assay discussed in example 2. Strains that are biotypes of a deposited bacterium as discussed above and that are suitable for use in the invention may be identified by sequencing other nucleotide sequences for a bacterium deposited under accession number DSM 3319, DSM 108065, DSM 22094, DSM 14662 or DSM 26241. For example, substantially the whole genome may be sequenced and a biotype strain for use in the invention may have at least 95%, 96%, 97%, 98%, 99%, 99 5% or 99.9% sequence identity across at least 80% of its whole genome (e.g. across at least 85%, 90%, 95% or 99%, or across its whole genome).

Strains that are biotypes of the bacteria strain deposited under accession number NCIMB 43457 or NCIMB 43526 are suitable for use in the invention may be identified by sequencing other nucleotide sequences for the bacteria strain deposited under accession number NCIMB 43457 or NCIMB 43526. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome). For example, in some embodiments, a biotype strain has at least 98% sequence identity across at least 98% of its genome or at least 99% sequence identity across 99% of its genome. Preferably, a biotype strain has at least 99.5% or at least 99.9% sequence identity across 100% of its genome.

Other suitable sequences for use in identifying biotype strains may include hsp60 or repetitive sequences such as BOX, ERIC, (GTG)₅, or REP [47]. Biotype strains may have sequences with at least 95%, 96%, 97%, 98%, 99%, 99.5% or 99.9% sequence identity to the corresponding sequence of a deposited bacterium. Biotype strains may have sequences with at least 95%, 96%, 97%, 98%, 99%, 99.5% or 99.9% sequence identity to the corresponding sequence of the bacterium deposited under accession number NCIMB 43457. In some embodiments, a biotype strain has a sequence with at least 95%, 96%, 97%, 98%, 99%, 99.5% or 99.9% sequence identity to the 16S rRNA sequence of SEQ ID NO: 6. Biotype strains may have sequences with at least 95%, 96%, 97%, 98%, 99%, 99.5% or 99.9% sequence identity to the corresponding sequence of the bacterium deposited under accession number NCIMB 43526. In some embodiments, a biotype strain has a sequence with at least 95%, 96%, 97%, 98%, 99%, 99.5% or 99.9% sequence identity to the 16S rRNA sequence of SEQ ID NO: 7.

Alternatively, strains that are biotypes of a deposited bacterium as discussed above and that are suitable for use in the invention may be identified by using the accession number DSM 3319, DSM 108065, DSM 22094, DSM 14662 or DSM 26241, and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Anaerostipes* strains.

Alternatively, strains that are biotypes of the bacteria strain deposited under accession number NCIMB 43457 or NCIMB 43526 and that are suitable for use in the invention may be identified by using the accession number NCIMB 43457 deposit and restriction fragment analysis and/or PCR analysis, for example by using fluorescent amplified fragment length polymorphism (FAFLP) and repetitive DNA element (rep)-PCR fingerprinting, or protein profiling, or partial 16S or 23s rDNA sequencing. In preferred embodiments, such techniques may be used to identify other *Anaerostipes* strains (e.g. *Anaerostipes butyraticus, Anaerostipes caccae, Anaerostipes hadrus* or *Anaerostipes rhamnosivorans* strains). In certain embodiments, strains that are biotypes of a bacterium deposited under accession number DSM 3319, DSM 108065, DSM 22094, DSM 14662, DSM 26241, NCIMB 43457 or NCIMB 43526 and that are suitable for use in the invention are strains that provide the same pattern as a bacterium deposited under accession number DSM 3319, DSM 108065, DSM 22094, DSM 14662 or DSM 26241, NCIMB 43457 or NCIMB 43526 when analysed by amplified ribosomal DNA restriction analysis (ARDRA), for example when using Sau3AI restriction enzyme (for exemplary methods and guidance see, for example, [48]. Alternatively, biotype strains are identified as strains that have the same carbohydrate fermentation patterns as a bacterium deposited under accession numberDSM 3319, DSM 108065, DSM 22094, DSM 14662, DSM 26241 , NCIMB 43457 or NCIMB 43526.

Other *Anaerostipes* strains that are useful in the compositions and methods of the invention, such as biotypes of a bacterium deposited under accession number DSM 3319, DSM 108065, DSM 22094, DSM 14662, DSM 26241, NCIMB 43457 or NCIMB 43526, may be identified using any appropriate method or strategy, including the assays described in the examples. For instance, strains for use in the invention may be identified by administering the bacteria to the HDAC activity assay and assessing HDAC activity inhibition. Bacterial strains with comparable HDAC inhibitory activity to DSM 3319, NCIMB 43457 or NCIMB 43526 are suitable for use in the invention. In particular, bacterial strains that have similar growth patterns, metabolic type and/or surface antigens to a bacterium deposited under accession number DSM 3319, NCIMB 43457 or NCIMB 43526 may be useful in the invention, A useful strain will have comparable HDAC inhibitory activity and/or comparable effects of the reduction of hyperactivity in the assays used in the Examples to the DSM 3319 strain, which may be identified by using the culturing and administration protocols described in the Examples. For example, a suitable *Anaerostipes* strain can inhibit the activity of HDAC 1 by at least 40% (e.g. at least 50%, at least 60%, at least 70%, at least 80%, at least 90% or 100%) when assayed using a fluoregenic assay, for example the assay discussed in example 6. In addition, or alternatively, a suitable *Anaerostipes* strain can inhibit the activity of HDAC 2 by at least 60% (e.g. at least 70%, at least 80%, at least 90% or 100%) when assayed using a fluoregenic assay, for example the assay discussed in example 6. In addition, or alternatively, a suitable *Anaerostipes* strain can inhibit the activity of HDAC 3 by at least 50% (e.g. at least 60%, at least 70%, at least 80%, at least 90% or 100%) when assayed using a fluoregenic assay, for example the assay discussed in example 6.

A particularly preferred strain of the invention is the *Anaerostipes hadrus* strain deposited under accession number DSM 3319. This is the exemplary train tested in the examples and shown to be effective for reducing HDAC activity and reducing hyperactivity. Therefore, the invention provides a cell, such as an isolated cell, of the *Anaerostipes hadrus* strain deposited under accession number DSM 3319, or a derivative thereof, for use in therapy.

A particularly preferred strain of the invention is the *Anaerostipes hadrus* strain deposited under accession number NCIMB 43457. This is the exemplary train tested in the examples and shown to be effective for reducing HDAC activity and reducing hyperactivity. Therefore, the invention provides a cell, such as an isolated cell, of the *Anaerostipes hadrus* strain deposited under accession number NCIMB 43457, or a derivative thereof, for use in therapy.

A particularly preferred strain of the invention is the *Anaerostipes hadrus* strain deposited under accession number NCIMB 43526. This is the exemplary train tested in the examples and shown to be effective for reducing HDAC activity and reducing hyperactivity. Therefore, the invention provides a cell, such as an isolated cell, of the *Anaerostipes hadrus* strain deposited under accession number NCIMB 43526, or a derivative thereof, for use in therapy. In certain embodiments, strains that are biotypes of the bacteria strain deposited under accession numbers DSM 3319, DSM 108065, DSM 22094, DSM 14662, DSM 26241, NCIMB 43457 or NCIMB 43526 NCIMB 43457 and that are suitable for use in the invention are strains that provide the same pattern as the bacteria deposited under any one of the accession number DSM 3319, DSM 108065, DSM 22094, DSM 14662, DSM 26241, NCIMB 43457 or NCIMB 43526 when analysed by amplified ribosomal DNA restriction analysis (ARDRA), for example when using Sau3AI restriction enzyme (for exemplary methods and guidance see, for example, [49]).

Other *Anaerostipes* strains (e.g. *Anaerostipes hadrus, Anaerostipes butyraticus, Anaerostipes caccae* or *Anaerostipes rhamnosivorans* strains) that are useful in the compositions and methods of the invention, such as biotypes of any one of the bacteria deposited under any one of the accession numbers NCIMB 43457, DSM 3319, DSM 108065, DSM 22094, DSM 14662 or DSM 26241, may be identified using any appropriate method or strategy, including the assays described in the examples. For instance, strains for use in the invention may be identified by culturing in anaerobic YCFA and/or administering the bacteria to an autism spectrum disorder mouse model and then assessing cytokine levels. In particular, bacterial strains that have similar growth patterns, metabolic type and/or surface antigens to the bacteria deposited under any one of the accession numbers NCIMB 43457, DSM 3319, DSM 108065, DSM 22094, DSM 14662 or DSM 26241 may be useful in the invention. A useful strain will have comparable immune modulatory activity to the NCIMB 43457, DSM 3319, DSM 108065, DSM 22094, DSM 14662 or DSM 26241 strain. In particular, a biotype strain will elicit comparable effects on the autism spectrum disorder models to the effects shown in the examples, which may be identified by using the culturing and administration protocols described in the examples.

A particularly preferred strain of the invention is the *Anaerostipes hadrus* strain deposited under accession number NCIMB 43457. This is the exemplary *Anaerostipes hadrus* strain tested in the examples and shown to be effective for treating disease, for example for treating autism. Therefore, the invention provides a cell, such as an isolated cell, of the *Anaerostipes hadrus* strain deposited under accession number NCIMB 43457, or a derivative thereof. The invention also provides a composition comprising a cell of the *Anaerostipes hadrus* strain deposited under accession number NCIMB 43457, or a derivative thereof. The invention also provides a biologically pure culture of the *Anaerostipes hadrus* strain deposited under accession number NCIMB 43457. The invention also provides a cell of the *Anaerostipes hadrus* strain deposited under accession number NCIMB 43457, or a derivative thereof, for use in therapy, in particular for the diseases described herein. In particular, in the treatment of for use in treating or preventing a central nervous system disorder or condition, such as autism.

A particularly preferred strain of the invention is the *Anaerostipes hadrus* strain deposited under accession number NCIMB 43526. This is the exemplary *Anaerostipes hadrus* strain tested in the examples and shown to be effective for treating disease, for example for treating autism. Therefore, the invention provides a cell, such as an isolated cell, of the *Anaerostipes hadrus* strain deposited under accession number NCIMB 43526, or a derivative thereof. The invention also provides a composition comprising a cell of the *Anaerostipes hadrus* strain deposited under accession number NCIMB 43526, or a derivative thereof. The invention also provides a biologically pure culture of the *Anaerostipes hadrus* strain deposited under accession number NCIMB 43526. The invention also provides a cell of the *Anaerostipes hadrus* strain deposited under accession number NCIMB 43526, or a derivative thereof, for use in therapy, in particular for the diseases described herein. In particular, in the treatment of for use in treating or preventing a central nervous system disorder or condition, such as autism.

A derivative of the strain deposited under accession number DSM 3319 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable HDAC inhibitory activity to the original DSM 3319 strain. In particular, a derivative strain will elicit comparable effects on HDAC inhibitory activity or hyperactivity models shown in the Examples, which may be identified by using the culturing and administration protocols described in the Examples, A derivative of the DSM 3319 strain will generally be a biotype of the DSM 3319 strain. A derivative of the strain deposited under accession number NCIMB 43457 or NCIMB 43526 may be a daughter strain (progeny) or a strain cultured (subcloned) from the original. A derivative of a strain of the invention may be modified, for example at the genetic level, without ablating the biological activity, In particular, a derivative strain of the invention is therapeutically active. A derivative strain will have comparable immune modulatory activity to the deposited strain. In particular, a derivative strain will elicit comparable effects on the autism spectrum disorder models to the effects shown in the examples, which may be identified by using the culturing and administration protocols described in the examples, A derivative of the NCIMB 43457 or NCIMB 43526 strain will generally be a biotype of the NCIMB 43457 or NCIMB 43526 strain.

The bacterial strain may also be a strain that has the same safety and therapeutic efficacy characteristics as the strains deposited under any one of accession numbers NCIMB 43457, DSM 3319, DSM 108065, DSM 22094, DSM 14662 or DSM 26241, and such cells are encompassed by the invention.

The compositions of the invention may comprise a bacterial strain of the *Eubacterium* or *Faecalicatena* genera. The examples also demonstrate that bacteria of this genus are useful in therapy, particularly for treating or preventing autism spectrum disorders and central nervous system disorders mediated by the microbiota-gut-brain axis. The mouse model experiments used in this application for the assessment of the symptoms of autism spectrum disorders are known in the art to be applicable for the assessment of symptoms of other central nervous system disorders including those listed above.

The invention therefore also provides a composition comprising a bacterial strain of the *Eubacterium* or *Faecalicatena* genera for use in therapy, for example, for use in treating or preventing a central nervous system disorder or condition, in particular a central nervous system disorder or condition mediated by the microbiota-gut-brain axis. In certain embodiments, the compositions of the invention comprise strains of the *Eubacterium* or *Faecalicatena* genera and do not contain any other bacterial genera. In certain embodiments, the compositions of the invention comprise a single strain of the *Eubacterium* or *Faecalicatena* genera and do not contain any other bacterial strains, genera or species.

Examples of *Eubacterium* strains for use in the invention include those belonging to the species *Eubacterium callanderi* (*E. callanderi*), *E. limosum, E. eligens, E. hallii, E. rectale, E. barkeri, E. biforme, E. cylindroides, E. dolichum, E. moniliforme* and *E. ventriosum.* Preferably, *Eubacterium* strains for use in the invention belong to the species *E. callanderi, E. limosum, E. eligens, E. hallii* or *E. rectale.* More preferred species for use in the invention are either *E. callanderi* or *E*. *limosum.* The most preferred species for use in the invention is *E. callanderi. E. callanderi* is also sometimes spelled as *E. callenderi* in the literature (see, e.g. [50]), although the same species is being referred to. Bacteria of *Eubacterium* genus have been described as Gram-positive, nonsporulating, strictly anaerobic bacilli.

Examples of *Faecalicatena* strains for use in the invention include those belonging to the species *Faecalicatena fissicatena (F. fissicatena*), *F. contorta* and *F. orotica.* Preferably, *Faecalicatena* strains for use in the invention belong to the species *F. fissicatena* or *F. contorta.* Bacteria of *Faecalicatena* genus have been described as Gram-stain-positive, obligately anaerobic, spore-forming or non-spore-forming, non-motile, non-pigmented rods found in chains or pairs.

The *Faecalicatena* genus was proposed in 2017, when the former species *Eubacterium contortum, Eubacterium fissicatena* and *Clostridium contortum* were reclassified to be part of the new genus, and renamed as *Faecalicatena contorta, Faecalicatena fissicatena* and *Faecalicatena orotica* respectively [51]. *Eubacterium contortum* and *Eubacterium fissicatena* (now *Faecalicatena contorta* and *Faecalicatena fissicatena*) were originally classified as part of the *Eubacterium* genus on the basis of common phenotypic characteristics, including being Gram-positive, obligatory anaerobic, rod-shaped, non-spore-forming, and producing acetic acid, CO₂ and H₂ during glucose fermentation [52], [53]. Hence, there are close phenotypic (and thus also genotypic) similarities between bacterial strains of the *Eubacterium* and *Faecalicatena* genera, as used in compositions of the invention.

The bacterial strain of *E*. *callanderi* deposited under accession number NCIMB 43455 was tested in the Examples. A 16s rRNA gene sequence for this strain is provided in SEQ ID NO: 8. The strain was deposited with the international depositary authority NCIMB, Ltd. (Ferguson Building, Aberdeen, AB21 9YA, Scotland) by 4D Pharma Research Ltd. (Life Sciences Innovation Building, Aberdeen, AB25 2ZS, Scotland) on 9th August 2019 and was assigned accession number NCIMB 43455.

The invention therefore also provides a cell, such as an isolated cell, of bacterial strain deposited under accession number NCIMB 43455, or a derivative thereof. The invention also provides a composition comprising a cell of the bacterial strain deposited under accession number NCIMB 43455, or a derivative thereof. The invention also provides a biologically pure culture of the bacterial strain deposited under accession number NCIMB 43455. The invention also provides a cell of the bacterial strain deposited under accession number NCIMB 43455, or a derivative thereof, for use in therapy, in particular for the diseases described herein.

The invention also provides a bacterial strain having a 16s rRNA gene sequence that is at least 98%, 99%, 99.5% or 99.9% identical to SEQ ID NO: 8, preferably wherein the bacterial strain has the 16s rRNA gene sequence represented by SEQ ID NO: 8. Preferably such a bacterial strain is of the species *Eubacterium callanderi.* Alternatively, such a bacterial strain is of the species *Eubacterium limosum.* The invention also provides such a bacterial strain (or a composition comprising such a bacterial strain) for use in therapy, in particular for the diseases described herein. The invention also provides a bacterial strain of the species *Eubacterium callanderi* (or a composition comprising a bacterial strain of the species *Eubacterium callanderi*), for use in therapy, in particular for the diseases described herein. The invention also provides a bacterial strain of the species *Eubacterium limosum* (or a composition comprising a bacterial strain of the species *Eubacterium limosum*), for use in therapy, in particular for the diseases described herein.

A further preferred bacterial strain of the invention is the strain *E. eligens* strain ref. 1, which was also tested in the Examples. A 16s rRNA gene sequence for *E*. *eligens* strain ref. 1 is provided in SEQ ID NO: 9.

The invention also provides a bacterial strain having a 16s rRNA gene sequence that is at least 98%, 99%, 99.5% or 99.9% identical to SEQ ID NO: 9, preferably wherein the bacterial strain has the 16s rRNA gene sequence represented by SEQ ID NO: 9. Preferably such a bacterial strain is of the species *Eubacterium eligens.* The invention also provides such a bacterial strain for use in therapy, in particular for the diseases described herein. The invention also provides a bacterial strain of the species *Eubacterium eligens* (or a composition comprising a bacterial strain of the species *Eubacterium eligens*), for use in therapy, in particular for the diseases described herein.

A further preferred bacterial strain of the invention is the strain *E. rectale* strain ref. 2, which was also tested in the Examples. A 16s rRNA gene sequence for *E. rectale* strain ref. 2 is provided in SEQ ID NO: 10.

The invention also provides a bacterial strain having a 16s rRNA gene sequence that is at least 98%, 99%, 99.5% or 99.9% identical to SEQ ID NO: 10, preferably wherein the bacterial strain has the 16s rRNA gene sequence represented by SEQ ID NO: 10. Preferably such a bacterial strain is of the species *Eubacterium rectale.* The invention also provides such a bacterial strain for use in therapy, in particular for the diseases described herein. The invention also provides a bacterial strain of the species *Eubacterium rectale* (or a composition comprising a bacterial strain of the species *Eubacterium rectale*), for use in therapy, in particular for the diseases described herein.

A further preferred strain of the invention is the strain *E. rectale* strain ref. 1, which was also tested in the Examples. A 16s rRNA gene sequence for *E. rectale* strain ref. 1 is provided in SEQ ID NO: 11.

The invention also provides a bacterial strain having a 16s rRNA gene sequence that is at least 98%, 99%, 99.5% or 99.9% identical to SEQ ID NO: 11, preferably wherein the bacterial strain has the 16s rRNA gene sequence represented by SEQ ID NO: 11. Preferably such a bacterial strain is of the species *Eubacterium rectale.* The invention also provides such a bacterial strain for use in therapy, in particular for the diseases described herein.

A further preferred strain of the invention is the strain *E*. *hallii* strain ref. 1, which was also tested in the Examples. A 16s rRNA gene sequence for *E. hallii* strain ref. 1 is provided in SEQ ID NO: 14.

The invention also provides a bacterial strain having a 16s rRNA gene sequence that is at least 98%, 99%, 99.5% or 99.9% identical to SEQ ID NO: 14, preferably wherein the bacterial strain has the 16s rRNA gene sequence represented by SEQ ID NO: 14. Preferably such a bacterial strain is of the species *Eubacterium hallii.* The invention also provides such a bacterial strain for use in therapy, in particular for the diseases described herein. The invention also provides a bacterial strain of the species *Eubacterium hallii* (or a composition comprising a bacterial strain of the species *Eubacterium hallii*) for use in therapy, in particular for the diseases described herein.

A further preferred strain of the invention is the strain *F fissicatena* strain ref. 1, which was also tested in the Examples. A 16s rRNA gene sequence for *F. fissicatena* strain ref. 1 is provided in SEQ ID NO: 12.

The invention also provides a bacterial strain having a 16s rRNA gene sequence that is at least 98%, 99%, 99.5% or 99.9% identical to SEQ ID NO: 12, preferably wherein the bacterial strain has the 16s rRNA gene sequence represented by SEQ ID NO: 12. Preferably such a bacterial strain is of the species *Faecalicatena fissicatena.* The invention also provides such a bacterial strain for use in therapy, in particular for the diseases described herein. The invention also provides a bacterial strain of the species *Faecalicatena fissicatena* (or a composition comprising a bacterial strain of the species *Faecalicatena fissicatena*), for use in therapy, in particular for the diseases described herein.

A further preferred strain of the invention is the strain *F. contorta* strain ref. 1, which was also tested in the Examples. A 16s rRNA gene sequence for this strain is provided in SEQ ID NO: 13. This strain was deposited with the international depositary authority NCIMB, Ltd. (Ferguson Building, Aberdeen, AB21 9YA, Scotland) by 4D Pharma Research Ltd. (Life Sciences Innovation Building, Aberdeen, AB25 2ZS, Scotland) on 15th November 2016 and was assigned accession number NCIMB 42689.

The invention provides a cell, such as an isolated cell, of bacterial strain deposited under accession number NCIMB 42689, or a derivative thereof. The invention also provides a composition comprising a cell of the bacterial strain deposited under accession number NCIMB 42689, or a derivative thereof The invention also provides a biologically pure culture of the bacterial strain deposited under accession number NCIMB 42689. The invention also provides a cell of the bacterial strain deposited under accession number NCIMB 42689, or a derivative thereof, for use in therapy, in particular for the diseases described herein.

The invention also provides a bacterial strain having a 16s rRNA gene sequence that is at least 98%, 99%, 99.5% or 99.9% identical to SEQ ID NO: 13, preferably wherein the bacterial strain has the 16s rRNA gene sequence represented by SEQ ID NO: 13. Preferably such a bacterial strain is of the species *Faecalicatena contorta.* The invention also provides such a bacterial strain for use in therapy, in particular for the diseases described herein. The invention also provides a bacterial strain of the species *Faecalicatena contorta* (or a composition comprising a bacterial strain of the species *Faecalicatena contorta*), for use in therapy, in particular for the diseases described herein.

Bacterial strains closely related to the strain tested in the examples are also expected to be effective for therapy, including for treating or preventing autism spectrum disorders and central nervous system disorders and conditions, in particular central nervous system disorders and conditions mediated by the microbiota-gut-brain axis. Bacterial strains for use in the invention preferably have a 16s rRNA gene sequence that is at least 95%, 96%, 97%, 98%, 99%, 99.5% or 99.9% identical any of SEQ ID NO: 8, 9, 10, 11, 12, 13 or 14.

In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium.* In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium callanderi* or *Eubacterium limosum* (preferably *Eubacterium callanderi*). Preferably, the bacterial strain for use in the invention has a 16s rRNA gene sequence that is at least 95%, 96%, 97%, 98%, 99%, 99.5% or 99.9% identical to SEQ ID NO: 8. Preferably, the bacterial strain for use in the invention has the 16s rRNA gene sequence represented by SEQ ID NO: 8. Such bacterial strains (having a 16s rRNA gene sequence with identity to SEQ ID NO: 8, or having the 16s rRNA gene sequence represented by SEQ ID NO: 8), are especially preferred for use in the invention. In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium eligens.* Preferably, the bacterial strain for use in the invention has a 16s rRNA gene sequence that is at least 95%, 96%, 97%, 98%, 99%, 99.5% or 99.9% identical to SEQ ID NO: 9. Preferably, the bacterial strain for use in the invention has the 16s rRNA gene sequence represented by SEQ ID NO: 9. In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium hallii.* Preferably, the bacterial strain for use in the invention has a 16s rRNA gene sequence that is at least 95%, 96%, 97%, 98%, 99%, 99.5% or 99.9% identical to SEQ ID NO: 10. Preferably, the bacterial strain for use in the invention has the 16s rRNA gene sequence represented by SEQ ID NO: 10. In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium rectale.* Preferably, the bacterial strain for use in the invention has a 16s rRNA gene sequence that is at least 95%, 96%, 97%, 98%, 99%, 99.5% or 99.9% identical to SEQ ID NO: 10. Preferably, the bacterial strain for use in the invention has the 16s rRNA gene sequence represented by SEQ ID NO: 10. Preferably, the bacterial strain for use in the invention has a 16s rRNA gene sequence that is at least 95%, 96%, 97%, 98%, 99%, 99.5% or 99.9% identical to SEQ ID NO: 11. Preferably, the bacterial strain for use in the invention has the 16s rRNA gene sequence represented by SEQ ID NO: 11.

In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Faecalicatena.* In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Faecalicatena fissicatena.* Preferably, the bacterial strain for use in the invention has a 16s rRNA gene sequence that is at least 95%, 96%, 97%, 98%, 99%, 99.5% or 99.9% identical to SEQ ID NO: 12. Preferably, the bacterial strain for use in the invention has the 16s rRNA gene sequence represented by SEQ ID NO: 12. In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Faecalicatena contorta.* Preferably, the bacterial strain for use in the invention has a 16s rRNA gene sequence that is at least 95%, 96%, 97%, 98%, 99%, 99.5% or 99.9% identical to SEQ ID NO: 13. Preferably, the bacterial strain for use in the invention has the 16s rRNA gene sequence represented by SEQ ID NO: 13.

In certain embodiments, the bacterial strain for use in the invention has a 16s rRNA gene sequence that is at least 95%, 96%, 97%, 98%, 99%, 99.5% or 99.9% identical to the 16s rRNA gene sequence of a bacterial strain of *Eubacterium hallii.* Preferably, the bacterial strain for use in the invention has a 16s rRNA gene sequence that is at least 95%, 96%, 97%, 98%, 99%, 99.5% or 99.9% identical to SEQ ID NO: 14. Preferably, the bacterial strain for use in the invention has the 16s rRNA gene sequence represented by SEQ ID NO: 14.

Preferably, the bacterial strain has a 16s rRNA gene sequence having at least 98.65% sequence similarity to SEQ ID NO: 8, 9, 10, 11, 12, 13 or 14. Preferably, the bacterial strain has a 16s rRNA gene sequence having at least 98.65% sequence similarity to SEQ ID NO: 8. Pairwise similarities between 16S rRNA gene sequences can be calculated based on robust global sequence alignment algorithms such as the EzTaxon server described in [54].

Bacterial strains that are biotypes of the bacterium deposited under accession number NCIMB 43455 are also expected to be effective for use in therapy, including for treating or preventing autism spectrum disorder and central nervous system disorders and conditions, in particular central nervous system disorders and conditions mediated by the microbiota-gut-brain axis. Bacterial strains that are biotypes of the bacterium deposited under accession number NCIMB 42689 are also expected to be effective for treating or preventing autism spectrum disorder and central nervous system disorders and conditions, in particular central nervous system disorders and conditions mediated by the microbiota-gut-brain axis. A biotype is a closely related strain that has the same or very similar physiological and biochemical characteristics.

Strains that are biotypes of the bacterium deposited under accession number NCIMB 43455 or NCIMB 42689 and that are suitable for use in the invention may be identified by sequencing other nucleotide sequences for the bacterium deposited under accession number NCIMB 43455 or NCIMB 42689. For example, substantially the whole genome may be sequenced and a biotype strain for use in the invention may have at least 95%, 96%, 97%, 98%, 99%, 99.5% or 99.9% sequence identity across at least 80% of its whole genome (e.g. across at least 85%, 90%, 95% or 99%, or across its whole genome), to either of the bacterial strains deposited under accession number NCIMB 43455 or NCIMB 42689, preferably NCIMB 43455. For example, in some embodiments, a biotype strain has at least 98% sequence identity across at least 98% of its genome or at least 99% sequence identity across 99% of its genome, to either of the bacterial strains deposited under accession number NCIMB 43455 or NCIMB 42689, preferably NCIMB 43455.

Other suitable sequences for use in identifying biotype strains may include hsp60 or repetitive sequences such as BOX, ERIC, (GTG)₅, or REP or [55]. Biotype strains may have sequences with at least 95%, 96%, 97%, 98%, 99%, 99.5% or 99.9% sequence identity to the corresponding sequence of the bacterium deposited under accession number NCIMB 43455 or NCIMB 42689.

In some, especially preferred, embodiments, a biotype strain has a sequence with at least 95%, 96%, 97%, 98%, 99%, 99.5% or 99.9% sequence identity to the corresponding sequence of the strain deposited as NCIMB 43455 and comprises a 16s rRNA gene sequence that is at least 99% identical (e.g. at least 99.5% or at least 99.9% identical) to SEQ ID NO: 8. In some embodiments, a biotype strain has a sequence with at least 95%, 96%, 97%, 98%, 99%, 99.5% or 99.9% sequence identity to the corresponding sequence of strain deposited as NCIMB 43455 and has the 16s rRNA gene sequence of SEQ ID NO: 8.

In other embodiments, a biotype strain has a sequence with at least 95%, 96%, 97%, 98%, 99%, 99.5% or 99.9% sequence identity to the corresponding sequence of strain *F*. *contorta* strain ref. 1 deposited as NCIMB 42689 and comprises a 16s rRNA gene sequence that is at least 99% identical (e.g. at least 99.5% or at least 99.9% identical) to SEQ ID NO: 13. In some embodiments, a biotype strain has a sequence with at least 95%, 96%, 97%, 98%, 99%, 99.5% or 99.9% sequence identity to the corresponding sequence of strain *F. contorta* strain ref. 1 deposited as NCIMB 42689 and has the 16s rRNA gene sequence of SEQ ID NO: 13.

In other embodiments, a biotype strain has a sequence with at least 95%, 96%, 97%, 98%, 99%, 99.5% or 99.9% sequence identity to the corresponding sequence of strain *E. hallii* strain ref. 1 and comprises a 16s rRNA gene sequence that is at least 99% identical (e.g. at least 99.5% or at least 99.9% identical) to SEQ ID NO: 14. In some embodiments, a biotype strain has a sequence with at least 95%, 96%, 97%, 98%, 99%, 99.5% or 99.9% sequence identity to the corresponding sequence of strain *E. hallii* strain ref. 1 and has the 16s rRNA gene sequence of SEQ ID NO: 14.

Alternatively, strains that are biotypes of a bacterium deposited under accession number NCIMB NCIMB 43455 or NCIMB 42689 and that are suitable for use in the invention may be identified by using the accession number NCIMB 43455 or NCIMB 42689 deposit, and restriction fragment analysis and/or PCR analysis, for example by using fluorescent amplified fragment length polymorphism (FAFLP) and repetitive DNA element (rep)-PCR fingerprinting, or protein profiling, or partial 16S or 23S rDNA sequencing. In preferred embodiments, such techniques may be used to identify other *Eubacterium* or *Faecalicatena* strains.

In certain embodiments, strains that are biotypes of a bacterium deposited under accession number NCIMB 43455 or NCIMB 42689 and that are suitable for use in the invention are strains that provide the same pattern as a bacterium deposited under accession number NCIMB 43455 or NCIMB 42689 when analysed by amplified ribosomal DNA restriction analysis (ARDRA), for example when using Sau3AI restriction enzyme (for exemplary methods and guidance see, for example, [56]). Alternatively, biotype strains are identified as strains that have the same carbohydrate fermentation patterns as a bacterium deposited under accession number NCIMB 43455 or NCIMB 42689.

Other *Eubacterium* or *Faecalicatena* strains that are useful in the compositions and methods of the invention, such as biotypes of the bacterium deposited under accession number NCIMB 43455 or NCIMB 42689, may be identified using any appropriate method or strategy, including the assays described in the examples. For instance, strains for use in the invention may be identified by culturing in anaerobic YCFA and/or administering the bacteria to an autism spectrum disorder mouse model and then assessing cytokine levels. In particular, bacterial strains that have similar growth patterns, metabolic type and/or surface antigens to the bacterium deposited under accession number NCIMB 43455 or NCIMB 42689 may be useful in the invention A useful strain will have comparable immune modulatory activity to the NCIMB 43455 or NCIMB 42689 strain. In particular, a biotype strain will elicit comparable effects on the autism spectrum disorder models to the effects shown in the Examples.

A derivative of the strain deposited under accession number NCIMB 43455 or NCIMB 42689 may be a daughter strain (progeny) or a strain cultured (subcloned) from the original. A derivative of the strain deposited under accession number NCIMB 43455 or NCIMB 42689 may be a daughter strain (progeny) or a strain cultured (subcloned) from the original. A derivative of a strain of the invention may be modified, for example at the genetic level, without ablating the biological activity. In particular, a derivative strain of the invention is therapeutically active. A derivative strain will have comparable immune modulatory activity to the original NCIMB 43455 or NCIMB 42689 strain. In particular, a derivative strain will elicit comparable effects on the central nervous system disorder or condition models and comparable effects on cytokine levels to the effects shown in the Examples, which may be identified by using the culturing and administration protocols described in the Examples. A derivative of the NCIMB 43455 or NCIMB 42689 strain will generally be a biotype of the NCIMB 43455 or NCIMB 42689 strain.

References to cells deposited under accession number NCIMB 43455 or NCIMB 42689 encompass any cells that have the same safety and therapeutic efficacy characteristics as the strains deposited under accession number NCIMB 43455 or NCIMB 42689, and such cells are encompassed by the invention.

In preferred embodiments, the bacterial strains in the compositions of the invention are viable. Such viable bacterial strains may be capable of partially or totally colonising the intestine.

Compositions of the invention comprise therapeutically effective amounts of the bacterial strain or strains. Likewise, the bacterial strain or strains for use according to the invention are used at therapeutically effective amounts.

Preferably, the compositions disclosed herein are to be administered to the gastrointestinal tract in order to enable delivery to and / or partial or total colonisation of the intestine with the bacterial strain of the invention. In other words, the bacteria may have colonised some or all of the gastrointestinal tract and / or such colonisation may be transient or permanent.

More specifically, in some embodiments, the "total colonisation of the intestine" means that bacteria have colonised all parts of the intestine (i.e. the small intestine, large intestine and rectum). Additionally or alternatively, the term "total colonisation" means that the bacteria engraft permanently in the some or all parts of the intestine.

In some embodiments, "partial colonisation of the intestine" means that bacteria have colonised some but not all parts of the intestine. Additionally or alternatively, the term "partial colonisation" means that the bacteria engraft transiently in some or all parts of the intestine.

The transience of engraftment can be determined by assessing (e.g. in a fecal sample) the abundance of the bacterial strain of the invention periodically (e.g. daily) following the end of a dosing interval to determine the washout period, i.e. the period between conclusion of the dosing interval and there being no detectable levels of the bacterial strain of the invention present. In embodiments of the invention, the washout period is 14 days or less, 12 days or less, 10 days or less, 7 days or less, 4 days or less, 3 days or less, 2 days or less or 1 day or less.

In embodiments of the invention, the bacteria of the present invention engraft transiently in the large intestine.

In certain embodiments, the bacterial strain for use in the invention is resistant to one of more of tetracycline, bacitracin, amoxicillin, ampicillin, arbekacin and dibekacin, azlocillin, bacampicillin, carbenicillin, ceftobiprole, clarithromycin, doripenem, erythromycin, fusidic acid, gentamicin, grepafloxacin, imipenem, josamycin, meropenem, meziocillin, piperacillin, rifampin, rifaximin, rokitamycin, rosaramicin, roxithromycin, spiramycin, streptomycin, sulfamethoxazole/trimethoprim, telithromycin, ticarcillin, ticarcillin/clavulanate, tosufloxacin, trimethoprim and virginiamycin. In certain embodiments, the bacterial strain for use in the invention is susceptible to Quinopristin-dalfopristin. In preferred embodiments, the bacterial strain for use in the invention is resistant to tetracycline and/or bacitracin.

In certain embodiments, the bacterial strain for use in the invention is resistant to β-lactam antibiotics. In certain embodiments, the bacterial strain for use in the invention is resistant to vancomycin. In certain embodiments, the bacterial strain for use in the invention is resistant to ampicillin.

In preferred embodiments, the bacterial strain for use in the invention is naturally-occurring. For example, the bacterial strain has been isolated from the mammalian digestive tract.

In preferred embodiments, the bacterial strain for use in the invention has not been not genetically engineered. For example, the bacterial strain has not been transformed with recombinant DNA.

In some embodiments, the composition for use in the invention does not comprise both of the bacterial strains: *Anaerostipes caccae* DSM 14662 and *Anaerostipes hadrus* DSM 3319 / ATCC 29173.

In some embodiments, the composition for use in the invention comprises fewer than 40 different bacterial strains. In some embodiments, the composition for use in the invention comprises fewer than 30 different bacterial strains. In some embodiments, the composition for use in the invention comprises fewer than 20 different bacterial strains. In some embodiments, the composition for use in the invention comprises fewer than 10 different bacterial strains.

### Therapeutic uses

As demonstrated in the examples, the bacterial compositions of the invention are effective for reducing the HDAC activity. In particular, treatment with compositions of the invention achieves a reduction in Class 1 HDAC activity. In particular, treatment with the compositions of the invention achieves a reduction in HDAC2 activity. The compositions of the invention also show clinical improvements in animal models of hyperactivity. Therefore, the compositions of the invention may be useful for treating or preventing diseases or conditions mediated by HDAC activity. A condition may be a symptom of a disease. In particular, the compositions of the invention may be useful for reducing or preventing diseases or conditions mediated by elevated levels of HDAC activity. In particular, the compositions of the invention may be useful for reducing or preventing diseases or conditions mediated by elevated levels of Class I HDAC activity. In particular, the compositions of the invention may be useful for reducing or preventing diseases or conditions mediated by elevated levels of HDAC2 activity.

Histone deacetylases are a class of enzymes that remove acetyl groups from protein targets. The most abundant HDAC target are histones, but HDACs are known to deacetylate lysine residues of non-histone protein targets to temporally regulate protein activity. As such, HDACs are sometimes referred to as lysine deacetylases. There are currently 13 known HDACs which are categorised into four main classes class 1 (HDACs 1, 2, 3 and 8), class IIa (HDACs 4,5,7 and 9) and class IIb (HDACs 6 and 10), Class III (sirt1-sirt7) and class IV (HDAC 11) [7]. Each class generally has a different tissue expression pattern and subcellular localisation.

Protein acetylation/deacetylation is generally used a mechanism of post-translational control of protein activity Histone acetylation/deacetylation is a well-established mechanism of transcriptional regulation. Genetic regulation is caused by histone deacetylase-mediated cleavage of an acetyl group from a ε-N-acetyl of a lysine amino acid in a histone tail. Removal of the acetyl group restores positive charge to the histone tail, leading to more favourable binding to the negative charged phosphodiester DNA backbone. Improved binding leads to tighter chromosome compaction and an overall reduction in gene expression at the site of histone deacetylation.

Histone deacetylase activity has been implicated in a wide array of diseases and conditions. Inhibition of histone deacetylase activity can be used to alleviate or ameliorate these diseases or conditions. Pan-inhibitors of histone deacetylases may be useful in the treatment or prevention of HDAC-mediated diseases. Isoform specific HDAC inhibitors may be useful in the treatment or prevention of diseases mediated by specific HDAC isoform activity.

Inhibition of HDAC activity is an established treatment modality and a number of HDAC inhibitors are approved medicines, including: Vorinostat (CTCL), Romidepsin (CTCL), Chidamide (PTCL), Panobinostat (multiple myeloma), Belinostat (T cell lymphoma), and many are in clinical trials, including: Panobinostat (CTCL), valproic acid (cervical cancer and ovarian cancer, spinal muscular atrophy), Mocetinostat (follicular lymphoma, Hodgkin lymphoma and acute myeloid leukemia), Abexinostat (sarcoma), Entinostat (Hodgkin lymphoma, lung cancer and breast cancer), SB939 (Recurrent or Metastatic Prostate Cancer), Resminostat (Hodgkin lymphoma), Givinostat (refractory leukemias and myelomas), HBI-800 (Advanced Solid Tumors Including Melanoma, Renal Cell Carcinoma (RCC), and Non-Small Cell Lung Cancer (NSCLC)), Kevetrin (ovarian cancer), CUDC-101, AR-42 (relapsed or treatment-resistant multiple myeloma, chronic lymphocytic leukemia or lymphoma), CHR-2845, CHR-3996, 4SC-202 (advanced haematological indications), CG200745 (solid tumours), ACY-1215 (multiple myeloma), ME-344 (solid refractory tumours), sulforaphane, and Trichostatin (anti-inflammatory).

Examples of diseases or conditions mediated by HDAC activity include neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer. In certain embodiments the compositions of the invention are used to treat or prevent one of these diseases or conditions. In certain embodiments, the compositions of the invention are used to treat or prevent one of these diseases or conditions mediated by HDAC activity. In certain embodiments, the compositions of the invention are used to treat or prevent one of these diseases or conditions mediated by Class I HDAC activity. In certain embodiments, the compositions of the invention are used to treat or prevent one of these diseases or conditions mediated by HDAC2.

In certain embodiments, the compositions of the invention are for use in therapy. In certain embodiments, the compositions of the invention are for use in the treatment of prevention of a disease or condition mediated by HDAC activity. In certain embodiments, the compositions of the invention are for use in a method of reducing HDAC activity in the treatment or prevention of a disease or condition mediated by HDAC activity. In some embodiments, the compositions of the invention are for use in treating or preventing a disease or condition mediated by Class I HDAC activity. In certain embodiments, the compositions of the invention are for use in a method of inhibiting Class I HDAC activity. In certain embodiments, the compositions of the invention are for use in a method of selectively inhibiting Class I HDAC activity in the treatment or prevention of a disease mediated by Class I HDAC activity. The inventors have identified that certain compositions of the invention selectively inhibit Class I HDACs. As used herein "selective" refers to compositions that have the greatest inhibitory effect on Class I HDACs, for example, in comparison to their inhibitory effect of HDACs from other classes. Selective inhibition of HDACs is advantageous for the treatment of diseases that require long-term administration of a therapeutic agent, for example where a disease or condition needs to be treated throughout the lifetime of a patient. In certain embodiments, the compositions of the invention that are Class I HDAC selective inhibitors are for use in the palliative treatment or prevention of a disease or condition mediated by Class I HDAC activity. Selective inhibitors are advantageous over pan-inhibitors known in the art by reducing side effects associated with the unwanted inhibition of other classes of HDACs. In certain embodiments, the compositions of the invention are HDAC2 selective inhibitors. In certain embodiments, the compositions of the invention are for use in a method of selectively reducing HDAC2 activity. In certain embodiments, the compositions of the invention are for use in the treatment or prevention of a disease mediated by HDAC2 activity.

### Modulation of the microbiota-gut-brain axis

Communication between the gut and the brain (the microbiota-gut-brain axis) occurs via a bidirectional neurohumoral communication system. Recent evidence shows that the microbiota that resides in the gut can modulate brain development and produce behavioural phenotypes via the microbiota-gut-brain axis. Indeed, a number of reviews suggest a role of the microbiota-gut-brain axis in maintaining central nervous system functionality and implicate dysfunction of the microbiota-gut-brain axis in the development of central nervous system disorders and conditions [20],[23],[24],[57].

The bidirectional communication between the brain and the gut (i.e. the-gut-brain axis) includes the central nervous system, neuroendocrine and neuroimmune systems, including the hypothalamus-pituitary-adrenal (HPA) axis, sympathetic and parasympathetic arms of the autonomic nervous system (ANS), including the enteric nervous system (ENS) and the vagus nerve, and the gut microbiota.

As demonstrated in the examples, the compositions of the present invention can modulate the microbiota-gut-brain axis and reduce behavioural symptoms associated with a CNS disorder. Accordingly, the compositions disclosed herein (compositions of the invention) may be useful for treating or preventing disorders of the central nervous system (CNS), in particular those disorders and conditions associated with dysfunction of the microbiota-gut-brain axis.

The compositions disclosed herein (compositions of the invention) may also be useful for treating or preventing neurodevelopmental disorders and/or neuropsychiatric conditions. Neurodevelopmental diseases and neuropsychiatric conditions are often associated with the microbiota-gut-brain axis. The compositions disclosed herein (compositions of the invention) may be useful for treating or preventing neurodevelopmental diseases and/or neuropsychiatric conditions mediated by dysfunction of the microbiota-gut-brain axis. In further preferred embodiments, the compositions disclosed herein (compositions of the invention) are for use in treating or preventing a neurodevelopmental disorder or a neuropsychiatric condition.

In particular embodiments, the compositions disclosed herein (compositions of the invention) may be useful for treating or preventing a disease or condition selected from the group consisting of: autism spectrum disorders (ASDs); child developmental disorder; obsessive compulsive disorder (OCD); major depressive disorder; depression; seasonal affective disorder; anxiety disorders; schizophrenia spectrum disorders; schizophrenia; bipolar disorder; psychosis; mood disorder; chronic fatigue syndrome (myalgic encephalomyelitis); stress disorder; post-traumatic stress disorder; dementia; Alzheimer's; Parkinson's disease; and/or chronic pain, such as central sensitisation or fibromyalgia. In further embodiments, the compositions disclosed herein (compositions of the invention) may be useful for treating or preventing motor neuron disease; Huntington's disease; Guillain-Barre syndrome and/or meningitis.

The compositions disclosed herein (compositions of the invention) may be particularly useful for treating or preventing chronic disease, treating or preventing disease in patients that have not responded to other therapies (such as treatment with anti-psychotics and/or anti-depressants), and/or treating or preventing the tissue damage and symptoms associated with dysfunction of the microbiota-gut-brain axis.

In certain embodiments, the compositions disclosed herein (compositions of the invention) modulate the CNS. In some embodiments, the compositions disclosed herein (compositions of the invention) modulate the autonomic nervous system (ANS). In some embodiments, the compositions disclosed herein (compositions of the invention) modulate the enteric nervous system (ENS). In some embodiments, the compositions disclosed herein (compositions of the invention) modulate the hypothalamic, pituitary, adrenal (HPA) axis. In some embodiments, the compositions disclosed herein (compositions of the invention) modulate the neuroendocrine pathway. In some embodiments, the compositions disclosed herein (compositions of the invention) modulate the neuroimmune pathway. In some embodiments, the compositions disclosed herein (compositions of the invention) modulate the CNS, the ANS, the ENS, the HPA axis and/or the neuroendocrine and neuroimmune pathways. In certain embodiments, the compositions disclosed herein (compositions of the invention) module the levels of commensal metabolites and/or the gastrointestinal permeability of a subject.

The signalling of the microbiota-gut-brain axis is modulated by neural systems. Accordingly, in some embodiments, the compositions disclosed herein (compositions of the invention) modulate signalling in neural systems. In certain embodiments, the compositions disclosed herein (compositions of the invention) modulate the signalling of the central nervous system. In some embodiments, the compositions disclosed herein (compositions of the invention) modulate signalling in sensory neurons, In other embodiments, the compositions disclosed herein (compositions of the invention) modulate signalling in motor neurons. In some embodiments, the compositions disclosed herein (compositions of the invention) modulate the signalling in the ANS. In some embodiments, the ANS is the parasympathetic nervous system. In preferred embodiments, the compositions disclosed herein (compositions of the invention) modulate the signalling of the vagus nerve. In other embodiments, the ANS is the sympathetic nervous system. In other embodiments, the compositions disclosed herein (compositions of the invention) modulate the signalling in the enteric nervous system. In certain embodiments, the signalling of ANS and ENS neurons responds directly to luminal contents of the gastrointestinal tract. In other embodiments, the signalling of ANS and ENS neurons responds indirectly to neurochemicals produced by luminal bacteria. In other embodiments, the signalling of ANS and ENS neurons responds to neurochemicals produced by luminal bacteria or enteroendocrine cells. In certain preferred embodiments, the neurons of the ENS activate vagal afferents that influence the functions of the CNS In some embodiments, the compositions disclosed herein (compositions of the invention) regulate the activity of enterochromaffin cells.

In certain embodiments, the compositions disclosed herein (compositions of the invention) modulate fear conditioning in an animal model. In certain embodiments, the compositions disclosed herein (compositions of the invention) can be used to modulate the development of fear and/or anxiety, and/or modulate the extent to which the fear and/or anxiety becomes extinct in a subject. In certain embodiments, the compositions disclosed herein (compositions of the invention) can be used to modulate the extent of stress-induced hyperthermia in an animal model. In certain embodiments, the compositions disclosed herein (compositions of the invention) modulate the level of stress and/or anxiety in a subject.

### Autism spectrum disorder (ASD)

Autism spectrum disorder is a set of heterogeneous neurodevelopmental conditions, characterised by early-onset difficulties in social interaction, communication and unusually restricted, repetitive behaviour and interests. Symptoms can be recognised from a very early age but ASD is often diagnosed in more able children starting mainstream education. Autism represents the primary type of ASD.

Historically, autism has been diagnosed on the basis of three core domains: impaired social interaction, abnormal communication, and restricted and repetitive behaviours and interests. In the International Classification of Diseases (ICD-10R, WHO 1993) and the Diagnostic and Statistical Manual (DSM-IV, American Psychiatric Association, 2000), autism comes under the umbrella term of Pervasive Developmental Disorder (PDD), with four possible diagnostic subtypes: Asperger Syndrome, Childhood Autism/Autistic Disorder, Atypical Autism, and PDD-not otherwise specified. In DSM-5, these diagnostic subtypes are combined into a single category of autism spectrum disorder (ASD) and the previous use of three core domains of impairment has been reduced to two main areas, namely social communication and interaction, and repetitive behaviour, which include sensory integration dysfunctions.

ASD is a 'spectrum disorder' as it affects each person in a variety of different ways and can range from very mild to severe. The functioning of the affected individual varies substantially depending on language abilities, level of intelligence, co-morbidity, composition of symptoms and access to services. Cognitive functioning, learning, attention and sensory processing are usually impaired.

DSM-IV states that the diagnosis of autism requires the presence of at least six symptoms, including a minimum of two measures of qualitative impairment in social interaction, one symptom of qualitative impairment in communication, and one symptom of restricted and repetitive behaviour. DSM-5 redefines diagnosis of ASD into two symptom domains: (i) social interaction and social communication deficits; and (ii) restricted, repetitive patterns of behaviour, interests or activities.

Co-morbid medical conditions are highly prevalent in ASDs. Co-morbid include anxiety and depression, seizures, attention deficits, aggressive behaviours, sleep problems, gastrointestinal disorders, epilepsy, mental retardation, intellectual disabilities and feeding difficulties.

The examples demonstrate that the compositions disclosed herein (compositions of the invention) achieve a reduction in disease incidence and disease severity in an animal model of autism spectrum disorder and so they may be useful in the treatment or prevention of autism spectrum disorders.

ASD is a central nervous system disorder that is partially triggered by environmental factors. Therefore, dysfunction of the microbiota-gut-brain axis may be responsible for development and persistence of ASDs. Accordingly, in preferred embodiments, the composition of the invention are for use in treating or preventing autism spectrum disorders. In some embodiments, the compositions disclosed herein (compositions of the invention) are for use in treating or preventing autism. In some embodiments, the autism is Pervasive Developmental Disorder (PDD). In another embodiment, the PDD is Asperger Syndrome, Childhood Autism/Autistic Disorder, Atypical Autism and/or PDD-not otherwise specified. Accordingly, in some embodiments, the compositions disclosed herein (compositions of the invention) are for use in treating or preventing autism spectrum disorders, autism, pervasive developmental disorder; Asperger Syndrome; Childhood Autism/Autistic Disorder, Atypical Autism and/or PDD-not otherwise specified.

The compositions disclosed herein (compositions of the invention) may be useful for modulating the microbiota-gut-brain axis of a subject. Accordingly, in preferred embodiments the compositions disclosed herein (compositions of the invention) are for use in preventing an ASD in a patient that has been identified as at risk of an ASD, or that has been diagnosed with an ASD at a prenatal or an early developmental stage; in childhood and/or in adulthood. The compositions disclosed herein (compositions of the invention) may be useful for preventing the development of ASDs.

The compositions disclosed herein (compositions of the invention) may be useful for managing or alleviating ASDs. Treatment or prevention of ASDs may refer to, for example, an alleviation of the severity of symptoms or a reduction in the frequency of exacerbations or the range of triggers that are a problem for the patient.

In preferred embodiments, the compositions disclosed herein (compositions of the invention) prevent, reduce or alleviate at least one core symptom of ASDs.

In some embodiments, the compositions disclosed herein (compositions of the invention) prevent, reduce or alleviate at least one of the two symptom domains of ASD classified in the DSM-5. In some embodiments, the compositions disclosed herein (compositions of the invention) prevent, reduce or alleviate social interaction and/or social communication deficits. In some embodiments, the compositions disclosed herein (compositions of the invention) prevent, reduce or alleviate restrictive, repetitive patterns of behaviour, interests or activities. In some embodiments, the compositions disclosed herein (compositions of the invention) prevent, reduce or alleviate social interaction, social communication deficits and/or restrictive, repetitive patterns of behaviour, interests or activities.

In some embodiments, the compositions disclosed herein (compositions of the invention) prevent, reduce or alleviate repetitive behaviour, stereotyped behaviour, compulsive behaviour, routine behaviour, sameness behaviour and restricted behaviour. In some embodiments, the compositions disclosed herein (compositions of the invention) improve social awareness, social information processing, capacity for social communication, social anxiety/avoidance, and autistic preoccupations and traits in a subject with ASDs.

In some embodiments, the compositions disclosed herein (compositions of the invention) prevent, reduce or alleviate additional symptoms associated with the core symptoms of ASDs. In some embodiments, the compositions disclosed herein (compositions of the invention) prevent, reduce or alleviate irritability (including aggression, deliberate self-injury and temper tantrums), agitation, crying, lethargy, social withdrawal, stereotypic behaviour, hyperactivity, non-compliance, inappropriate speech, anxiety, depression, and/or over or under-controlled behaviour in a subject with ASDs. In some embodiments, the compositions disclosed herein (compositions of the invention) improve cognitive functioning, learning, attention and/or sensory processing in a subject with ASD.

In other embodiments, the compositions disclosed herein (compositions of the invention) improve secondary outcome measures in a subject with ASDs. In some embodiments, the secondary outcome measures include additional symptom and/or functional rating scales, behavioural scales and miscellaneous measures of interest.

In some embodiments, the compositions disclosed herein (compositions of the invention) cause in a positive change in the diagnostic and/or symptomatic scale for the assessment of core symptoms of a subject with ASDs. In some embodiments, the diagnostic and/or symptomatic scale is the Autism Diagnostic Interview - Revised (ASI-R). In some embodiments, the diagnostic or symptomatic scale is the Autism Diagnostic Observation Schedule-Generic (ADOS-G) now ADOS-2. In other embodiments, the diagnostic or symptomatic scale is the Autism Diagnostic Interview Revised (ADI-R). In other embodiments, the diagnostic or symptomatic scale is the Diagnostic Interview for Social and Communication Disorders (DISCO). In yet other embodiments, the diagnostic or symptomatic scale is the Childhood Autism Rating Scale (CARS and CARS2).

In some embodiments, the compositions disclosed herein (compositions of the invention) cause a positive change in generic measures of the efficacy endpoints of ASDs. In certain embodiments, the generic measures include, but are not limited to the Aberrant Behaviour Checklist (ABC), the Child Behaviour Checklist (CBCL), the Vineland-II Adaptive Behaviour Scales (VABS), the Social Responsiveness Scale (SRS), and/or the Repetitive Behaviour Scale - Revised (RBS-R).

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global functioning of the subject with ASDs.

Additional scales would be known to a person skilled in the art. In some embodiments, the compositions disclosed herein (compositions of the invention) would improve the outcome of diagnostic and/or symptomatic scales known to a person skilled in the art.

In certain embodiments, the compositions disclosed herein (compositions of the invention) prevent, reduce or alleviate the incidence of comorbidities of ASDs. In some embodiments, the compositions disclosed herein (compositions of the invention) prevent, reduce or alleviate the incidence of anxiety and depression, seizures, attention deficits, aggressive behaviours, sleep problems, gastrointestinal disorders (including irritable bowel syndrome (IBS)), epilepsy, mental retardation, intellectual disabilities and/or feeding difficulties. In certain embodiments, the compositions disclosed herein (compositions of the invention) prevent, reduce or alleviate gastrointestinal comorbidities, such as abdominal pain, diarrhoea and flatulence.

In some embodiments, the compositions disclosed herein (compositions of the invention) prevent, reduce or alleviate the symptoms of certain psychiatric and behavioural disorders that may present clinically with similarities to autism. Accordingly, in some embodiments, the compositions disclosed herein (compositions of the invention), prevent, reduce or alleviate attention deficit disorder (ADHD); affective/anxiety disorders; attachment disorders; oppositional defiant disorder (ODD); obsessive compulsive disorder (OCD) and/or psychoses including schizophrenia (cognitive impairment).

In some embodiments, the compositions disclosed herein (compositions of the invention) are particularly effective at preventing, reducing or alleviating ASDs when used in combination with another therapy for treating ASDs. Such therapies include anti-psychotic, anti-anxiety and antidepressant drugs. Such drugs include risperidone (Risperdal^{®}); olanzapine (Zyprexa^{®}); fluoxetine (Prozac^{®}); sertraline (Zoloft^{®}); fluvoxamine (Luvox^{®}); clomipramine (Anafranil^{®}); haloperidol (Haldol^{®}); thioridazine; fluphenazine; chlorpromazine; ziprasidone (Geogon^{®}); carbamazepine (Tegretol^{®}); lamotrigine (Lamictal^{®}); topiramate (Topomax^{®}); valproic acid (Depakote^{®}), methylphenidate (Ritalin^{®}); diazepam (Valium^{®}) and lorazepam (Ativan^{®}).

The EMA Guidelines on the clinical development of medicinal products for the treatment of autism spectrum disorder state that, due to the heterogeneity of the disease, it may not be possible to achieve a significant effect on all core symptoms with a single compound, and so short term efficacy has to be demonstrated on at least one core symptom. The live biotherapeutic strains used in the Examples have shown effective treatment of at least one core symptom of autistic spectrum disorder, so bacterial strains of the genus *Anaerostipes, Eubacterium* or *Faecalicatena* are expected to be effective against human disease.

### Obsessive compulsive disorder (OCD)

OCD is a heterogeneous, chronic and disabling disorder belonging to the anxiety disorders. According to the DSM-IV definition, the essential features of OCD are recurrent obsessions and/or compulsions (criterion A) that are severe and time consuming (more than one hour a day) or cause marked distress or significantly interfere with the subject's normal routine, occupational functioning, usual social activities or relationships (criterion C). At some point during the course of the disorder, the person has recognised that the obsessions or compulsions are excessive or unreasonable (criterion B).

Obsessions are defined as recurrent and persistent thoughts, impulses or images that are experienced as intrusive and inappropriate and cause marked anxiety or distress. The thoughts, impulses or images are not simply excessive worries about real-life problems, they are recognised by the patient as a product of his own mind (e.g. fear for contamination, symmetry obsession). The person attempts to ignore, suppress or neutralise the obsessions with some other thoughts or actions.

Compulsions are defined as repetitive behaviours (e.g. hand washing, ordering, hoarding, checking) or mental acts (e.g. praying, counting, repeating words silently) that the person feels driven to perform in response to an obsession or according to rules that must be applied rigidly.

OCD is often associated with co-morbidity rates of other psychiatric diseases including major depressive disorder, other anxiety disorders (generalised anxiety disorder, social anxiety disorder, panic disorder), substance abuse and eating disorders (anorexia and bulimia).

OCD is a psychiatric disorder that may develop or persist due to dysfunction of the microbiota-gut-brain axis. Accordingly, in preferred embodiments, the compositions disclosed herein (compositions of the invention) are for use in treating or preventing OCD in a subject.

In certain embodiments, the compositions disclosed herein (compositions of the invention) prevent, reduce or alleviate the essential symptomatic features of OCD. In certain embodiments, the compositions disclosed herein (compositions of the invention) prevent, reduce or alleviate recurrent obsessions and/or compulsions in a subject. In certain embodiments, the obsessions are recurrent or persistent thoughts, impulses or images that are experiences as intrusive and inappropriate and cause marked anxiety or distress. In certain embodiments, the compulsions are repetitive behaviours that the subject feels driven to perform in response to an obsession or according to rules that must be applied rigidly.

In certain embodiments, the compositions disclosed herein (compositions of the invention) improve symptoms of OCD in a subject accordingly to the Y-BOCS and/or the NIMH-OC diagnostic and/or symptomatic scales. In some embodiments, the Y-BOCS scale is used to monitor improvement of primary endpoints. In some embodiments, the NIMH-OC scale is used to monitor improvement of secondary parameters.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social functioning (relationships, work, *etc*.) of the subject with ASDs. In some embodiments, the global scale is the Sheehan disability scale.

In preferred embodiments, the compositions disclosed herein (compositions of the invention) prevent, reduce or alleviate at least one comorbidity of OCD. The comorbidities of OCD include major depressive disorder, other anxiety disorders (generalised anxiety disorder, social anxiety disorder, panic disorder), substance abuse and eating disorders (anorexia and bulimia) Gilles de la Tourette syndrome, ADHD (Attention-Deficit/Hyperactivity Disorder) and developmental disorders.

In some embodiments, the compositions disclosed herein (compositions of the invention) are particularly effective at preventing, reducing or alleviating OCD when used in combination with another therapy for treating OCD. Such therapies include serotonin and dopamine reuptake inhibitors; clomipramine and anti-psychotics.

### Major depressive disorder (MDD)

MDD is associated with substantial psychosocial dysfunction and high individual mental strain as well as with excess morbidity and mortality (the risk of suicide is considerable). The term major depressive disorder encompasses clinical depression, major depression, unipolar depression, unipolar disorder, recurrent depression and simply depression. The term major depressive disorder covers mood disorders; dysthymia; chronic depression; seasonal affective disorder and borderline personality disorder.

According to the DSM-5 criteria, MDD symptoms include a depressed mood, or loss of interest or pleasure in daily activities for more than two weeks; and impaired social, occupational and educational function. Specific symptoms, at least five of the following nine, present nearly every day: depressed mood or irritable most of the day; decreased interest or pleasure in most activities, most of each day; significant weight change or change in appetite; change in sleep (insomnia or hypersomnia); change in activity (psychomotor agitation or retardation); fatigue or loss of energy; guilt or worthlessness (feelings of worthlessness or excessive or inappropriate guilt); reduced concentration (diminished ability to think or concentrate, or more indecisiveness, and suicidality (thoughts of death or suicide, or subject has a suicide plan). In addition, MDD is associated with anxiety symptoms including irrational worry; preoccupation with unpleasant worries; trouble relaxing and/or feeling tense. MDD episodes can be mild, moderate or severe.

MDD episodes are often associated with comorbidity with other psychiatric disorders or with somatic disorders like Parkinson's disease, Alzheimer's disease, cerebrovascular disorders, cancer and chronic pain syndromes. MDD is frequently associated with a wide spectrum of other mental disorders as comorbidities including generalised anxiety disorder; anxiety disorder; substance use disorders; post-traumatic stress disorder (PTSD); personality disorders; pain; stress; irritable bowel syndrome; insomnia; headaches and interpersonal problems.

Major depressive disorder is a psychiatric disorder that may develop or persist due to dysfunction of the microbiota-gut-brain axis. Accordingly, in preferred embodiments, the compositions disclosed herein (compositions of the invention) are for use in treating or preventing MDD in a subject.

In certain embodiments, the compositions disclosed herein (compositions of the invention) are for use in treating or preventing acute major depressive episodes and/or the prevention of new episodes (recurrence prevention). In certain embodiments, the compositions disclosed herein (compositions of the invention) prevent, reduce or alleviate the occurrence of mild, moderate or severe MDD episodes.

In certain embodiments, the compositions disclosed herein (compositions of the invention) prevent, reduce or alleviate one or more of the symptoms of MDD as classified by the DSM-5 criteria listed herein. In a preferred embodiment, the compositions disclosed herein (compositions of the invention) prevent, reduce or alleviate a depressed mood in a subject. In a preferred embodiment, the compositions disclosed herein (compositions of the invention) prevent, reduce or alleviate a decreased interest or pleasure in most activities in a subject. In some embodiments, the compositions disclosed herein (compositions of the invention) reduce the occurrence of symptoms of MDD within a 2-week period.

In some embodiments, the compositions disclosed herein (compositions of the invention) improve the symptoms of MDD according to a symptomatic or diagnostic scale. Such scales for assessing symptomatic improvement include the Hamilton Rating Scale of Depression (HAMD) and the Montgomery Asberg Depression Rating Scale. In addition, the Zung Self-Rating Depression Scale (SDS) and Zung Self-Rating Anxiety Scale (SAS) are also suitable symptomatic improvement scales.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social and occupational functioning of the subject with MDD.

In certain embodiments, the compositions disclosed herein (compositions of the invention) are for use in treating or preventing treatment resistant MDD.

In preferred embodiments, the compositions disclosed herein (compositions of the invention) prevent, reduce or alleviate at least one comorbidity of MDD. The comorbidities of MDD include generalised anxiety disorder; anxiety disorder; substance use disorders; post-traumatic stress disorder (PTSD); personality disorders; pain; stress; IBS; insomnia; headaches and interpersonal problems.

In some embodiments, the compositions disclosed herein (compositions of the invention) are particularly effective at preventing, reducing or alleviating MDD when used in combination with another therapy for treating MDD. Such therapies include antidepressants, augmentation strategies (e.g. combination therapy, lithium and other mood stabilizers, thyroid hormones and atypical antipsychotics) or even second generation antipsychotics.

### Anxiety disorders

Anxiety disorders are a group of mental disorders characterised by feelings of anxiety and fear. There are a number of anxiety disorders including generalised anxiety disorder (GAD); specific phobia; social anxiety disorder; separation anxiety disorder; agroraphobia; panic disorder and selective mutism.

GAD is diagnosed according to DMS-5 in six criterion. The first criterion is too much anxiety or worry over more than six months wherein the anxiety or worry is present most of the time in regards to many activities. The second criterion is that the subject is unable to manage the symptoms of the first criterion. The third criterion is that at least three (one in children) of the following occurs: restlessness; tires easily; problems concentrating; irritability; muscle tension and problems with sleep. The final three criterion are that the symptoms results in significant social, occupational and functional impairment; the symptoms are not due to medications, drugs, or other physical health problems; and the symptoms do not fit better with another psychiatric problem such as panic disorder. All other anxiety disorders may be considered as differential diagnoses of GAD.

GAD is frequently associated with a wide spectrum of other mental disorders as comorbidities including depression; substance use disorders; stress; IBS; insomnia; headaches; pain; cardiac events; interpersonal problems and ADHD

Anxiety disorders are psychiatric disorders that may develop or persist due to dysfunction of the microbiota-gut-brain axis. Accordingly, in preferred embodiments, the compositions disclosed herein (compositions of the invention) are for use in treating or preventing anxiety disorders in a subject. In certain embodiments, the anxiety disorder is generalised anxiety disorder (GAD); specific phobia; social anxiety disorder; separation anxiety disorder; agoraphobia; panic disorder and selective mutism.

In certain embodiments, the compositions disclosed herein (compositions of the invention) prevent, reduce or alleviate one or more of the symptoms of GAD in a subject as classified by the DMS-5 criteria listed herein. According to DMS-5, the same symptoms are associated with other anxiety disorders. Therefore, in certain embodiments, the compositions disclosed herein (compositions of the invention) prevent, reduce or alleviate one or more of the symptoms of anxiety disorders in a subject In preferred embodiments, the compositions disclosed herein (compositions of the invention) prevent, reduce or alleviate the anxiety or worry of the subject. In certain embodiments, the compositions disclosed herein (compositions of the invention) reduce the occurrence of symptoms within a six month period. In certain embodiments, the composition di prevents, reduces or alleviates restlessness; fatigue; loss of concentration; irritability; muscle tension; and/or problems with sleep. In some embodiments, the compositions disclosed herein (compositions of the invention) prevent, reduce or alleviate social, occupational and functional impairment associated with anxiety disorders.

In some embodiments, the compositions disclosed herein (compositions of the invention) improve the symptoms of anxiety disorders according to a symptomatic or diagnostic scale. In certain embodiments, the scale for assessing symptomatic improvement includes the Hamilton Anxiety Rating Scale (HAM-A). In some embodiments, the HAM-A total scale is used to assess primary endpoint. In other embodiments, the HAM-A psychic anxiety factor may be useful as a secondary endpoint.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social, occupational and functional impairment of the subject with anxiety disorder. In some embodiments, the global scale is the Sheehan disability scale.

In preferred embodiments, the compositions disclosed herein (compositions of the invention) prevent, reduce or alleviate at least one comorbidity of GAD and anxiety disorders. The comorbidities of GAD include depression; substance use disorders; stress; IBS; insomnia; headaches; pain; cardiac events; interpersonal problems and ADHD

In some embodiments, the compositions disclosed herein (compositions of the invention) are particularly effective at preventing, reducing or alleviating anxiety disorders when used in combination with another therapy for treating anxiety disorders. Such therapies include selective serotonin reuptake inhibitors (venlafaxine, duloxetine, escitalopram and paroxetine); benzodiazepines (alprazolam, lorazepam and clonazepam); pregabalin (Lyrica^{®}) and gabapentin (Neurontin ^{®}); serotonin receptor partial agonists (buspirone and tandospirone); atypical serotonergic antidepressants (such as imipramine and clomipramine); monoamine oxidase inhibitors (MAOIs) (such as moclobemide and phenelzine); hydroxyzine; propranolol; clonidine; guanfacine and prazosin.

### Post-traumatic stress disorder (PTSD)

PTSD is a severe and disabling disorder, an essential feature of which is the inclusion of a traumatic event as a precipitating factor of this disorder.

The symptoms of PTSD are grouped into four main clusters according to the DSM-5 criteria: (i) intrusion: examples include nightmares, unwanted thoughts of the traumatic events, flashbacks, and reacting to traumatic reminders with emotional distress or physiological reactivity; (ii) avoidance: examples include avoiding triggers for traumatic memories including places, conversations, or other reminders; (iii) negative alterations in cognitions and mood: examples include distorted blame of self or others for the traumatic event, negative beliefs about oneself or the world, persistent negative emotions (e.g., fear, guilt, shame), feeling alienated, and constricted affect (e.g., inability to experience positive emotions); (iv) alterations in arousal and reactivity: examples include angry, reckless, or self-destructive behaviour, sleep problems, concentration problems, increased startle response, and hypervigilance.

Symptoms that resolve within 4 weeks of the traumatic event meet the criteria for an Acute Stress Disorder. The DSM distinguishes between acute (duration of symptoms for less than three months) and chronic PTSD (duration of symptoms longer than 3 months). If the symptoms begin more than 6 months after the stressor, the disorder is defined as delayed onset PTSD.

PTSD carries high comorbidities with major depressive disorder and substance use disorders.

PTSD is a psychiatric disorder that may develop or persist due to dysfunction of the microbiota-gut-brain axis. Accordingly, in preferred embodiments, the compositions disclosed herein (compositions of the invention) are for use in treating or preventing PTSD in a subject. According to a similar pathogenesis, in certain embodiments, the compositions disclosed herein (compositions of the invention) are for use in treating or preventing stress disorders. In certain embodiments, the compositions disclosed herein (compositions of the invention) treat acute stress disorder. In some embodiments, the compositions disclosed herein (compositions of the invention) treat acute and/or chronic PTSD. In some embodiments, the compositions disclosed herein (compositions of the invention) treat delayed onset PTSD.

In certain embodiments, the compositions disclosed herein (compositions of the invention) prevent, reduce or alleviate one or more of the symptoms of PTSD (or stress disorder) in a subject as classified by the DSM-5 criteria listed herein. In preferred embodiments, the compositions disclosed herein (compositions of the invention) prevent, reduce or alleviate intrusive thoughts in a subject with PTSD. In preferred embodiments, the compositions disclosed herein (compositions of the invention) prevent, reduce or alleviate avoidance behaviour in a subject with PTSD. In preferred embodiments, the compositions disclosed herein (compositions of the invention) prevent, reduce or alleviate negative alterations in cognitions and mood in a subject with PTSD. In preferred embodiments, the compositions disclosed herein (compositions of the invention) prevent alterations in arousal and reactivity in a subject with PTSD.

In some embodiments, the compositions disclosed herein (compositions of the invention) improve the symptoms of PTSD and stress disorders according to a symptomatic or diagnostic scale. In certain embodiments, the scale for assessing symptomatic improvement is the Clinical-Administered PTSD (CAPS) scale.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social, occupational and functional impairment of the subject with PTSD and stress disorders. In some embodiments, the global scale is the Sheehan disability scale.

In preferred embodiments, the compositions disclosed herein (compositions of the invention) prevent, reduce or alleviate at least one comorbidity of PTSD and stress disorders. The comorbidities of PTSD and stress disorders include MDD, substance use disorders; stress and anxiety.

In some embodiments, the compositions disclosed herein (compositions of the invention) are particularly effective at preventing, reducing or alleviating PTSD and stress disorders when used in combination with another therapy for treating PTSD and stress disorders. Such therapies include serotoninergic agents, tricyclic antidepressants, mood stabilisers, adrenergic inhibiting agents, antipsychotics, benzodiazepines, sertraline (Zoloft^{®}), fluoxetine (Prozac^{®}) and/or paroxetine (Paxil^{®}).

### Schizophrenia spectrum and psychotic disorders

These diseases affect a subject's ability to think clearly, make good judgements, respond emotionally, communicate effectively, understand reality, and behave appropriately. Psychotic diseases include schizophrenia (symptoms listed below); schizoaffective disorder (the subject has symptoms of both schizophrenia and a mood disorder, such as depression or bipolar disorder); schizophreniform disorder (displays the symptoms of schizophrenia, but the symptoms last for a shorter time: between 1 and 6 months); brief psychotic disorder (subjects display a sudden, short period of psychotic behaviour, often in response to a very stressful event, such as a death in the family - recovery is usually less than a month); delusional disorder (delusions last for at least 1 month); shared psychotic disorder; substance-induced psychotic disorder; psychotic disorder due to another medical condition; paraphrenia (displaying symptoms similar to schizophrenia and starting late in life, when people are elderly). The most well-known psychotic disorder is schizophrenia and the majority of psychotic disorders display similar symptoms to schizophrenia.

Schizophrenia is a severe psychiatric disease with a heterogeneous course and symptom profile. Schizophrenia presents clinically with so-called positive and negative symptoms. The positive symptoms include delusions, hallucinations, disorganised speech, and disorganised or catatonic behaviours. Negative symptoms include affective flattening, restriction in the fluency and productivity of thought and speech and in the initiation of goal directed behaviour. The positive symptoms appear to reflect an excess or distortion of normal functions, whereas negative symptoms appear to reflect a diminution or loss of normal function. In addition, cognitive deficits (defects of working memory, information processing, attention/vigilance, learning, reasoning and social cognition) are common. Cognitive deficits generally show poor improvement with current antipsychotic treatment, Schizophrenic patients also suffer from mood symptoms. Besides these predominant symptoms, schizophrenia is associated with a comorbidity with other psychiatric symptoms such as manic and depressive symptoms, anxiety or obsessive-compulsive symptoms, substance abuse and dependence, and personality disorder.

According to the DSM-5, for the diagnosis of schizophrenia, a subject must have at least two of the following symptoms: delusions; hallucinations; disorganised speech; disorganised or catatonic behaviour and negative symptoms. At least one of the symptoms must be the presence of delusions, hallucinations or disorganised speech. Continuous signs of disturbance must persist for at least 6 months, during which the subject must experience at least 1 month of active symptoms, with social or occupational deterioration problems occurring over a significant amount of time.

Schizophrenia spectrum and psychotic disorders are psychiatric disorders that may develop or persist due to dysfunction of the microbiota-gut-brain axis. Therefore, in preferred embodiments, the compositions disclosed herein (compositions of the invention) are for use in treating or preventing schizophrenia spectrum and/or psychotic disorders in a subject. In certain embodiments, the schizophrenia spectrum and psychotic disorder is selected from schizophrenia; schizoaffective disorder; schizophreniform disorder; brief psychotic disorder; delusional disorder; shared psychotic disorder; substance-induced psychotic disorder; psychotic disorder due to another medical condition and paraphrenia. In preferred embodiments, the compositions disclosed herein (compositions of the invention) are for use in treating or preventing schizophrenia. In certain embodiments, the schizophrenia is selected from paranoid, disorganised, catatonic, undifferentiated and residual schizophrenia.

In certain embodiments, the compositions disclosed herein (compositions of the invention) prevent, reduce or alleviate one or more of the symptoms of schizophrenia in a subject as classified by the DSM-5 criteria listed herein. These embodiments apply to the prevention, reduction or alleviation of symptoms of other schizophrenia spectrum and psychotic disorders. In certain embodiments, the compositions disclosed herein (compositions of the invention) prevent, reduce or alleviate negative symptoms of schizophrenia. In certain embodiments, the compositions disclosed herein (compositions of the invention) prevent, reduce or alleviate positive symptoms of schizophrenia. In certain embodiments, the compositions disclosed herein (compositions of the invention) prevent, reduce or alleviate negative and positive symptoms of schizophrenia. In preferred embodiments, the compositions disclosed herein (compositions of the invention) prevent, reduce or alleviate delusions, hallucinations, disorganised speech, and disorganised or catatonic behaviours in a subject with schizophrenia. In preferred embodiments, the compositions disclosed herein (compositions of the invention) prevent, reduce or alleviate affective flattening, restriction in the fluency and productivity of thought and speech and in the initiation of goal directed behaviour in a subject with schizophrenia. In preferred embodiments, the compositions disclosed herein (compositions of the invention) prevent, reduce or alleviate the cognitive defects and/or mood disorders in a subject with schizophrenia.

In certain embodiments, the compositions disclosed herein (compositions of the invention) reduce the occurrence of positive and/or negative symptoms of schizophrenia in a subject within a 6 month period. In certain embodiments, the compositions disclosed herein (compositions of the invention) improve social and/or occupational functionality in a subject with schizophrenia spectrum or psychotic disorder.

In some embodiments, the compositions disclosed herein (compositions of the invention) improve the symptoms of schizophrenia spectrum or psychotic disorders according to a symptomatic or diagnostic scale. In certain embodiments, the scale for assessing symptomatic improvement is the Positive and Negative Symptom Scale (PANSS) and Brief Psychiatric Rating Scale (BPRS). In certain embodiments, the Scale for Assessment of Negative Symptoms (SANS) is used.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social and occupational impairment of the subject with schizophrenia spectrum or psychotic disorders.

In preferred embodiments, the compositions disclosed herein (compositions of the invention) prevent, reduce or alleviate at least one comorbidity of schizophrenia spectrum or psychotic disorder. In certain embodiments, the comorbidity is as manic and depressive symptoms, anxiety or obsessive-compulsive symptoms, substance abuse and dependence, and personality disorder.

In certain embodiments, the compositions disclosed herein (compositions of the invention) are for use in treating or preventing treatment resistant of refractory schizophrenia.

In some embodiments, the compositions disclosed herein (compositions of the invention) are particularly effective at preventing, reducing or alleviating schizophrenia spectrum or psychotic disorders when used in combination with another therapy for treating PTSD and stress disorders. In certain embodiments, such therapies include first generation antipsychotics including chlorpromazine, fluphenazine, haloperidol and/or perphenazine. In certain embodiments, such therapies include second generation therapies including aripiprazole (Abilify^{®}); asenapine (Saphns^{®}), brexpiprazole (Rexulti^{®}); cariprazine (Vraylar^{®}); clozapine (Clozaril^{®}); iloperidone (Fanapt^{®}); lurasidone (Latuda^{®}); olanzapine (Zyprexa^{®}); paliperidone (Invega); quetiapine (Seroquel^{®}); risperidone (Risperdal^{®}); ziprasidone (Geodon^{®}).

### Bipolar disorder

Bipolar disorder in general is a chronic disease. Mania is the cardinal symptom of bipolar disorder. There are several types of bipolar disorder based upon the specific duration and pattern of manic and depressive episodes. In DSM-5, a distinction is made between bipolar I disorder, bipolar II disorder, cyclothymic disorder, rapid-cycling bipolar disorder and bipolar disorder NOS.

According to the DSM, mania is a distinct period of abnormally and persistently elevated, expansive, or irritable mood. The episode must last a week, and the mood must have at least three of the following symptoms: high self-esteem; reduced need for sleep; increase rate of speech; rapid jumping of ideas; easily distracted; an increased interest in goals or activities; psychomotor agitation; increased pursuit of activities with a high risk of danger.

Bipolar I disorder involves one or more manic or mixed (mania and depression) episodes and at least one major depressive episode (see above for symptoms of MDD episodes). Bipolar II disorder has one or more major depressive episodes accompanied by at least one hypomanic episode. There are no manic or mixed episodes. Hypomania is a lesser form of mania. The symptoms are responsible for significant social, occupational and functional impairments. Cyclothymia is characterized by changing low-level depression along with periods of hypomania. The symptoms must be present for at least two years in adults or one year in children before a diagnosis can be made. Symptom free periods in adults and children last no longer than two months or one month, respectively. Rapid cycling bipolar disorder is a severe form of bipolar disorder. It occurs when a person has at least four episodes of major depression, mania, hypomania, or mixed states within a year. Not-otherwise specified (NOS) bipolar disorder classified bipolar symptoms that do not clearly fit into other types. NOS is diagnosed when multiple bipolar symptoms are present but not enough to meet the label for any of the other subtypes.

Bipolar disorder is associated with the following comorbidities: ADHD; anxiety disorders; substance disorders; obesity and metabolic syndrome.

Bipolar disorder is a psychiatric disorder that may develop or persist due to dysfunction of the microbiota-gut-brain axis. Therefore, in preferred embodiments, the compositions disclosed herein (compositions of the invention) are for use in treating or preventing bipolar disorder in a subject. In certain embodiments, the bipolar disorder is bipolar I disorder. In certain embodiments, the bipolar disorder is bipolar II disorder. In certain embodiments, the bipolar disorder is cyclothymic disorder. In certain embodiments, the bipolar disorder is rapid-cycling bipolar disorder. In certain embodiments, the bipolar disorder is bipolar disorder NOS.

In preferred embodiments, the compositions disclosed herein (compositions of the invention) prevent, reduce or alleviate one or more of the symptoms of bipolar disorder in a subject. In certain embodiments, the compositions disclosed herein (compositions of the invention) prevent, reduce or alleviate the occurrence of manic episodes in a subject. In certain embodiments, the compositions disclosed herein (compositions of the invention) prevent, reduce or alleviate the occurrence of an abnormally and persistently elevated, expansive, or irritable mood. In certain embodiments, the compositions disclosed herein (compositions of the invention) prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger. In certain embodiments, the compositions disclosed herein (compositions of the invention) prevent, reduce or alleviate the occurrence of one or more manic or mixed episodes in a subject. In certain embodiments, the compositions disclosed herein (compositions of the invention) reduce the occurrence of at least one major depressive episode in a subject. In certain embodiments, the compositions disclosed herein (compositions of the invention) prevent, reduce or alleviate the occurrence of at least one major depressive episode accompanied by at least one hypomanic episode.

In preferred embodiments, the compositions disclosed herein (compositions of the invention) treat the acute phase of bipolar disorder and/or prevent the occurrence of further episodes. In certain embodiments, the compositions disclosed herein (compositions of the invention) treat the acute phase of manic/depressive episodes in a subject with bipolar disorder and prevent occurrence of further manic/depressive episodes.

In some embodiments, the compositions disclosed herein (compositions of the invention) improve the symptoms of bipolar disorder according to a symptomatic or diagnostic scale. In certain embodiments, the scale for assessing symptomatic improvement of manic episodes is the Manic State Rating Scale and the Young Mania Rating Scale. In certain embodiments, the scale is the Bech-Rafaelsen Mania Scale (BRMAS). In certain embodiments, scales for assessing symptomatic improvement of the switch from manic to depressive episodes include the Hamilton Depression Rating Scale, the Montgomery-Asberg Rating Scale, and the Bech-Rafaelsen Depression Scale.

In some embodiments, the compositions disclosed herein (compositions of the invention) improve the Clinical Global Impression - Global Improvement (CGI-I) scale for assessing psychiatric and neurological disorders. In some embodiments, the compositions disclosed herein (compositions of the invention) display a positive effect on global social, occupational and functional impairments of the subject with bipolar disorder.

In preferred embodiments, the compositions disclosed herein (compositions of the invention) prevent, reduce or alleviate at least one comorbidity of bipolar disorder. In certain embodiments, the comorbidity is selected from ADHD, anxiety disorders, substance disorder, obesity and metabolic syndrome.

In certain embodiments, the compositions disclosed herein (compositions of the invention) are for use in treating or preventing manic-depressive illness and bipolar disorder unresponsive to lithium and divalproex.

In some embodiments, the compositions disclosed herein (compositions of the invention) are particularly effective at preventing, reducing or alleviating bipolar disorder when used in combination with another therapy for treating bipolar disorder. In certain embodiments, such therapies include lithium carbonate, anticonvulsant drugs (including valproate, divalproex, carbamazepine and lamotrigine) and antipsychotic drugs (including aripiprazole, olanzapine, quetiapine and risperidone).

### Neurocognitive disorders and Alzheimer's disease

### - Alzheimer's disease and dementia

Aberrant accumulation of hyperphosphorylated tau is a hallmark of neurodegenerative tauopathies such Alzheimer's disease. Reduction in HDAC activity can reduce levels of hyperphosphorylated tau and alleviate symptoms of tau-driven neurological disorders [58]. Therefore, in certain embodiments, the compositions of the invention are for use in the treatment or prevention of neurodegenerative tauopathies. In certain embodiments, the compositions of the invention are for use in the treatment of Alzheimer's disease.

In DSM-5, the term dementia was replaced with the terms major neurocognitive disorder and mild neurocognitive disorder. Neurocognitive disorder is a heterogeneous class of psychiatric diseases. The most common neurocognitive disorder is Alzheimer's disease, followed by vascular dementias or mixed forms of the two. Other forms of neurodegenerative disorders (e.g. Lewy body disease, frontotemporal dementia, Parkinson's dementia, Creutzfeldt-Jakob disease, Huntington's disease, and Wernicke-Korsakoff syndrome) are accompanied by dementia.

Alzheimer's disease and dementia are also characterised by neuronal loss, so the neuroprotective and neuroproliferative effects shown in the examples for the compositions of the invention indicate that they may be useful for treating or preventing these conditions.

The symptomatic criteria for dementia under DSM-5 are evidence of significant cognitive decline from a previous level of performance in one or more cognitive domains selected from: learning and memory; language; executive function; complex attention; perceptual-motor and social cognition. The cognitive deficits must interfere with independence in everyday activities. In addition, the cognitive deficits do not occur exclusively in the context of a delirium and are not better explained by another mental disorder (for example MDD or schizophrenia).

In addition to the primary symptom, subjects with neurodegenerative disorders display behavioural and psychiatric symptoms including agitation, aggression, depression, anxiety, apathy, psychosis and sleep-wake cycle disturbances.

Neurodegenerative and neurocognitive disorders are psychiatric disorders that may develop or persist due to dysfunction of the microbiota-gut-brain axis. Therefore, in preferred embodiments, the compositions of the invention are for use in treating or preventing neurodegenerative or neurocognitive disorders in a subject. In preferred embodiments, the neurodegenerative or neurocognitive disorder is Alzheimer's disease. In other embodiments, the neurodegenerative or neurocognitive disorder is selected from vascular dementias; mixed form Alzheimer's disease and vascular dementia; Lewy body disease; frontotemporal dementia; Parkinson's dementia; Creutzfeldt-Jakob disease; Huntington's disease, and Wernicke-Korsakoff syndrome.

In preferred embodiments, the compositions of the invention prevent, reduce or alleviate one or more of the symptoms of neurodegenerative or neurocognitive disorders in a subject In certain embodiments, the compositions of the invention prevent, reduce or alleviate the occurrence of cognitive decline in a subject. In certain embodiments, the compositions of the invention improve the level of performance of a subject with neurodegenerative or neurocognitive disorders in one or more cognitive domains selected from: learning and memory; language; executive function; complex attention; perceptual-motor and social cognition. In some embodiments, the compositions of the invention prevent, reduce or alleviate the occurrence of one or more behavioural and psychiatric symptoms associated with neurodegenerative or neurocognitive disorders selected from agitation, aggression, depression, anxiety, apathy, psychosis and sleep-wake cycle disturbances.

In certain embodiments, the compositions of the invention prevent, reduce or alleviate symptomatic disease by intervention in suspected pathogenic mechanisms at a preclinical stage. In certain embodiments, the compositions of the invention improve disease modification, with slowing or arrest of symptom progression. In some embodiments, the slowing or arrest of symptom progression correlates with evidence in delaying the underlying neuropathological process. In preferred embodiments, the compositions of the invention improve symptoms of neurodegenerative or neurocognitive disorders comprising enhanced cognitive and functional improvement. In preferred embodiments, the compositions of the invention improve the behavioural and psychiatric symptoms of dementia (BPSD). In preferred embodiments, the compositions of the invention improve the ability of a subject with neurodegenerative or neurocognitive disorder to undertake everyday activities.

In preferred embodiments, the compositions of the invention improve both cognition and functioning in a subject with Alzheimer's disease. In some embodiments, the composition of the invention improve the cognitive endpoint in a subject with Alzheimer's disease. In some embodiments, the compositions of the invention improve the functional endpoint in a subject with Alzheimer's disease. In preferred embodiments, the compositions of the invention improve the cognitive and functional endpoint in a subject with Alzheimer's disease. In yet further preferred embodiments, the compositions of the invention improve the overall clinical response (the global endpoint) in a subject with Alzheimer's disease.

In some embodiments, the compositions of the invention improve the symptoms of neurodegenerative or neurocognitive disorders according to a symptomatic or diagnostic test. In certain embodiments, the tests for assessing symptomatic improvement of Alzheimer's disease (and other neurodegenerative or neurocognitive disorders) are selected from objective cognitive, activities of daily living, global assessment of change, health related quality of life tests and tests assessing behavioural and psychiatric symptoms of neurodegenerative or neurocognitive disorders.

In certain embodiments, the objective cognitive tests for assessment of symptomatic improvement use the Alzheimer's disease Assessment Scale cognitive subscale (ADAS-cog) and the classic ADAS scale. In certain embodiments, symptomatic improvement of cognition is assessed using the Neurophysiological Test Battery for Use in Alzheimer's Disease (NTB).

In some embodiments, the global assessment of change test uses the Clinical Global Impression - Global Improvement (CGI-I) scale for assessing psychiatric and neurological disorders. In some embodiments, the global scale is the Clinician's Interview Based Impression of Change plus (CIBIC-plus). In some embodiments, the global scale is the Alzheimer's Disease Cooperative Study Unit Clinician's Global Impression of Change (ADCS-CGIC).

In certain embodiments, the health related quality of life measures are the Alzheimer's Disease-Related QOL (ADRQL) and the QOL-Alzheimer's Disease (QOL-AD).

In certain embodiments, the tests assessing behavioural and psychiatric symptoms of neurodegenerative or neurocognitive disorders are selected from the Behavioural pathology in Alzheimer's Disease Rating Scale (BEHAVE-AD); the Behavioural Rating Scale for Dementia (BRSD); the Neuropsychiatric Inventory (NPI); and the Cohen-Mansfield Agitation Inventory (CMAI).

In some embodiments, the compositions of the invention are particularly effective at preventing, reducing or alleviating neurodegenerative or neurocognitive disorders when used in combination with another therapy for treating neurodegenerative or neurocognitive disorders. In certain embodiments, such therapies include acetylcholinesterase inhibitors including donepezil (Aricept^{®}), galantamine (Razadyne^{®}) and rivastigmine (Exelon ^{®}), and memantine.

### - Parkinson's disease

Parkinson's disease is a common neurodegenerative disease neuropathologically characterised by degeneration of heterogeneous populations of neural cells (dopamine-producing cells). The clinical diagnosis of Parkinson's disease requires bradykinesia and at least one of the following core symptoms: resting tremor; muscle rigidity and postural reflex impairment. Other signs and symptoms that may be present or develop during the progression of the disease are autonomic disturbances (sialorrhoea, seborrhoea, constipation, micturition disturbances, sexual functioning, orthostatic hypotension, hyperhydrosis), sleep disturbances and disturbances in the sense of smell or sense of temperature. Parkinson's disease is a neurodegenerative disease that may develop or persist due to HDAC activity. For example, HDAC activity has been shown to regulate aggregation and deposition toxic intracellular proteinaceous filaments that are a hallmark of neurodegenerative diseases such as Parkinson's disease [59]. Inhibition of HDAC activity has been shown to reduce toxic protein misfolding events in Parkinson's disease models. In addition, Parkinson's disease is also a psychiatric disorder that may develop or persist due to dysfunction of the microbiota gut brain axis. Therefore, in preferred embodiments, the compositions of the invention are for use in treating or preventing Parkinson's disease in a subject. Depressive symptoms and cognitive dysfunction comorbidities develop in many Parkinson's disease patients, as well as neurocognitive disorders related to Lewy Bodies.

In further preferred embodiments, compositions of the invention are for use in a method of treating or preventing Parkinson's disease. Compositions of the invention may improve motor and cognitive functions in models of Parkinson's disease. Treatment with the compositions may modulate signalling in the central, autonomic and enteric nervous systems; may modulate the activity of the HPA axis pathway; may modulate neuroendocrine and/or neuroimmune pathways; and may modulate the levels of commensal metabolites, inflammatory markers and/or gastrointestinal permeability of a subject, all of which are implicated in the neuropathology of Parkinson's disease. In preferred embodiments, the invention provides a composition comprising a bacterial strain of the species *Anaerostipes hadrus* for use in a method of treating or preventing Parkinson's disease. Compositions using *Anaerostipes* may be particularly effective for treating Parkinson's disease. The composition may further comprise an organic acid.

In preferred embodiments, the compositions of the invention prevent, reduce or alleviate one or more of the symptoms of Parkinson's disease in a subject. In preferred embodiments, the compositions of the invention prevent, reduce or alleviate one or more core symptoms of Parkinson's disease in a subject. In certain embodiments, the compositions of the invention prevent, reduce or alleviate bradykinesia in a subject. In certain embodiments, the compositions of the invention prevent, reduce or alleviate resting tremor; muscle rigidity and/or postural reflex impairment in a subject. In certain embodiments, the compositions of the invention prevent, reduce or alleviate one or more symptoms associated with Parkinson's disease progression selected from autonomic disturbances (sialorrhoea, seborrhoea, constipation, micturition disturbances, sexual functioning, orthostatic hypotension, hyperhydrosis), sleep disturbances and disturbances in the sense of smell or sense of temperature.

In preferred embodiments, the compositions of the invention prevent, reduce or alleviate depressive symptoms comorbid with Parkinson's disease. In certain embodiments, the compositions of the invention improve verbal memory and/or executive functions. In certain embodiments, the compositions of the invention improve attention, working memory, verbal fluency and/or anxiety.

In other preferred embodiments, the compositions of the invention prevent, reduce or alleviate cognitive dysfunctions comorbid with Parkinson's disease.

In certain embodiments, the compositions of the invention prevent, reduce or alleviate hyperactivity or anxiety-like behaviour comorbid with Parkinson's disease. Mice models of Parkinson's disease have been shown to exhibit hyperactivity. Certain models have indicated that hyperactivity may be a consequence of imbalanced neurotransmitter levels in the brain or functional changes in other structures within the brain that precede degeneration of dopaminergic neurons. Thus, behavioural disturbances, such as hyperactivity, may be symptoms of Parkinson's disease that precede the onset of motor disturbances. The compositions of the invention have been shown to reduce hyperactivity in mice models of Parkinson's disease. Therefore, in certain embodiments, the compositions of the invention may be for use in the prevention of motor disturbances in Parkinson's disease. In certain embodiments, the compositions of the invention are for use in the treatment or prevention of behavioural disturbances associated with Parkinson's disease.

In certain embodiments, the compositions of the invention prevent, reduce or alleviate Parkinson's disease progression. In certain embodiments, the compositions of the invention prevent, reduce or alleviate later motor complications. In certain embodiments, the compositions of the invention prevent, reduce or alleviate late motor fluctuations. In certain embodiments, the compositions of the invention prevent, reduce or alleviate neuronal loss. In certain embodiments, the compositions of the invention improve symptoms of Parkinson's disease dementia (PDD). In certain embodiments, the compositions of the invention prevent, reduce or alleviate impairment of executive function, attention and/or working memory. In certain embodiments, the compositions of the invention improve dopaminergic neurotransmission. In certain embodiments, the compositions of the invention prevent, reduce or alleviate impaired dopaminergic neurotransmission.

In some embodiments, the compositions of the invention improve the symptoms of Parkinson's disease according to a symptomatic or diagnostic scale. In certain embodiments, the tests for assessing symptomatic improvement of motor function in Parkinson's disease is the Unified Parkinson's Disease Rating Scale. In particular, UPDRS II considers the activity of daily life and UPDRS III considers motor-examination.

In some embodiments, the compositions of the invention improve the symptoms associated with PDD according to a symptomatic or diagnostic test and/or scale. In certain embodiments, the test or scale is selected from the Hopkins Verbal Learning Test - Revised (HVLT-R); the Delis-Kaplan Executive Function System (D-KEFS) Color-Word Interference Test; the Hamilton Depression Rating Scale (HAM-D 17; depression); the Hamilton Anxiety Rating Scale (HAM-A; anxiety) and the Unified Parkinson's Disease Rating Scale (UPDRS; PD symptom severity).

In some embodiments, the compositions of the invention improve the Clinical Global Impression - Global Improvement (CGI-I) scale for assessing psychiatric and neurological disorders. In some embodiments, the compositions of the invention display a positive effect on global social and occupational impairment of the subject with Parkinson's disease.

In certain embodiments, the compositions of the invention are for use in treating or preventing neurological disorders such as Parkinson's disease in a subject wherein said use involves reducing or preventing loss of dopaminergic cells in the substantia nigra. In certain embodiments, the compositions of the invention are for use in treating or preventing neurological disorders such as Parkinson's disease in a subject wherein said use involves reducing or preventing the degeneration of dopaminergic neurons in the substantia nigra pars compacta. In certain embodiments, the compositions of the invention are for use in treating or preventing neurological disorders such as Parkinson's disease in a subject wherein said use involves reducing or preventing the degeneration of dopaminergic neurons in the substantia nigra pars compacta and the consequent loss of their projecting nerve fibers in the striatum. In certain embodiments, the compositions of the invention are for use in treating or preventing neurological disorders such as Parkinson's disease in a subject wherein said use involves reducing or preventing loss of nigrostriatal dopaminergic neurons.

In certain embodiments, the compositions of the invention are for use in treating or preventing neurological disorders such as Parkinson's disease in a subject wherein said use involves increasing dopamine levels. In certain embodiments, the compositions of the invention are for use in treating or preventing neurological disorders such as Parkinson's disease in a subject wherein said use involves increasing DOPAC levels. In certain embodiments, the compositions of the invention are for use in treating or preventing neurological disorders such as Parkinson's disease in a subject wherein said use involves increasing dopamine and DOPAC levels. In certain embodiments, the dopamine and/or DOPAC levels are increased in the striatum.

The examples demonstrate that the compositions of the invention activate MAP2 (Microtubule - associated protein 2) activation. MAP2 is a gene associated with neuronal differentiation of MAP2 and is thought to be essential for microtubule formation in neuritogenesis, so compositions of the invention may be particularly useful for treating neurodegenerative diseases. In some embodiments, the compositions of the invention are for use in treating a neurodegenerative disease, such as Alzheimer's disease or Parkinson's disease, by activating or increasing the levels of MAP2. Moreover, as MAP2 promotes neurite outgrowth, which play a major role in re-networking of damaged neurons and synaptogenesis, MAP2 expression might go beyond being a marker of neuronal differentiation and indicate "neuronal re-wiring" associated with the therapeutic outcome of neuropathological disease.

The examples demonstrate that the compositions of the invention modulate the expression of a number of proteins in the brain. In particular, compositions of the invention increase the expression of BDNF in the hippocampus and the prefrontal cortex. BDNF is essential for adult synaptic plasticity and the formation of memories and a decrease in the levels of BDNF is observed in Alzheimer's and Huntington's patients. The compositions of the invention are therefore particularly useful for the treatment of Alzheimer's and Huntington's disease. In certain embodiments, compositions of the invention increase expression of BDNF in the brain.

In some embodiments, the compositions of the invention are particularly effective at preventing, reducing or alleviating neurocognitive disorders when used in combination with another therapy for treating neurocognitive disorders. In certain embodiments, such therapies include dopamine agonists (including L-Dopa+); monoamine oxidase inhibitors, catecholamine-O-methyl transferase inhibitors, anticholinergics and glutamate modulators.

### Other central nervous system disorders

In preferred embodiments, the compositions disclosed herein (compositions of the invention) are for use in treating or preventing a central nervous system disorder associated with dysfunction of the microbiota-gut-brain axis. In addition to the embodiments above, the compositions disclosed herein (compositions of the invention) are for use in treating or preventing psychosis; chronic fatigue syndrome (myalgic encephalomyelitis) and/or chronic pain. In further embodiments, the compositions disclosed herein (compositions of the invention) may be useful for treating or preventing motor neuron disease; Huntington's disease; Guillain-Barre syndrome and/or meningitis.

### Huntington's disease

Huntington's disease is an inherited brain condition, caused by an inherited faulty gene, which damages certain nerve cells in the brain. This brain damage gets progressively worse over time and can affect movement, cognition (perception, awareness, thinking, judgement) and behaviour. Early features of the disease can include personality changes, mood swings, fidgety movements, irritability and altered behaviour.

In certain embodiments, the compositions disclosed herein (compositions of the invention) are for use in treating or preventing Huntington's disease. In certain embodiments, the compositions disclosed herein (compositions of the invention) manage the symptoms of Huntington's disease, such as irritability or excessive movement. In certain embodiments, the compositions disclosed herein (compositions of the invention) treat the depression associated with Huntington's disease and/or improve symptoms such as social withdrawal, lack or interest and sleep disturbance. In certain embodiments, the compositions disclosed herein (compositions of the invention) improve memory and ability to concentrate on tasks. In certain embodiments, the compositions disclosed herein (compositions of the invention) treat disabling abnormal movements. In certain embodiments, the compositions disclosed herein (compositions of the invention) treat behavioural problems, antisocial behaviour, irritability and psychosis associated with Huntington's disease. In certain embodiments, the compositions disclosed herein (compositions of the invention) induce neuroprotection and prevent nerve damage. In certain embodiments, the compositions disclosed herein (compositions of the invention) increase the levels of dopamine and/or the levels of dopamine-containing cells.

### Neurochemical factors, neuropeptides and neurotransmitters and the microbiota-gut-brain axis

As outlined above, the microbiota-gut-brain axis is modulated by a number of different physiological systems. The microbiota-gut-brain axis is modulated by a number of signalling molecules. Alterations in the levels of these signalling molecules results in defects in central nervous system development and/or functionality. Indeed, many of the molecules disclosed in this section have been implicated in the functionality of the microbiota-gut-brain axis and the pathogenesis of central nervous system disorders or conditions ([20], [24], [63], [60]). The experiments performed by the inventors indicate that behavioural changes can be triggered by administration of a bacterial strain of the genus *Anaerostipes,* such as an *Anaerostipes hadrus* strain and of *Eubacterium* or *Faecalicatena* strains. This effect may be mediated by an effect on levels of the signalling molecules, in particular those listed in this section. These alterations may be responsible for the therapeutic benefits associated with *Anaerostipes*; or *Eubacterium* or *Faecalicatena* strains. Accordingly, due to the fact that the central nervous system disorders and conditions disclosed herein display a similar fundamental biochemical and physiological pathogenesis (i.e. via the microbiota-gut-brain axis), a similar therapeutic benefit of *Anaerostipes;* or *Eubacterium* or *Faecalicatena* strains may be also achieved for these disorders and conditions. Administration of *Eubacterium* or *Faecalicatena* strains may be particularly effective for triggering behavioural changes associated with central nervous system disorders or conditions.

The signalling of the microbiota-gut-brain axis is modulated by levels of neurochemical factors, neuropeptides and neurotransmitters. Accordingly, in certain embodiments, the compositions disclosed herein (compositions of the invention) modulates levels of neurochemical factors, neuropeptides and neurotransmitters. Accordingly, in certain preferred embodiments, the compositions disclosed herein (compositions of the invention) directly alter CNS biochemistry. In preferred embodiments, the compositions disclosed herein (compositions of the invention) modulate the levels of brain-derived neurotrophic factor (BDNF). In certain embodiments, the compositions disclosed herein (compositions of the invention) modulate the levels of monoamines. In certain embodiments, the monoamines are serotonin (5-hydroxytryptamine (5-HT)), dopamine, norepinephrine and/or epinephrine. In certain embodiments, the monoamines are catecholamines. In certain embodiments, the catecholamines are dopamine, norepinephrine and epinephrine. In certain embodiments, the monoamines are tryptamines. In certain embodiments, the tryptamines are serotonin and melatonin. In certain embodiments, the compositions disclosed herein (compositions of the invention) modulate the levels of acetylcholine. The levels of BDNF, monoamines or acetylcholine can be measured relative to the levels of BDNF, monoamines or acetylcholine levels observed in the patient before treatment or in a healthy individual.

In certain preferred embodiments, the compositions disclosed herein (compositions of the invention) modulate the levels of oxytoxin. Oxytocin is associated with emotional, social, cognitive and neuroendocrine physiologies as well as autoregulation. In particular, oxytocin release is involved in anxiolysis; positive mood; maternal behaviour, pair bonding; sexual behaviour; social memory; olfactory memory; anorexiant effects; attenuation of the HPA axis response to stress; autoexcitation during birth and suckling as well as other physiological and psychological processes. In certain embodiments, the compositions disclosed herein (compositions of the invention) increase the levels of oxytocin. In certain embodiments, the compositions disclosed herein (compositions of the invention) decrease the levels of oxytocin. In certain embodiments, the compositions disclosed herein (compositions of the invention) increase or decrease oxytocin signalling. In certain embodiments, the compositions disclosed herein (compositions of the invention) modulate the levels of oxytocin receptors. In certain embodiments, the compositions disclosed herein (compositions of the invention) modulate the flux of calcium ions into or out of neuronal, muscle and gastrointestinal cells. In preferred embodiments, the compositions disclosed herein (compositions of the invention) treat and prevent neurodevelopmental and neuropsychiatric disorders and diseases associated with the microbiota-gut-brain axis by modulating the levels of oxytocin, The level of oxytocin can be measured relative to the oxytocin levels observed in the patient before treatment or in a healthy individual.

In certain embodiments, the compositions disclosed herein (compositions of the invention) modulate the levels of brain monoamines and metabolites thereof. In preferred embodiments, the monoamine is serotonin. In certain embodiments, the compositions disclosed herein (compositions of the invention) modulate the serotonergic and/or kynurenine routes of tryptophan metabolism. In certain embodiments, the compositions disclosed herein (compositions of the invention) modulate the levels of serotonin metabolites, such as 5-Hydroxyindoleacetic acid (5-HIAA). In certain embodiments, the compositions disclosed herein (compositions of the invention) modulate the levels of dopamine metabolites, such as Homovanillic acid (HVA). Modulation of these neurotransmitters and neurochemical factors is useful for treating stress, depression and anxiety-related disorders. The level of brain monoamines can be measured relative to the brain monoamines levels observed in the patient before treatment or in a healthy individual.

The signalling of the microbiota-gut-brain axis is modulated by levels of γ-aminobutyric acid (GABA). Accordingly, in preferred embodiments, the compositions disclosed herein (compositions of the invention) modulate the levels of GABA. GABA is an inhibitory neurotransmitter that reduces neuronal excitability. In certain embodiments, the compositions disclosed herein (compositions of the invention) increase the levels of GABA. In certain embodiments, the compositions disclosed herein (compositions of the invention) decrease the levels of GABA. In certain embodiments, the compositions disclosed herein (compositions of the invention) alter GABAergic neurotransmission. In certain embodiments, the compositions disclosed herein (compositions of the invention) modulate the level of GABA transcription in different regions of the central nervous system. In certain embodiments, the commensal derived GABA crosses the blood-brain barrier and affects neurotransmission directly. In certain embodiments, the compositions disclosed herein (compositions of the invention) lead to a reduction of GABA in the hippocampus, amygdala and/or locus coeruleus. In certain embodiments, the compositions disclosed herein (compositions of the invention) lead to an increase of GABA in cortical regions. The level of GABA can be measured relative to the GABA levels observed in the patient before treatment or in a healthy individual.

The levels of neuroactive molecules, such as serotonin, melatonin, GABA, histamines and acetylcholine are linked to the pathophysiology of central nervous system diseases such as dementia, Alzheimer's disease and Huntington's disease. Thus, the compositions of the invention may be used for treating or preventing a disease mediated by GABA, for example epilepsy.

The signalling of the microbiota-gut-brain axis is modulated by levels of histamines. Accordingly, in certain embodiments, the compositions disclosed herein (compositions of the invention) modulate the levels of histamines. In certain embodiments, the histamines has an immunoregulatory effect. In certain embodiments, histamine levels enable translocation of bacteria from the lumen into systemic circulation. Therefore, in some embodiments, the compositions disclosed herein (compositions of the invention) alter gastrointestinal tract permeability and/or barrier function. In certain other embodiments, the histamine acts as a neurotransmitter linked to central processes.

The signalling of the microbiota-gut-brain axis is modulated by the HPA axis. Accordingly, in certain embodiments, the compositions disclosed herein (compositions of the invention) modulate HPA activity. In certain embodiments, the compositions disclosed herein (compositions of the invention) attenuate the HPA stress response. In certain preferred embodiments, the compositions disclosed herein (compositions of the invention) modulate inflammatory responses associated with HPA activity, In certain embodiments, the compositions disclosed herein (compositions of the invention) modulate the levels of glucocorticoids. In certain preferred embodiments, the compositions disclosed herein (compositions of the invention) modulate the levels of corticosterone and adrenaline. In certain embodiments, the compositions disclosed herein (compositions of the invention) modulate the levels of corticotrophin-releasing factor and/or vasopressin. In certain embodiments, the compositions disclosed herein (compositions of the invention) modulate the levels of vasopressin and/or other neurohypophysial or antidiuretic hormones. Alterations in HPA axis activity are associated with anxiety and stress disorders.

The signalling of the microbiota-gut-brain axis is modulated by alterations in the immune response and inflammatory factors and markers. Accordingly, in certain embodiments, the compositions disclosed herein (compositions of the invention) may modulate the immune response. In certain embodiments, the compositions disclosed herein (compositions of the invention) modulate the systemic levels of circulating neuroimmune signalling molecules. In certain preferred embodiments, the compositions disclosed herein (compositions of the invention) modulate pro-inflammatory cytokine production and inflammation. In certain embodiments, the compositions disclosed herein (compositions of the invention) modulate the inflammatory state. In certain embodiments, the compositions disclosed herein (compositions of the invention) modulate the splenocyte proliferative response. In certain embodiments, the compositions disclosed herein (compositions of the invention) modulate the systemic and/or plasma levels of C-reactive protein; IL-1 family cytokines; IL-1β; IL-2; IL-4; IL-6; IL-8; IL-10; IL-12p40; IL-17; IL-17A; IL-21; IL-23; TNF- α and IFN-γ. In some embodiments the compositions disclosed herein (compositions of the invention) module the levels of anti-inflammatory cytokines, for example IL-10. In preferred embodiments, the compositions disclosed herein (compositions of the invention) increase the levels of IL-10. In some embodiments, the compositions disclosed herein (compositions of the invention) modulate the levels of TNF-α. In preferred embodiments, the compositions disclosed herein (compositions of the invention) modulate the levels of IFN-γ. In some embodiments, the compositions disclosed herein (compositions of the invention) modulate the IFN-γ:IL-10 ratio. In certain preferred embodiments, the compositions disclosed herein (compositions of the invention) decrease the IFN-y:IL-10 ratio. In preferred embodiments, the compositions disclosed herein (compositions of the invention) decrease the levels of the pro-inflammatory cytokines TNF-α and IFN-γ. In preferred embodiments, the compositions disclosed herein (compositions of the invention) (e.g. comprising an *Anaerostipes hadrus* strain) decreases the levels of TNF-α and/or IL-1β. In preferred embodiments, the compositions disclosed herein (compositions of the invention) (e.g. comprising an *Anaerostipes hadrus* strain) increase the levels of the IL-6. Increased circulating levels of cytokines are closely associated with various neuropsychiatric disorders, including depression, anxiety, schizophrenia and ASD. Evidence of inflammatory state alteration is highlighted in disorders such as schizophrenia, major depressive disorder and bipolar disorder. The level of cytokines can be measured relative to the cytokines levels observed in the patient before treatment or in a healthy individual.

In certain embodiments, the compositions disclosed herein (compositions of the invention) modulates the levels of tolerance-mediating dendritic cells and reciprocally regulate pro and anti-inflammatory cytokine responses. In certain embodiments, the compositions disclosed herein (compositions of the invention) decrease the systemic level of myeloperoxidase (a marker for inflammation and oxidation).Therapeutic modulators of the immune system and of inflammatory responses are useful for treating autism spectrum disorders and mood disorders.

In certain embodiments, the compositions disclosed herein (compositions of the invention) modulate the immune response to an infection or vaccination. In certain embodiments, the compositions disclosed herein (compositions of the invention) modulate the level of inflammation in response to infection or vaccination. In certain preferred embodiments, the compositions disclosed herein (compositions of the invention) modulate maternal immune activation in response to an infection or vaccination during pregnancy. Accordingly, the compositions disclosed herein (compositions of the invention) can be administered during pregnancy in order to treat or prevent a central nervous system disorder in the offspring.

The signalling of the microbiota-gut-brain axis is modulated by levels commensal metabolites. Accordingly, in certain embodiments, the compositions disclosed herein (compositions of the invention) modulate the systemic levels of microbiota metabolites. In certain preferred embodiments, the compositions disclosed herein (compositions of the invention) modulate the level of short chain fatty acids (SCFAs). In certain embodiments the level of SCFAs is increased or decreased. In some embodiments, the SCFA is butyric acid (BA) (or butyrate). In some embodiments, the SCFA is propionic acid (PPA). In some embodiments, the SCFA is acetic acid. In some embodiments, the compositions disclosed herein (compositions of the invention) (e.g. comprising an *Anaeroslipes hadrus gnavus* strain) increases the levels of one, two, three, four, five or all of the following: acetate, propionate, valerate, butyrate, isobutyrate and isovalerate. In certain embodiments, the compositions disclosed herein (compositions of the invention) modulate the ability of SCFAs to cross the blood-brain barrier. The level of SCFAs can be measured relative to the SCFAs levels observed in the patient before treatment or in a healthy individual.

In certain embodiments, the compositions disclosed herein (compositions of the invention) modulate the level of Polysaccharide A (PSA). In certain embodiments, the compositions disclosed herein (compositions of the invention) modulate the levels of the potent pro-inflammatory endotoxin lipopolysaccharide (LPS). LPS leads to the production of inflammatory cytokines that alter physiological brain activity and modulate neuropeptide synthesis. LPS has an important influence on the modulation of the CNS, increasing the activity of areas devoted to the control of emotions (e.g. the amygdala). In certain embodiments, the compositions disclosed herein (compositions of the invention) modulate the level of tryptophan and/or its metabolites. In certain embodiments, the compositions disclosed herein (compositions of the invention) modulate the levels of 4-ethylphenylsulphate (4EPS; a uremic toxic associated with ASD-related behavioural abnormalities). In preferred embodiments, the compositions disclosed herein (compositions of the invention) decrease the levels of 4-ethylphenylsulphate in a subject. The signals generated by the stimulation of neuronal signalling pathways caused by intraluminal gut stimuli strongly modulate brain activity, including pain perception, immune-response modulation, emotional control and other homeostatic functions. Accordingly, a composition able to modulate levels of these factors would have broad therapeutic applications for treating or preventing CNS disorders.

The compositions disclosed herein may modulate tight junction proteins and so are useful, for example, in the treatment or prevention of disorders or conditions associated with dysregulated or otherwise abnormal expression of tight junction proteins and functional markers in the gut. In some embodiments, the compositions disclosed herein (e.g. comprising an *Anaerostipes hadrus* strain) may modulate one, two or all three of IDOl, TPH1 and TJP1 gene expression. In some embodiments, the compositions disclosed herein (e.g. comprising an *Anaerostipes hadrus* strain) may modulate one, two or all three of IDOl, TPH1 and TJP1 gene expression. Increases in TPH1 correlate with improved serotonin production and so may be useful for treating depression and related conditions.

The signalling of the microbiota-gut-brain axis is modulated by levels gastrointestinal permeability. Accordingly, in some embodiments, the compositions disclosed herein (compositions of the invention) alter the integrity of the gastrointestinal tract epithelium. In certain embodiments, the compositions disclosed herein (compositions of the invention) modulate the permeability of the gastrointestinal tract. In certain embodiments, the compositions disclosed herein (compositions of the invention) modulate the barrier function and integrity of the gastrointestinal tract. In certain embodiments, the compositions disclosed herein (compositions of the invention) modulate gastrointestinal tract motility. In certain embodiments, the compositions disclosed herein (compositions of the invention) modulate the translocation of commensal metabolites and inflammatory signalling molecules into the bloodstream from the gastrointestinal tract lumen.

The signalling of the microbiota-gut-brain axis is modulated by microbiome composition in the gastrointestinal tract. Accordingly, in certain embodiments, the compositions disclosed herein (compositions of the invention) modulates the microbiome composition of the gastrointestinal tract. In certain embodiments, the compositions disclosed herein (compositions of the invention) prevents microbiome dysbiosis and associated increases in toxic metabolites (e.g. LPS). In certain embodiments, the compositions disclosed herein (compositions of the invention) modulate the levels of *Clostridium* in the gastrointestinal tract. In preferred embodiments, the compositions disclosed herein (compositions of the invention) reduce the level of *Clostridium* in the gastrointestinal tract. In certain embodiments, the compositions disclosed herein (compositions of the invention) reduce the levels of *Campylobacter jejuni.* In certain embodiments, the compositions disclosed herein (compositions of the invention) modulate the proliferation of harmful anaerobic bacteria and the production of neurotoxins produced by these bacteria. In certain embodiments, the compositions disclosed herein (compositions of the invention) modulate the microbiome levels of *Lactobacillus* and/or *Bifidobacterium.* In certain embodiments, the compositions disclosed herein (compositions of the invention) modulate the microbiome levels of *Sutterella*, *Prevotella, Ruminoccucs* genera and/or the Alcaligenaceae family. In certain embodiments, the compositions disclosed herein (compositions of the invention) increase the level of *Lactobacillus plantarum* and/or *Saccharomyces boulardii.*

In certain embodiments, the compositions disclosed herein (compositions of the invention) prevent the dysregulation of the composition of the microbiome by extensive antibiotic use. In certain preferred embodiments, the compositions disclosed herein (compositions of the invention) maintain a functional maternal microbiome composition upon administration of antibiotics during pregnancy. Accordingly, the compositions disclosed herein (compositions of the invention) can be administered during pregnancy in order to treat or prevent a central nervous system disorder in the offspring.

Modulation of the microbiome has been shown to be effective at improving psychiatric disorder-related behaviours, including anxiety, depression, autism spectrum disorder, obsessive-compulsive disorder and memory abilities (including spatial and non-spatial memory), as well as other CNS-related disorders including Parkinson's disease. Certain studies have suggested that probiotics can reduce psychological stress, somatisation, depression and anger-hostility. The levels of *Lactobacillus* are associated with depression and have been implicated in pain signalling associated with gastrointestinal discomfort.

In certain embodiments, the compositions disclosed herein (compositions of the invention) prevent, reduce or alleviate at least one of the behavioural symptoms associated with a central nervous system disorder described herein. In preferred embodiments, the compositions disclosed herein (compositions of the invention) improve the overall clinical response in a subject.

In preferred embodiments, the compositions disclosed herein (compositions of the invention) prevent, reduce or alleviate stereotyped, repetitive behaviour in a subject. In preferred embodiments, the compositions disclosed herein (compositions of the invention) prevent, reduce or alleviate the occurrence of unusually restrictive behaviours and/or interests. In certain embodiments, the compositions disclosed herein (compositions of the invention) prevent, reduce or alleviate recurrent obsessions and/or compulsions in a subject. In preferred embodiments, the compositions disclosed herein (compositions of the invention) prevent, reduce or alleviate deficits in social behaviour in a subject. In preferred embodiments, the compositions disclosed herein (compositions of the invention) prevent, reduce or alleviate avoidance behaviour in a subject. In preferred embodiments, the compositions disclosed herein (compositions of the invention) prevent, reduce or alleviate deficits in communication behaviour in a subject.

In preferred embodiments, the compositions disclosed herein (compositions of the invention) prevent, reduce or alleviate negative alterations in cognitions and mood in a subject. In preferred embodiments, the compositions disclosed herein (compositions of the invention) prevent, reduce or alleviate anxiety-related behaviour in a subject. In preferred embodiments, the compositions disclosed herein (compositions of the invention) prevent, reduce or alleviate stress-related behaviour in a subject. In preferred embodiments, the compositions disclosed herein (compositions of the invention) prevent, reduce or alleviate depression-related behaviour in a subject. In preferred embodiments, the compositions disclosed herein (compositions of the invention) prevent, reduce or alleviate aggressive behaviour in a subject. In preferred embodiments, the compositions disclosed herein (compositions of the invention) prevent, reduce or alleviate the occurrence of an abnormally and persistently elevated, expansive, or irritable mood.

In preferred embodiments, the compositions disclosed herein (compositions of the invention) prevent, reduce or alleviate intrusive thoughts in a subject. In preferred embodiments, the compositions disclosed herein (compositions of the invention) prevent alterations in arousal and reactivity in a subject. In preferred embodiments, the compositions disclosed herein (compositions of the invention) prevent, reduce or alleviate delusions, hallucinations, disorganised speech, and disorganised or catatonic behaviours in a subject. In preferred embodiments, the compositions disclosed herein (compositions of the invention) prevent, reduce or alleviate affective flattening, restriction in the fluency and productivity of thought and speech and in the initiation of goal directed behaviour in a subject. In preferred embodiments, the compositions disclosed herein (compositions of the invention) prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger.

In preferred embodiments, the compositions disclosed herein (compositions of the invention) improve spatial and/or non-spatial memory deficits in a subject. In preferred embodiments, the compositions disclosed herein (compositions of the invention) improve both cognition and functioning in a subject. In preferred embodiments, the compositions disclosed herein (compositions of the invention) improve locomotor activity in a subject. In preferred embodiments, the compositions disclosed herein (compositions of the invention) prevent, reduce or alleviate bradykinesia in a subject. In preferred embodiments, the compositions disclosed herein (compositions of the invention) prevent, reduce or alleviate resting tremor; muscle rigidity and/or postural reflex impairment in a subject.

In preferred embodiments, the compositions disclosed herein (compositions of the invention) prevent, reduce or alleviate at least one comorbidity associated with a CNS disorder disclosed herein.

In preferred embodiments, the compositions disclosed herein (compositions of the invention) improve the scores of a subject on at least one of the symptomatic and/or diagnostic scales for CNS disorders described herein. In certain other embodiments, the symptomatic and/or diagnostic scale is selected from the General Health Questionnaire (GHQ); the Depression Anxiety and Stress Scale (DASS); the Leiden Index of Depression Sensitivity-Revised (LEIDS-r); the Positive and Negative Symptom Scale (PANSS); the State-Trait Anxiety Inventory (STAI); the Development Behavior Checklist (DBC); the Beck Depression Inventory (BDI); the Beck Anxiety Inventory (BAI); the Hopkins Symptom Checklist (HSCL-90); the Hospital Anxiety and Depression Scale (HADS); the Perceived Stress Scale (PSS); the Coping Checklist (CCL) (also used to counter the stress of daily life); and the questionnaire-based Profile of Mood State (POMS).

In certain embodiments, the compositions disclosed herein (compositions of the invention) may improve the symptomatic and/or diagnostic scale when assessing therapeutic efficacy in other animal models of CNS disorders known to a person skilled in the art. In addition to the behavioural assays disclosed in the examples, the compositions disclosed herein (compositions of the invention) may improve reciprocal social interactions; olfactory communication; ultrasonic vocalisation; motor stereotypes (such as circling and vertical jumping), repetitive behaviour such as self-grooming and diffing; and perseverance in spatial tasks.

In addition, the compositions disclosed herein (compositions of the invention) will be useful in treating and/or preventing CNS disorders in other animal models of CNS disorders. Other mouse models include inbred mice strains (including BALB/cJ and C58/J) and also genetically modified mice strains (including *NEUREXIN1*, *NEUROLIGIN3, NEUROLIGIN4, SHANK2, SHANK3, CNTNAP2*, *Tsc1*/*2* and *Fmr1* gene mutant mice strains).

In certain embodiments, the compositions disclosed herein (compositions of the invention) improve social behaviour of a subject. In preferred embodiments, the compositions disclosed herein (compositions of the invention) improve the recognition of social novelty in a subject. In preferred embodiments, the compositions disclosed herein (compositions of the invention) improve the ability to discriminate between familiar and novel objects and familiar and novel subjects. In preferred embodiments, the composition of the invention improve ability to recognise other subjects.

In certain embodiments, the composition disclosed herein may improve depressive or depressive-like behaviour of a subject. In certain embodiments, the compositions disclosed herein (compositions of the invention) improve learned helplessness in a subject.

In certain embodiments, the compositions disclosed herein regulate plasma levels of amino acids. In certain embodiments, the compositions disclosed herein regulate the biosynthesis or catabolism of amino acids. In preferred embodiments, the compositions disclosed herein regulate plasma levels of proline. In preferred embodiments, the compositions disclosed herein reduce the plasma levels of proline. Elevated proline is known to negatively affect brain function by an increase in dopamine in the prefrontal cortex [61]. In addition, proline is considered to be a neurotransmitter that modulates glutamatergic neurotransmission in the hippocampus, and neurotransmission elsewhere in the brain Accordingly, proline has been implicated in CNS disorders and psychiatric disorders, in particular psychosis. In preferred embodiments, the reduction in plasma levels of proline treats or prevents CNS disorders, in particular, ADHD, OCD, mood disorders, autism spectrum disorder, psychosis and schizophrenia.

In certain embodiments, the compositions disclosed herein prevent, reduce or alleviate the symptoms of psychiatric disorders, for example schizophrenia and bipolar disorder, associated with 22q11.2 deletion syndrome (22q11DS) [65]. In certain embodiments, the compositions disclosed herein improve the social behavioural and social cognitive problems in subjects with 22q11DS. In preferred embodiments, the compositions disclosed herein modulate the associated cognitive and behavioural outcomes in 22q11DS subjects. In preferred embodiments, the modulation of these outcomes is a consequence of reduced plasma levels of proline. In certain embodiments, the compositions disclosed herein modulate the activity of proline hydrogenase.

In certain embodiments, the compositions disclosed herein modulate the levels of NMDA receptors and/or the subunits thereof. In preferred embodiments, the compositions disclosed herein modulate the levels of the NMDA receptor 2B. In certain embodiments, the compositions disclosed herein increase the levels of the NMDA receptor 2B. In preferred embodiments, the compositions disclosed herein decrease the levels of the NMDA receptor 2B. Dysregulation of NMDA receptors have been associated with CNS disorders, in particular ASD and schizophrenia. There have been suggestions that NMDA receptor antagonists may be effective in treating ASD [62]. In addition, suppression of NMDA receptor function has been demonstrated to improve social deficits and reduce repetitive behaviour in valproic acid induced models of ASD [63]. In certain embodiments, the compositions disclosed herein cause hypofunction of the NMDA receptor 2B. In certain embodiments, the compositions disclosed herein cause hyperfunction of the NMDA receptor 2B. In certain embodiments, the compositions disclosed herein prevent, reduce or alleviate the symptoms of CNS disorders, for example ASD or schizophrenia as a consequence of the modulation of NMDA receptor 2B activity. In preferred embodiments, the compositions disclosed hereins suppress NMDA receptor activity and reduce social deficits and stereotypical behaviour in subjects with CNS disorders.

In certain embodiments, the compositions disclosed herein modulate the levels of BDNF. In preferred embodiments, the compositions disclosed herein reduce the levels of BDNF. In certain embodiments, the reduction in BDNF is localised to the amygdalar. Meta-analyses of ASD populations have shown that higher levels of BDNF are detected in ASD subjects compared to controls [64]. In preferred embodiments, the compositions disclosed herein prevent, reduce or alleviate the symptoms of CNS disorders, in particular ASD, as a consequence of the reduction in levels of BDNF. Altered levels of BDNF have been associated with a number of neurodevelopmental disorders, as well as psychosis and schizophrenia. In certain embodiments, the compositions disclosed herein modulate levels of BDNF in order to prevent, reduce or alleviate the symptoms of neurodevelopmental and psychiatric disorders.

In preferred embodiments, the compositions disclosed herein are for use in treating or preventing a central nervous system disorder associated with dysfunction of the microbiota-gut-brain axis. In addition to the embodiments above, the compositions disclosed herein are for use in treating or preventing psychosis; chronic fatigue syndrome (myalgic encephalomyelitis) and/or chronic pain. In further embodiments, the compositions disclosed herein may be useful for treating or preventing motor neuron disease; Huntington's disease; Guillain-Barre syndrome and/or meningitis.

Huntington's disease is an inherited brain condition, caused by an inherited faulty gene, which damages certain nerve cells in the brain. This brain damage gets progressively worse over time and can affect movement, cognition (perception, awareness, thinking, judgement) and behaviour. Early features of the disease can include personality changes, mood swings, fidgety movements, irritability and altered behaviour.

In certain embodiments, the compositions disclosed herein are for use in treating or preventing Huntington's disease. In certain embodiments, the compositions disclosed herein manage the symptoms of Huntington's disease, such as irritability or excessive movement. In certain embodiments, the compositions disclosed herein treat the depression associated with Huntington's disease and/or improve symptoms such as social withdrawal, lack or interest and sleep disturbance. In certain embodiments, the compositions disclosed herein improve memory and ability to concentrate on tasks. In certain embodiments, the compositions disclosed herein treat disabling abnormal movements. In certain embodiments, the compositions disclosed herein treat behavioural problems, antisocial behaviour, irritability and psychosis associated with Huntington's disease. In certain embodiments, the compositions disclosed herein induce neuroprotection and prevent nerve damage. In certain embodiments, the compositions disclosed herein increase the levels of dopamine and/or the levels of dopamine-containing cells.

### Brain injury

The examples demonstrate that the compositions of the invention are neuroprotective and have HDAC inhibitory activity. HDAC2 is a crucial target for functional recovery from stroke [65] and HDAC inhibition can prevent white matter injury [66], so the compositions of the invention may be useful in the treatment of brain injury.

In certain embodiments, the compositions of the invention are for use in treating brain injury. In some embodiments, the brain injury is a traumatic brain injury. In some embodiments, the brain injury is an acquired brain injury. In some embodiments, the compositions of the invention are for use in treating brain injury resulting from trauma. In some embodiments, the compositions of the invention are for use in treating brain injury resulting from a tumour. In some embodiments, the compositions of the invention are for use in treating brain injury resulting from a stroke. In some embodiments, the compositions of the invention are for use in treating brain injury resulting from a brain haemorrhage, In some embodiments, the compositions of the invention are for use in treating brain injury resulting from encephalitis. In some embodiments, the compositions of the invention are for use in treating brain injury resulting from cerebral hypoxia. In some embodiments, the compositions of the invention are for use in treating brain injury resulting from cerebral anoxia.

In preferred embodiments, the compositions of the invention are for use in treating stroke. The effects shown in the examples are particularly relevant to the treatment of stroke. Stroke occurs when blood flow to at least a part of the brain is interrupted. Without an adequate supply of blood to provide oxygen and nutrients to the brain tissue and to remove waste products from the brain tissue, brain cells rapidly begin to die. The symptoms of stroke are dependent on the region of the brain which is affected by the inadequate blood flow. Symptoms include paralysis, numbness or weakness of the muscles, loss of balance, dizziness, sudden severe headaches, speech impairment, loss of memory, loss of reasoning ability, sudden confusion, vision impairment, coma or even death. A stroke is also referred to as a brain attack or a cerebrovascular accident (CVA). The symptoms of stroke may be brief if adequate blood flow is restored within a short period of time. However, if inadequate blood flow continues for a significant period of time, the symptoms can be permanent.

In some embodiments, the stroke is cerebral ischemia. Cerebral ischemia results when there is insufficient blood flow to the tissues of the brain to meet metabolic demand. In some embodiments, the cerebral ischemia is focal cerebral ischemia, i.e. confined to a specific region of the brain. In some embodiments the cerebral ischemia is global cerebral ischemia, i.e. encompassing a wide area of the brain tissue. Focal cerebral ischemia commonly occurs when a cerebral vessel has become blocked, either partially or completely, reducing the flow of blood to a specific region of the brain. In some embodiments the focal cerebral ischemia is ischemic stroke. In some embodiments, the ischemic stroke is thrombotic, i.e. caused by a thrombus or blood clot, which develops in a cerebral vessel and restricts or blocks blood flow. In some embodiments the ischemic stroke is a thrombotic stroke. In some embodiments, the ischemic stroke is embolic, i.e. caused by an embolus, or an unattached mass that travels through the bloodstream and restricts or blocks blood flow at a site distant from its point of origin. In some embodiments the ischemic stroke is an embolic stroke. Global cerebral ischemia commonly occurs when blood flow to the brain as a whole is blocked or reduced. In some embodiments the global cerebral ischemia is caused by hypoperfusion, i.e. due to shock. In some embodiments the global cerebral ischemia is a result of a cardiac arrest.

In some embodiments the subject diagnosed with brain injury has suffered cerebral ischemia. In some embodiments, the subject diagnosed with brain injury has suffered focal cerebral ischemia. In some embodiments, the subject diagnosed with brain injury has suffered an ischemic stroke. In some embodiments, the subject diagnosed with brain injury has suffered a thrombotic stroke. In some embodiments, the subject diagnosed with brain injury has suffered an embolic stroke. In some embodiments, the subject diagnosed with brain injury has suffered global cerebral ischemia. In some embodiments, the subject diagnosed with brain injury has suffered hypoperfusion. In some embodiments, the subject diagnosed with brain injury has suffered a cardiac arrest.

In some embodiments, the compositions of the invention are for use in treating cerebral ischemia. In some embodiments, the compositions of the invention are for use in treating focal cerebral ischemia. In some embodiments, the compositions of the invention are for use treating ischemic stroke. In some embodiments, the compositions of the invention are for use in treating thrombotic stroke. In some embodiments, the compositions of the invention are for use in treating embolic stroke. In some embodiments, the compositions of the invention are for use in treating global cerebral ischemia. In some embodiments, the compositions of the invention are for use in treating hypoperfusion.

In some embodiments, the stroke is hemorrhagic stroke. Hemorrhagic stroke is caused by bleeding into or around the brain resulting in swelling, pressure and damage to the cells and tissues of the brain. Hemorrhagic stroke is commonly a result of a weakened blood vessel that ruptures and bleeds into the surrounding brain. In some embodiments, the hemorrhagic stroke is an intracerebral hemorrhage, i.e. caused by bleeding within the brain tissue itself. In some embodiments the intracerebral hemorrhage is caused by an intraparenchymal hemorrhage. In some embodiments the intracerebral hemorrhage is caused by an intraventricular hemorrhage. In some embodiments the hemorrhagic stroke is a subarachnoid hemorrhage i.e. bleeding that occurs outside of the brain tissue but still within the skull. In some embodiments, the hemorrhagic stroke is a result of cerebral amyloid angiopathy. In some embodiments, the hemorrhagic stroke is a result of a brain aneurysm. In some embodiments, the hemorrhagic stroke is a result of cerebral arteriovenous malformation (AVM).

In some embodiments the subject diagnosed with brain injury has suffered hemorrhagic stroke. In some embodiments, the subject diagnosed with brain injury has suffered an intracerebral hemorrhage. In some embodiments, the subject diagnosed with brain injury has suffered an intraparenchymal hemorrhage. In some embodiments, the subject diagnosed with brain injury has suffered an intraventricular hemorrhage. In some embodiments, the subject diagnosed with brain injury has suffered a subarachnoid hemorrhage. In some embodiments, the subject diagnosed with brain injury has suffered cerebral amyloid angiopathy. In some embodiments, the subject diagnosed with brain injury has suffered a brain aneurysm. In some embodiments, the subject diagnosed with brain injury has suffered cerebral AVM

In some embodiments, the compositions of the invention are for use in treating hemorrhagic stroke. In some embodiments, the compositions of the invention are for use in treating an intracerebral hemorrhage. In some embodiments, the compositions of the invention are for use in treating an intraparenchymal hemorrhage. In some embodiments, the compositions of the invention are for use in treating an intraventricular hemorrhage. In some embodiments, the compositions of the invention are for use in treating a subarachnoid hemorrhage. In some embodiments, the compositions of the invention are for use in treating a cerebral amyloid angiopathy. In some embodiments, the compositions of the invention are for use in treating a brain aneurysm. In some embodiments, the compositions of the invention are for use in treating cerebral AVM.

Restoration of adequate blood flow to the brain after a period of interruption, though effective in alleviating the symptoms associated with stroke, can paradoxically result in further damage to the brain tissue. During the period of interruption, the affected tissue suffers from a lack of oxygen and nutrients, and the sudden restoration of blood flow can result in inflammation and oxidative damage through the induction of oxidative stress. This is known as reperfusion injury, and is well documented not only following stroke, but also following a heart attack or other tissue damage when blood supply returns to the tissue after a period of ischemia or lack of oxygen. In some embodiments the subject diagnosed with brain injury has suffered from reperfusion injury as a result of stroke. In some embodiments, the compositions of the invention are for use in treating reperfusion injury as a result of stroke.

A transient ischemic attack (TIA), often referred to as a mini-stroke, is a recognised warning sign for a more serious stroke. Subjects who have suffered one or more TIAs are therefore at greater risk of stroke. In some embodiments the subject diagnosed with brain injury has suffered a TIA. In some embodiments, the compositions of the invention are for use in treating a TIA. In some embodiments, the compositions of the invention are for use in treating brain injury in a subject who has suffered a TIA.

High blood pressure, high blood cholesterol, a familial history of stroke, heart disease, diabetes, brain aneurysms, arteriovenous malformations, sickle cell disease, vasculitis, bleeding disorders, use of nonsteroidal anti-inflammatory drugs (NSAIDs), smoking tobacco, drinking large amounts of alcohol, illegal drug use, obesity, lack of physical activity and an unhealthy diet are all considered to be risk factors for stroke. In particular, lowering blood pressure has been conclusively shown to prevent both ischemic and hemorrhagic strokes [67, 68]. In some embodiments, the compositions of the invention are for use in treating brain injury in a subject who has at least one risk factor for stroke. In some embodiments the subject has two risk factors for stroke. In some embodiments the subject has three risk factors for stroke. In some embodiments the subject has four risk factors for stroke. In some embodiments the subject has more than four risk factors for stroke. In some embodiments the subject has high blood pressure. In some embodiments the subject has high blood cholesterol. In some embodiments the subject has a familial history of stroke. In some embodiments the subject has heart disease. In some embodiments the subject has diabetes, In some embodiments the subject has a brain aneurysm. In some embodiments the subject has arteriovenous malformations. In some embodiments the subject has vasculitis. In some embodiments the subject has sickle cell disease. In some embodiments the subject has a bleeding disorder. In some embodiments the subject has a history of use of nonsteroidal anti-inflammatory drugs (NSAIDs). In some embodiments the subject smokes tobacco. In some embodiments the subject drinks large amounts of alcohol. In some embodiments the subject uses illegal drugs. In some embodiments the subject is obese. In some embodiments the subject is overweight. In some embodiments the subject has a lack of physical activity. In some embodiments the subject has an unhealthy diet.

The examples indicate that the compositions of the invention may be useful for treating brain injury and aiding recovery when administered before the injury event occurs. Therefore, the compositions of the invention may be particularly useful for treating brain injury when administered to subjects at risk of brain injury, such as stroke.

In certain embodiments, the compositions of the invention are for use in reducing the damage caused by a potential brain injury, preferably a stroke. The compositions may reduce the damage caused when they are administered before the potential brain injury occurs, in particular when administered to a patient identified as at risk of a brain injury.

The examples indicate that the compositions of the invention may be useful for treating brain injury and aiding recovery when administered after the injury event occurs. Therefore, the compositions of the invention may be particularly useful for treating brain injury when administered to subjects following a brain injury, such as stroke.

In some embodiments, the compositions of the invention treat brain injury by reducing motoric damage. In some embodiments, the compositions of the invention treat brain injury by improving motor function. In some embodiments, the compositions of the invention treat brain injury by improving muscle strength. In some embodiments, the compositions of the invention treat brain injury by improving memory. In some embodiments, the compositions of the invention treat brain injury by improving social recognition. In some embodiments, the compositions of the invention treat brain injury by improving neurological function.

Treatment of brain injury may refer to, for example, an alleviation of the severity of symptoms. Treatment of brain injury may also refer to reducing the neurological impairments following stroke. Compositions of the invention for use in treating stroke may be provided to the subject in advance of the onset of stroke, for example in a patient identified as being at risk of stroke. Compositions of the invention for use in treating stroke may be provided after a stroke has occurred, for example, during recovery. Compositions of the invention for use in treating stroke may be provided during the acute phase of recovery (i.e. up to one week after stroke). Compositions of the invention for use in treating stroke may be provided during the subacute phase of recovery (i.e. from one week up to three months after stroke). Compositions of the invention for use in treating stroke may be provided during the chronic phase of recovery (from three months after stroke).

In certain embodiments, the compositions of the invention are for use in combination with a secondary active agent. In certain embodiments, the compositions of the invention are for use in combination with aspirin or tissue plasminogen activator (tPA). Other secondary agents include other antiplatelets (such as clopidogrel), anticoagulants (such as heparins, warfarin, apixaban, dabigatran, edoxaban or rivaroxaban), antihypertensives (such as diuretics, ACE inhibitors, calcium channel blockers, beta-blockers or alpha-blockers) or statins. The compositions of the invention may improve the patient's response to the secondary active agent.

In certain embodiments, the compositions of the invention reduce the effect of ischemia on tissues. In certain embodiments, the compositions of the invention reduce the amount of damage to tissues caused by ischemia. In certain embodiments, the tissues damaged by ischemia are the cerebral tissues. In certain embodiments, the compositions of the invention reduce necrosis or the number of necrotic cells. In certain embodiments, the compositions of the invention reduce apoptosis or the number of apoptotic cells. In certain embodiments, the compositions of the invention reduce the number of necrotic and apoptotic cells. In certain embodiments, the compositions of the invention prevent cell death by necrosis and/or apoptosis. In certain embodiments, the compositions of the invention prevent cell death by necrosis and/or apoptosis caused by ischemia. In certain embodiments, the compositions of the invention improve the recovery of the tissue damaged by ischemia. In certain embodiments, the compositions of the invention improve the speed of clearance of necrotic cells and/or apoptotic cells. In certain embodiments, the compositions of the invention improve the efficacy of the clearance of necrotic cells and/or apoptotic cells. In certain embodiments, the compositions of the invention improve the replacement and/or regeneration of cells within tissues. In certain embodiments, the compositions of the invention improve the replacement and/or regeneration of cells within tissues damaged by ischemia. In certain embodiments, the compositions of the invention improve the overall histology of the tissue (for example upon a biopsy).

### Inflammatory and autoimmune disorders

The examples demonstrate that the compositions of the invention have HDAC inhibitory activity and that they further have anti-inflammatory properties. HDAC activity is central to the pathology of many inflammatory and autoimmune disorders, and HDAC inhibitors have shown efficacy in the treatment of many inflammatory and autoimmune disorders, as discussed below in relation to specific conditions (see also [69]). Therefore, the compositions of the invention may be useful for treating inflammatory and autoimmune disorders, in particular inflammatory and autoimmune disorders mediated by histone deacetylase (HDAC) activity.

In certain embodiments, the compositions of the invention are for use in a method of treating or preventing an inflammatory or autoimmune disorder. In certain embodiments, the compositions of the invention are for use in treating or preventing an inflammatory or autoimmune disease, wherein said treatment or prevention is achieved by reducing or preventing HDAC activation. In certain embodiments, the compositions of the invention are for use in treating a patient with an inflammatory or autoimmune disease, wherein the patient has elevated HDAC levels or activity. In certain embodiments, the patient may have been diagnosed with a chronic inflammatory or autoimmune disease or condition, or the composition of the invention may be for use in preventing an inflammatory or autoimmune disease or condition developing into a chronic inflammatory or autoimmune disease or condition. In certain embodiments, the disease or condition may not be responsive to treatment with TNF-α inhibitors.

HDAC may be associated with chronic inflammatory and autoimmune diseases, so the compositions of the invention may be particularly useful for treating or preventing chronic diseases or conditions as listed above. In certain embodiments, the compositions are for use in patients with chronic disease. In certain embodiments, the compositions are for use in preventing the development of chronic disease.

The compositions of the invention may be useful for treating diseases and conditions mediated by HDAC and for addressing HDAC activation, so the compositions of the invention may be particularly useful for treating or preventing chronic disease, treating or preventing disease in patients that have not responded to other therapies (such as treatment with TNF-α inhibitors), and/or treating or preventing the tissue damage and symptoms associated with HDAC.

The examples demonstrate that the compositions of the invention reduce IL-6 production and secretion, which may be particularly useful for treating inflammatory and autoimmune disorders. In certain embodiments, the compositions of the invention are for use in reducing inflammation in the treatment of disease. In certain embodiments, the compositions of the invention decrease IL-6 production and secretion. In certain embodiments, the compositions of the invention decrease the activation of the NPκB promoter. In certain embodiments, the compositions of the invention are able to modulate the activation of IL-6 production by the potent pro-inflammatory endotoxin lipopolysaccharide (LPS).

### - Inflammatory bowel disease

The examples demonstrate that the compositions of the invention have HDAC inhibitory activity and that they also have anti-inflammatory properties, and so they may be useful in the treatment of inflammatory bowel disease. Overexpression of different HDAC isoforms have been implicated in a variety of disease pathologies, including colitis. Additionally, valproic acid has been associated with class I HDAC inhibition and amelioration of colitis in a DSS-colitis murine model [70]. This study suggested a role for HDAC class I inhibitors in IFN-γ, IL-10, IL-1β and TNF-α suppression, assigning functionality to HDAC inhibition and efficacy in colitis. Therefore, the examples indicate that the compositions of the invention may be useful for treating inflammatory bowel diseases.

In certain embodiments, the compositions of the invention are for use in treating or preventing inflammatory bowel disease. In certain embodiments, the compositions of the invention are for use in treating or preventing inflammatory bowel disease, wherein said treatment or prevention is achieved by reducing or preventing HDAC activation. In certain embodiments, the compositions of the invention are for use in treating a patient with inflammatory bowel disease, wherein the patient has elevated HDAC levels or activity.

Inflammatory bowel disease (IBD) is a complex disease that can be caused by multiple environmental and genetic factors. Factors contributing to the onset of IBD include diet, microbiota, intestinal permeability, and genetic susceptibility to increased inflammatory response to gut infection. Symptoms of inflammatory bowel disease include abdominal pain, vomiting, diarrhoea, rectal bleeding, severe internal cramps/muscle spasms in the pelvic region, weight loss and anaemia. In certain embodiments, the compositions are for use in reducing one or more symptoms associated with IBD. In certain embodiments, the compositions of the invention are for use in preventing one or more symptoms of IBD.

IBD may accompany other diseases or conditions, such as arthritis, pyoderma gangrenosum, primary sclerosing cholangitis, non-thyroidal illness syndrome, deep vein thrombosis, bronchiolitis obliterans organizing pneumonia. In certain embodiments, the compositions of the invention are for use in the treatment or prevention of one or more diseases or conditions that accompany IBD.

Inflammatory bowel disease is generally diagnosed by biopsy or colonoscopy. Measurements of faecal calprotectin is useful for the preliminary diagnosis of IBD. Other laboratory test for the diagnosis of IBD include, complete blood count, erythrocyte sedimentation rate, comprehensive metabolic panel, faecal occult blood test or C-reactive protein test. Typically a combination of laboratory testing and biopsy/colonoscopy will be used to confirm diagnosis of IBD. In certain embodiments, the compositions of the invention are for use in a subject diagnosed with IBD.

In certain embodiments the inflammatory bowel disease is Crohn's disease. Studies have shown that several HDACs are upregulated in the inflammatory muscosa of patients with Crohn's disease. Therefore, inhibition of HDAC activity may be useful in the treatment of Crohn's disease. In certain embodiments, the compositions of the invention are for use in the treatment or prevention of Crohn's disease.

Crohn's disease is a complex disease with an array of probable causes, including genetic risk factors, diet, other lifestyle factors, such as smoking and alcohol consumption, and microbiome composition. Crohn's disease can manifest anywhere along the gastrointestinal tract.

Gastrointestinal symptoms of Crohn's disease range from mild to severe and include abdominal pain, diarrhoea, faecal blood, ileitis, increased bowel movements, increased flatulence, intestinal stenosis, vomiting, and perianal discomfort. The compositions of the invention may be for use in the treatment of prevention of one or more gastrointestinal symptoms of Crohn's disease.

Systemic symptoms of Crohn's disease include growth defects, such as the inability to maintain growth during puberty, decreased appetite, fever and weight loss. Extra-intestinal features of Crohn's disease include uveitis, photobia, episcleritis, gall stones, seronegative spondyloarthropathy, arthritis, enthesitis, erythema nodosum, pyoderma gangrenosum, deep venous thrombosis, pulmonary embolism, autoimmune haemolytic anaemia, clubbing and osteoporosis. Extra-intestinal features are additional conditions associated with Crohn's disease that manifest outside the GI tract. Subjects with Crohn's disease also exhibit increased susceptibility to neurological complications such as seizures, strokes, myopathy, peripheral neuropathy, headache and depression. In certain embodiments, the compositions of the invention are for use in the treatment or prevention of one or more systemic symptoms of Crohn' disease. In certain embodiments, the compositions of the invention are for use in the treatment or prevention of one or more extra-intestinal features of Crohn's disease.

The diagnosis of Crohn's disease usually involves carrying out multiple tests and surgical procedures, such as gastroscopy and/or colonoscopy and biopsy, typically of the ileum, radiologic tests, complete blood counts, C-reactive protein tests and erythrocyte sedimentation rates. In certain embodiments, the compositions of the invention are for use in subjects diagnosed with Crohn's disease. In some embodiments, compositions of the invention are for use in treating a subject who has been diagnosed with Crohn's disease.

Crohn's disease is classified depending on the extent of the region of the GI tract affected [71]. A disease of both the ileum and colon is classified as Ileocolic Crohn's. In some embodiments, the compositions are for use in the treatment or prevention of Ileocolic Crohn's. In some embodiments, the compositions are for use in a subject diagnosed with Ileocolic Crohn's/ Crohn's ileitis is classified if only the ileum is affected. Crohn's colitis is classified if only the colon is affected. In certain embodiments, the compositions are for use in the treatment or prevention of Crohn's ileitis. In some embodiments, the compositions are for use in a subject diagnosed with Crohn's ileitis. In certain embodiments, the compositions are for use in the treatment or prevention of Crohn's colitis. In some embodiments, the compositions are for use in a subject diagnosed with Crohn's colitis.

Crohn's disease may be treated with a number of therapeutic agents, such as corticosteroids, such as prednisone, immunosuppressive agents, such as azathioprine, or biologics, such as infliximab, adalimumab, and golimumab, vedolizumab and etrolizumab. In certain embodiments, the compositions of the invention are for use in the treatment or prevention of Crohn's disease in combination with an additional therapeutic agent. In certain embodiments, the additional therapeutic agent is for use in the treatment or prevention of Crohn's disease.

### - Multiple Sclerosis

Multiple sclerosis (MS) is an autoimmune inflammatory disorder of the central nervous system. MS can be modelled in animals by the induction of experimental autoimmune encephalomyelitis (EAE). HDAC inhibitors have been shown to reduce clinical symptoms and inhibit disease progress in mice with adoptive EAE (Dasgupta et al., 2003, J Immunol, 170 (7), 3874-3882). Injection of an HDAC inhibitor has also been shown to significantly reduce neurological impairment and disability in mice with an experimental model of chronic MS (Camelo et al., 2005, J Neuroimmunol, 164(1-2), 10-21). Inhibition of HDAC activity has been suggested as a promising therapy for MS (Gray et al., 2006, Epigenetics, 1:2, 67-75). Therefore, the compositions of the invention may be useful for treating or preventing multiple sclerosis in a subject.

In certain embodiments, the compositions of the invention are for use in treating or preventing multiple sclerosis, wherein said treatment or prevention is achieved by reducing or preventing HDAC activation. In certain embodiments, the compositions of the invention are for use in treating a patient with multiple sclerosis, wherein the patient has elevated HDAC levels or activity.

In preferred embodiments, the compositions of the invention are for use in treating or preventing multiple sclerosis. The compositions of the invention may achieve HDAC inhibition, and so they may be useful in the treatment or prevention of multiple sclerosis. Multiple sclerosis is an inflammatory disorder associated with damage to the myelin sheaths of neurons, particularly in the brain and spinal column. Multiple sclerosis is a chronic disease, which is progressively incapacitating and which evolves in episodes.

In certain embodiments, treatment with the compositions of the invention results in a reduction in disease incidence or disease severity. In certain embodiments, the compositions of the invention are for use in reducing disease incidence or disease severity. In certain embodiments, treatment with the compositions of the invention prevents a decline in motor function or results in improved motor function. In certain embodiments, the compositions of the invention are for use in preventing a decline in motor function or for use in improving motor function. In certain embodiments, treatment with the compositions of the invention prevents the development of paralysis. In certain embodiments, the compositions of the invention are for use in preventing paralysis in the treatment of multiple sclerosis.

The compositions of the invention may be useful for modulating a patient's immune system, so in certain embodiments the compositions of the invention are for use in preventing multiple sclerosis in a patient that has been identified as at risk of multiple sclerosis, or that has been diagnosed with early-stage multiple sclerosis or "relapsing-remitting" multiple sclerosis. The compositions of the invention may be useful for preventing the development of sclerosis.

The compositions of the invention may be useful for managing or alleviating multiple sclerosis. The compositions of the invention may be particularly useful for reducing symptoms associated with multiple sclerosis. Treatment or prevention of multiple sclerosis may refer to, for example, an alleviation of the severity of symptoms or a reduction in the frequency of exacerbations or the range of triggers that are a problem for the patient.

### - Arthritis

Arthritis is a disease characterised by chronic joint inflammation. Rheumatoid arthritis is a chronic autoimmune disorder that typically results in swollen and painful joints HDAC inhibition has been proposed to treat rheumatoid arthritis by a variety of mechanisms, including influencing cytokine production, inhibiting T-cell differentiation, suppressing proliferation of synovial fibroblasts and reducing bone loss by influencing osteoclasts and osteoblasts (Vojinov et al., 2011, Mol Med, 17 (5-6) 397-403). HDAC inhibition has been shown to have a strong anti-inflammatory effect in several animal models of arthritis (Joosten et al., 2011, Mol Med, 17 (5-6), 391-396). Therefore, the compositions of the invention may be useful for treating or preventing arthritis in a subject.

In preferred embodiments, the compositions of the invention are for use in treating or preventing rheumatoid arthritis (RA). In certain embodiments, the compositions of the invention are for use in treating or preventing rheumatoid arthritis, wherein said treatment or prevention is achieved by reducing or preventing HDAC activation. In certain embodiments, the compositions of the invention are for use in treating a patient with rheumatoid arthritis, wherein the patient has elevated HDAC levels or activity.

In certain embodiments, treatment with the compositions of the invention results in a reduction in the swelling of joints. In certain embodiments, the compositions of the invention are for use in patients with swollen joints or patients identified as at risk of having swollen joints. In certain embodiments, the compositions of the invention are for use in a method of reducing joint swelling in RA.

In certain embodiments, treatment with the compositions of the invention results in a reduction in cartilage damage or bone damage. In certain embodiments, the compositions of the invention are for use in reducing or preventing cartilage or bone damage in the treatment of RA. In certain embodiments, the compositions are for use in treating patient with severe RA that are at risk of cartilage or bone damage.

In certain embodiments, the compositions of the invention are for use in preventing bone erosion or cartilage damage in the treatment of RA. In certain embodiments, the compositions are for use in treating patients that exhibit bone erosion or cartilage damage or patients identified as at risk of bone erosion or cartilage damage.

The compositions of the invention may be useful for modulating a patient's immune system, so in certain embodiments the compositions of the invention are for use in preventing RA in a patient that has been identified as at risk of RA, or that has been diagnosed with early-stage RA. The compositions of the invention may be useful for preventing the development of RA.

The compositions of the invention may be useful for managing or alleviating RA. The compositions of the invention may be particularly useful for reducing symptoms associated with joint swelling or bone destruction. Treatment or prevention of RA may refer to, for example, an alleviation of the severity of symptoms or a reduction in the frequency of exacerbations or the range of triggers that are a problem for the patient.

### - Asthma

Asthma is a chronic inflammatory respiratory disease. HDAC inhibitors have been shown to have anti-inflammatory effects that relieve airway inflammation, airway remodelling and airway hypersensitivity in a mouse model of chronic asthma (Ren et al., 2016, Inflamm Res, 65, 995-1008). Therefore, the compositions of the invention may be useful for treating or preventing asthma in a subject.

In preferred embodiments, the compositions of the invention are for use in treating or preventing asthma. In certain embodiments, the compositions of the invention are for use in treating or preventing asthma, wherein said treatment or prevention is achieved by reducing or preventing HDAC activation, In certain embodiments, the compositions of the invention are for use in treating a patient with asthma, wherein the patient has elevated HDAC levels or activity.

In certain embodiments, the asthma is eosinophilic or allergic asthma. Eosinophilic and allergic asthma are characterised by increased numbers of eosinophils in peripheral blood and in airway secretions and is associated pathologically with thickening of the basement membrane zone and pharmacologically by corticosteroid responsiveness [72]. Compositions that reduce or inhibit eosinophil recruitment or activation may be useful for treating or preventing eosinophilic and allergic asthma. Eosinophilic and allergic asthma are also characterised by a cascade of inflammatory events mediated by T helper type 2 lymphocyte (Th2) processes. Compositions that reduce or inhibit T helper type 2 lymphocyte (Th2) processes may be useful for treating or preventing eosinophilic and allergic asthma.

In additional embodiments, the compositions of the invention are for use in treating or preventing neutrophilic asthma (or non-eosinophilic asthma). High neutrophil numbers are associated with severe asthma that may be insensitive to corticosteroid treatment. Compositions that reduce or inhibit neutrophil recruitment or activation may be useful for treating or preventing neutrophilic asthma.

Eosinophilic asthma (also referred to as Th2-high asthma) and neutrophilic asthma (also referred to as Th2-low or non-Th2 asthma) have different underlying pathophysiological mechanisms and present different clinical features. For example, Th2-high asthma generally presents early onset and exhibits seasonal variations of symptoms, whereas Th2-low asthma has a much later onset, typically around the age of 40 or later. Th2-high asthma is also characterised by increased immunoglobulin E (IgE) blood levels, whereas this feature is absent in Th2-low asthma. Th2 high asthma is also characterised by high sputum levels of eosinophils. By contrast, Th2-low asthma may be characterised by elevated levels of sputum neutrophils. In certain embodiments, the compositions of the invention are for use in treating Th2-low or non-Th2 asthma. In certain embodiments, the compositions of the invention are for use in treating Th2-high asthma.

Eosinophilic and neutrophilic asthma are not mutually exclusive conditions and treatments that help address either the eosinophil and neutrophil responses may be useful for treating asthma in general.

In certain embodiments, the compositions of the invention are for use in methods reducing an eosinophilic inflammatory response in the treatment or prevention of asthma, or for use in methods of reducing a neutrophilic inflammatory response in the treatment or prevention of asthma. As noted above, high levels of eosinophils in asthma is associated pathologically with thickening of the basement membrane zone, so reducing eosinophilic inflammatory response in the treatment or prevention of asthma may be able to specifically address this feature of the disease. Also, elevated neutrophils, either in combination with elevated eosinophils or in their absence, is associated with severe asthma and chronic airway narrowing. Therefore, reducing the neutrophilic inflammatory response may be particularly useful for addressing severe asthma.

In certain embodiments, the compositions reduce peribronchiolar infiltration in allergic asthma, or are for use in reducing peribronchiolar infiltration in the treatment of allergic asthma. In certain embodiments, the compositions reduce peribronchiolar and/or perivascular infiltration in neutrophilic asthma, or are for use in reducing peribronchiolar and/or perivascular infiltration in the treatment of allergic neutrophilic asthma.

In certain embodiments, treatment with compositions of the invention provides a reduction or prevents an elevation in TNFa levels.

In certain embodiments, the compositions of the invention are for use in a method of treating asthma that results in a reduction of the eosinophilic and/or neutrophilic inflammatory response. In certain embodiments, the patient to be treated has, or has previously been identified as having, elevated neutrophil or eosinophil levels, for example as identified through blood sampling or sputum analysis.

The compositions of the invention may be useful for preventing the development of asthma in a new-born when administered to the new-born, or to a pregnant woman. The compositions may be useful for preventing the development of asthma in children. The compositions of the invention may be useful for treating or preventing adult-onset asthma. The compositions of the invention may be useful for managing or alleviating asthma. The compositions of the invention may be particularly useful for reducing symptoms associated with asthma that is aggravated by allergens, such as house dust mites.

Treatment or prevention of asthma may refer to, for example, an alleviation of the severity of symptoms or a reduction in the frequency of exacerbations or the range of triggers that are a problem for the patient.

### - Psoriasis

Psoriasis is a chronic inflammatory skin disease. Overexpression of HDAC1 has been reported for in skin biopsies from psoriatic pateints (Tovar-Castillo et al., 2007, Int J Dermatol, 46, 239-46) and a HDAC inhibitor has been shown to block the conversion of Foxp3+ Tregs into Foxp3-RORγt+ IL-17/Tregs (a shift associated with psoriasis disease progression) (Bovenschen et al., 2011, J Invest Dermatol, 131, 1853-60). Therefore, the compositions of the invention may be useful for treating or preventing psoriasis in a subject.

In preferred embodiments, the compositions of the invention are for use in treating or preventing psoriasis. In certain embodiments, the compositions of the invention are for use in treating or preventing psoriasis, wherein said treatment or prevention is achieved by reducing or preventing HDAC activation. In certain embodiments, the compositions of the invention are for use in treating a patient with psoriasis, wherein the patient has elevated HDAC levels or activity.

### - Systemic lupus erythematosus

Systemic lupus erythematosus (SLE) is an autoimmune disease. HDAC inhibition is believed to be a promising therapeutic approach for treating SLE based on studies on cell cultures and mouse models of SLE (Reilly et al., 2011, Mol Med, 17 (5-6), 417-425). Therefore, the compositions of the invention may be useful for treating or preventing systemic lupus erythematosus in a subject.

In preferred embodiments, the compositions of the invention are for use in treating or preventing SLE. In certain embodiments, the compositions of the invention are for use in treating or preventing SLE, wherein said treatment or prevention is achieved by reducing or preventing HDAC activation. In certain embodiments, the compositions of the invention are for use in treating a patient with SLE, wherein the patient has elevated HDAC levels or activity.

### - Allograft rejection

Allograft rejection occurs when transplanted tissues are rejected by the recipient's immune system. Studies on murine cardiac transplants have shown that HDAC inhibition increases intra-graft histone 3 acetylation and is associated with increased intra-graft levels of Foxp3 protein (a forkhead transcription family member involved in controlling immune responses), maintenance of tissue architecture and a lack of the stigmata of chronic rejection relative to controls (Wang et al., Immunol Cell Biol, 1-8). Therefore, the compositions of the invention may be useful for treating or preventing allograft rejection in a subject.

In preferred embodiments, the compositions of the invention are for use in treating or preventing allograft rejection. In certain embodiments, the compositions of the invention are for use in treating or preventing allograft rejection, wherein said treatment or prevention is achieved by reducing or preventing HDAC activation. In certain embodiments, the compositions of the invention are for use in treating a patient with allograft rejection, wherein the patient has elevated HDAC levels or activity.

### - Diabetes

Diabetes mellitus is a group of diseases in which low levels of insulin and/or peripheral insulin resistance lead to hyperglycermia. HDAC inhibition has been proposed to treat diabetes by a variety of mechanisms, including de-repression of *Pdx1* (Park et al., 2008, J Clin Invest, 118, 2316-24), enhancing expression of transcription factor *Ngn3* to increase the pool of endocrine progenitor cells (Haumaitre et al., 2008, Mol Cell Biol, 28, 6373-83) and enhancing insulin expression (Molsey et al., 2003, J Biol Chem, 278, 19660-6) amongst others. HDAC inhibition is also a promising treatment for late diabetic complications such as diabetic nephropathy and retinal ischemia (Christensen et al., 2011, Mol Med, 17 (5-6), 370-390). Therefore, the compositions of the invention may be useful for treating or preventing diabetes in a subject.

In preferred embodiments, the compositions of the invention are for use in treating or preventing diabetes. In preferred embodiments, the compositions of the invention are for use in treating or preventing type I diabetes. In preferred embodiments, the compositions of the invention are for use in treating or preventing type II diabetes. In certain embodiments, the compositions of the invention are for use in treating or preventing diabetes, wherein said treatment or prevention is achieved by reducing or preventing HDAC activation. In certain embodiments, the compositions of the invention are for use in treating a patient with diabetes, wherein the patient has elevated HDAC levels or activity.

### - Graft-versus-host Disease (GVHD)

The compositions of the invention may be for use in the treatment or prevention of Graft-versus-host disease (GVHD). GVHD is a medical complication following transplantation of allogeneic tissue into a subject. GVHD commonly occurs following stem cell or bone marrow transplantation or solid organ transplantation, particularly where the genetic background of the graft (i.e. the donor) and the host (i.e. the recipient) are distinct.

The pathophysiology of GVHD comprises three distinct phases. Firstly, host antigen presenting cells (APCs), such as dendritic cells (DCs) are activated following recognition of the transplanted tissue as a foreign substance. APC activation precedes the recruitment and activation of effector immune cells, such as conventional cytotoxic T cells, which leads to destruction or rejection of the foreign tissue.

HDAC inhibition has been shown to mediate potent pleiotropic anti-inflammatory effects useful in the treatment or prevention of GVHD HDAC inhibition may inhibit at multiple points of the GVHD pathophysiological cascade. For example, HDAC inhibition prevents antigen presenting cell and dendritic cell activation against allogeneic tissues *in vivo* by enhancing the expression of indoleamine 2,3-dioxygenase in a STAT-3 dependent manner [73]. HDAC inhibition of STAT-1 activity has also been shown to be beneficial in the treatment or prevention of GVHD [74]. In certain embodiments, the composition of the invention may be for use in the treatment or prevention of GVHD by inhibiting APC activation.

HDAC inhibition has also been shown to expand Treg cell populations and activity in vivo [75]. HDAC inhibition-mediated upregulation of Treg cell activity has been shown to supress conventional cytotoxic T cell activity, which may be useful in the treatment or prevention of GVHD by supressing the 2nd phase of the GVHD pathophysiological cascade. In certain embodiments, the compositions of the invention are for use in the treatment or prevention of GVHD by reducing conventional cytotoxic T cell activity. In certain embodiments, the compositions of the invention may be for use in reducing conventional cytotoxic T cell activity. In certain embodiments, the composition of the invention may be for use in the treatment or prevention of GVHD by upregulating Treg cell activity.

Donor NK cells have been shown to reduce GVHD by eliminating host APCs. HDAC inhibition has been shown to increase NK cell activity. Therefore, the compositions of the invention may be for use to increase NK cell activity, which may be useful in the treatment or prevention of GVHD by increasing the elimination of APCs. In certain embodiments, the compositions of the invention may be for use in the treatment or prevention of GVHD by enhancing the elimination of host APCs. In certain embodiments, the compositions of the invention may be for use in the treatment or prevention of GVHD by enhancing NK cell activity. In certain embodiments, the compositions of the invention may be for use in the treatment or prevention of GVHD by enhancing NK cell activity-mediated elimination of host APCs.

In certain embodiments, the compositions of the invention may be administered after the host has received the transplant. In certain embodiments, the compositions of the invention may be administered to the host before the subject has received the transplant. Administration of the compositions of the invention before the transplant has been received may be useful in priming the immune system of the subject to not elicit an inflammatory or autoimmune response against the transplanted tissue. In certain embodiments, the compositions of the invention may be used for preventing or preventing the onset of GVHD. In certain embodiments, the composition of the invention may be for use in the treatment or prevention of GVHD prophylactically. In certain embodiments, the compositions of the invention may be used in the prophylaxis of GVHD. In certain embodiments, the compositions of the invention may be for use in a method of preventing transplant tissue rejection in a subject.

In certain embodiments, the compositions of the invention may be useful for treating, delaying, preventing, or preventing the onset of acute GVHD. Symptoms of acute GVHD typically manifest within the first 100 days of transplantation. Delaying, treatment or prevention of acute GVHD may be particularly beneficial to aid the recovery of subjects in the immediate aftermath of transplant surgery. In certain embodiments, the compositions may treat, delay the onset of, prevent or prevent the onset of acute GVHD by inhibiting HDAC activity. In certain embodiments, the compositions may treat, delay the onset of, prevent, or prevent the onset of acute GVHD by upregulating Treg cell activity. The compositions may treat, delay the onset of, prevent or prevent the onset of acute GVHD by inhibiting conventional cytotoxic T cell activity. The compositions of the invention may treat, delay the onset of, prevent or prevent the onset of acute GVHD by enhancing NK cell activity. The compositions of the invention may treat, delay the onset of, prevent or prevent the onset of acute GVHD by inhibiting APC activation.

In certain embodiments, the compositions of the invention may treat, delay the onset of, prevent, or prevent the onset of acute GVHD when administered to a subject within 100 days following transplantation. In certain embodiments, the compositions of the invention may treat, delay the onset of, prevent, or prevent the onset of acute GVHD when administered to a subject prophylactically, for example, when the composition is administered to the subject before the transplant. In certain embodiments, the compositions of the invention may treat, delay the onset of, prevent, or prevent the onset of persistent, late-onset or recurrent acute GVHD, such as acute GVHD that occurs or recurs more than 100 days after transplantation.

In certain embodiments, the composition of the invention may treat, delay the onset of, prevent, or prevent the onset one or more symptoms of acute GVHD selected from the list consisting of macropaular skin rash, nausea, anorexia, diarrhea, severe abdominal pain, ileus and cholestatic hyperbilirubinemia.

In certain embodiments, the compositions of the invention may be useful for treating, delaying the onset of, preventing, or preventing the onset of chronic GVHD. Chronic GVHD is a complex, multisystem disorder that can involve any organ and is typically characterised by fibrosis. Chronic GVHD may evolve from acute GVHD, or may emerge after a period of quiescence following acute GVHD, or may emerge de novo. Symptoms of chronic GVHD may emerge at any time following transplantation. In certain embodiments, the compositions may be useful for treating, preventing, preventing the onset of, or delaying the onset of chronic GVHD by inhibiting HDAC activity. The compositions may treat, delay the onset of, prevent, or prevent the onset of chronic GVHD by upregulating Treg cell activity. The compositions may treat, delay the onset of, prevent, or prevent the onset of chronic GVHD by inhibiting conventional cytotoxic T cell activity. The compositions of the invention may treat, delay the onset of, prevent, or prevent the onset of chronic GVHD by enhancing NK cell activity. The compositions of the invention may treat, delay the onset of, prevent, or prevent the onset of chronic GVHD by inhibiting APC DC activation.

In certain embodiments, the compositions of the invention are for administration to a patient that has recently undergone a stem cell, bone marrow or solid organ transplant. In certain embodiments, the compositions of the invention are for administration to a patient is in need of a stem cell, bone marrow or solid organ transplant.

In certain embodiments, the composition of the invention may treat, delay the onset of, prevent, or prevent the onset of one or more symptoms of chronic GVHD selected from the list consisting of: dyspigmentation, new-onset alopecia, poikiloderma,lichen planuslike eruptions or sclerotic features, nail dystrophy or loss, xerostomia, mouth ulcers (such as aphthous stomatitis), lichen-type features in the mouth (such as lichen sclerosis), keratoconjunctivitis sicca, sicca syndrome, cicatricial conjunctivitis, fascititis, myostitis, joint stiffness, vaginal sclerosis, ulcerations, anorexia, weight loss, oesophageal web, jaundice, transaminitis, pleural effusions, bronchiolitis obliterans, nephrotic syndrome, pericarditis, thrombocytopenia, anemia, and neutropenia.

The inventors have also shown that the compositions of the invention can reduce colitis associated with GVHD. Colitis is an inflammatory side effect observed in patients with GVHD. The compositions of the invention may also be useful for treating colonic inflammation in a subject with GVHD Therefore, in some embodiments, the compositions of the invention are for use in treating colitis in a subject with GVHD. In some embodiments, the compositions of the invention are for use in reducing the severity of colitis in a subject with GVHD. In some embodiments, the compositions of the invention are for use in reducing the severity of colitis in the treatment of GVHD. In some embodiments, the compositions of the invention are for use in treating colonic inflammation in a subject with GVHD. In some embodiments, the compositions of the invention are for use in reducing the severity of colonic inflammation in a subject with GVHD. In some embodiments, the compositions of the invention are for use in reducing colonic inflammation in the treatment of GVHD

The inventors have also found that the compositions of the invention are useful for maintaining gut-barrier function in subjects with GVHD. Maintaining gut-barrier function reduces the translocation of inflammatory cytokines through the gut-barrier, which aggravates toxicity in GVHD [76]. In certain embodiments, the compositions of the invention are for use in maintaining gut-barrier function in the treatment of GVHD. In some embodiments, the compositions of the invention are for use in reducing translocation of inflammatory cytokines across the gut-barrier in the treatment of GVHD

In certain embodiments, the compositions of the invention may be for use in combination with one or more pharmacological agents for the treatment or prevention of GVHD. In certain embodiments, the one or more pharmacological agents are for the pharmacological prevention or treatment of GVHD. In certain embodiments, the compositions of the invention are for use in the treatment or prevention of GVHD in a subject who is receiving, has received, or is about to receive, one or more of said pharmacological agents. In certain embodiments, the one or more pharmacological agents are selected from the list consisting of: suberoylanilide, vorisnostat, ITF2357 cyclosporine, ciclosporin, sirolimus, pentostatin, rituximab, imatinib, mycophenolate mofetil, tacrolimus, prednisone, methotrexate, remestemcel-L and Prochymal, wherein the pharmacological agent is administered in a therapeutically effective amount for the treatment or prevention of GVHD. In some embodiments, the compositions of the invention are for use in the treatment of GVHD in a subject who has received, is receiving, or is about to receive extracorporeal photophoreses.

### Behavioural and psychiatric disorders

The compositions of the invention may be useful in reducing hyperactivity in a subject. Hyperactivity is a symptom of behavioural and psychiatric disorders, such as attention deficit hyperactive disorder (ADHD), post-traumatic stress disorder, anxiety disorders, bipolar affective disorder and obsessive compulsive disorder. Hyperactivity may be a symptom of hormonal disorders, such as hyperthyroidism, hyperkinetic and resistance to thyroid hormone. Hyperactivity may also be a symptom of neuronal disorders, such as adrenoleukodystrophy. Hyperactivity may also be a symptom of hyperkinetic disorder, catatonic schizophrenia, anorexia nervosa, Fragile X Syndrome (FXS), phenylketonuria (PKU), foetal alcohol syndrome (FAS), anxiety, depression and Tourette's syndrome. In certain embodiments, the compositions of the invention are for use in the treatment or prevention of behavioural disorders. In certain embodiments, the compositions of the invention are for use in the treatment or prevention of psychiatric disorders. In certain embodiments, the compositions of the invention are for use in the treatment of emotional and behavioural disorders.

In certain embodiments, the compositions are for use in reducing hyperactivity in the treatment of hyperthyroidism or resistance to thyroid hormone. In certain embodiments, the compositions of the invention are for use in treating hyperactivity in a patient diagnosed with hyperthyroidism or resistance to thyroid hormone.

In certain embodiments, the compositions are for use in reducing hyperactivity in the treatment of adrenoleukodystrophy. In certain embodiments, the compositions of the invention are for use in treating hyperactivity in a patient diagnosed with adrenoleukodystrophy.

In certain embodiments, the compositions are for use in reducing hyperactivity in the treatment of catatonic schizophrenia. In certain embodiments, the compositions of the invention are for use in treating hyperactivity in a patient diagnosed with catatonic schizophrenia.

In certain embodiments, the compositions are for use in reducing hyperactivity in the treatment of anorexia nervosa. In certain embodiments, the compositions of the invention are for use in treating hyperactivity in a patient diagnosed with anorexia nervosa.

In certain embodiments, the compositions are for use in reducing hyperactivity in the treatment of Fragile X Syndrome. In certain embodiments, the compositions of the invention are for use in treating hyperactivity in a patient diagnosed with Fragile X Syndrome (FXS).

In certain embodiments, the compositions are for use in reducing hyperactivity in the treatment of phenylketonuria. In certain embodiments, the compositions of the invention are for use in treating hyperactivity in a patient diagnosed with phenylketonuria.

In certain embodiments, the compositions are for use in reducing hyperactivity in the treatment of foetal alcohol syndrome. In certain embodiments, the compositions of the invention are for use in treating hyperactivity in a patient diagnosed with foetal alcohol syndrome.

In certain embodiments, the compositions are for use in reducing hyperactivity in the treatment of anxiety. In certain embodiments, the compositions of the invention are for use in treating hyperactivity in a patient diagnosed with anxiety.

In certain embodiments, the compositions are for use in reducing hyperactivity in the treatment of depression. In certain embodiments, the compositions of the invention are for use in treating hyperactivity in a patient diagnosed with depression.

In certain embodiments, the compositions are for use in reducing hyperactivity in the treatment of Tourette's syndrome. In certain embodiments, the compositions of the invention are for use in treating hyperactivity in a patient diagnosed with Tourette's syndrome.

### - ADHD

In certain embodiments, composition of the invention are for use in treating or preventing ADHD. In certain embodiments, the compositions of the invention are for use in the treatment or prevention of hyperactivity in subjects with behavioural disorders. In certain embodiments, the compositions of the invention are for use in the treatment or prevention of hyperactivity in subjects with ADHD. ADHD can manifest in both children and in adults. In some embodiments, the compositions of the invention are for use in the treatment or prevention of ADHD in adults. In some embodiments, the compositions are for use in the treatment or prevention of ADHD in children.

In some embodiments, the compositions are for use in subjects diagnosed with ADHD. Diagnosis of ADHD is complex procedure often involving psychological evaluation of a subject displaying symptoms of ADHD, coupled with physical examination and possibly the detection of biological markers associated with ADHD, such as platelet monoamine oxidase expression, urinary norepinephrine, urinary MHPG, and urinary phenethylamine levels.

Formal diagnosis is typically made by a psychiatric health care professional. Different countries use different metrics for the diagnosis and classification of ADHD. In some countries, diagnosis and classification is made according to the criteria defined by the American Psychiatric Association in the Diagnostic and Statistical Manual of Mental Disorders (DSM). The DSM classifies ADHD in different sub-types depending on the array of symptoms exhibited by the subject. ADHD may be diagnosed as ADHD predominantly inattentive type (ADHD-pi). In certain embodiments, the compositions of the invention are for use in the treatment or prevention of ADHD-pi. In some embodiments, the compositions of the invention are for use in a subject diagnosed with ADHD-pi. In some embodiments, the compositions of the invention are for use in a method of treating a subject diagnosed with ADHD-pi. ADHD may also be diagnosed as ADHD predominantly hyperactive-impulsive type. In some embodiments, the compositions are for use in the treatment or prevention of ADHD predominantly hyperactive-impulsive type. In some embodiments, the compositions of the invention are for use in a subject diagnosed with ADHD predominantly hyperactive-impulsive type. In some embodiments, the compositions of the invention are for use in a method of treatment of a subject diagnosed with ADHD predominantly hyperactive-impulsive type.

Symptoms of ADHD include being easily distracted, forgetful, daydreaming, disorganization, poor concentration, and difficulty completing tasks, with excessive fidgetiness and restlessness, hyperactivity, difficulty waiting and remaining seated, immature behavior. Destructive behaviors may also be present. For symptoms to be associated with ADHD, they must be present for more than six months, and must appear in more than one environment (such as at home and at school or work). In certain embodiments, the compositions are for use in treating or preventing one or more symptoms of ADHD. In certain embodiments, the compositions are for use in the treatment or prevention of a subject displaying one or more symptoms of ADHD. In some embodiments, the compositions of the invention are for use in the treatment of prevention of hyperactivity. In some embodiments, the compositions are for use in a method of reducing hyperactivity in a subject. In some embodiments, the compositions of the invention are for use as anti-hyperactivity medicaments.

Other methods of treatment of ADHD include psychological therapy, behavioral therapy, cognitive behavioral therapy, interpersonal psychotherapy, stimulant medications, such as methtylphenidate, non-stimulant medications, such as atomoxetine, bupropion, guanfacine and clonidine. In certain embodiments, the compositions of the invention are for use in combination with an additional method of treatment for ADHD

### - Obsessive compulsive disorder (OCD)

In certain embodiments, the compositions of the invention are for use in treating or preventing OCD. In certain embodiments, the compositions are for use in reducing hyperactivity in the treatment of OCD. In certain embodiments, the compositions of the invention are for use in treating hyperactivity in a patient diagnosed with OCD.

OCD is a heterogeneous, chronic and disabling disorder belonging to the anxiety disorders. According to the DSM-IV definition, the essential features of OCD are recurrent obsessions and/or compulsions (criterion A) that are severe and time consuming (more than one hour a day) or cause marked distress or significantly interfere with the subject's normal routine, occupational functioning, usual social activities or relationships (criterion C). As some point during the course of the disorder, the person has recognised that the obsessions or compulsions are excessive or unreasonable (criterion B).

Obsessions are defined as recurrent and persistent thoughts, impulses or images that are experienced as intrusive and inappropriate and cause marked anxiety or distress. The thoughts, impulses or images are not simply excessive worries about real-life problems, they are recognised by the patient as a product of his own mind (e.g. fear for contamination, symmetry obsession). The person attempts to ignore, suppress or neutralise the obsessions with some other thoughts or actions.

Compulsions are defined as repetitive behaviours (e.g. hand washing, ordering, hoarding, checking) or mental acts (e.g. praying, counting, repeating words silently) that the person feels driven to perform in response to an obsession or according to rules that must be applied rigidly.

OCD is often associated with co-morbidity rates of other psychiatric diseases including major depressive disorder, other anxiety disorders (generalised anxiety disorder, social anxiety disorder, panic disorder), substance abuse and eating disorders (anorexia and bulimia).

OCD is a psychiatric disorder that may develop or persist due to dysfunction of the microbiota-gut-brain axis. Accordingly, in preferred embodiments, the compositions of the invention are for use in treating or preventing OCD in a subject.

In certain embodiments, the compositions of the invention prevent, reduce or alleviate the essential symptomatic features of OCD. In certain embodiments, the compositions of the invention prevent, reduce or alleviate recurrent obsessions and/or compulsions in a subject. In certain embodiments, the obsessions are recurrent or persistent thoughts, impulses or images that are experiences as intrusive and inappropriate and cause marked anxiety or distress. In certain embodiments, the compulsions are repetitive behaviours that the subject feels driven to perform in response to an obsession or according to rules that must be applied rigidly.

In certain embodiments, the compositions of the invention improve symptoms of OCD in a subject accordingly to the Y-BOCS and/or the NIMH-OC diagnostic and/or symptomatic scales. In some embodiments, the Y-BOCS scale is used to monitor improvement of primary endpoints. In some embodiments, the NIMH-OC scale is used to monitor improvement of secondary parameters.

In some embodiments, the compositions of the invention improve the Clinical Global Impression - Global Improvement (CGI-I) scale for assessing psychiatric and neurological disorders. In some embodiments, the compositions of the invention display a positive effect on global social functioning (relationships, work, *etc*.) of the subject with ASDs. In some embodiments, the global scale is the Sheehan disability scale.

In preferred embodiments, the compositions of the invention prevent, reduce or alleviate at least one comorbidity of OCD. The comorbidities of OCD include major depressive disorder, other anxiety disorders (generalised anxiety disorder, social anxiety disorder, panic disorder), substance abuse and eating disorders (anorexia and bulimia) Gilles de la Tourette syndrome, ADHD (Attention-Deficit/Hyperactivity Disorder) and developmental disorders.

In some embodiments, the compositions of the invention are particularly effective at preventing, reducing or alleviating OCD when used in combination with another therapy for treating OCD. Such therapies include serotonin and dopamine reuptake inhibitors; clomipramine and anti-psychotics.

### - Anxiety disorders

In certain embodiments, the compositions of the invention are for use in treating or preventing anxiety disorders. In certain embodiments, the compositions are for use in reducing hyperactivity in the treatment of an anxiety disorder. In certain embodiments, the compositions of the invention are for use in treating hyperactivity in a patient diagnosed with an anxiety disorder.

Anxiety disorders are a group of mental disorders characterised by feelings of anxiety and fear. There are a number of anxiety disorders including generalised anxiety disorder (GAD); specific phobia; social anxiety disorder; separation anxiety disorder; agroraphobia; panic disorder and selective mutism.

GAD is diagnosed according to DMS-5 in six criterion. The first criterion is too much anxiety or worry over more than six months wherein the anxiety or worry is present most of the time in regards to many activities. The second criterion is that the subject is unable to manage the symptoms of the first criterion. The third criterion is that at least three (one in children) of the following occurs: restlessness; tires easily; problems concentrating; irritability; muscle tension and problems with sleep. The final three criterion are that the symptoms results in significant social, occupational and functional impairment; the symptoms are not due to medications, drugs, or other physical health problems; and the symptoms do not fit better with another psychiatric problem such as panic disorder. All other anxiety disorders may be considered as differential diagnoses of GAD.

GAD is frequently associated with a wide spectrum of other mental disorders as comorbidities including depression; substance use disorders; stress; IBS; insomnia; headaches; pain; cardiac events; interpersonal problems and ADHD

Anxiety disorders are psychiatric disorders that may develop or persist due to dysfunction of the microbiota-gut-brain axis. Accordingly, in preferred embodiments, the compositions of the invention are for use in treating or preventing anxiety disorders in a subject. In certain embodiments, the anxiety disorder is generalised anxiety disorder (GAD); specific phobia; social anxiety disorder; separation anxiety disorder; agoraphobia; panic disorder and selective mutism.

In certain embodiments, the compositions of the invention prevent, reduce or alleviate one or more of the symptoms of GAD in a subject as classified by the DMS-5 criteria listed herein. According to DMS-5, the same symptoms are associated with other anxiety disorders. Therefore, in certain embodiments, the compositions of the invention prevent, reduce or alleviate one or more of the symptoms of anxiety disorders in a subject. In preferred embodiments, the compositions of the invention prevent, reduce or alleviate the anxiety or worry of the subject. In certain embodiments, the compositions of the invention reduce the occurrence of symptoms within a six month period. In certain embodiments, the composition of the invention prevents, reduces or alleviates restlessness; fatigue, loss of concentration; irritability; muscle tension; and/or problems with sleep. In some embodiments, the compositions of the invention prevent, reduce or alleviate social, occupational and functional impairment associated with anxiety disorders.

In some embodiments, the compositions of the invention improve the symptoms of anxiety disorders according to a symptomatic or diagnostic scale. In certain embodiments, the scale for assessing symptomatic improvement includes the Hamilton Anxiety Rating Scale (HAM-A). In some embodiments, the HAM-A total scale is used to assess primary endpoint. In other embodiments, the HAM-A psychic anxiety factor may be useful as a secondary endpoint.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with anxiety disorder. In some embodiments, the global scale is the Sheehan disability scale.

In preferred embodiments, the compositions of the invention prevent, reduce or alleviate at least one comorbidity of GAD and anxiety disorders. The comorbidities of GAD include depression; substance use disorders; stress; IBS; insomnia; headaches; pain; cardiac events; interpersonal problems and ADHD

In some embodiments, the compositions of the invention are particularly effective at preventing, reducing or alleviating anxiety disorders when used in combination with another therapy for treating anxiety disorders. Such therapies include selective serotonin reuptake inhibitors (venlafaxine, duloxetine, escitalopram and paroxetine); benzodiazepines (alprazolam, lorazepam and clonazepam); pregabalin (Lyrica^{®}) and gabapentin (Neurontin ^{®}); serotonin receptor partial agonists (buspirone and tandospirone); atypical serotonergic antidepressants (such as imipramine and clomipramine); monoamine oxidase inhibitors (MAOIs) (such as moclobemide and phenelzine); hydroxyzine; propranolol; clonidine; guanfacine and prazosin.

### - Post-traumatic stress disorder (PTSD)

In certain embodiments, the compositions of the invention are for use in treating or preventing PTSD. In certain embodiments, the compositions are for use in reducing hyperactivity in the treatment of PTSD. In certain embodiments, the compositions of the invention are for use in treating hyperactivity in a patient diagnosed with PTSD.

PTSD is a severe and disabling disorder, an essential feature of which is the inclusion of a traumatic event as a precipitating factor of this disorder.

The symptoms of PTSD are grouped into four main clusters according to the DMS-V criteria: (i) intrusion: examples include nightmares, unwanted thoughts of the traumatic events, flashbacks, and reacting to traumatic reminders with emotional distress or physiological reactivity; (ii) avoidance: examples include avoiding triggers for traumatic memories including places, conversations, or other reminders; (iii) negative alterations in cognitions and mood: examples include distorted blame of self or others for the traumatic event, negative beliefs about oneself or the world, persistent negative emotions (e.g., fear, guilt, shame), feeling alienated, and constricted affect (e.g., inability to experience positive emotions); (iv) alterations in arousal and reactivity: examples include angry, reckless, or self-destructive behaviour, sleep problems, concentration problems, increased startle response, and hypervigilance.

Symptoms that resolve within 4 weeks of the traumatic event meet the criteria for an Acute Stress Disorder. The DSM distinguishes between acute (duration of symptoms for less than three months) and chronic PTSD (duration of symptoms longer than 3 months). If the symptoms begin more than 6 months after the stressor, the disorder is defined as delayed onset PTSD.

PTSD carries high comorbidities with major depressive disorder and substance use disorders.

PTSD is a psychiatric disorder that may develop or persist due to dysfunction of the microbiota-gut-brain axis. Accordingly, in preferred embodiments, the compositions of the invention are for use in treating or preventing PTSD in a subject. According to a similar pathogenesis, in certain embodiments, the compositions of the invention are for use in treating or preventing stress disorders. In certain embodiments, the compositions of the invention treat acute stress disorder. In some embodiments, the compositions of the invention treat acute and/or chronic PTSD. In some embodiments, the compositions of the invention treat delayed onset PTSD.

In certain embodiments, the compositions of the invention prevent, reduce or alleviate one or more of the symptoms of PTSD (or stress disorder) in a subject as classified by the DMS-5 criteria listed herein. In preferred embodiments, the compositions of the invention prevent, reduce or alleviate intrusive thoughts in a subject with PTSD. In preferred embodiments, the compositions of the invention prevent, reduce or alleviate avoidance behaviour in a subject with PTSD. In preferred embodiments, the compositions of the invention prevent, reduce or alleviate negative alterations in cognitions and mood in a subject with PTSD. In preferred embodiments, the compositions of the invention prevent alterations in arousal and reactivity in a subject with PTSD.

In some embodiments, the compositions of the invention improve the symptoms of PTSD and stress disorders according to a symptomatic or diagnostic scale. In certain embodiments, the scale for assessing symptomatic improvement is the Clinical-Administered PTSD (CAPS) scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairment of the subject with PTSD and stress disorders. In some embodiments, the global scale is the Sheehan disability scale.

In preferred embodiments, the compositions of the invention prevent, reduce or alleviate at least one comorbidity of PTSD and stress disorders . The comorbidities of PTSD and stress disorders include MDD, substance use disorders; stress and anxiety.

In some embodiments, the compositions of the invention are particularly effective at preventing, reducing or alleviating PTSD and stress disorders when used in combination with another therapy for treating PTSD and stress disorders . Such therapies include serotoninergic agents, tricyclic antidepressants, mood stabilisers, adrenergic inhibiting agents, antipsychotics, benzodiazepines, sertraline (Zoloft^{®}), fluoxetine (Prozac^{®}) and/or paroxetine (Paxil^{®}).

### - Bipolar disorder

In certain embodiments, the compositions of the invention are for use in treating or preventing bipolar disorder. In certain embodiments, the compositions are for use in reducing hyperactivity in the treatment of bipolar disorder. In certain embodiments, the compositions of the invention are for use in treating hyperactivity in a patient diagnosed with bipolar disorder.

Bipolar disorder in general is a chronic disease. Mania is the cardinal symptom of bipolar disorder, There are several types of bipolar disorder based upon the specific duration and pattern of manic and depressive episodes. In DMS-5, a distinction is made between bipolar I disorder, bipolar II disorder, cyclothymic disorder, rapid-cycling bipolar disorder and bipolar disorder NOS.

According to the DSM, mania is a distinct period of abnormally and persistently elevated, expansive, or irritable mood. The episode must last a week, and the mood must have at least three of the following symptoms: high self-esteem; reduced need for sleep; increase rate of speech; rapid jumping of ideas; easily distracted; an increased interest in goals or activities; psychomotor agitation; increased pursuit of activities with a high risk of danger.

Bipolar I disorder involves one or more manic or mixed (mania and depression) episodes and at least one major depressive episode (see above for symptoms of MDD episodes). Bipolar lI disorder has one or more major depressive episodes accompanied by at least one hypomanic episode. There are no manic or mixed episodes. Hypomania is a lesser form of mania. The symptoms are responsible for significant social, occupational and functional impairments. Cyclothymia is characterized by changing low-level depression along with periods of hypomania. The symptoms must be present for at least two years in adults or one year in children before a diagnosis can be made. Symptom free periods in adults and children last no longer than two months or one month, respectively. Rapid cycling bipolar disorder is a severe form of bipolar disorder. It occurs when a person has at least four episodes of major depression, mania, hypomania, or mixed states within a year. Not-otherwise specified (NOS) bipolar disorder classified bipolar symptoms that do not clearly fit into other types. NOS is diagnosed when multiple bipolar symptoms are present but not enough to meet the label for any of the other subtypes.

Bipolar disorder is associated with the following comorbidities: ADHD; anxiety disorders; substance disorders; obesity and metabolic syndrome.

Bipolar disorder is a psychiatric disorder that may develop or persist due to dysfunction of the microbiota-gut-brain axis. Therefore, in preferred embodiments, the compositions of the invention are for use in treating or preventing bipolar disorder in a subject. In certain embodiments, the bipolar disorder is bipolar I disorder. In certain embodiments, the bipolar disorder is bipolar II disorder. In certain embodiments, the bipolar disorder is cyclothymic disorder . In certain embodiments, the bipolar disorder is rapid-cycling bipolar disorder. In certain embodiments, the bipolar disorder is bipolar disorder NOS.

In preferred embodiments, the compositions of the invention prevent, reduce or alleviate one or more of the symptoms of bipolar disorder in a subject. In certain embodiments, the compositions of the invention prevent, reduce or alleviate the occurrence of manic episodes in a subject. In certain embodiments, the compositions of the invention prevent, reduce or alleviate the occurrence of an abnormally and persistently elevated, expansive, or irritable mood. In certain embodiments, the compositions of the invention prevent, reduce or alleviate one or more of the following symptoms: high self-esteem; reduced need for sleep; increase rate of speech; rapid jumping of ideas; easily distracted; an increased interest in goals or activities; psychomotor agitation; increased pursuit of activities with a high risk of danger. In certain embodiments, the compositions of the invention prevent, reduce or alleviate the occurrence of one or more manic or mixed episodes in a subject. In certain embodiments, the compositions of the invention reduce the occurrence of at least one major depressive episode in a subject. In certain embodiments, the compositions of the invention prevent, reduce or alleviate the occurrence of at least one major depressive episode accompanied by at least one hypomanic episode.

In preferred embodiments, the compositions of the invention treat the acute phase of bipolar disorder and/or prevent the occurrence of further episodes. In certain embodiments, the compositions of the invention treat the acute phase of manic/depressive episodes in a subject with bipolar disorder and prevent occurrence of further manic/depressive episodes.

In some embodiments, the compositions of the invention improve the symptoms of bipolar disorder according to a symptomatic or diagnostic scale. In certain embodiments, the scale for assessing symptomatic improvement of manic episodes is the Manic State Rating Scale and the Young Mania Rating Scale. In certain embodiments, the scale is the Bech-Rafaelsen Mania Scale (BRMAS). In certain embodiments, scales for assessing symptomatic improvement of the switch from manic to depressive episodes include the Hamilton Depression Rating Scale, the Montgomery-Asberg Rating Scale, and the Bech-Rafaelsen Depression Scale.

In some embodiments, the compositions of the invention improve the Clinical Global Impression - Global Improvement (CGI-I) scale for assessing psychiatric and neurological disorders. In some embodiments, the compositions of the invention display a positive effect on global social, occupational and functional impairments of the subject with bipolar disorder

In preferred embodiments, the compositions of the invention prevent, reduce or alleviate at least one comorbidity of bipolar disorder. In certain embodiments, the comorbidity is selected from ADHD, anxiety disorders, substance disorder, obesity and metabolic syndrome.

In certain embodiments, the compositions of the invention are for use in treating or preventing manic-depressive illness and bipolar disorder unresponsive to lithium and divalproex.

In some embodiments, the compositions of the invention are particularly effective at preventing, reducing or alleviating bipolar disorder when used in combination with another therapy for treating bipolar disorder. In certain embodiments, such therapies include lithium carbonate, anticonvulsant drugs (including valproate, divalproex, carbamazepine and lamotrigine) and antipsychotic drugs (including aripiprazole, olanzapine, quetiapine and risperidone).

### Cancer

HDAC function and expression is perturbed in a variety of cancers and often leads to poor prognosis. HDAC function in cancer is associated with the aberrant expression or function of genes that promote cellular proliferation and tumorigenic phenotypes. In certain cancers HDACs primarily regulate the onset of cancer and are described as oncogenes. In other cancers onco-fusion proteins recruit Class I HDACs to repress the expression of genes that regulate cellular differentiation or cell cycle control, leading to cellular transformation. The knockdown or inhibition of HDAC expression has been shown to have multiple anti-cancer effects, such as cell cycle arrest and inhibition of proliferation, apoptosis, differentiation and senescence and disruption of angiogenesis. Therefore, the compositions of the invention may be useful in the treatment of cancers mediated by HDAC activity, by inhibiting HDAC activity.

In certain embodiments, the compositions of the invention are for use in treating or preventing cancer. In certain embodiments, the composition of the invention are for use in treating or preventing cancers mediated by HDAC activity. In certain embodiments, the compositions of the invention are for use in treating or preventing colorectal cancer,

In certain embodiments, treatment with the compositions of the invention results in a reduction in tumour size or a reduction in tumour growth. In certain embodiments, the compositions of the invention are for use in reducing tumour size or reducing tumour growth. The compositions of the invention may be effective for reducing tumour size or growth. In certain embodiments, the compositions of the invention are for use in patients with solid tumours. In certain embodiments, the compositions of the invention are for use in reducing or preventing angiogenesis in the treatment of cancer. Genes regulated by HDACs have central roles in angiogenesis. In certain embodiments, the compositions of the invention are for use in preventing metastasis.

In certain embodiments, the compositions of the invention are for use in treating or preventing gastric cancer. HDAC2 has been shown to play a functional role in the development of gastric cancers and colorectal tumorigenesis [77,78]. In mice models of colorectal cancer, inhibition of HDAC2 resulted in a reduced rates of tumour development. In certain embodiments, the compositions of the invention that selectively inhibit HDAC2 are for use in treating or preventing colorectal cancer, in particular colorectal cancer mediated by HDAC2 activity.

In certain embodiments, the compositions of the invention are for use in treating or preventing breast cancer. The compositions of the invention may be effective for treating breast cancer, and HDACs have been shown to be upregulated in breast cancer [79] In certain embodiments, the compositions of the invention are for use in reducing tumour size, reducing tumour growth, or reducing angiogenesis in the treatment of breast cancer.

In certain embodiments, the compositions of the invention are for use in treating or preventing prostate cancer. The compositions of the invention may be effective for treating prostate cancer, as HDAC activity play a major role in the development of prostate cancer [80]. In certain embodiments, the compositions of the invention are for use in reducing tumour size, reducing tumour growth, or reducing angiogenesis in the treatment of prostate cancer. In certain embodiments, the cancer is hormone refractory prostate cancer.

In certain embodiments, the compositions of the invention are for use in treating or preventing lung cancer. The compositions of the invention may be effective for treating lung cancer, and HDACs have been shown to be upregulated in lung cancer [81]. In certain embodiments, the compositions of the invention are for use in reducing tumour size, reducing tumour growth, or reducing angiogenesis in the treatment of lung cancer. In preferred embodiments the cancer is lung carcinoma. In preferred embodiments, the compositions are for use in the treatment of lung cancer with high levels of expression of HDAC2. Certain lung cancer tissues have be shown to abundantly express HDAC2. Inactivation of HDAC2 represses lung cancer cell growth. High levels of HDAC2 activity has been shown to repress p53 activity [82]. Active p53 arrests cell division and ultimately leads to the onset of apoptosis. In certain embodiments, compositions of the invention that inhibit HDAC2 are for use in the treatment of lung cancers with high levels of HDAC2 activity.

In certain embodiments, the compositions of the invention are for use in treating or preventing liver cancer. The compositions of the invention may be effective for treating liver cancer, and HDACs have been shown to be upregulated in liver cancer [83]. In certain embodiments, the compositions of the invention are for use in reducing tumour size, reducing tumour growth, or reducing angiogenesis in the treatment of liver cancer. In preferred embodiments the cancer is hepatoma (hepatocellular carcinoma). In certain embodiments, the cancer is a low-grade or early-stage tumour

In certain embodiments, the compositions of the invention are for use in treating or preventing carcinoma. The compositions of the invention may be particularly effective for treating carcinoma. In certain embodiments, the compositions of the invention are for use in treating or preventing non-immunogenic cancer. The compositions of the invention may be effective for treating non-immunogenic cancers.

In further embodiments, the compositions of the invention are for use in treating or preventing acute lymphoblastic leukemia (ALL), acute myeloid leukemia, adrenocortical carcinoma, basal-cell carcinoma, bile duct cancer, bladder cancer, bone tumor, osteosarcoma/malignant fibrous histiocytoma, brainstem glioma, brain tumor, cerebellar astrocytoma, cerebral astrocytoma/malignant glioma, ependymoma, medulloblastoma, supratentorial primitive neuroectodermal tumors, breast cancer, bronchial adenomas/carcinoids, Burkitt's lymphoma, carcinoid tumor, cervical cancer, chronic lymphocytic leukemia, chronic myelogenous leukemia, chronic myeloproliferative disorders, colon cancer, cutaneous T-cell lymphoma, endometrial cancer, ependymoma, esophageal cancer, Ewing's sarcoma, intraocular melanoma, retinoblastoma, gallbladder cancer, gastric cancer, gastrointestinal carcinoid tumor, gastrointestinal stromal tumor (GIST), germ cell tumor, glioma, childhood visual pathway and hypothalamic, Hodgkin lymphoma, melanoma, islet cell carcinoma, Kaposi sarcoma, renal cell cancer, laryngeal cancer, leukaemias, lymphomas, mesothelioma, neuroblastoma, non-Hodgkin lymphoma, oropharyngeal cancer, osteosarcoma, ovarian cancer, pancreatic cancer, parathyroid cancer, pharyngeal cancer, pituitary adenoma, plasma cell neoplasia, prostate cancer, renal cell carcinoma, retinoblastoma, sarcoma, testicular cancer, thyroid cancer, or uterine cancer.

The compositions of the invention may be particularly effective when used in combination with further therapeutic agents. The HDAC inhibitory effects of the compositions of the invention may be effective when combined with more direct anti-cancer agents. Therefore, in certain embodiments, the invention provides a composition comprising a bacterial strain of the genus *Anaerostipes* and an anticancer agent. In preferred embodiments the anticancer agent is an immune checkpoint inhibitor, a targeted antibody immunotherapy, a CAR-T cell therapy, an oncolytic virus, or a cytostatic drug. In preferred embodiments, the composition comprises an anti-cancer agent selected from the group consisting of: Yervoy (ipilimumab, BMS); Keytruda (pembrolizumab, Merck); Opdivo (nivolumab, BMS); MEDI4736 (AZ/MedImmune); MPDL3280A (Roche/Genentech); Tremelimumab (AZ/MedImmune); CT-011 (pidilizumab, CureTech); BMS-986015 (lirilumab, BMS); MEDI0680 (AZ/MedImmune); MSB-0010718C (Merck); PF-05082566 (Pfizer); MEDI6469 (AZ/MedImmune); BMS-986016 (BMS); BMS-663513 (urelumab, BMS); IMP321 (Prima Biomed); LAG525 (Novartis); ARGX-110 (arGEN-X); PF-05082466 (Pfizer); CDX-1127 (varlilumab; CellDex Therapeutics); TRX-518 (GITR Inc.); MK-4166 (Merck); JTX-2011 (Jounce Therapeutics); ARGX-115 (arGEN-X); NLG-9189 (indoximod, NewLink Genetics); INCB024360 (Incyte); IPH2201 (Innate Immotherapeutics/AZ); KLG-919 (NewLink Genetics); anti-VISTA (JnJ); Epacadostat (INCB24360, Incyte); F001287 (Flexus/BMS); CP 870893 (University of Pennsylvania); MGA271 (Macrogenix); Emactuzumab (Roche/Genentech); Galunisertib (Eli Lilly); Ulocuplumab (BMS); BKT140/BL8040 (Biokine Therapeutics); Bavituximab (Peregrine Pharmaceuticals), CC 90002 (Celgene); 852A (Pfizer); VTX-2337 (VentiRx Pharmaceuticals); IMO-2055 (Hybridon, Idera Pharmaceuticals); LY2157299 (Eli Lilly), EW-7197 (Ewha Women's University, Korea); Vemurafenib (Plexxikon); Dabrafenib (Genentech/GSK); BMS-777607 (BMS); BLZ945 (Memorial Sloan-Kettering Cancer Centre); Unituxin (dinutuximab, United Therapeutics Corporation); Blincyto (blinatumomab, Amgen), Cyramza (ramucirumab, Eli Lilly); Gazyva (obinutuzumab, Roche/Biogen); Kadcyla (ado-trastuzumab emtansine, Roche/Genentech); Perjeta (pertuzumab, Roche/Genentech); Adcetris (brentuximab vedotin, Takeda/Millennium); Arzerra (ofatumumab, GSK); Vectibix (panitumumab, Amgen); Avastin (bevacizumab, Roche/Genentech); Erbitux (cetuximab, BMS/Merck); Bexxar (tositumomab-I131, GSK); Zevalin (ibritumomab tiuxetan, Biogen); Campath (alemtuzumab, Bayer); Mylotarg (gemtuzumab ozogamicin, Pfizer); Herceptin (trastuzumab, Roche/Genentech); Rituxan (rituximab, Genentech/Biogen); volociximab (Abbvie); Enavatuzumab (Abbvie); ABT-414 (Abbvie); Elotuzumab (Abbvie/BMS); ALX-0141 (Ablynx); Ozaralizumab (Ablynx); Actimab-C (Actinium); Actimab-P (Actinium); Milatuzumab-dox (Actinium); Emab-SN-38 (Actinium); Naptumonmab estafenatox (Active Biotech); AFM13 (Affimed); AFM11 (Affimed); AGS-16C3F (Agensys); AGS-16M8F (Agensys); AGS-22ME (Agensys); AGS-15ME (Agensys); GS-67E (Agensys); ALXN6000 (samalizumab, Alexion); ALT-836 (Altor Bioscience); ALT-801 (Altor Bioscience); ALT-803 (Altor Bioscience); AMG780 (Amgen); AMG 228 (Amgen); AMG820 (Amgen); AMG172 (Amgen); AMG595 (Amgen); AMG110 (Amgen); AMG232 (adecatumumab, Amgen); AMG211 (Amgen/MedImmune); BAY20-10112 (Amgen/Bayer); Rilotumumab (Amgen); Denosumab (Amgen); AMP-514 (Amgen); MEDI575 (AZ/MedImmune); MEDI3617 (AZ/MedImmune); MEDI6383 (AZ/MedImmune); MEDI551 (AZ/MedImmune); Moxetumomab pasudotox (AZ/Medlmmune); MEDI565 (AZ/Medlmmune); MEDI0639 (AZ/MedImmune); MEDI0680 (AZ/Medlmmune); MEDI562 (AZ/MedImmune); AV-380 (AVEO); AV203 (AVEO); AV299 (AVEO); BAY79-4620 (Bayer); Anetumab ravtansine (Bayer); vantictumab (Bayer); BAY94-9343 (Bayer); Sibrotuzumab (Boehringer Ingleheim); BI-836845 (Boehringer Ingleheim); B-701 (BioClin); BIIB015 (Biogen); Obinutuzumab (Biogen/Genentech); BI-505 (Bioinvent); BI-1206 (Bioinvent); TB-403 (Bioinvent); BT-062 (Biotest) BIL-010t (Biosceptre); MDX-1203 (BMS); MDX-1204 (BMS); Necitumumab (BMS); CAN-4 (Cantargia AB); CDX-011 (Celldex); CDX1401 (Celldex); CDX301 (Celldex); U3-1565 (Daiichi Sankyo); patritumab (Daiichi Sankyo); tigatuzumab (Daiichi Sankyo); nimotuzumab (Daiichi Sankyo); DS-8895 (Daiichi Sankyo); DS-8873 (Daiichi Sankyo); DS-5573 (Daiichi Sankyo); MORab-004 (Eisai); MORab-009 (Eisai); MORab-003 (Eisai); MORab-066 (Eisai); LY3012207 (Eli Lilly); LY2875358 (Eli Lilly); LY2812176 (Eli Lilly); LY3012217(Eli Lilly); LY2495655 (Eli Lilly); LY3012212 (Eli Lilly); LY3012211 (Eli Lilly); LY3009806 (Eli Lilly); cixutumumab (Eli Lilly); Flanvotumab (Eli Lilly); IMC-TR1 (Eli Lilly); Ramucirumab (Eli Lilly); Tabalumab (Eli Lilly); Zanolimumab (Emergent Biosolution); FG-3019 (FibroGen); FPA008 (Five Prime Therapeutics); FP-1039 (Five Prime Therapeutics); FPA144 (Five Prime Therapeutics), catumaxomab (Fresenius Biotech); IMAB362 (Ganymed); IMAB027 (Ganymed); HuMax-CD74 (Genmab); HuMax-TFADC (Genmab); GS-5745 (Gilead); GS-6624 (Gilead); OMP-21M18 (demcizumab, GSK); mapatumumab (GSK); IMGN289 (ImmunoGen); IMGN901 (ImmunoGen); IMGN853 (ImmunoGen); IMGN529 (ImmunoGen); IMMU-130 (Immunomedics); milatuzumab-dox (Immunomedics); IMMU-115 (Immunomedics); IMMU-132 (Immunomedics); IMMU-106 (Immunomedics), IMMU-102 (Immunomedics); Epratuzumab (Immunomedics); Clivatuzumab (Immunomedics); IPH41 (Innate Immunotherapeutics); Daratumumab (Janssen/Genmab); CNTO-95 (Intetumumab, Janssen); CNTO-328 (siltuximab, Janssen); KB004 (KaloBios); mogamulizumab (Kyowa Hakko Kirrin); KW-2871 (ecromeximab, Life Science); Sonepcizumab (Lpath); Margetuximab (Macrogenics); Enoblituzumab (Macrogenics); MGD006 (Macrogenics); MGF007 (Macrogenics); MK-0646 (dalotuzumab, Merck); MK-3475 (Merck); Sym004 (Symphogen/Merck Serono); DI17E6 (Merck Serono); MOR208 (Morphosys); MOR202 (Morphosys); Xmab5574 (Morphosys); BPC-1C (ensituximab, Precision Biologics); TAS266 (Novartis); LFA102 (Novartis); BHQ880 (Novartis/Morphosys); QGE031 (Novartis); HCD122 (lucatumumab, Novartis); LJM716 (Novartis); AT355 (Novartis); OMP-21M18 (Demcizumab, OncoMed); OMP52M51 (Oncomed/GSK); OMP-59R5 (Oncomed/GSK); vantictumab (Oncomed/Bayer); CMC-544 (inotuzumab ozogamicin, Pfizer); PF-03446962 (Pfizer); PF-04856884 (Pfizer); PSMA-ADC (Progenics); REGN1400 (Regeneron); REGN910 (nesvacumab, Regeneron/Sanofi); REGN421 (enoticumab, Regeneron/Sanofi); RG7221, RG7356, RG7155, RG7444, RG7116, RG7458, RG7598, RG7599, RG7600, RG7636, RG7450, RG7593, RG7596, DCDS3410A, RG7414 (parsatuzumab), RG7160 (imgatuzumab), RG7159 (obintuzumab), RG7686, RG3638 (onartuzumab), RG7597 (Roche/Genentech); SAR307746 (Sanofi); SAR566658 (Sanofi); SAR650984 (Sanofi); SAR153192 (Sanofi); SAR3419 (Sanofi); SAR256212 (Sanofi), SGN-LIV1A (lintuzumab, Seattle Genetics); SGN-CD33A (Seattle Genetics); SGN-75 (vorsetuzumab mafodotin, Seattle Genetics); SGN-19A (Seattle Genetics) SGN-CD70A (Seattle Genetics); SEA-CD40 (Seattle Genetics); ibritumomab tiuxetan (Spectrum); MLN0264 (Takeda); ganitumab (Takeda/Amgen); CEP-37250 (Teva); TB-403 (Thrombogenic); VB4-845 (Viventia); Xmab2512 (Xencor); Xmab5574 (Xencor); nimotuzumab (YM Biosciences); Carlumab (Janssen); NY-ESO TCR (Adaptimmune); MAGE-A-10 TCR (Adaptimmune); CTL019 (Novartis); JCAR015 (Juno Therapeutics); KTE-C19 CAR (Kite Pharma); UCART19 (Cellectis); BPX-401 (Bellicum Pharmaceuticals); BPX-601 (Bellicum Pharmaceuticals); ATTCK20 (Unum Therapeutics); CAR-NKG2D (Celyad); Onyx-015 (Onyx Pharmaceuticals); H101 (Shanghai Sunwaybio); DNX-2401 (DNAtrix); VCN-01 (VCN Biosciences); Colo-Adl (PsiOxus Therapeutics); ProstAtak (Advantagene); Oncos-102 (Oncos Therapeutics); CG0070 (Cold Genesys); Pexa-vac (JX-594, Jennerex Biotherapeutics); GL-ONC1 (Genelux); T-VEC (Amgen); G207 (Medigene); HF10 (Takara Bio); SEPREHVIR(HSV1716, Virttu Biologics); OrienX010 (OrienGene Biotechnology); Reolysin (Oncolytics Biotech); SVV-001 (Neotropix); Cacatak (CVA21, Viralytics); Alimta (Eli Lilly), cisplatin, oxaliplatin, irinotecan, folinic acid, methotrexate, cyclophosphamide, 5-fluorouracil, Zykadia (Novartis), Tafinlar (GSK), Xalkori (Pfizer), Iressa (AZ), Gilotrif (Boehringer Ingelheim), Tarceva (Astellas Pharma), Halaven (Eisai Pharma), Veliparib (Abbvie), AZD9291 (AZ), Alectinib (Chugai), LDK378 (Novartis), Genetespib (Synta Pharma), Tergenpumatucel-L (NewLink Genetics), GV1001 (Kael-GemVax), Tivantinib (ArQule); Cytoxan (BMS); Oncovin (Eli Lilly); Adriamycin (Pfizer); Gemzar (Eli Lilly); Xeloda (Roche); Ixempra (BMS); Abraxane (Celgene); Trelstar (Debiopharm); Taxotere (Sanofi); Nexavar (Bayer); IMMU-132 (Immunomedics); E7449 (Eisai); Thermodox (Celsion); Cometriq (Exellxis); Lonsurf (Taiho Pharmaceuticals); Camptosar (Pfizer); UFT (Taiho Pharmaceuticals); and TS-1 (Taiho Pharmaceuticals).

### Modes of administration

Preferably, the compositions of the invention are to be administered to the gastrointestinal tract in order to enable delivery to and/or partial or total colonisation of the intestine with the bacterial strain of the invention. Preferably, the compositions of the invention are formulated to be administered to the gastrointestinal tract in order to enable delivery to and/or partial or total colonisation of the intestine with the bacterial strain of the invention. In some embodiments, the term "total colonisation of the intestine" means that bacteria have colonised all parts of the intestine (i.e. the small intestine, large intestine and rectum). In further embodiments of the invention, the term "total colonisation" or "partial colonisation" means that the bacteria are retained permanently or temporarily in the intestine, respectively. Generally, the compositions of the invention are administered orally, but they may be administered rectally, intranasally, or via buccal or sublingual routes. In other words, the bacteria may have colonised some or all of the gastrointestinal tract and / or such colonisation may be transient or permanent.

More specifically, in some embodiments, the "total colonisation of the intestine" means that bacteria have colonised all parts of the intestine (i.e. the small intestine, large intestine and rectum). Additionally or alternatively, the term "total colonisation" means that the bacteria engraft permanently in the some or all parts of the intestine.

In some embodiments, "partial colonisation of the intestine" means that bacteria have colonised some but not all parts of the intestine. Additionally or alternatively, the term "partial colonisation" means that the bacteria engraft transiently in some or all parts of the intestine.

The transience of engraftment can be determined by assessing (e.g. in a fecal sample) the abundance of the bacterial strain of the invention periodically (e.g. daily) following the end of a dosing interval to determine the washout period, i.e. the period between conclusion of the dosing interval and there being no detectable levels of the bacterial strain of the invention present. In embodiments of the invention, the washout period is 14 days or less, 12 days or less, 10 days or less, 7 days or less, 4 days or less, 3 days or less, 2 days or less or 1 day or less.

In embodiments of the invention, the bacteria of the present invention engraft transiently in the large intestine.

In certain embodiments, the compositions of the invention may be administered as a foam, as a spray or a gel.

In certain embodiments, the compositions of the invention may be administered as a suppository, such as a rectal suppository, for example in the form of a theobroma oil (cocoa butter), synthetic hard fat (e.g. suppocire, witepsol), glycero-gelatin, polyethylene glycol, or soap glycerin composition.

In certain embodiments, the compositions of the invention are administered to the gastrointestinal tract via a tube, such as a nasogastric tube, orogastric tube, gastric tube, jejunostomy tube (J tube), percutaneous endoscopic gastrostomy (PEG), or a port, such as a chest wall port that provides access to the stomach, jejunum and other suitable access ports.

The compositions of the invention may be administered once, or they may be administered sequentially as part of a treatment regimen. In certain embodiments, the compositions of the invention are to be administered daily (either once or several times). In certain embodiments, the compositions disclosed herein are administered regularly, such as daily, every two days, or weekly, for an extended period of time, such as for at least one week, two weeks, one month, two months, six months, or one year.

In some embodiments the compositions disclosed herein are administered for 7 days, 14 days, 16 days, 21 days or 28 days or no more than 7 days, 14 days, 16 days, 21 days or 28 days. For example, in some embodiments the compositions disclosed herein are administered for 16 days.

In certain embodiments of the invention, treatment according to the invention is accompanied by assessment of the patient's gut microbiota. Treatment may be repeated if delivery of and / or partial or total colonisation with the strain of the invention is not achieved such that efficacy is not observed, or treatment may be ceased if delivery and/or partial or total colonisation is successful and efficacy is observed.

In certain embodiments, the composition of the invention may be administered to a pregnant animal, for example a mammal such as a human in order to prevent an inflammatory or autoimmune disease; or a central nervous system disorder or condition (such as those disclosed herein) developing in her child *in utero* and/or after it is born.

The compositions of the invention may be administered to a patient that has been diagnosed with: a disease or condition mediated by histone deacetylase activity, or that has been identified as being at risk of a disease or condition mediated by histone deacetylase activity; or a central nervous system disorder or condition (such as those disclosed herein). The compositions may also be administered as a prophylactic measure to prevent the development of diseases or conditions mediated by histone deacetylase activity in a healthy patient.

The compositions disclosed herein may be administered to a patient that has been diagnosed with a central nervous system disorder or condition, in particular a central nervous system disorder or condition mediated by the microbiota-gut-brain axis, or that has been identified as being at risk of a central nervous system disorder or condition, in particular central nervous system disorder or condition mediated by the microbiota-gut-brain axis. The compositions may also be administered as a prophylactic measure to prevent the development of central nervous system disorders or conditions, in particular central nervous system disorders or conditions mediated by the microbiota-gut-brain axis in a healthy patient.

The compositions of the invention may be administered to a patient that has been identified as having an abnormal gut microbiota. For example, the patient may have reduced or absent colonisation by *Anaerostipes,* in particular *Anaerostipes hadrus*; and/or *Eubacterium* or *Faecalicatena*, in particular *Eubacterium callanderi.*

The compositions of the invention may be administered as a food product, such as a nutritional supplement.

Generally, the compositions of the invention are for the prevention or treatment of human diseases, although they may be used to treat animals including monogastric mammals such as poultry, pigs, cats, dogs, horses or rabbits. The compositions of the invention may be useful for enhancing the growth and performance of animals. If administered to animals, oral gavage may be used.

In some embodiments, the subject to whom the composition is to be administered is an adult human. In some embodiments, the subject to whom the composition is to be administered is an infant human.

### Compositions

The compositions of the invention comprise bacteria. In preferred embodiments of the invention, the composition is formulated in freeze-dried form. The composition of the invention may comprise granules or gelatin capsules, for example hard gelatin capsules, comprising a bacterial strain of the invention.

Preferably, the composition of the invention comprises lyophilised bacteria. Lyophilisation of bacteria is a well-established procedure and relevant guidance is available in, for example, references [84,86].The examples demonstrate that lyophilised compositions are particularly effective.

Alternatively, the composition of the invention may comprise a live, active bacterial culture. The examples demonstrate that cultures of the bacteria of the invention are therapeutically effective.

In some embodiments, the bacterial strain in the composition of the invention has not been inactivated, for example, has not been heat-inactivated. In some embodiments, the bacterial strain in the composition of the invention has not been killed, for example, has not been heat-killed. In some embodiments, the bacterial strain in the composition of the invention has not been attenuated, for example, has not been heat-attenuated. For example, in some embodiments, the bacterial strain in the composition of the invention has not been killed, inactivated and/or attenuated. For example, in some embodiments, the bacterial strain in the composition of the invention is live. For example, in some embodiments, the bacterial strain in the composition of the invention is viable. For example, in some embodiments, the bacterial strain in the composition of the invention is capable of partially or totally colonising the intestine. For example, in some embodiments, the bacterial strain in the composition of the invention is viable and capable of partially or totally colonising the intestine.

In some embodiments, the composition comprises a mixture of live bacterial strains and bacterial strains that have been killed. In preferred embodiments, the composition of the invention is encapsulated to enable delivery of the bacterial strain to the intestine. Encapsulation protects the composition from degradation until delivery at the target location through, for example, rupturing with chemical or physical stimuli such as pressure, enzymatic activity, or physical disintegration, which may be triggered by changes in pH. Any appropriate encapsulation method may be used. Exemplary encapsulation techniques include entrapment within a porous matrix, attachment or adsorption on solid carrier surfaces, self-aggregation by flocculation or with cross-linking agents, and mechanical containment behind a microporous membrane or a microcapsule. Guidance on encapsulation that may be useful for preparing compositions of the invention is available in, for example, references [87] and [88].

The composition may be administered orally and may be in the form of a tablet, capsule or powder. Encapsulated products are preferred because bacteria of the genus *Anaerostipes* and *Eubacterium* are anaerobes, while *Faecalicatena* are obligate anaerobes.

The composition may be administered orally and may be in the form of a tablet, capsule or powder, Encapsulated products are preferred because *Anaerostipes* are anaerobes and *Eubacterium* are anaerobes, while *Faecalicatena* are obligate anaerobes. Other ingredients (such as vitamin C, for example), may be included as oxygen scavengers and prebiotic substrates to improve the delivery and / or partial or total colonisation and survival *in vivo.* Alternatively, the probiotic composition of the invention may be administered orally as a food or nutritional product, such as milk or whey based fermented dairy product, or as a pharmaceutical product.

The composition may be formulated as a probiotic.

A composition of the invention includes a therapeutically effective amount of a bacterial strain of the invention. A therapeutically effective amount of a bacterial strain is sufficient to exert a beneficial effect upon a patient. A therapeutically effective amount of a bacterial strain may be sufficient to result in delivery to and / or partial or total colonisation of the patient's intestine.

A suitable daily dose of the bacteria, for example for an adult human, may be from about 1 × 10³ to about 1 × 10¹¹ colony forming units (CFU); for example, from about 1 × 10⁷ to about 1 × 10¹⁰ CFU; in another example from about 1 × 10⁶ to about 1 × 10¹⁰ CFU; in another example from about 1 × 10⁷ to about 1 × 10¹¹ CFU; in another example from about 1 × 10⁸ to about 1 × 10¹⁰ CFU; in another example from about 1 × 10⁸ to about 1 × 10¹¹ CFU.

In certain embodiments, the dose of the bacteria is at least 10⁹ cells per day, such as at least 10¹⁰, at least 10¹¹, or at least 10¹² cells per day.

In certain embodiments, a dose of the composition may comprise the bacterial strain in an amount of from about 1 × 10⁶ to about 1 × 10¹¹ colony forming units (CFU)/g, respect to the weight of the composition. The dose may be suitable for an adult human. For example, the composition may comprise the bacterial strain from about 1 × 10³ to about 1 × 10¹¹ CFU/g; for example, from about 1 × 10⁷ to about 1 × 10¹⁰ CFU/g; in another example from about 1 × 10⁶ to about 1 × 10¹⁰ CFU/g; in another example from about 1 × 10⁷ to about 1 × 10¹¹ CFU/g; in another example from about 1 × 10⁸ to about 1 × 10¹⁰ CFU/g; in another example from about 1 × 10⁸ to about 1 × 10¹¹ CFU/g, from about 1 × 10⁸ to about 1 × 10¹⁰ CFU/g. For example, from about 1 × 10⁸ to about 1 × 10¹⁰ CFU/g. The dose may be, for example, 1g, 3g, 5g, and 10g.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of between 500mg and 1000mg, between 600mg and 900mg, between 700mg and 800mg, between 500mg and 750mg or between 750mg and 1000mg. In certain embodiments, the invention provides the above pharmaceutical composition, wherein the lyophilised bacteria in the pharmaceutical composition is administered at a dose of between 500mg and 1000mg, between 600mg and 900mg, between 700mg and 800mg, between 500mg and 750mg or between 750mg and 1000mg.

The composition may be formulated as a probiotic. A probiotic is defined by the FAO/WHO as a live microorganism that, when administered in adequate amounts, confers a health benefit on the host.

Typically, a probiotic, such as the composition of the invention, is optionally combined with at least one suitable prebiotic compound. A prebiotic compound is usually a non-digestible carbohydrate such as an oligo- or polysaccharide, or a sugar alcohol, which is not degraded or absorbed in the upper digestive tract. Known prebiotics include commercial products such as inulin and transgalacto-oligosaccharides.

Other prebiotic compounds (such as vitamin C, for example), may be included as oxygen scavengers and to improve the delivery and / or partial or total colonisation and survival *in vivo.* Alternatively, the probiotic composition of the invention may be administered orally as a food or nutritional product, such as milk or whey based fermented dairy product, or as a pharmaceutical product.

In certain embodiments, the probiotic composition of the present invention includes a prebiotic compound in an amount of from about 1 to about 30% by weight, respect to the total weight composition, (e.g. from 5 to 20% by weight). Known prebiotics include commercial products such as inulin and transgalacto-oligosaccharides.

A prebiotic compound is usually a non-digestible carbohydrate such as an oligo- or polysaccharide, or a sugar alcohol, which is not degraded or absorbed in the upper digestive tract. The carbohydrate may be selected from the group consisting of: fructo- oligosaccharides (or FOS), short-chain fructo-oligosaccharides, inulin, isomalt-oligosaccharides, pectins, xylo-oligosaccharides (or XOS), chitosan-oligosaccharides (or COS), beta-glucans, arable gum modified and resistant starches, polydextrose, D-tagatose, acacia fibers, carob, oats, and citrus fibers. In one aspect, the prebiotics are the short-chain fructo-oligosaccharides (for simplicity shown herein below as FOSs-c.c); said FOSs-c.c. are not digestible carbohydrates, generally obtained by the conversion of the beet sugar and including a saccharose molecule to which three glucose molecules are bonded.

Other prebiotic compounds (such as vitamin C, for example), may be included as oxygen scavengers and to improve the delivery and/or partial or total colonisation and survival *in vivo.* Alternatively, the probiotic composition of the invention may be administered orally as a food or nutritional product, such as milk or whey based fermented dairy product, or as a pharmaceutical product.

The compositions of the invention may comprise pharmaceutically acceptable excipients or carriers, Examples of such suitable excipients may be found in the reference [89]. Acceptable carriers or diluents for therapeutic use are well known in the pharmaceutical art and are described, for example, in reference [90]. Examples of suitable carriers include lactose, starch, glucose, methyl cellulose, magnesium stearate, mannitol, sorbitol and the like. Examples of suitable diluents include ethanol, glycerol and water. The choice of pharmaceutical earner, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid, cysteine and esters of p-hydroxybenzoic acid, for example, in some embodiments the preservative is selected from sodium benzoate, sorbic acid and esters of p-hydroxybenzoic acid. Antioxidants and suspending agents may be also used. A further example of a suitable carrier is saccharose. A further example of a preservative is cysteine.

The compositions of the invention may be formulated as a food product. For example, a food product may provide nutritional benefit in addition to the therapeutic effect of the invention, such as in a nutritional supplement. Similarly, a food product may be formulated to enhance the taste of the composition of the invention or to make the composition more attractive to consume by being more similar to a common food item, rather than to a pharmaceutical composition. In certain embodiments, the composition of the invention is formulated as a milk-based product. The term "milk-based product" means any liquid or semi-solid milk- or whey- based product having a varying fat content. The milk-based product can be, e.g., cow's milk, goat's milk, sheep's milk, skimmed milk, whole milk, milk recombined from powdered milk and whey without any processing, or a processed product, such as yoghurt, curdled milk, curd, sour milk, sour whole milk, butter milk and other sour milk products, Another important group includes milk beverages, such as whey beverages, fermented milks, condensed milks, infant or baby milks; flavoured milks, ice cream; milk-containing food such as sweets.

In some embodiments, the compositions disclosed herein comprise one or more bacterial strains of the genus *Anaerostipes* and do not contain bacteria from any other species, or which comprise only *de minimis* or biologically irrelevant amounts of bacteria from another species. Thus, in some embodiments, the invention provides a composition comprising one or more bacterial strains of the genus *Anaerostipes,* which does not contain bacteria from any other species or which comprises only *de minimis* or biologically irrelevant amounts of bacteria from another species, for use in therapy.

In some embodiments, the compositions comprise one or more bacterial strains of the genus *Anaerostipes, Eubacterium* or *Faecalicatena* and do not contain bacteria from any other genus or comprise only *de minimis* or biologically irrelevant amounts of bacteria from another. In some embodiments, the compositions comprise one or more bacterial strains of the genus *Eubacterium* or *Faecalicatena* and do not contain bacteria from any other genus or comprise only *de minimis* or biologically irrelevant amounts of bacteria from another.

In certain embodiments, the compositions disclosed herein contain a single bacterial species and do not contain any other bacterial species. In certain embodiments, the compositions disclosed herein contain a single bacterial strain and do not contain any other bacterial strains. For example, the compositions of the invention may comprise bacteria only of a strain of *Eubacterium callanderi,* a strain of *Eubacterium limosum,* a strain of *Eubacterium rectale,* a strain of *Eubacterium eligens,* a strain of *Eubacterium hallii,* a strain of *Faecalicatena fissicatena* or a strain of *Faecalicatena contorta.* For example, the compositions of the invention may comprise bacteria only of a strain of *Anaerostipes hadrus*, a strain of *Anaerostipes butyraticus*, a strain of *Anaerostipes rhamnosivorans* or a strain of *Anaerostipes caccae.* Such compositions may comprise only *de minimis* or biologically irrelevant amounts of other bacterial strains or species. Such compositions may be a culture that is substantially free from other species of organism. In some embodiments, such compositions may be a lyophilisate that is substantially free from other species of organism.

In some embodiments, the invention provides a composition comprising a single bacterial strain of the genus *Anaerostipes,* which does not contain bacteria from any other strains or which comprises only *de minimis* or biologically irrelevant amounts of bacteria from another strain for use in therapy.

In certain embodiments, the compositions of the invention contain a single bacterial strain or species and do not contain any other bacterial strains or species. Such compositions may comprise only *de minimis* or biologically irrelevant amounts of other bacterial strains or species. Such compositions may be a culture that is substantially free from other species of organism.

In certain embodiments, the compositions of the invention consist of 1, 2, 3, 4, 5, 6, 7, 8, 9, 10, 11, 12, 13, 14, 15 or 16 bacterial strains or species. In certain embodiments, the compositions consist of from 1 to 10, preferably from 1 to 5 bacterial strains or species. In some embodiments, the compositions disclosed herein comprise more than one strain from within the same species (e.g. more than 1, 2, 3, 4, 5, 6, 7, 8, 9, 10, 15, 20, 25, 30, 35, 40 or 45 strains), and, optionally, do not contain bacteria from any other species. In some embodiments, the compositions disclosed herein comprise less than 50 strains from within the same species (e.g. less than 45, 40, 35, 30, 25, 20, 15, 12, 10, 9, 8, 7, 6, 5, 4 or 3 strains), and, optionally, do not contain bacteria from any other species. In some embodiments, the compositions disclosed herein comprise 1-40, 1-30, 1-20, 1-19, 1-18, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 strains from within the same species and, optionally, do not contain bacteria from any other species. In some embodiments, the compositions disclosed herein comprise more than one species from within the same genus (e.g. more than 1, 2, 3, 4, 5, 6, 7, 8, 9, 10, 12, 15, 17, 20, 23, 25, 30, 35 or 40 species), and, optionally, do not contain bacteria from any other genus. In some embodiments, the compositions disclosed herein comprise less than 50 species from within the same genus (e.g. less than 50, 45, 40, 35, 30, 25, 20, 15, 12, 10, 8, 7, 6, 5, 4 or 3 species), and, optionally, do not contain bacteria from any other genus. In some embodiments, the compositions disclosed herein comprise 1-50, 1-40, 1-30, 1-20, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 species from within the same genus and, optionally, do not contain bacteria from any other genus. The invention comprises any combination of the foregoing.

In some embodiments, the compositions of the invention comprise more than one bacterial strain or species. For example, in some embodiments, the compositions of the invention comprise more than one strain from within the same species (e.g. more than 1, 2, 3, 4, 5, 6, 7, 8, 9, 10, 15, 20, 25, 30, 35, 40 or 45 strains), and, optionally, do not contain bacteria from any other species. In some embodiments, the compositions of the invention comprise less than 50 strains from within the same species (e.g. less than 45, 40, 35, 30, 25, 20, 15, 12, 10, 9, 8, 7, 6, 5, 4 or 3 strains), and, optionally, do not contain bacteria from any other species. In some embodiments, the compositions of the invention comprise 1-40, 1-30, 1-20, 1-19, 1-18, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 strains from within the same species and, optionally, do not contain bacteria from any other species. In some embodiments, the compositions of the invention comprise more than one species from within the same genus (e.g. more than 1, 2, 3, 4, 5, 6, 7, 8, 9, 10, 12, 15, 17, 20, 23, 25, 30, 35 or 40 species), and, optionally, do not contain bacteria from any other genus. In some embodiments, the compositions of the invention comprise less than 50 species from within the same genus (e.g. less than 50, 45, 40, 35, 30, 25, 20, 15, 12, 10, 8, 7, 6, 5, 4 or 3 species), and, optionally, do not contain bacteria from any other genus. In some embodiments, the compositions of the invention comprise 1-50, 1-40, 1-30, 1-20, 1-15, 1-10, 1-9, 1-8, 1-7, 1-6, 1-5, 1-4, 1-3, 1-2, 2-50, 2-40, 2-30, 2-20, 2-15, 2-10, 2-5, 6-30, 6-15, 16-25, or 31-50 species from within the same genus and, optionally, do not contain bacteria from any other genus. The invention comprises any combination of the foregoing.

In certain embodiments, the pharmaceutical composition of the invention comprises between 1-50 distinct bacterial strains, such as between 1-50,1-40,1-30,1-20,1-19,1-18,1-17,1-16,1-15,1-14,1-13, 1-12, 1-11, 1-10, 1-9, 1-8, 1-7, 1-6, 1-5, 1-4, 1-3 or 2 distinct bacterial strains. In certain embodiments, the pharmaceutical composition of the invention comprises between 1-50 distinct bacterial species, such as between 1-50, 1-40, 1-30, 1-20, 1-19. 1-18, 1-17, 1-16, 1-15, 1-14, 1-13, 1-12, 1-11, 1-10, 1-9, 1-8, 1-7, 1-6, 1-5, 1-4, 1-3 or 2 distinct bacterial species.

In certain embodiments, the pharmaceutical composition comprises 16, 15, 14, 13, 12, 11, 10, 9, 8, 7, 6, 5 or fewer distinct bacterial species. In certain embodiments, the pharmaceutical composition comprises 4 or fewer distinct bacterial species. In certain embodiments, the pharmaceutical composition comprises 3 or fewer distinct bacterial species. In certain embodiments, the pharmaceutical composition comprises 2 or fewer distinct bacterial species. In certain embodiments, the pharmaceutical composition comprises *Eubacterium callanderi*, *Eubacterium limosum*, *Eubacterium rectale, Eubacterium eligens, Eubacterium hallii, Faecalicatena fissicatena* or *Faecalicatena contorta* (in particular, *Eubacterium callanderi*) and no other bacterial species. In certain embodiments, the pharmaceutical composition comprises *Anaerostipes hadrus*, *Anaerostipes buₜyraticus*, *Anaerostipes rhamnosivorans* or *Anaerostipes caccae* (in particular *Anaerostipes hadrus*) and no other bacterial species. In preferred embodiments, the compositions of the invention comprise a single strain of *Eubacterium callanderi, Eubacterium limosum, Eubacterium rectale, Eubacterium eligens, Eubacterium hallii, Faecalicatena fissicatena* or *Faecalicatena contort* (in particular *Eubacterium callanderi*) and no other bacterial strains or species. In preferred embodiments, the compositions of the invention comprise a single strain of *Anaerostipes hadrus, Anaerostipes butyraticus, Anaerostipes rhamnosivorans* or *Anaerostipes caccae* (in particular *Anaerostipes hadrus*) and no other bacterial strains or species. Such compositions may comprise only *de minimis* or biologically irrelevant amounts of other bacterial strains or species. Strikingly, the examples demonstrate that compositions comprising only a single strain of the invention can have potent effects, with no reliance on co-administration with other strains or species.

In some embodiments, the composition comprises a microbial consortium. For example, in some embodiments, the composition comprises the *Anaerostipes* bacterial strain as part of a microbial consortium. For example, in some embodiments, the *Anaerostipes* bacterial strain is present in combination with one or more (e.g. at least 2, 3, 4, 5, 10, 15 or 20) other bacterial strains from the genus *Anaerostipes* and/or other genera with which it can live symbiotically *in vivo* in the intestine. For example, in some embodiments, the composition comprises a bacterial strain of the genus *Anaerostipes* in combination with a bacterial strain from a different genus. In another example, the composition comprises a bacterial strain of the genus *Anaerostipes* in combination with a second bacterial strain from the genus *Anaerostipes* or the composition comprises a bacterial strain of the genus *Anaerostipes* in combination with a second bacterial strain from the genus *Anaerostipes* and a bacterial strain from a different genus. In some embodiments, the composition comprises the *Eubacterium* or *Faecalicatena* bacterial strain as part of a microbial consortium. For example, in some embodiments, the *Eubacterium* or *Faecalicatena* bacterial strain is present in combination with one or more (e.g. at least 2, 3, 4, 5, 10, 15 or 20) other bacterial strains from the *Eubacterium* or *Faecalicatena* genera and/or other genera with which it can live symbiotically *in vivo* in the intestine, For example, in some embodiments, the composition comprises a strain of *Eubacterium* or *Faecalicatena* in combination with a bacterial strain from a different genus. In another example, the composition comprises a strain of *Eubacterium callanderi* in combination with a bacterial strain from the genus *Eubacterium,* or the composition comprises a strain of *Eubacterium callanderi* in combination with a bacterial strain from the genus *Eubacterium* and a bacterial strain from a different genus. In some embodiments, the microbial consortium comprises two or more bacterial strains obtained from a faeces sample of a single organism, e.g. a human. In some embodiments, the microbial consortium is not found together in nature. For example, in some embodiments, the microbial consortium comprises bacterial strains obtained from faeces samples of at least two different organisms. In some embodiments, the two different organisms are from the same species, e.g. two different humans. In some embodiments, the two different organisms are an infant human and an adult human. In some embodiments, the two different organisms are a human and a non-human mammal.

In some embodiments, the composition of the invention additionally comprises a bacterial strain that has the same safety and therapeutic efficacy characteristics as strain NCIMB 43457, but which is not NCIMB 43457, or which is not *Anaerostipes hadrus*

In some embodiments, the composition of the invention additionally comprises a bacterial strain that has the same safety and therapeutic efficacy characteristics as strain NCIMB 43526, but which is not NCIMB 43526, or which is not *Anaerostipes hadrus.*

In some embodiments, the composition of the invention additionally comprises a bacterial strain that has the same safety and therapeutic efficacy characteristics as the *Eubacterium callanderi* strain deposited under accession number NCIMB 43455, but which is not the *Eubacterium callanderi* strain deposited under accession number NCIMB 43455, or which is not *Eubacterium.*

In some embodiments, the composition of the invention additionally comprises a bacterial strain that has the same safety and therapeutic efficacy characteristics as the *Faecalicatena contorta* strain deposited under accession number NCIMB 42689, but which is not the *Faecalicatena contorta* strain deposited under accession number NCIMB 42689, or which is not *Faecalicatena.*

In some embodiments in which the composition of the invention comprises more than one bacterial strain, species or genus, the individual bacterial strains, species or genera may be for separate, simultaneous or sequential administration. For example, the composition may comprise all of the more than one bacterial strain, species or genera, or the bacterial strains, species or genera may be stored separately and be administered separately, simultaneously or sequentially. In some embodiments, the more than one bacterial strains, species or genera are stored separately but are mixed together prior to use.

In some embodiments, the bacterial strain for use in the invention is obtained from human adult faeces. In some embodiments in which the composition of the invention comprises more than one bacterial strain, all of the bacterial strains are obtained from human adult faeces or if other bacterial strains are present they are present only in *de minimis* amounts. The bacteria may have been cultured subsequent to being obtained from the human adult faeces and being used in a composition of the invention.

In some embodiments, the one or more *Anaerostipes* bacterial strain is/are the only therapeutically active agent(s) in a composition of the invention. In some embodiments, the bacterial strain(s) in the composition is/are the only therapeutically active agent(s) in a composition of the invention.

In some embodiments, the one or more *Eubacterium* or *Faecalicatena* bacterial strains is/are the only therapeutically active agent(s) in a composition of the invention. In some embodiments, the bacterial strain(s) in the composition is/are the only therapeutically active agent(s) in a composition of the invention.

The compositions for use in accordance with the invention may or may not require marketing approval.

In certain embodiments, the invention provides the above pharmaceutical composition, wherein said bacterial strain is lyophilised. In some cases, the bacterial strain is reconstituted prior to administration. In some cases, the reconstitution is by use of a diluent described herein. In certain embodiments, the invention provides the above pharmaceutical composition, wherein said bacterial strain is spray dried. In certain embodiments, the invention provides the above pharmaceutical composition, wherein the bacterial strain is lyophilised or spray dried and wherein it is live. In certain embodiments, the invention provides the above pharmaceutical composition, wherein the bacterial strain is lyophilised or spray dried and wherein it is viable. In certain embodiments, the invention provides the above pharmaceutical composition, wherein the bacterial strain is lyophilised or spray dried and wherein it is capable of partially or totally colonising the intestine. In certain embodiments, the invention provides the above pharmaceutical composition, wherein the bacterial strain is lyophilised or spray dried and wherein it is viable and capable of partially or totally colonising the intestine.

In some cases, the lyophilised or spray dried bacterial strain is reconstituted prior to administration. In some cases, the reconstitution is by use of a diluent described herein.

The compositions of the invention can comprise pharmaceutically acceptable excipients, diluents or carriers.

In certain embodiments, the invention provides a pharmaceutical composition comprising: a bacterial strain of the invention; and a pharmaceutically acceptable excipient, carrier or diluent; wherein the bacterial strain is in an amount sufficient to treat a disorder when administered to a subject in need thereof; and wherein the disorder is selected from the group consisting of neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer, a central nervous system disorder or condition (as described herein).

In certain embodiments, the invention provides pharmaceutical composition comprising: a bacterial strain of the invention; and a pharmaceutically acceptable excipient, carrier or diluent; wherein the bacterial strain is in an amount sufficient to treat or prevent a disease or condition mediated by HDAC. In preferred embodiments, said disease or condition is selected from the group consisting of neurodegenerative diseases, such as Alzheimer's disease, Huntington's disease or Parkinson's disease, brain injury, such as stroke, behavioural disorders, such as attention deficit hyperactivity disorder, inflammatory bowel diseases, such as Crohn's disease, cancer, such as prostate cancer, colorectal cancer, breast cancer, lung cancer, liver cancer or gastric cancer.

In preferred embodiments, pharmaceutical composition is for use in treating or preventing a central nervous system disorder or condition, in particular central nervous system disorder or condition mediated by the microbiota-gut-brain axis. In preferred embodiments, said the disorder or condition is selected from the group consisting of: autism spectrum disorders (ASDs); child developmental disorder; obsessive compulsive disorder (OCD); major depressive disorder; depression; seasonal affective disorder; anxiety disorders; schizophrenia spectrum disorders; schizophrenia; bipolar disorder; psychosis; mood disorder; chronic fatigue syndrome (myalgic encephalomyelitis); stress disorder; post-traumatic stress disorder; dementia; Alzheimer's; Parkinson's disease; and/or chronic pain. In further embodiments, the compositions disclosed herein may be useful for treating or preventing motor neuron disease; Huntington's disease; Guillain-Barre syndrome and/or meningitis.

In certain embodiments, the invention provides the above pharmaceutical composition, wherein the amount of the bacterial strain is from about 1 × 10³ to about 1 × 10¹¹ colony forming units per gram with respect to a weight of the composition.

In certain embodiments, the invention provides the above pharmaceutical composition, wherein the composition is administered at a dose of 1 g, 3 g, 5 g or 10 g.

In certain embodiments, the invention provides the above pharmaceutical composition, wherein the composition is administered by a method selected from the group consisting of oral, rectal, subcutaneous, nasal, buccal, and sublingual.

In certain embodiments, the invention provides the above pharmaceutical composition, comprising a carrier selected from the group consisting of lactose, starch, glucose, methyl cellulose, magnesium stearate, mannitol and sorbitol.

In certain embodiments, the invention provides the above pharmaceutical composition, comprising a diluent selected from the group consisting of ethanol, glycerol and water.

In certain embodiments, the invention provides the above pharmaceutical composition, comprising an excipient selected from the group consisting of starch, gelatin, glucose, anhydrous lactose, free-flow lactose, beta-lactose, corn sweetener, acacia, tragacanth, sodium alginate, carboxymethyl cellulose, polyethylene glycol, sodium oleate, sodium stearate, magnesium stearate, sodium benzoate, sodium acetate and sodium chloride.

In certain embodiments, the invention provides the above pharmaceutical composition, further comprising at least one of a preservative, an antioxidant and a stabilizer.

In certain embodiments, the invention provides the above pharmaceutical composition, comprising a preservative selected from the group consisting of sodium benzoate, sorbic acid and esters of p-hydroxybenzoic acid.

In certain embodiments, the invention provides the above pharmaceutical composition, wherein said bacterial strain is lyophilised.

In certain embodiments, the invention provides the above pharmaceutical composition, wherein when the composition is stored in a sealed container at about 4°C or about 25°C and the container is placed in an atmosphere having 50% relative humidity, at least 80% of the bacterial strain as measured in colony forming units, remains after a period of at least about: 1 month, 3 months, 6 months, 1 year, 1.5 years, 2 years, 2.5 years or 3 years.

In some embodiments, the composition of the invention is provided in a sealed container comprising a composition as described herein. In some embodiments, the sealed container is a sachet or bottle. In some embodiments, the composition of the invention is provided in a syringe comprising a composition as described herein.

The composition of the present invention may, in some embodiments, be provided as a pharmaceutical formulation. For example, the composition may be provided as a tablet or capsule. In some embodiments, the capsule is a gelatine capsule ("gel-cap"). The capsule can be a hard or a soft capsule. In some embodiments, the formulation is a soft capsule. Soft capsules are capsules which may, owing to additions of softeners, such as, for example, glycerol, sorbitol, maltitol and polyethylene glycols, present in the capsule shell, have a certain elasticity and softness. Soft capsules can be produced, for example, on the basis of gelatine or starch. Gelatine-based soft capsules are commercially available from various suppliers. Depending on the method of administration, such as, for example, orally or rectally, soft capsules can have various shapes, they can be, for example, round, oval, oblong or torpedo-shaped. Soft capsules can be produced by conventional processes, such as, for example, by the Scherer process, the Accogel process or the droplet or blowing process.

In some embodiments, the compositions disclosed herein are administered orally . Oral administration may involve swallowing, so that the compound enters the gastrointestinal tract.

Pharmaceutical formulations suitable for oral administration include solid plugs, solid microparticulates, semi-solid and liquid (including multiple phases or dispersed systems) such as tablets; soft or hard capsules containing multi- or nano-particulates, liquids (e.g. aqueous solutions), emulsions or powders; lozenges (including liquid-filled); chews; gels; fast dispersing dosage forms; films; ovules; sprays; and buccal/mucoadhesive patches.

In some embodiments the pharmaceutical formulation is an enteric formulation, i.e. a gastro-resistant formulation (for example, resistant to gastric pH) that is suitable for delivery of the composition of the invention to the intestine by oral administration. Enteric formulations may be particularly useful when the bacteria or another component of the composition is acid-sensitive, e.g. prone to degradation under gastric conditions.

In some embodiments, the enteric formulation comprises an enteric coating. In some embodiments, the formulation is an enteric-coated dosage form. For example, the formulation may be an enteric-coated tablet or an enteric-coated capsule, or the like. The enteric coating may be a conventional enteric coating, for example, a conventional coating for a tablet, capsule, or the like for oral delivery. The formulation may comprise a film coating, for example, a thin film layer of an enteric polymer, e.g. an acid-insoluble polymer.

In some embodiments, the enteric formulation is intrinsically enteric, for example, gastro-resistant without the need for an enteric coating. Thus, in some embodiments, the formulation is an enteric formulation that does not comprise an enteric coating. In some embodiments, the formulation is a capsule made from a thermogelling material. In some embodiments, the thermogelling material is a cellulosic material, such as methylcellulose, hydroxymethylcellulose or hydroxypropylmethylcellulose (HPMC). In some embodiments, the capsule comprises a shell that does not contain any film forming polymer. In some embodiments, the capsule comprises a shell and the shell comprises hydroxypropylmethylcellulose and does not comprise any film forming polymer (e.g. see [91]) In some embodiments, the formulation is an intrinsically enteric capsule (for example, Vcaps^{®} from Capsugel).

### Culturing methods

The bacterial strains for use in the present invention can be cultured using standard microbiology techniques as detailed in, for example, references [92,94].

The solid or liquid medium used for culture may, for example, be YCFA agar or YCFA medium. YCFA medium may include (per 100ml, approximate values): Casitone (1.0 g), yeast extract (0.25 g), NaHCO₃ (0.4 g), cysteine (0.1 g), K₂HPO₄ (0.045 g), KH₂PO₄ (0.045 g), NaCl (0.09 g), (NH₄)₂SO₄ (0.09 g), MgSO₄ · 7H₂O (0.009 g), CaCl₂ (0.009 g), resazurin (0.1 mg), hemin (1 mg), biotin (1 µg), cobalamin (1 µg), *p*-aminobenzoic acid (3 µg), folic acid (5 µg), and pyridoxamine (15 µg).

### Bacterial strains for use in vaccine compositions

The inventors have identified that the bacterial strains of the invention are useful for treating or preventing diseases or conditions mediated by HDAC. This is likely to be a result of the effect that the bacterial strains of the invention have on the host immune system. Therefore, the compositions of the invention may also be useful for preventing diseases or conditions mediated by HDAC, when administered as vaccine compositions. In certain such embodiments, the bacterial strains of the invention may be killed, inactivated or attenuated. In certain such embodiments, the compositions may comprise a vaccine adjuvant. In certain embodiments, the compositions are for administration via injection, such as via subcutaneous injection.

Furthermore, the compositions disclosed herein may also be useful for preventing central nervous system disorders or conditions, in particular central nervous system disorders or conditions mediated by the microbiota-gut-brain axis, when administered as vaccine compositions. In other certain such embodiments, the bacterial strains of the invention may be killed, inactivated or attenuated. In certain such embodiments, the compositions may comprise a vaccine adjuvant. In certain embodiments, the compositions are for administration via injection, such as via subcutaneous injection.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.*, references [95] and [96,102], *etc.*

The term "comprising" encompasses "including" as well as "consisting" e.g. a composition "comprising" X may consist exclusively of X or may include something additional *e*.*g*. X + Y.

The term "about" in relation to a numerical value *x* is optional and means, for example, *x*±10%.

The word "substantially" does not exclude "completely" e.g. a composition which is "substantially free" from Y may be completely free from Y. Where necessary, the word "substantially" may be omitted from the definition of the invention.

References to a percentage sequence identity between two nucleotide sequences means that, when aligned, that percentage of nucleotides are the same in comparing the two sequences. This alignment and the percent homology or sequence identity can be determined using software programs known in the art, for example those described in section 7.7.18 of ref. [103]. A preferred alignment is determined by the Smith-Waterman homology search algorithm using an affine gap search with a gap open penalty of 12 and a gap extension penalty of 2, BLOSUM matrix of 62. Another preferred alignment is determined by the Smith-Waterman homology search algorithm using an affine gap search with a gap open penalty of 5 and a gap extension penalty of 2, BLOSUM matrix of 62. The Smith-Waterman homology search algorithm is disclosed in ref. [104].

Unless specifically stated, a process or method comprising numerous steps may comprise additional steps at the beginning or end of the method, or may comprise additional intervening steps. Also, steps may be combined, omitted or performed in an alternative order, if appropriate.

Various embodiments of the invention are described herein. It will be appreciated that the features specified in each embodiment may be combined with other specified features, to provide further embodiments. In particular, embodiments highlighted herein as being suitable, typical or preferred may be combined with each other (except when they are mutually exclusive).

All patent and literature references cited in the present specification are hereby incorporated by reference in their entirety.

Any reference to a method for treatment comprising administering an agent to a patient, also covers that agent for use in said method for treatment, as well as the use of the agent in said method for treatment, and the use of the agent in the manufacture of a medicament.

The following examples are offered for illustrative purposes only, and are not intended to limit the scope of the present invention in any way.

### MODES FOR CARRYING OUT THE INVENTION

### Example 1 -Effect of Anaerostipes hadrus on short-chain fatty acid production in vitro

### Summary

This study investigated the effect of *Anaerostipes hadrus* on the production of short-chain fatty acids (SCFAs) in vitro. SCFAs, which include acetate, propionate, valerate, isobutyrate and isovalerate are microbial byproducts of dietary fibre. An increase in any SCFA suggests an increase in productivity of the microbiota and is a desirable trait.

### Materials and methods

Pure cultures of *Anaerostipes hadrus* DSM 3319 were grown anaerobically in YCFA+ broth [per litre: Casein hydrolysate 10.0 g, Yeast Extract 2.5 g, Sodium hydrogen carbonate 4.0 g, Glucose 2.0 g, Cellobiose 2.0 g, Soluble starch 2.0 g, Di-potassium hydrogen phosphate 0.45 g, Potassium dihydrogen phosphate 0.45 g, Resazurin 0.001 g, L-Cysteine HCl 1.0 g, Ammonium sulphate 0.9 g, Sodium chloride 0.9 g, Magnesium sulphate 0.09 g, Calcium chloride 0.09 g, Haemin 0.01 g, SCFA 3 1 ml (Acetic acid 2.026 ml/L, Propionic acid 0.715 ml/L, n-Valeric acid 0.119 ml/L, Iso-Valeric acid 0.119 ml/L, Iso-Butyric acid 0.119 ml/L), vitamin mix 1: 1 ml (Biotin 1mg/100 ml, Cyanocobalamine 1mg/100 ml, p-Aminobenzoic acid 3mg/100 ml, Pyridoxine 15mg/100 ml), vitamin mix 2: 1 ml (Thiamine 5mg/100 ml, Riboflavin 5mg/100 ml), vitamin mix 3: 1 ml (Folic acid 5mg/100 ml)] until they reached their stationary growth phase. Cultures were centrifuged at 5000 × g for 10 minutes and the cell-free supernatant (CFS) was filtered using a 0.45 µM followed by a 0.2 µM filter (Millipore, UK), after which 1 mL aliquots of the CFS were stored at -80 °C until use.

Short chain fatty acids (SCFAs) and medium chain fatty acids (MCFAs) from bacterial supernatants were analysed and quantified by MS Omics APS, Denmark. Samples were acidified using hydrochloride acid, and deuterium labelled internal standards were added. All samples were analyzed in a randomized order. Analysis was performed using a high polarity column (Zebron^{™} ZB-FFAP, GC Cap. Column 30 m × 0.25 mm × 0.25 µm) installed in a gas chromatograph (7890B, Agilent) coupled with a quadropole detector (5977B, Agilent). The system was controlled by ChemStation (Agilent). Raw data was converted to netCDF format using Chemstation (Agilent), before the data was imported and processed in Matlab R2014b (Mathworks, Inc.) using the PARADISe software described by reference [105].

### Results

The following pattern was observed for *Anaerostipes hadrus*:

| Acetic acid | Formic acid | Propionic acid | 2-methylpropanoic acid | Butanoic acid | methylbutanoic acid | Pentanoic acid | 4-methylpentanoic acid | Hexanoic acid | Heptanoic acid |
|---|---|---|---|---|---|---|---|---|---|
| 6.7 | 0.0 | -0.7 | 0.0 | 12.5 | 0.0 | 0.1 | 0.0 | 0.0 | 0.1 |

This shows that *Anaerostipes hadrus* mainly produces acetic acid and butyric acid, which is consistent with known data.

### Example 2 - Effect of Anaerostipes hadrus on short-chain fatty acid production in vivo

### Summary

This study investigated the effect of *Anaerostipes hadrus* on the production of short-chain fatty acids (SCFAs) in mice. SCFAs, which include acetate, propionate, valerate, isobutyrate and isovalerate are microbial byproducts of dietary fibre. An increase in any SCFA suggests an increase in productivity of the microbiota and is a desirable trait.

### Materials and methods

Male BALB/c mice received oral gavage (200µL volume) of 1 × 10⁹ CFU of NCIMB 43526 for 6 consecutive days. On day 7, the animals were euthanized. The caecum was removed, weighed and stored at -80 °C for SCFAs analysis. Caecum content was mixed and vortexed with MilliQ water and incubated at room temperature for 10 min. Supernatants were obtained by centrifugation (10000 g, 5 min, 4 °C) to pellet bacteria and other solids and filtration by 0.2µm. It was transferred to a clear GC vial and 2-Ethylbutyric acid (Sigma) was used as the internal standard. The concentration of SCFA was analyzed using a Varian 3500 GC flame-ionization system, fitted with a with a ZB-FFAP column (30 m × 0.32 mm × 0.25 mm; Phenomenex). A standard curve was built with different concentrations of a standard mix containing acetate, propionate, iso-butyrate, n-butyrate, isovalerate and valerate (Sigma). Peaks were integrated by using the Varian Star Chromatography Workstation version 6.0 software. All SCFA data are expressed as µmol/g.

### Results

Figure 1 shows that treatment with *Anaerostipes hadrus* resulted in a general increase in the production of acetate (A), propionate (B), isobutyrate (C), isovalerate (D) and valerate (E). Interestingly, this pattern differs from the one found *in vitro* and is digresses from the normal pattern of SCFA secretion, demonstrating that the *in vivo* results which take into account the whole microbiome rather than isolated strains differ.

SCFAs produced by bacteria in the microbiome are key mediators of the beneficial effects elicited by the gut microbiome. These data suggest that bacteria from the genus *Anaerostipes* may be useful in promoting productivity of the microbiota, and therefore useful in the treatment or prevention of diseases associated with a reduced productivity of the microbiota. SCFA can regulate the immune response, therefore these data suggest that bacteria from the genus *Anaerostipes* may be useful in the treatment of inflammatory conditions.

### Example 3 - Effect of Anaerostipes hadrus on peripheral immune markers

### Summary

This study investigated the effect of *Anaerostipes hadrus* on the production of several immune markers by isolated mouse splenocytes. Interleukin-1β (IL-1β), tumour necrosis factor-α (TNF-α), interferon-y (IFN-y), interleukin-6 (IL-6) are pro-inflammatory cytokines; while interleukin-10 (IL-10) has anti-inflammatory properties. Both pro-and anti-inflammatory cytokines are necessary for the maintenance of a healthy immune and inflammatory system, an imbalance in these cytokines can lead to negative physiological outputs.

### Materials and methods

Male BALB/c mice received oral gavage (200µL volume) of 1 × 10⁹ CFU of NCIMB 43526 for 6 consecutive days On day 7, the animals were euthanized. Spleen was removed, collected in 5 mL RPMI media (with L-glutamine and sodium bicarbonate, R8758 Sigma + 10 % FBS (F7524, Sigma) + 1% Pen/Strep (P4333, Sigma)) and processed immediately after culls for *ex-vivo* immune stimulation. Spleen cells were first homogenised in the RPMI media. The homogenate step was followed by RBC lysis step where the cells were incubated for 5 mins in 1ml of RBC lysis buffer (11814389001 ROCHE, Sigma). 10 ml of the media was added to stop the lysis and followed by 200g centrifugation for 5 mins This was followed by final step where the cells were passed through 40um strainer. The homogenate was then filtered over a 40um strainer, centrifuged at 200 g for 5 min and resuspended in media. Cells were counted and seeded (4,000,000/mL media). After 2.5 h of adaptation, cells were stimulated with lipopolysaccharide (LPS-2 µg/ml) or concanavalin A (ConA-2.5 µg/ml) for 24 h. Following stimulation, the supernatants were harvested to assess the cytokine release using Proinflammatory Panel 1 (mouse) V-PLEX Kit (Meso Scale Discovery, Maryland, USA) for TNFα, IL-10, IL-1β, Interferon γ, CXCL2 and IL6. The analyses were performed using MESO QuickPlex SQ 120, SECTOR Imager 2400, SECTOR Imager 6000, SECTOR S 600.

### Results

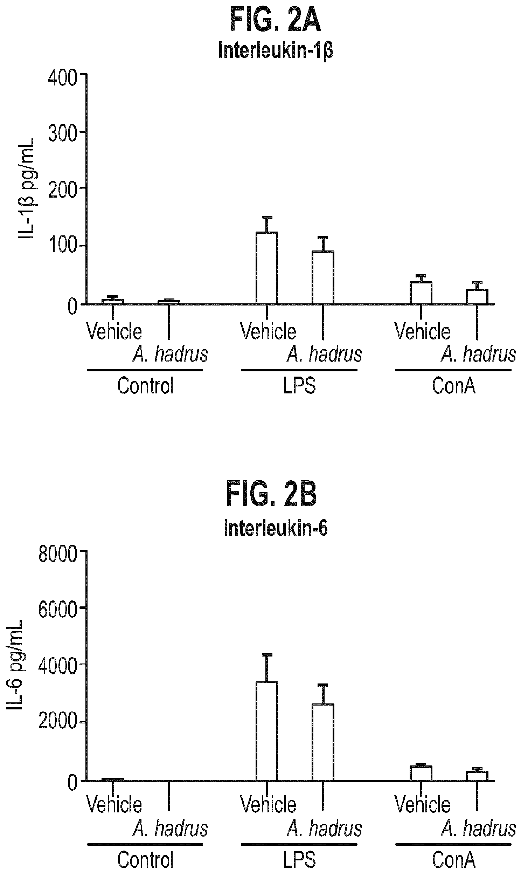

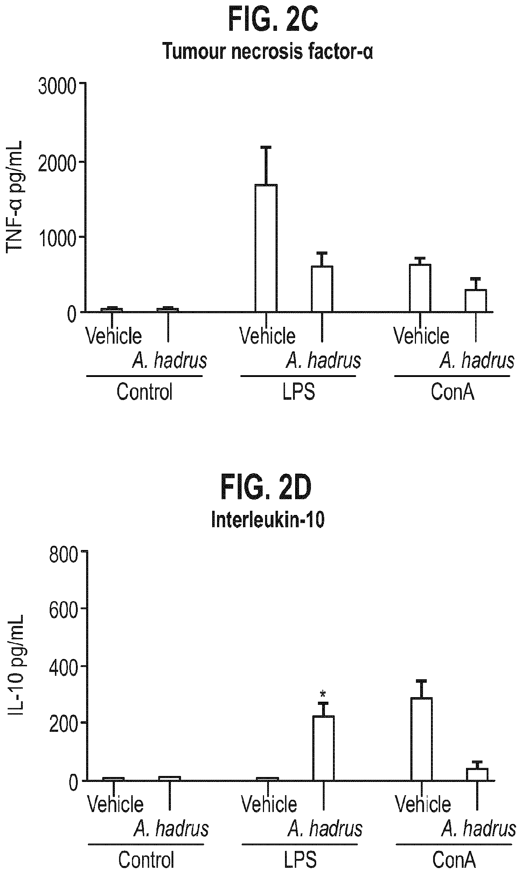

Figure 2 shows that mice treated with *Anaerostipes hadrus* produced less IL-1β, TNFα, IL-6, and showed increased production of IL-10 (when stimulated with LPS) compared to mice treated with the vehicle. These results suggest that *Anaerostipes hadrus* may have an immunomodulatory effect and therefore may be useful in the treatment or prevention of immune and/or inflammatory diseases.

### Example 4 - Effect of Anaerostipes hadrus on gene expression in the brain

### Summary

This study investigated the effect of *Anaerostipes hadrus* on the expression of certain genes of interest in the brain. mRNA levels for markers for the oxytocinergic system (oxytocin receptor and vasopressin receptor), endocrine system (mineralocorticoid (Nr3c1); glucocorticoid receptor (Nr3c2); corticosterone releasing factor (CRF) and receptors; Brain derived neurotrophic factor (BDNF)), immune system (11-6, TNF-a, TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala, hippocampus and prefrontal cortex (PFC), which are key brain regions of the limbic system involved in emotional response.

### Materials and Methods

### Method

Male BALB/c mice received oral gavage (200µL volume) of 1 × 10⁹ CFU of NCIMB 43526 for 6 consecutive days. On day 7, the animals were euthanized. The brain was quickly excised, dissected and each brain region was snap-frozen on dry ice and stored at -80 °C for further analysis. Total RNA was extracted using the mirVana^{™} miRNA Isolation kit (Ambion/Llife technologies, Paisley, UK) and DNase treated (Turbo DNA-free, Ambion/life technologies) according to the manufacturers recommendations. RNA was quantified using NanoDrop^{™} spectrophotometer (Thermo Fisher Scientific Inc., Wilmington, Delaware, USA) according to the manufacturer's instructions. RNA quality was assessed using the Agilent Bioanalyzer (Agilent, Stockport, UK) according to the manufacturer's procedure and an RNA integrity number (RIN) was calculated. RNA with RIN value >7 was used for subsequent experiments. RNA was reverse transcribed to cDNA using the Applied Biosystems High Capacity cDNA kit (Applied Biosystems, Warrington, UK) according to manufacturer's instructions. Briefly, Multiscribe Reverse Transcriptase (50 U/µL) was added as part of RT master mix, incubated for 25°C for 10 min, 37°C for 2 h, 85°C for 5 min and stored at 4°C. Quantitative PCR was carried out using probes (6 carboxy fluorescein - FAM) designed by Applied Biosystems to mouse specific targeted genes, while using β-actin as an endogenous control, Amplification reactions contained 1 µl cDNA, 5 µl of the 2X PCR Master mix (Roche), 900 nM of each primer and were brought to a total of 10 µl by the addition of RNase-free water. All reactions were performed in triplicate using 96-well plates on the LightCycler^{®}480 System. Thermal cycling conditions were as recommended by the manufacturer (Roche) for 55 cycles. To check for amplicon contamination, each run contained no template controls in triplicate for each probe used. Cycle threshold (Ct) values were recorded. Data was normalized using β-actin and transformed using the 2-ΔΔCT method and presented as a fold change vs. control group.

### Results

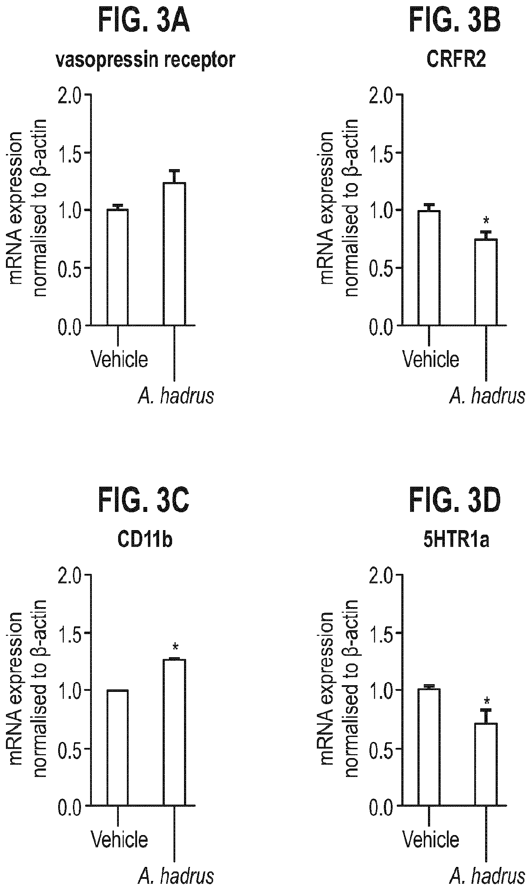

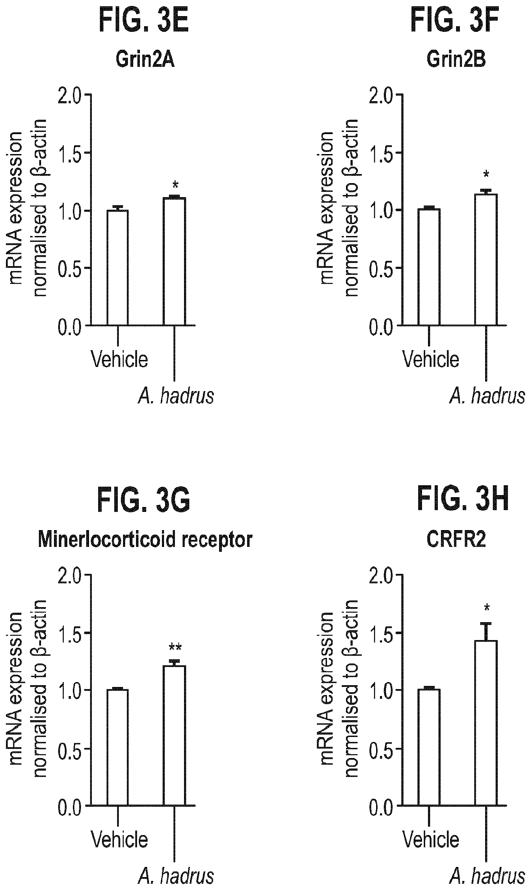

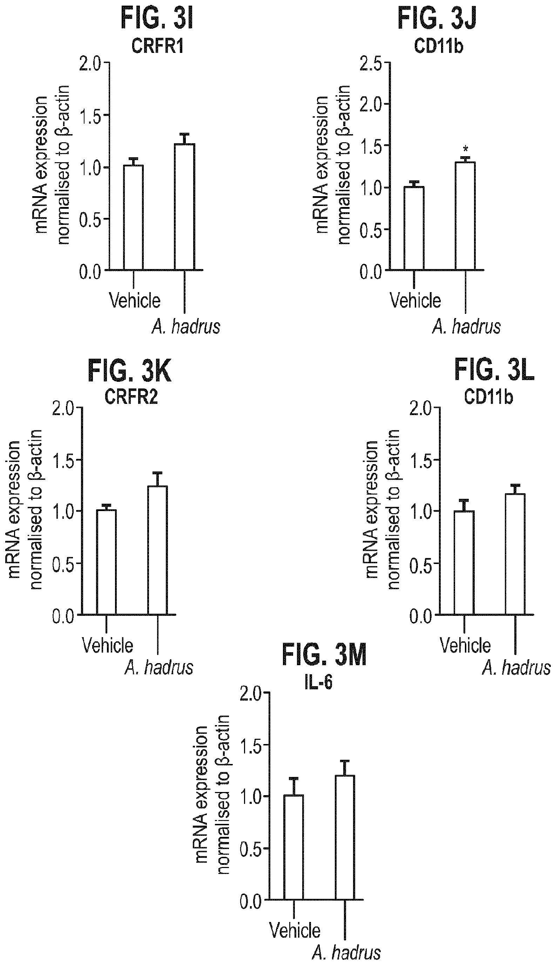

As shown in Figure 3, from the genes tested, a significant decrease in the expression of CRFR2 (B) and 5HTR1a (D), and a significant increase in the expression of CD11b (C), Grin2a (E) and Grin2b (F) were observed in the amygdala in mice which had been treated with *Anaerostipes hadrus*, relative to treatment with vehicle control. An increase in the expression of vasopressin receptor (A) was also observed in this brain region for the treated animals. Figure 3 shows that in the amygdala, treatment with *Anaerostipes hadrus* resulted in significantly higher expression of CRFR2 (B), CRFR1 (C) and CD11b (D), as well as a trend for an increase in the expression of mineralocorticoid receptor (A). As shown in Figure 3, mice treated with *Anaerostipes hadrus* also had an increased expression of CRFR2 (A), CD11b (B) and IL-6 (C) in the PFC. This indicates that bacteria from the genus *Anaerostipes* are capable of modulating the levels of expression of proteins in the brain, therefore they may be useful in the treatment or prevention of CNS diseases, disorders or conditions, such as autism.

### Example 5 - Efficacy of bacteria on histone deacetylase activity

### Introduction

The inventors sought to investigate the effectiveness of the *Anaerostipes hadrus* strain DSM 3319 and its metabolites on HDAC inhibition. As a control the *Megasphaera massiliensis* strain deposited under accession number NCIMB 42787 ("NCIMB 42787", see WO2018/229216) was included in the experiments which is known to be a potent HDAC inhibitor [106].

### Method

Specific HDAC inhibition activity was analysed for class I HDACs (1, 2, 3) and class II HDACs (4, 5, 6, 9) using fluorogenic assay kits for each isoform of HDAC (BPS Bioscience, CA). 10 % cell-free supernatant, or dilutions of SCFA, MCFA and BCFA (diluted in assay buffer) were exposed to specific HDAC isoforms provided in the kit. Assays were conducted according to manufacturer's instructions and fluorescence of untreated enzyme was used to calculate percentage inhibition of treated enzyme. Valproic acid (VPA) and trichostatin A (TSA), known HDAC inhibitors, were used as positive controls.

### Results

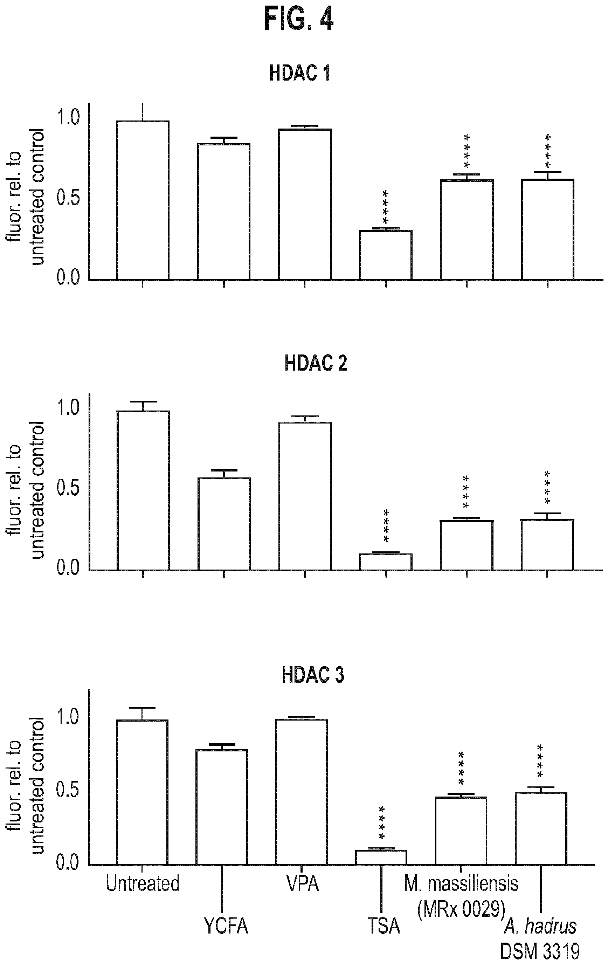

Figure 4 shows that DSM 3319 inhibited the activity of HDAC1, HDAC2 and HDAC 3 with high efficiency. In particular, the inhibition was comparable to the one seen with the known HDAC inhibitor NCIMB 42787which has been demonstrated as having strong neuroprotective effects *in vitro* and *in vivo* [107], exerting a robust anti-inflammatory effect [108] and also as being a promising anti-cancer therapy [109].

### Example 6 - Effect of Anaerostipes on intestinal permeability

### Summary

This study investigated the effect of *Anaerostipes* on intestinal permeability of the ileum and colon. Excessive permeability, or 'leakiness', of the intestine is associated with a number of inflammatory disorders of the gut.

### Materials and Methods

### Bacterial strains

*Anaerostipes hadrus* strain deposited under deposited under accession number NCIMB 43457.

### Method

Male BALB/c mice received oral gavage (200µL volume) of 1 × 10⁹ CFU of *Anaerostipes hadrus* for 6 consecutive days. On day 7, the animals were euthanized by cervical dislocation, and the distal ileum and colon were removed, placed in chilled Krebs solution, opened along the mesenteric line and carefully rinsed. Preparations were then placed in Ussing chambers (Harvard Apparatus, Kent, UK, exposed area of 0.12 cm2) as described previously (Hyland and Cox, 2005) with oxygenated (95% O₂, 5% CO₂) Krebs buffer maintained at 37°C. 4 kDa FITC-dextran was added to the mucosal chamber at a final concentration of 2.5 mg/mL; 200 µL samples were collected from the serosal chamber every 30 min for the following 3 h and fluorescence in those samples measured.

### Results

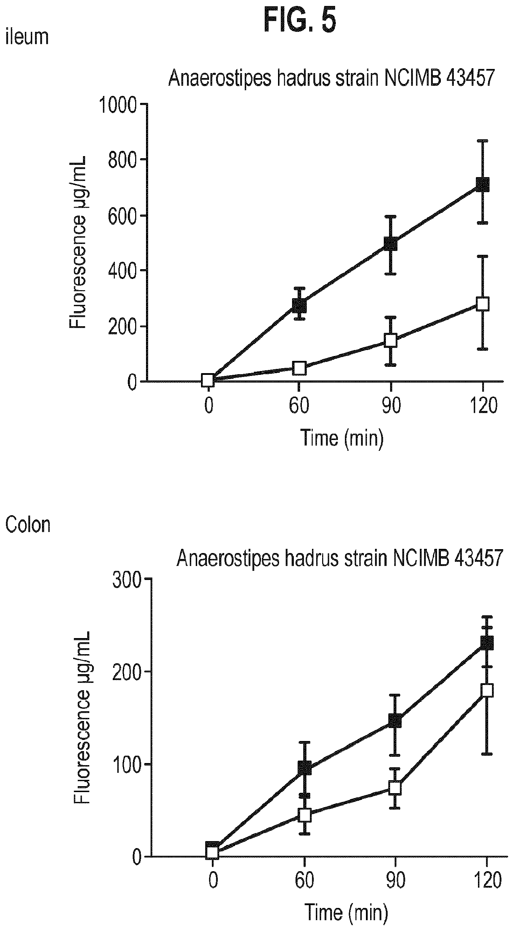

As shown in Figure 5, NCIMB 43457 decreases the permeability of both the ileum and colon, *Anaerostipes* strains may therefore be useful in modifying intestinal permeability, e.g. for the treatment or prevention of disorders or conditions associated with intestinal permeability.

### Example 7- Effect of Anaerostipes on short-chain fatty acid production in vivo

### Summary

This study investigated the effect of *Anaerostipes* on the production of short-chain fatty acids (SCFAs) in mice. SCFAs, which include acetate, propionate, valerate, butyrate, isobutyrate and isovalerate are microbial byproducts of dietary fibre. An increase in any SCFA suggests an increase in productivity of the microbiota and is a desirable trait.

### Materials and methods

### Bacterial strains

*Anaerostipes hadrus* strain NCIMB 43457.

### Method

Male BALB/c mice received oral gavage (200µL volume) of 1 × 10⁹ CFU of either of the two strains for 6 consecutive days. On day 7, the animals were euthanized. The caecum was removed, weighed and stored at -80 °C for SCFAs analysis. Caecum content was mixed and vortexed with MilliQ water and incubated at room temperature for 10 min. Supernatants were obtained by centrifugation (10000 g, 5 min, 4 °C) to pellet bacteria and other solids and filtration by 0.2µm. It was transferred to a clear GC vial and 2-Ethylbutyric acid (Sigma) was used as the internal standard. The concentration of SCFA was analyzed using a Varian 3500 GC flame-ionization system, fitted with a with a ZB-FFAP column (30 m × 0.32 mm × 0.25 mm; Phenomenex). A standard curve was built with different concentrations of a standard mix containing acetate, propionate, iso-butyrate, n-butyrate, isovalerate and valerate (Sigma). Peaks were integrated by using the Varian Star Chromatography Workstation version 6.0 software. All SCFA data are expressed as µmol/g.

### Results

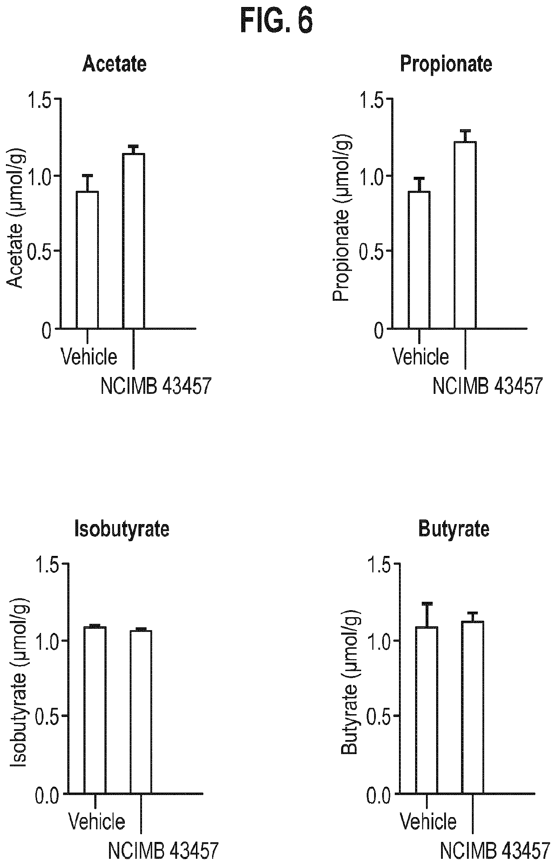

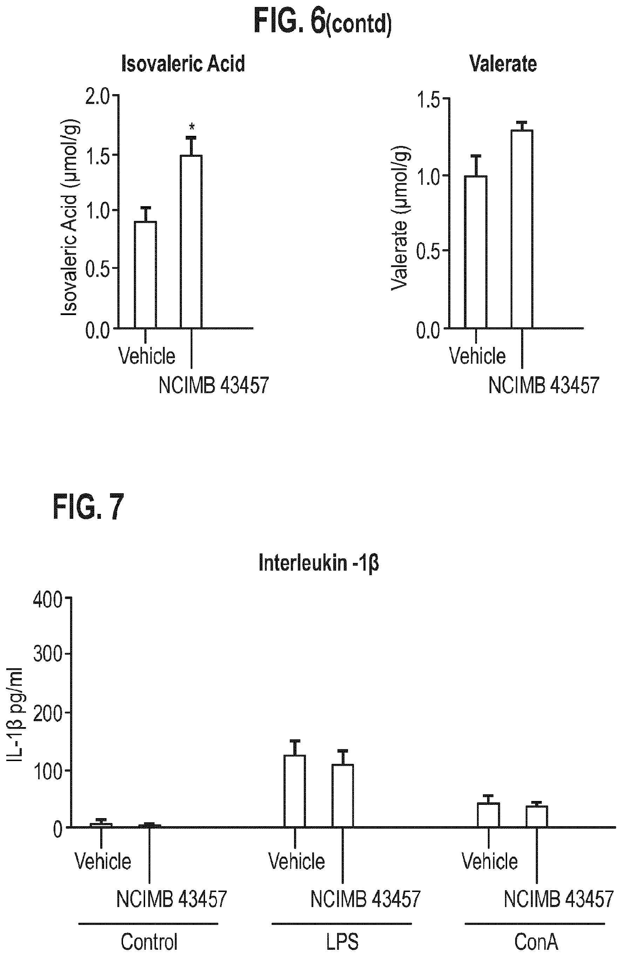

Figure 6 shows that treatment with NCIMB 43457 was able to increase the production of acetate, propionate, butyrate, isovaleric acid and valerate.

SCFAs produced by bacteria in the microbiome are key mediators of the beneficial effects elicited by the gut microbiome. These data suggest that bacteria from the genus *Anaerostipes* may be useful in promoting productivity of the microbiota, and therefore useful in the treatment or prevention of diseases associated with a reduced productivity of the microbiota. For example, SCFAs can regulate the immune response, therefore these data suggest that bacteria from the genus *Anaerostipes* may be useful in the treatment of inflammatory conditions.

Signalling of the microbiota-gut-brain axis is modulated by levels commensal metabolites. These data demonstrate that bacteria from the genus *Anaerostipes* can modulate the levels of commensal metabolites, for example by increasing the production of SCFAs. Accordingly, in certain embodiments, the compositions disclosed herein can treat or prevent a central nervous system disorder or condition by modulating the systemic levels of microbiota metabolites.

### Example 8 - Effect of Anaerostipes on peripheral immune markers

### Summary

This study investigated the effect of *Anaerostipes* on the production of several immune markers by isolated mouse splenocytes. Interleukin-1β (IL-1β), tumour necrosis factor-α (TNF-α), interferon-γ (IFN-y), interleukin-6 (IL-6) are pro-inflammatory cytokines; while interleukin-10 (IL-10) has anti-inflammatory properties. Both pro-and anti-inflammatory cytokines are necessary for the maintenance of a healthy immune and inflammatory system, an imbalance in these cytokines can lead to negative physiological outputs.

### Materials and methods

### Bacterial strains

*Anaerostipes hadrus* strain NCIMB 43457.

### Method

Male BALB/c mice received oral gavage (200µL volume) of 1 × 10⁹ CFU of the relevant bacterial strain for 6 consecutive days. On day 7, the animals were euthanized. Spleen was removed, collected in 5 mL RPMI media (with L-glutamine and sodium bicarbonate, R8758 Sigma + 10 % FBS (F7524, Sigma) + 1% Pen/Strep (P4333, Sigma)) and processed immediately after culls for ex-vivo immune stimulation. Spleen cells were first homogenised in the RPMI media. The homogenate step was followed by RBC lysis step where the cells were incubated for 5 mins in 1ml of RBC lysis buffer (11814389001 ROCHE, Sigma). 10 ml of the media was added to stop the lysis and followed by 200g centrifugation for 5 mins. This was followed by a final step where the cells were passed through a 40 µm strainer. The homogenate was then filtered over a 40 µm strainer, centrifuged at 200 g for 5 min and resuspended in media. Cells were counted and seeded (4,000,000/mL media). After 2.5 h of adaptation, cells were stimulated with lipopolysaccharide (LPS-2 µg/ml) or concanavalin A (ConA-2.5 µg/ml) for 24 h. Following stimulation, the supernatants were harvested to assess the cytokine release using Proinflammatory Panel 1 (mouse) V-PLEX Kit (Meso Scale Discovery, Maryland, USA) for TNFα, IL-10, IL-1β, Interferon γ, CXCL2 and IL6. The analyses were performed using MESO QuickPlex SQ 120, SECTOR Imager 2400, SECTOR Imager 6000, SECTOR S 600.

### Results

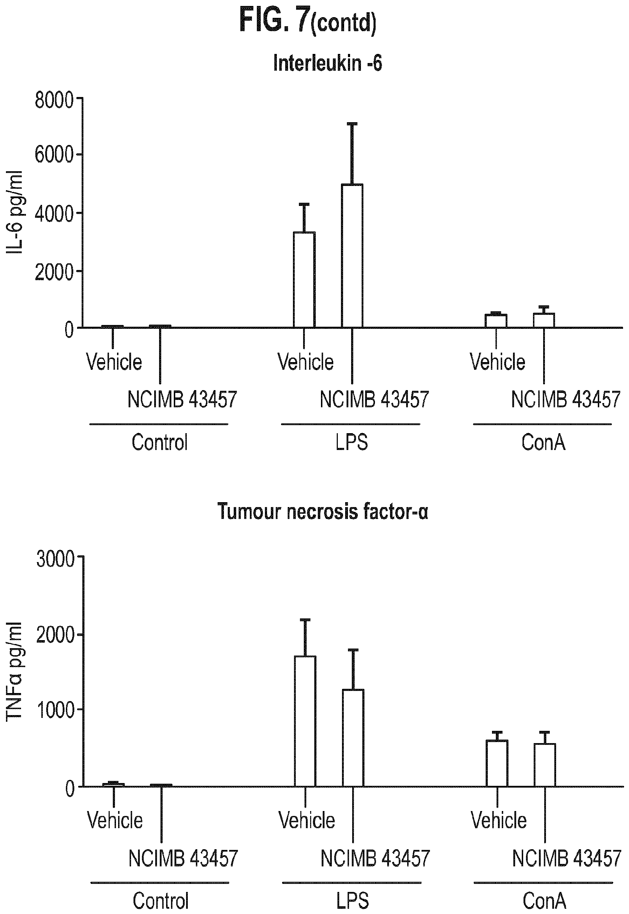

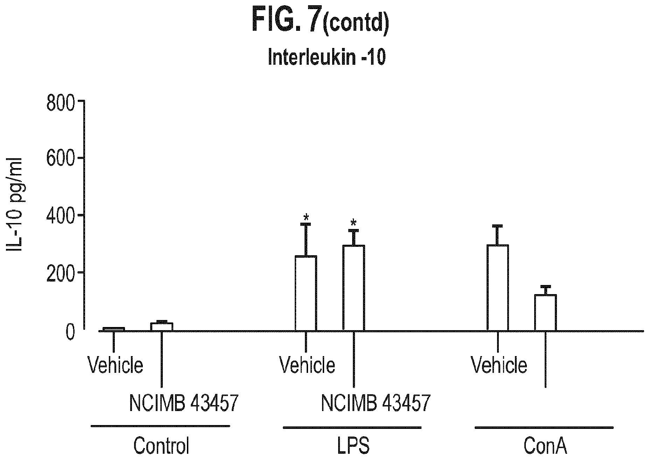

Figure 7 demonstrates that *Anaerostipes hadrus* strain NCIMB 43457 was able to decrease the levels of TNF-α and IL-1β when stimulated with LPS; and increased levels of IL-6 when stimulated with ConA. *Anaerostipes hadrus* strain NCIMB 43457 significantly increased IL-10 production when stimulated with LPS.

### Example 9 - Effect of Anaerostipes on gene expression in the brain

### Summary

This study investigated the effect of *Anaerostipes* on expression of certain genes of interest in the brain mRNA levels for markers for the oxytocinergic system (oxytocin receptor and vasopressin receptor), endocrine system (mineralocorticoid (Nr3c1); glucocorticoid receptor (Nr3c2); corticosterone releasing factor (CRF) and receptors; Brain derived neurotrophic factor (BDNF)), immune system (Il-6, TNF-a, TLR-4, CD11b); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala, hippocampus and prefrontal cortex (PFC), which are key brain regions of the limbic system involved in emotional response.

### Materials and Methods

### Bacterial strains

*Anaerostipes hadrus* strain NCIMB 43457.

### Method

Male BALB/c mice received oral gavage (200µL volume) of 1 X 10⁹ CFU of NCIMB 43457 for 6 consecutive days. On day 7, the animals were euthanized. The brain was quickly excised, dissected and each brain region was snap-frozen on dry ice and stored at -80 °C for further analysis. mRNA expression was quantified as described in Example 6.

### Results

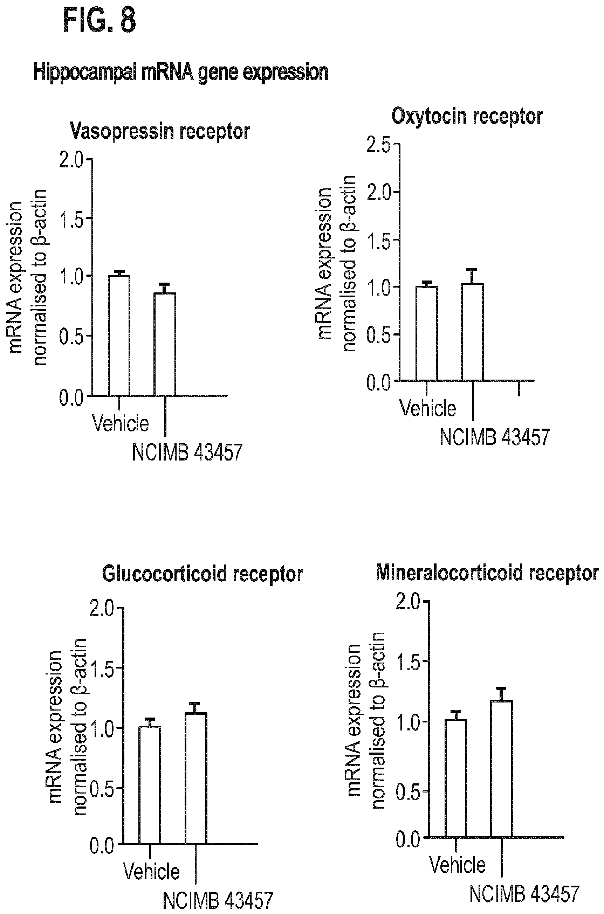

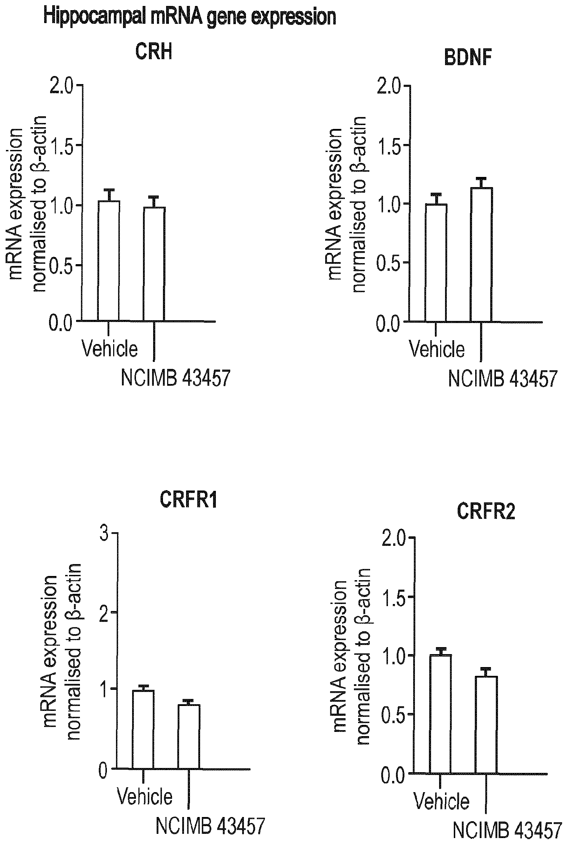

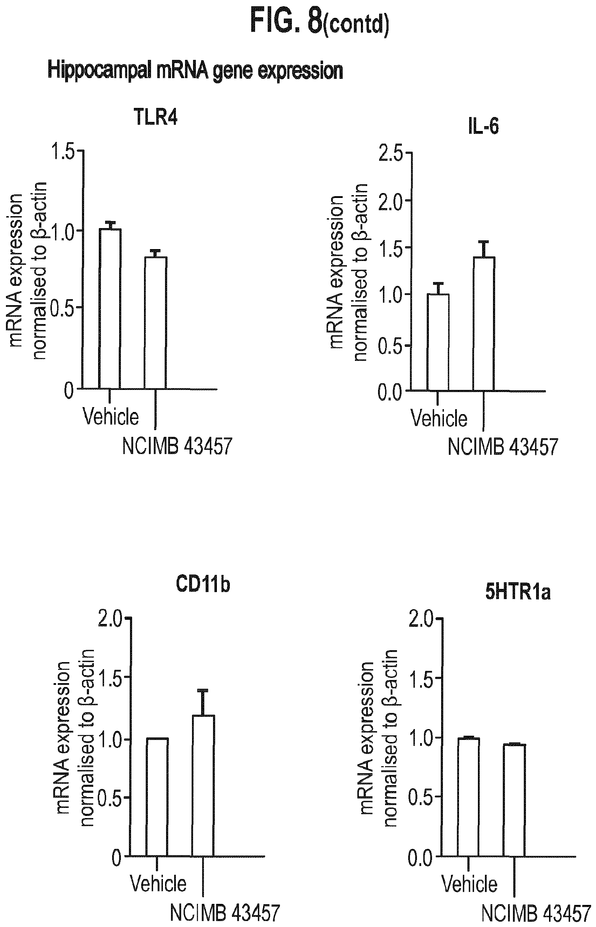

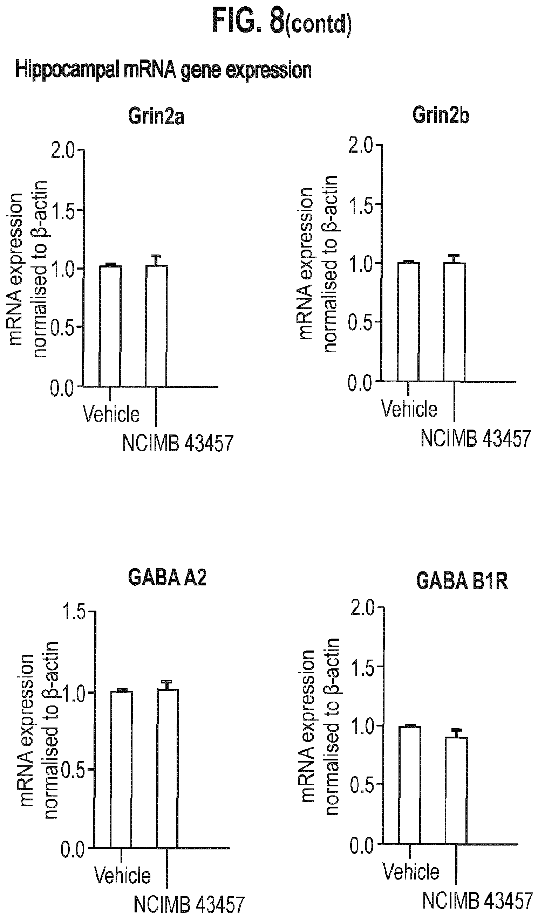

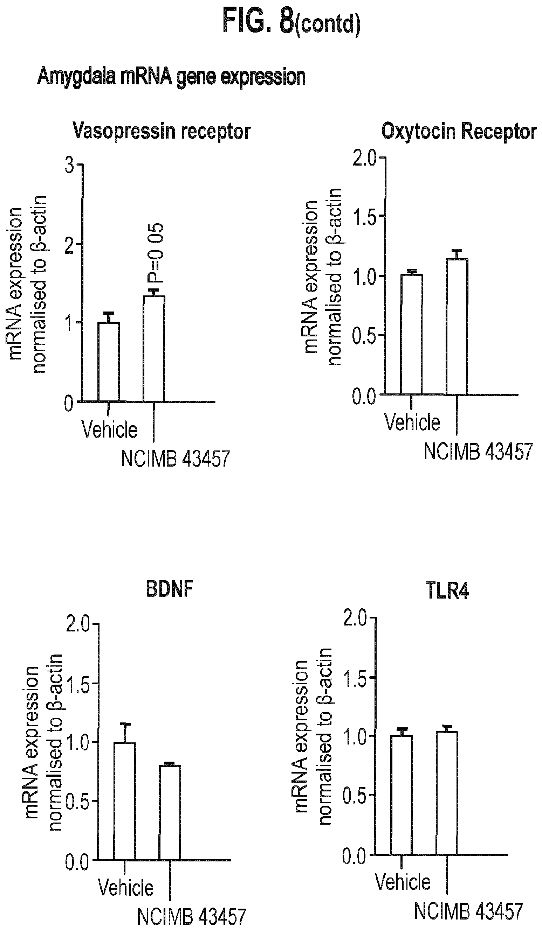

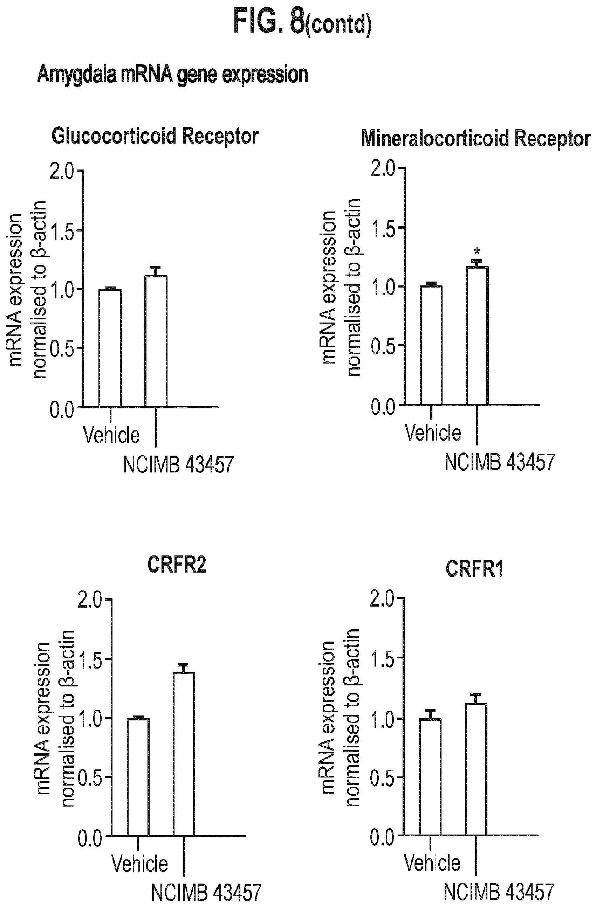

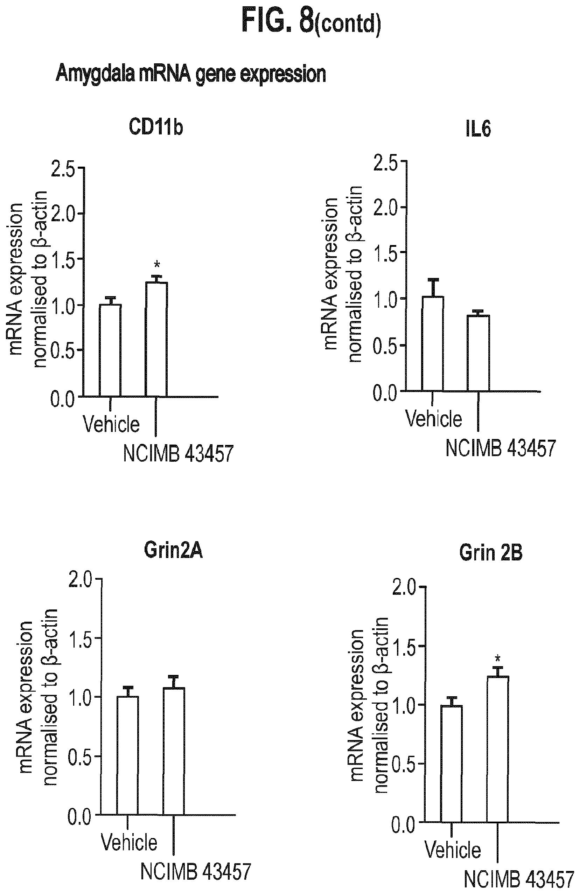

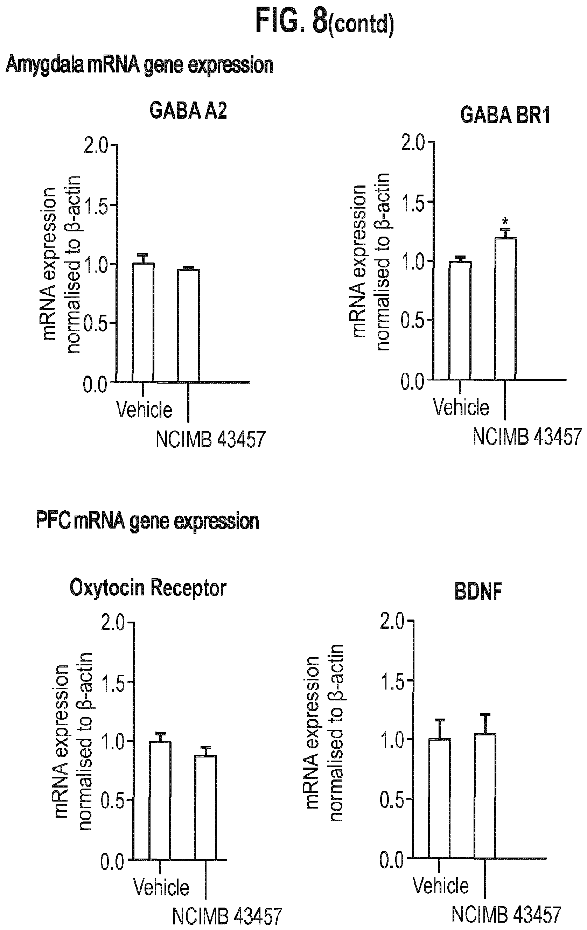

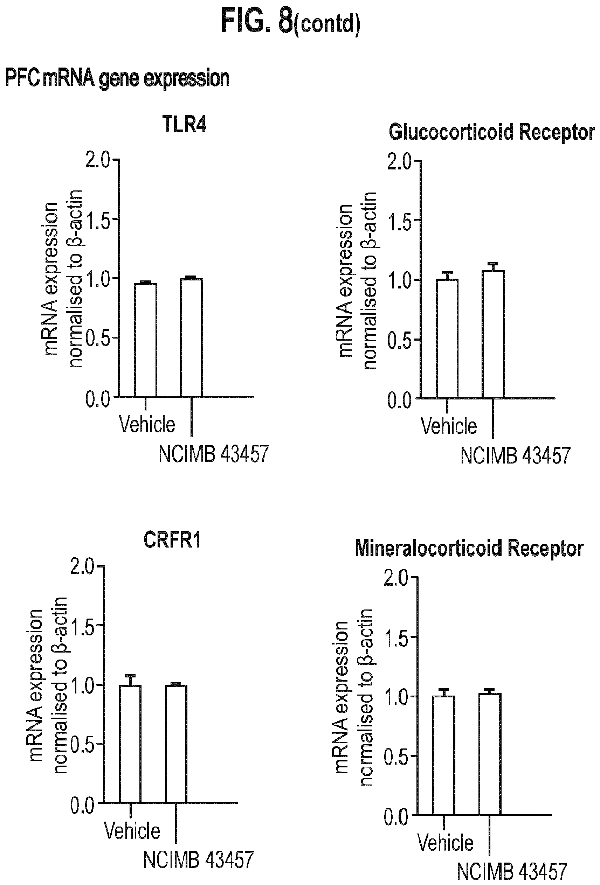

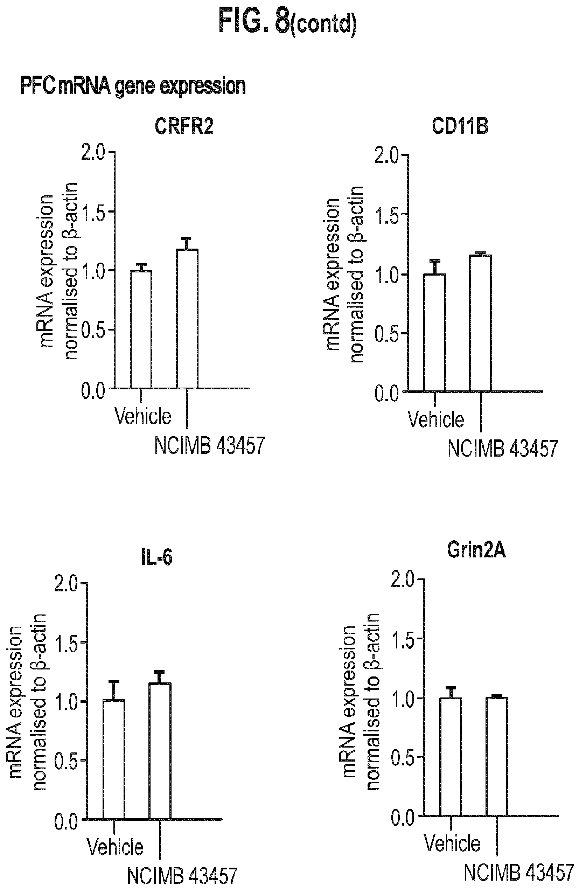

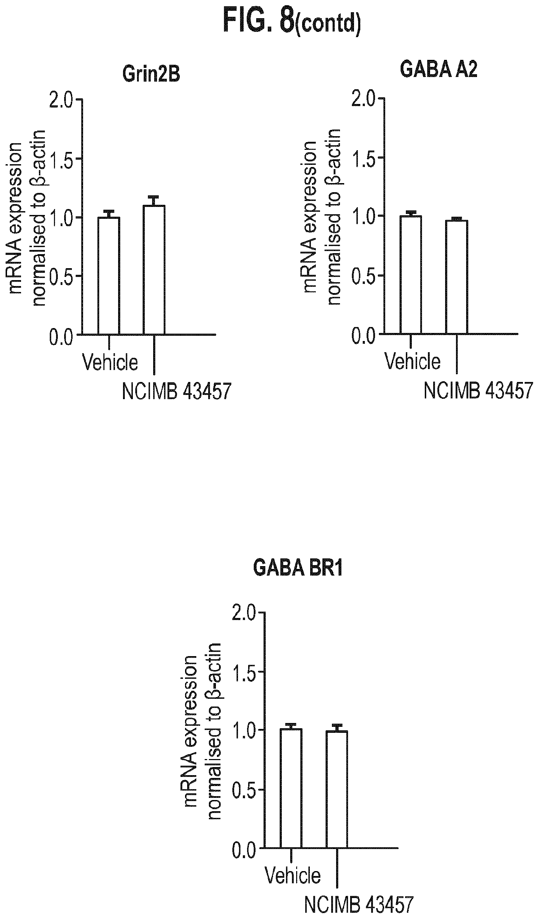

As shown in Figure 8, a significant change in the expression of TLR-4 (D) in the hippocampus; and mineralocorticoid receptor (E), CD11b (F), Grin2b (G), GABA A2 (H) and GABA BR1 (I) in the amygdala, was observed in mice that have been treated with Rfa1, relative to treatment with vehicle only. In addition, glucocorticoid receptor and CD11b expression were increased in the hippocampus, amygdala and PFC when treated with Rfa1, relative to the expression when treated with vehicle only.

As shown in Figure 8, from the genes tested, a significant decrease in the expression of CRFR2 (B) and 5HTR1a (D), and a significant increase in the expression of CD11b (C), Grin2a (E) and Grin2b (F) were observed in the amygdala in mice which had been treated with *Anaerostipes hadrus,* relative to treatment with vehicle control. An increase in the expression of vasopressin receptor (A) was also observed in this brain region for the treated animals. Figure 8 shows that in the amygdala, treatment with *Anaerostipes hadrus* resulted in significantly higher expression of CRFR2 (B), CRFR1 (C) and CD11b (D), as well as a trend for an increase in the expression of mineralocorticoid receptor (A). Mice treated with *Anaerostipes hadrus* also had an increased expression of CRFR2 (A), CD11b (B) and IL-6 (C) in the PFC. This indicates that bacteria from the genus *Anaerostipes* are capable of modulating the levels of expression of proteins in the brain, therefore they may be useful in the treatment or prevention of CNS diseases, disorders or conditions, such as autism.

The data in Figure 7 and 8 therefore indicate that *Anaerostipes* may be useful in the treatment or prevention of disorders or conditions that may benefit from the modulation in the levels of expression of these proteins in the brain, e.g. CNS diseases and disorders.

### Example 10 - Effect of Anaerostipes on behaviour in models of autism spectrum disorders

### Summary

This study investigated the effect of *Anaerostipes* on a number of behavioural readouts including anxiety-related behaviour (marble burying test, grooming test, elevated plus maze test, open field test), social behaviour (3-chamber social interaction test), cognitive performance (novel object recognition) and depression/acute stress (forced swim test) in an environmental animal model (maternal immune activation model) and/or a genetic animal model (Btbr mouse strain) of autism spectrum disorder.

### Materials and Methods

### BTRB mouse model

Btbr animals were bred in house with brother-sister mating. The male offspring from these animals were separated from their mothers at 3 weeks old and daily administration of the live biotherapeutic or control commenced at 8 weeks of age. Behavioural symptoms started at 11 weeks old. A control age-matched C57/B16 group was included as a reference control group.

### MIA mouse model

Female C57/Bl6 mice (8 weeks old) and age matched males were purchased from Harlan UK. After 1-week habituation these animals were mated. At embryonic day 12.5, females received either an injection of the viral mimetic poly-IC to activate the maternal immune system, or a saline vehicle injection. The male offspring from these animals was separated from their mothers at 3 weeks old and daily administration of the live biotherapeutic or control commenced at 8 weeks of age. Behavioural symptoms started at 11 weeks old.

### Methods

Animals received daily oral gavage of PBS or the live biotherapeutics prepared to 1 X 10⁹ CFU/mL in PBS. Dosing continued daily throughout the behavioural paradigm. Behaviour tests were performed after 4-7 weeks of initiation of dosing with the bacteria.

### Example 10a - The marble burying test

Mice were individually placed in a novel plexiglas cage (35 × 28 × 18.5 cm, L × W × H), filled up with sawdust (5-10 cm) and 20 marbles on top of it (five rows or marbles regularly spaced 2 cm away from the walls and 2 cm apart). Thirty minutes later, the number of marbles buried for more than 2/3 of their surface was counted. A higher number of marbles buried represents a heightened state of stereotype behaviour or higher levels of anxiety (neophobia). The marble burying test is a useful model of neophobia, anxiety and obsessive compulsive behaviour.

### Results

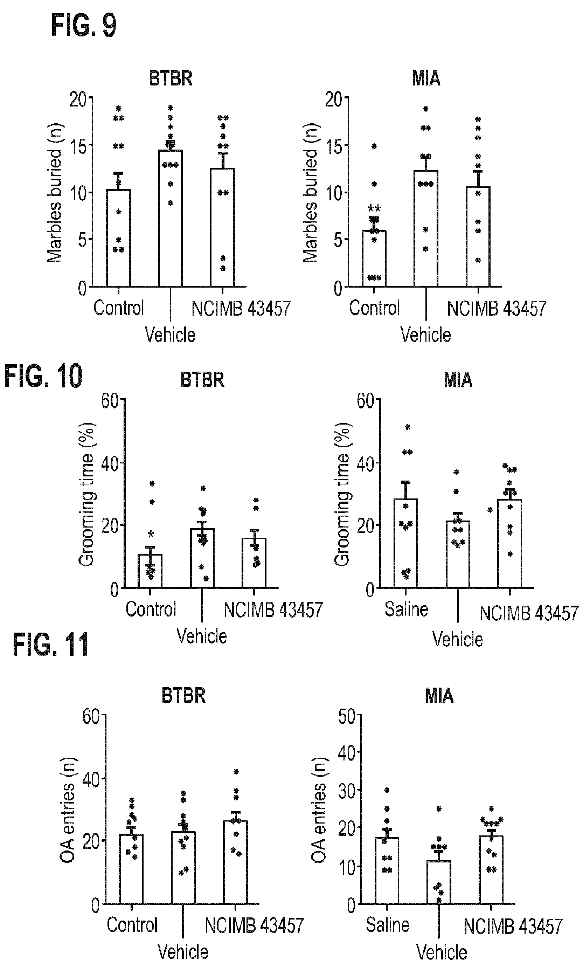

As shown in Figure 9, Btbr and MIA mice treated with NCIMB 43457 exhibited reduced stereotype-related behaviour, as they buried less marbles relative to mice administered vehicle.

### Example 10b - The grooming test

Self-grooming was evaluated in a 6.5 cm diameter × 10 cm tall, clear glass beaker covered with a filter top. Experimental animals were brought to the test room to habituate up to 1 hour prior to the test. The test was approximately 20 minutes in duration, and the cumulative time spent by the test animal grooming was recorded. Between tests the beakers were cleaned thoroughly for next use. This test is used as an index for stereotyped and repetitive behaviour. An increase in time spent grooming is indicative of increased stereotyped or repetitive behaviour.

### Results

As shown in Figure 10, Btbr mice treated with NCIMB 43457 exhibited reduced stereotype-related behaviour, as they spent less time grooming relative to mice administered vehicle.

### Example 10c - Elevated plus maze

The set up was made of a grey plastic cross-shaped maze 1 m elevated from the floor, comprising two open (fearful) and two closed (safe) arms (50 × 5 × 15 cm walls or 1 cm no wall). Experiments occurred under red light (~5 lux). Mice were individually placed into the center of the maze facing an open arm (to avoid direct entrance into a closed one) and were allowed 5-min free exploration. Experiments were videotaped using a ceiling camera for further parameters analysis using Ethovision software (3.1 version, Noldus, TrackSys, Nottingham, UK). The percentage of time spent, distance moved and the number of entries in each arm were measured, for anxiety behavior and locomotor activity, respectively (entrance in an arm was defined as all four paws inside the arm). An increase in time or number of entries into an open arm is an index of reduced anxiety.

### Results

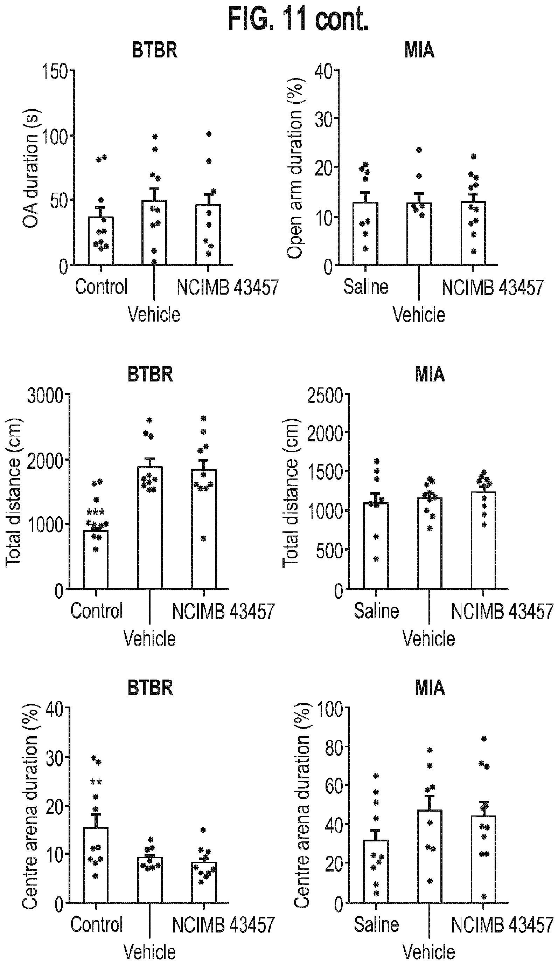

As shown in Figure 11, Btbr mice treated with NCIMB 43457 exhibited increased anxiety-related behaviour, as they spent less time in the open arm relative to mice administered vehicle.

### Example 10d - Social behaviour

The social testing apparatus was a rectangle, three-chambered box. Each chamber was 20 cm L × 40 cm W × 22 cm H. Dividing walls were made with small circular openings (5 cm in diameter) allowing access into each chamber. Two identical wire cup-like cages, with a bottom diameter of 10 cm, 13 cm in height and bars spaced 1.2 cm, allowing nose contact between the bars, but prevented fighting, were placed inside each side chamber in bilaterally symmetric positions. The test has three phases of 10 min each: 1) habitation 2) mouse versus object 3) novel mouse versus familiar mouse. Experiments were videotaped using a ceiling camera for further parameters analysis using Ethovision software (3.1 version, Noldus, TrackSys, Nottingham, UK). For the first phase the test mouse was placed into the middle chamber and allowed to explore the entire box with empty small wire cages inside for a 10-min habituation session. After the habituation period, the test mouse is removed from the testing box for a short interval while an object is placed in one side chamber and an unfamiliar conspecific male mouse (no prior contact with the test subject) in the other side chamber, both enclosed in a wire cup-like cage. During phase two, the test mouse is placed in the middle chamber and allowed to explore the entire box for 10 min. The amount of time spent exploring the object or mouse in each chamber and the number of entries into each chamber were evaluated. The location of the unfamiliar mouse in the left vs right side chamber was systematically alternated between trials. An entry was defined as all four paws in one chamber. During the third phase an object was replaced with an unfamiliar mouse serving as a novel mouse and in the other chamber the mouse used in phase two was kept the same, now serving as familiar mouse. After every trial, all chambers and cup-like wire cages were cleaned with 10% ethanol, dried and ventilated for a few minutes to prevent olfactory cue bias and to ensure proper disinfection. Lack of innate side preference was confirmed during the initial 10 min of habituation to the entire arena. Control animals are naturally interested in a conspecific mouse more than an inanimate object (sociability). In a similar vein, control animals spend more time interacting with a novel unfamiliar mouse then one they have already had interactions with. Some animal models, as used here, have deficits in this social paradigm.

### Results

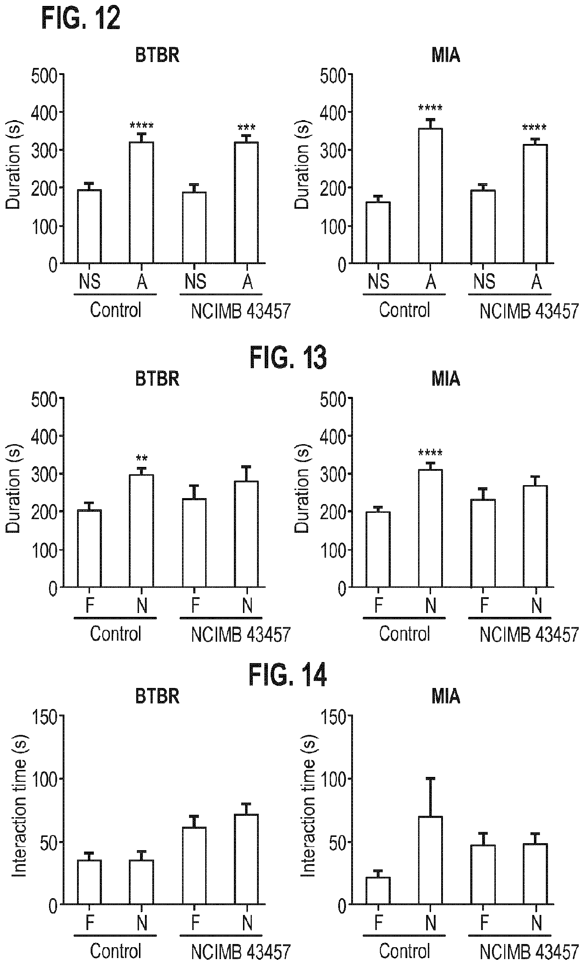

Figures 12 and 13 show that treatment with NCIMB 43457 resulted in more social behaviour. Both BtBR and MIA mice spent a significantly larger proportion of the time in the animal chamber than the non-social stimuli chamber (Figure 13), and also spent more time in the novel animal chamber than the familiar animal chamber (Figure 14), relative to mice administered vehicle.

### Example 10e - Novel object recognition

Mice were placed in the middle of a grey plastic rectangular box (40 × 32 × 23 cm, L × W × H) under a dimly light, 60 lux at the level of the arena, for 10 min. 24 h after mice were placed in the box with the two identical objects for a total time of 10 min (acquisition phase). After a 24 h, one of the two identical objects were substituted with a novel object and mice were placed in the middle of the box at the mid-point of the wall opposite the sample objects for a total time of 10 min (retention phase). Animals were acclimatized to the testing room for 30 min prior each experiment. Box and objects were cleaned with alcohol 10% to avoid any cue smell between each trial. Experiments were videotaped using a ceiling camera for further parameter analysis. Directed contacts with the objects, include any contact with mouth, nose or paw or minimal defined distance were counted as an interaction. Control animals will discriminate between an object they have had time to explore and a new object.

### Results

As shown in Figure 14, Btbr and MIA mice treated with NCIMB 43457 exhibited increased cognitive performance, as the amount of time spent interacting with a novel object relative to a familiar objection was significantly increased, as compared to mice administered vehicle.

### Example 10f - Forced swim

Mice were individually placed in a clear glass cylinder (24 × 21 cm diameter), containing 15-cm-depth water (25 ± 0.5°C). Water was changed between each animal. The test lasted 6 min and experiments were videotaped using a numeric tripod-fixed camera; data were further scored twice using the videos (Video Media Player software) and averaged by an experimenter blind to conditions. The latency to immobility was scored. The time of immobility (s) was measured for the last 4 min of the test, with immobility being defined as a total absence of movement except slight motions to maintain the head above the water. An increase in immobility means an increase in depressive-like behaviour as the animal has resigned itself to its situation (learned helplessness).

### Results

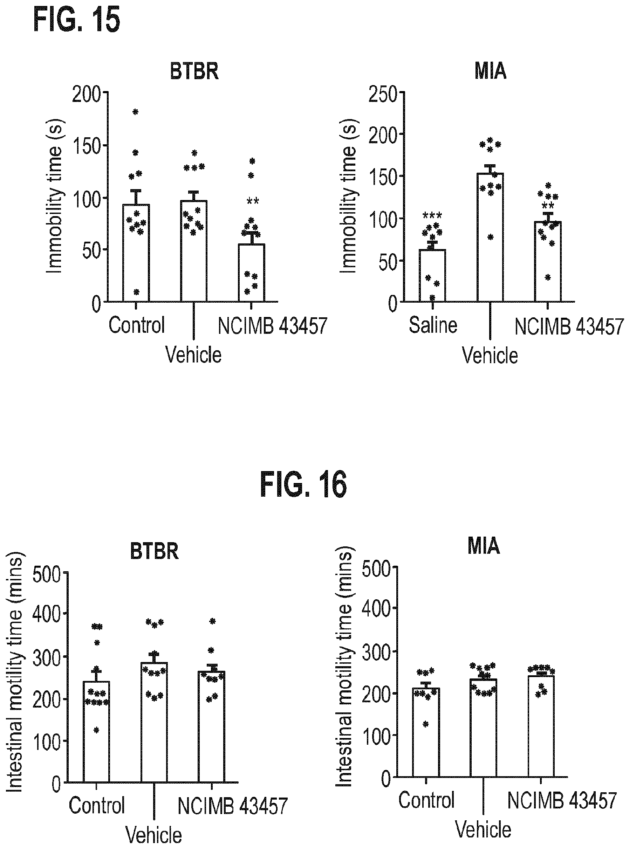

Treatment with NCIMB 43457 reduced depressive-like behaviour, as both Btbr and MIA mice spent significantly less time immobile (Figure 15). Together, the results indicate that *Anaerostipes* may be useful in therapy, e.g. in the treatment or prevention CNS disorders, such as autism, anxiety and depression.

### Example 11 - Effect of Anaerostipes on in vivo intestinal motility

This experiment tested for changes in gut barrier function to ascertain whether chronic treatment with the biotherapeutic alters intestinal motility.

### Methods

This experiment involves the oral administration of a given amount of a non-toxic, coloured marker (Carmin Red) to determine motility of the gut. Time to excretion of the first coloured faecal bolus is recorded as 'time of whole gut transit' and is used as an index of peristaltic motility in the whole intestine. Mice are single housed for 3h prior to the assay to allow habituation to a new cage. Carmin red dye (100-200 ul of 6% Carmin red in 0.5% methylcellulose per mouse) is given orally by gavage. Each cage is visually inspected every 10 min. The time of the first coloured bolus (red) is recorded. Following the appearance of the first coloured bolus mice are returned to normal housing conditions.

### Results

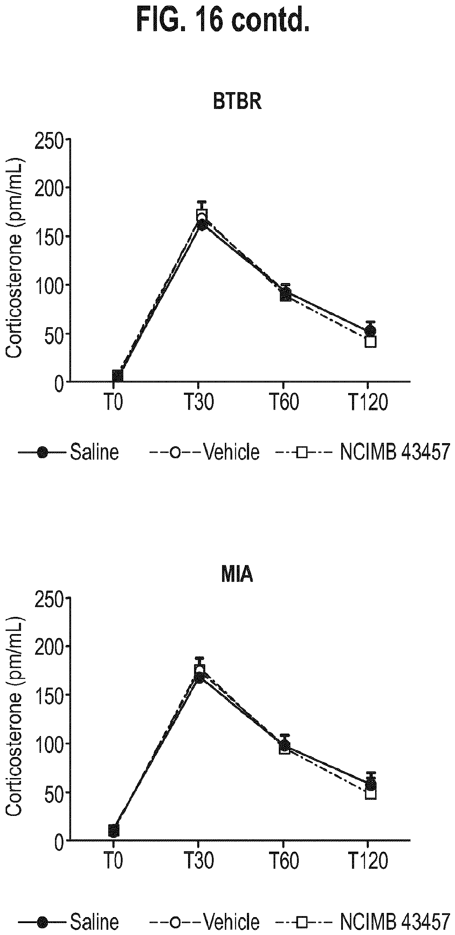

Treatment with NCIMB 43457 did not lead to a significant difference in intestinal motility time relative to vehicle-treated controls for BtBR (Figure 16).

### Example 12 - Effect of Anaerostipes on ex vivo gastrointestinal permeability

The permeability of the ileum and colon in Btbr and/or MIA models of autism spectrum disorder was assessed ex vivo using Ussing chambers. An increase in FITC concentration represents an undesired effect as it indicates an increase in the 'leakiness' of the intestinal barrier.

### Methods

The Btbr and MIA models were generated an maintained as described in Example 10. Mice were euthanized by cervical dislocation, and the distal ileum and colon were removed, placed in chilled Krebs solution opened along the mesenteric line and carefully rinsed. Preparations were then placed in Ussing chambers (Harvard Apparatus, Kent, UK, exposed area of 0.12 cm²) as described previously [110] with oxygenated (95% O2, 5% CO₂) Krebs buffer maintained at 37°C. 4 kDa FITC-dextran was added to the mucosal chamber at a final concentration of 2.5 mg/mL; 200 µL samples were collected from the serosal chamber every 30 min for the following 3 h.

### Results

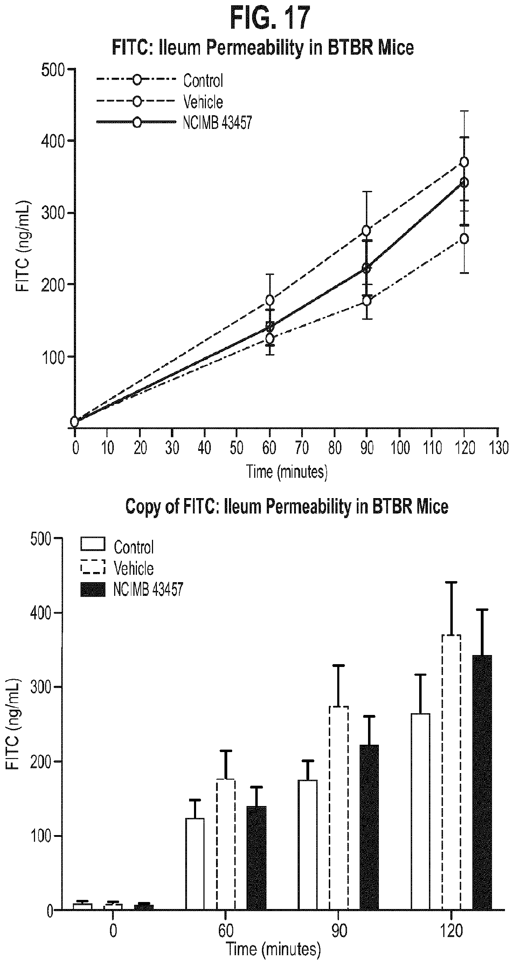

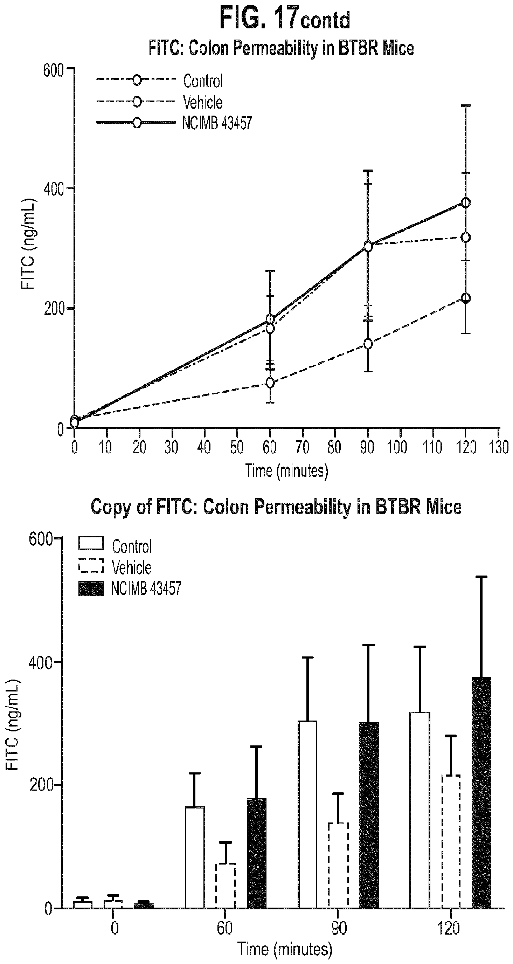

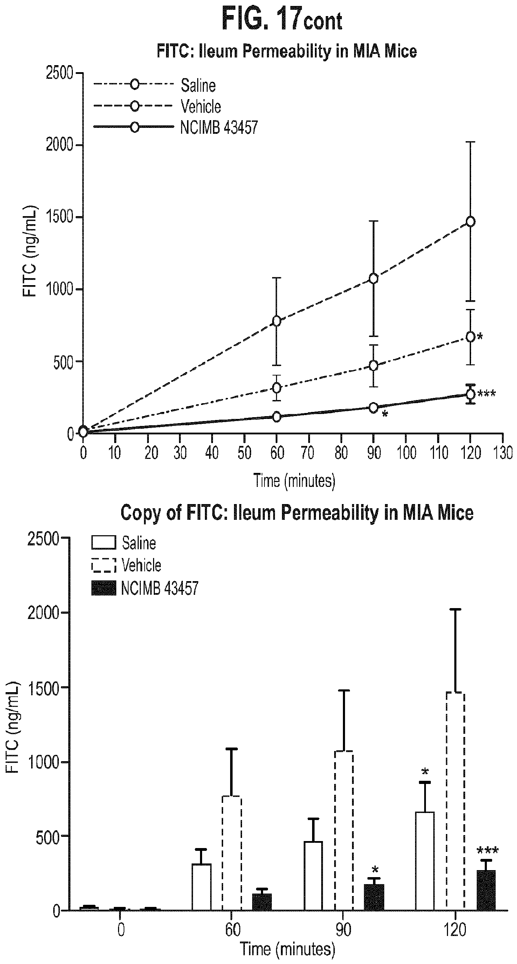

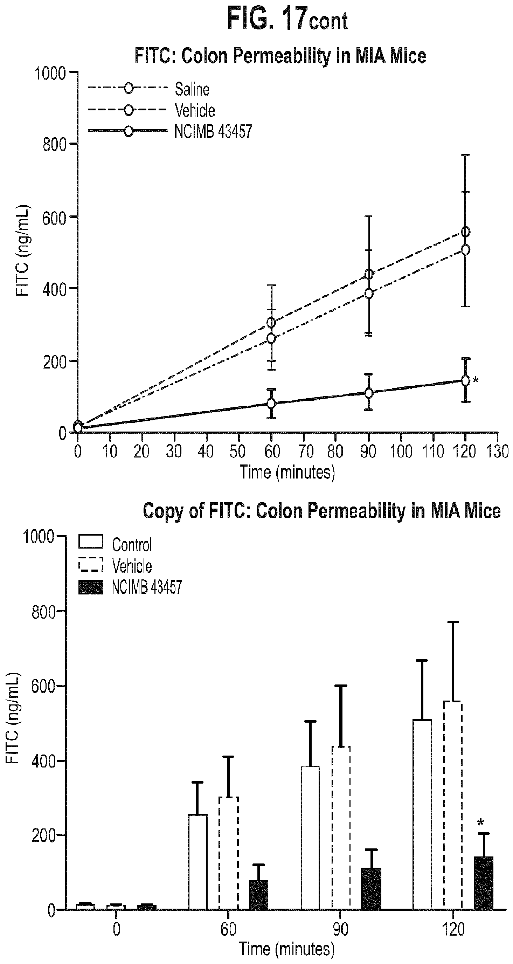

Figure 17 demonstrates that treatment with *Anaerostipes hadrus* leads to a reduction in permeability in the ileum and the colon.

These results suggest *Anaerostipes* strains may be useful in modifying intestinal permeability, e.g. for the treatment or prevention of disorders or conditions associated with intestinal permeability.

### Example 13 - Effect of Anaerostipes on brainstem monoamine levels in models of autism spectrum disorders

### Summary

This study investigated the effect of *Anaerostipes* strain NCIMB 43457 on brainstem monoamine levels in Btbr and/or MIA models of autism spectrum disorder. The brainstem encompasses a number of cell bodies for all the major neurotransmitter systems of the brain including serotonin and noradrenaline.

### Materials and Methods

### Mouse models

The Btbr and MIA models were generated and maintained as described in Example 10.

### Method

Animals received daily oral gavage of PBS or the live biotherapeutics prepared to 1 X 10⁹ CFU/mL in PBS. After 8 weeks, the animals were euthanized and neurotransmitter concentration was analysed by HPLC on samples from the brainstem. Briefly, brainstem tissue was sonicated in 500 µl of chilled mobile phase spiked with 4 ng/40 µl of N-Methyl 5-HT (Sigma Chemical Co., UK) as internal standard. The mobile phase contained 0.1 M citric acid, 5.6 mM octane-1-sulphonic acid (Sigma),

0.1 M sodium dihydrogen phosphate, 0.01 mM EDTA (Alkem/Reagecon, Cork) and 9% (v/v) methanol (Alkem/Reagecon), and was adjusted to pH 2.8 using 4 N sodium hydroxide (Alkem/Reagecon). Homogenates were then centrifuged for 15 min at 22,000 × g at 4°C and 40 µl of the supernatant injected onto the HPLC system which consisted of a SCL 10-Avp system controller, LECD 6A electrochemical detector (Shimadzu), a LC-10AS pump, a CTO-10A oven, a SIL-10A autoinjector (with sample cooler maintained at 40 C) and an online Gastorr Degasser (ISS, UK). A reverse-phase column (Kinetex 2.6 u C18 100 × 4.6 mm, Phenomenex) maintained at 30°C was employed in the separation (Flow rate 0.9 ml/min). The glassy carbon working electrode combined with an Ag/AgCl reference electrode (Shimdazu) operated a +08 V and the chromatograms generated were analyzed using Class-VP 5 software (Shimadzu). The neurotransmitters were identified by their characteristic retention times as determined by standard injections, which run at regular intervals during the sample analysis. The ratios of peak heights of analyte versus internal standard were measured and compared with standard injection. Results were expressed as ng of neurotransmitter per g fresh weight of tissue.

### Results

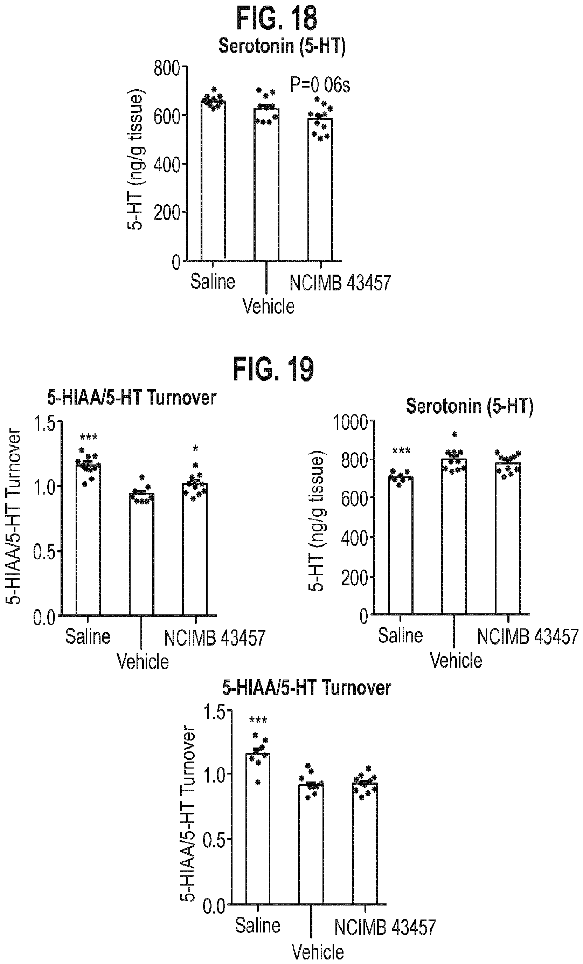

As shown in Figures 18 and 19, mice administered NCIMB 43457 have altered levels of certain monoamines in the brainstem. Btbr and MIA mice had lower serotonin levels (Figure 18) compared with animals administered vehicle, and the 5-HIAA/5-HT turnover was significantly increased in BtBR mice (Figure 19). This suggests that *Anaerostipes* is useful in the treatment or prevention of disorders associated with dysregulation of these neurotransmitter systems, e.g. CNS disorders such as autism.

### Example 14 - Effect of Anaerostipes on gene expression in the amygdala in models of autism spectrum disorders

### Summary

This study investigated the effect of *Anaerostipes* strain NCIMB 43457 on the expression of certain genes of interest in the brain of Btbr and MIA mice. mRNA levels for markers for the oxytocinergic system (oxytocin receptor and vasopressin receptor), endocrine system (mineralocorticoid (Nr3cl); glucocorticoid receptor (Nr3c2); corticosterone releasing factor (CRF) and receptors; and brain derived neurotrophic factor (BDNF)), immune system (Il-6, TNF-α and TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala.

### Materials and Methods

### Mouse models

The Btbr and MIA models were generated and maintained as described in Example 10.

### Method

Animals received daily oral gavage of PBS or the live biotherapeutics prepared to 1 X 10⁹ CFU/mL in PBS. After 8 weeks, the animals were euthanized. The brain was quickly excised, dissected and each brain region was snap-frozen on dry ice and stored at -80°C for further analysis. mRNA expression was quantified as described in Example 6.

### Results

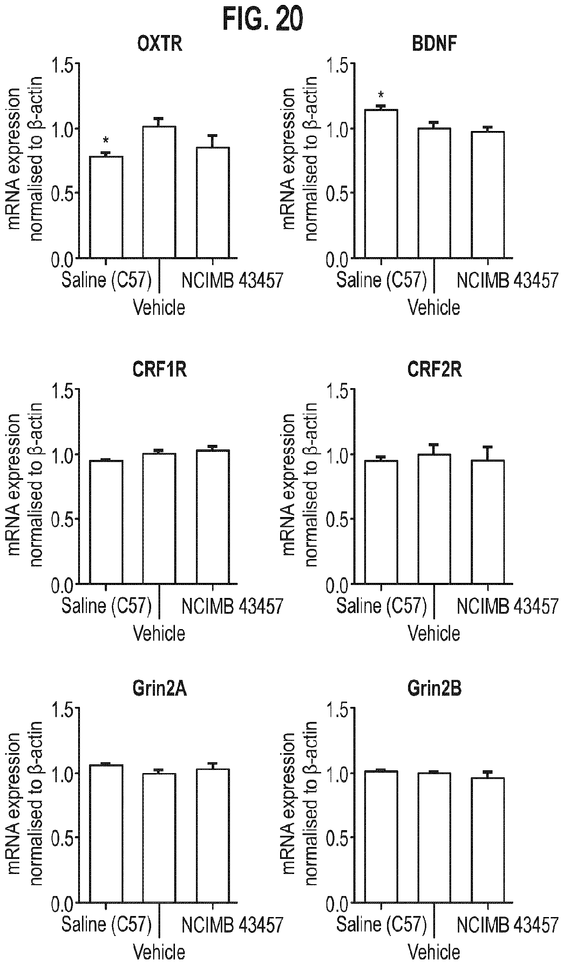

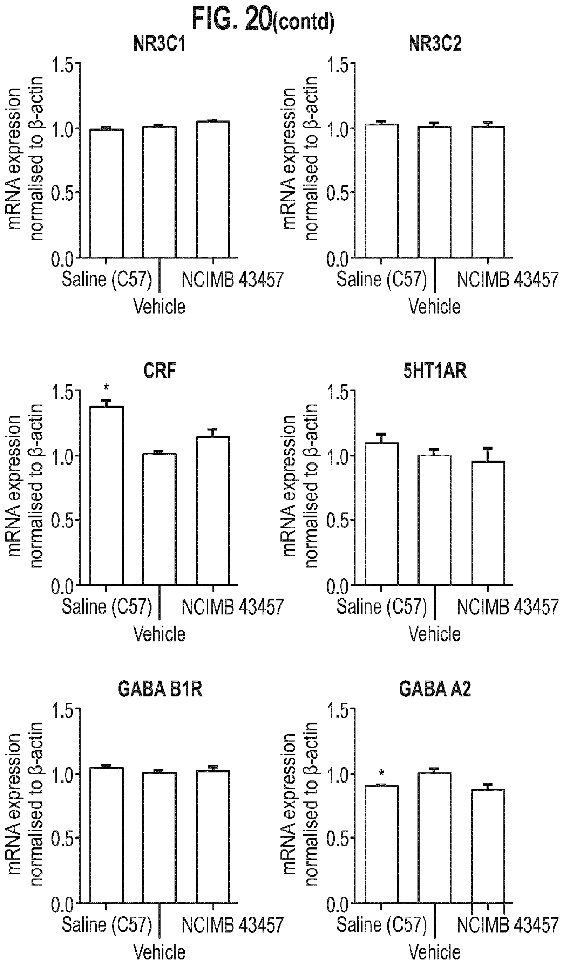

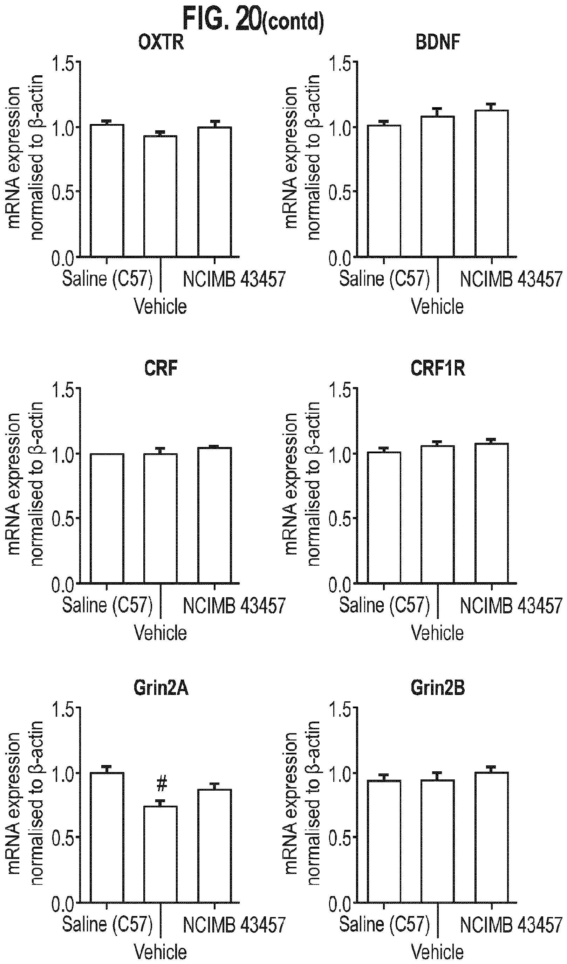

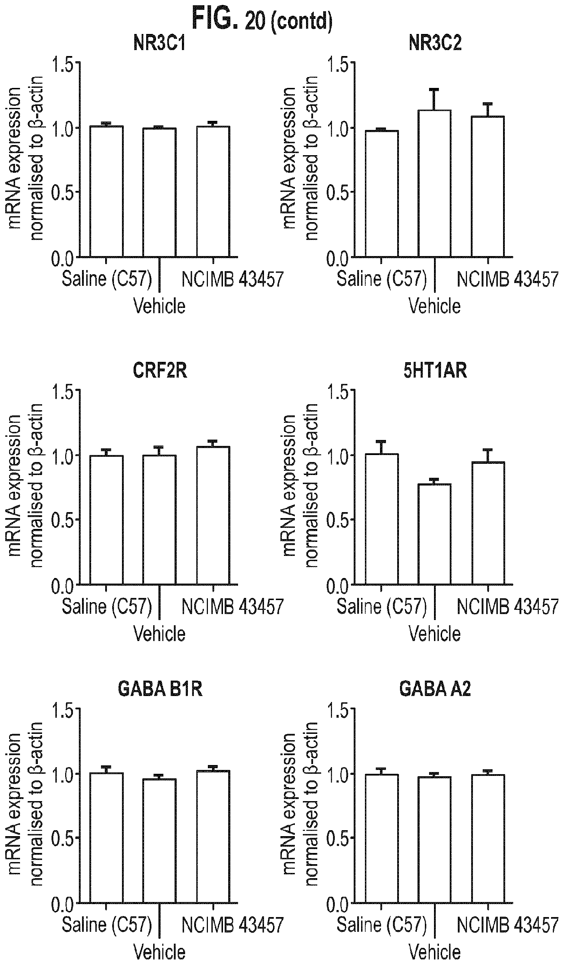

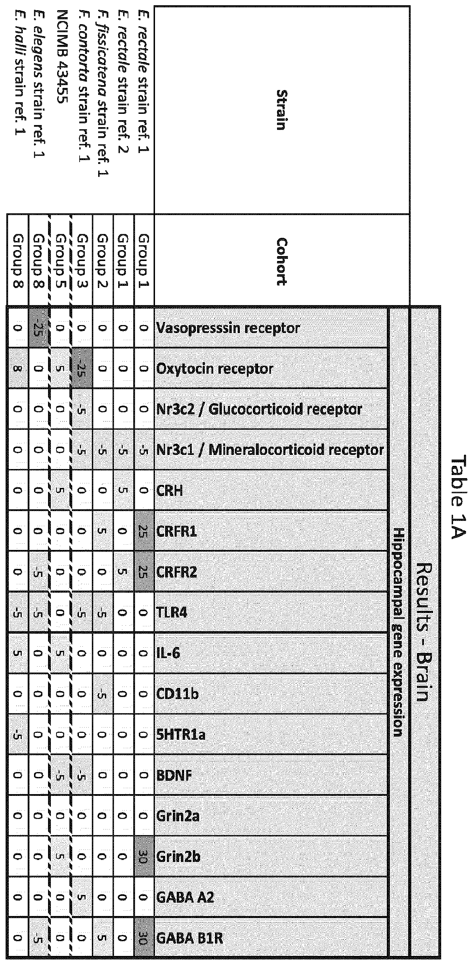

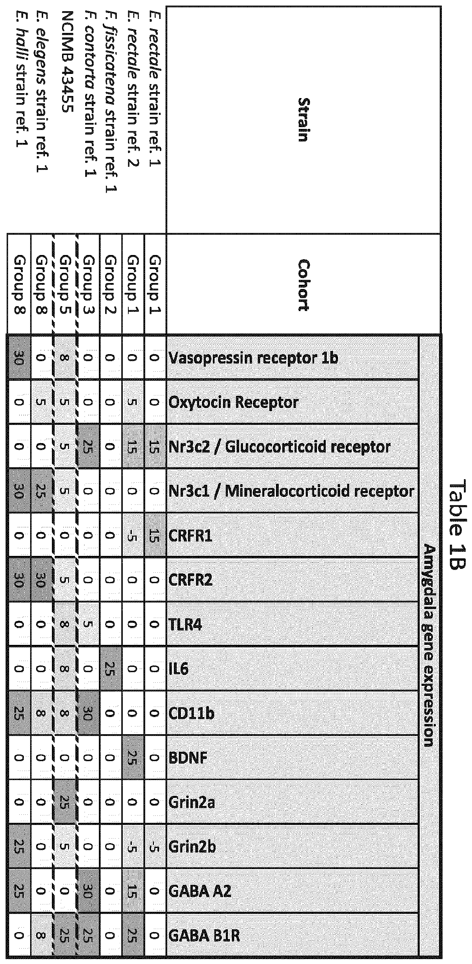

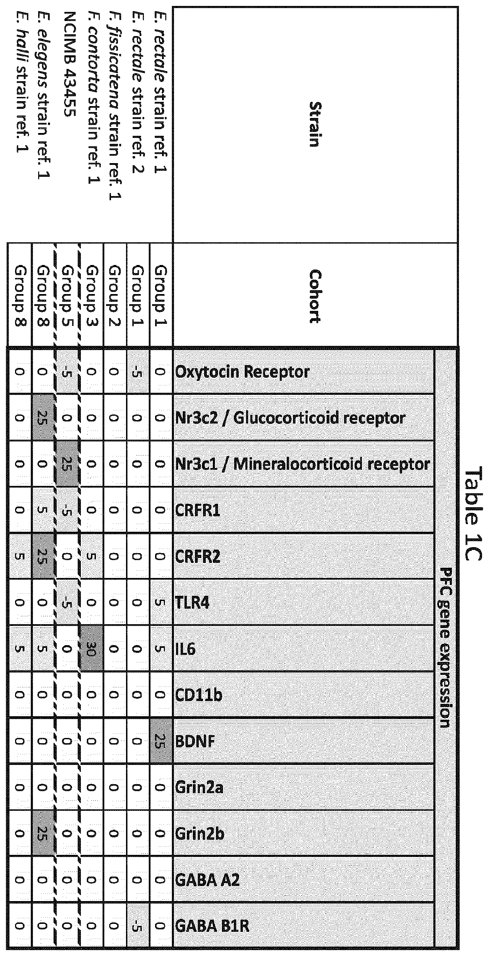

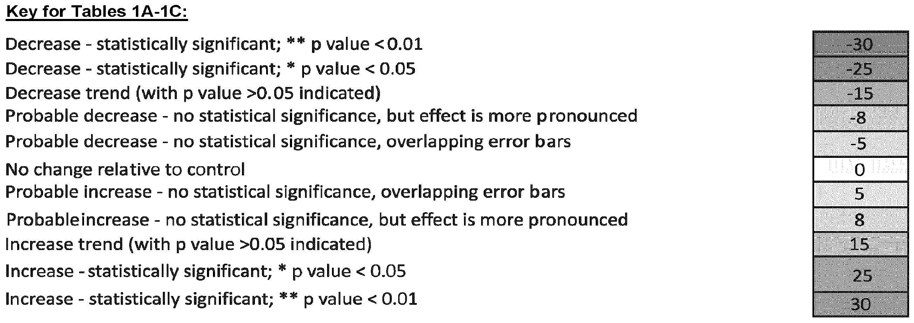

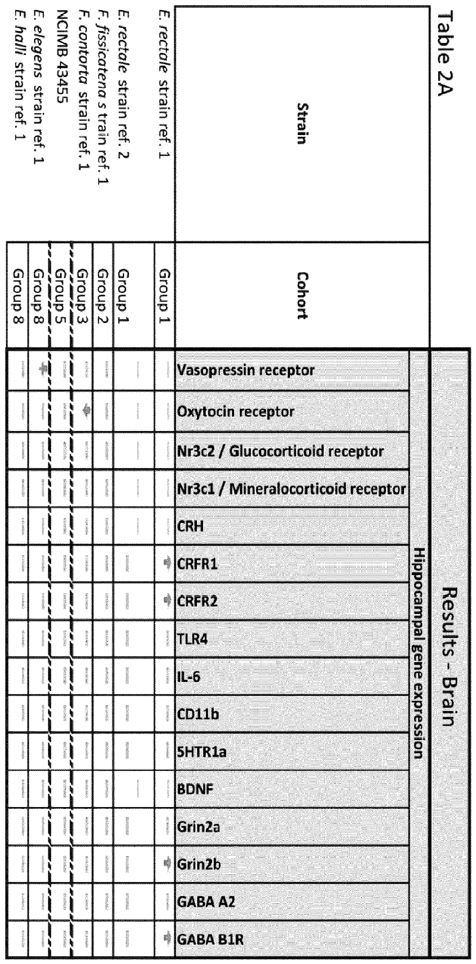

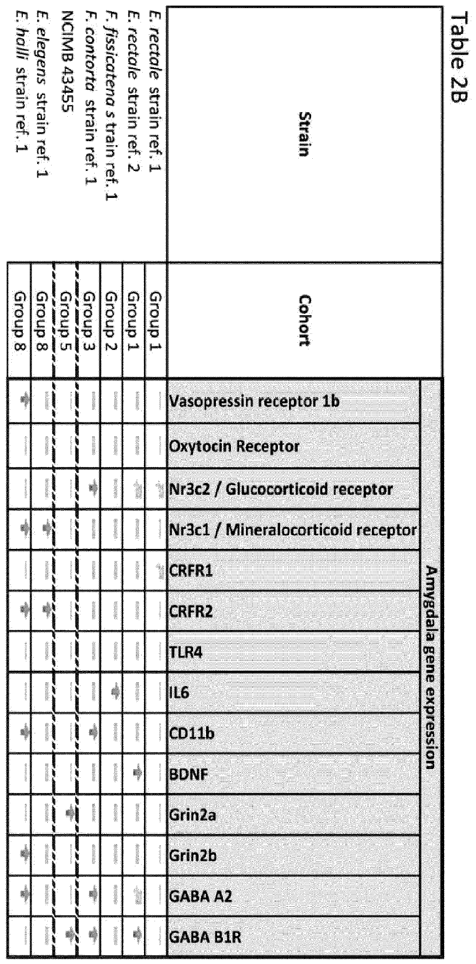

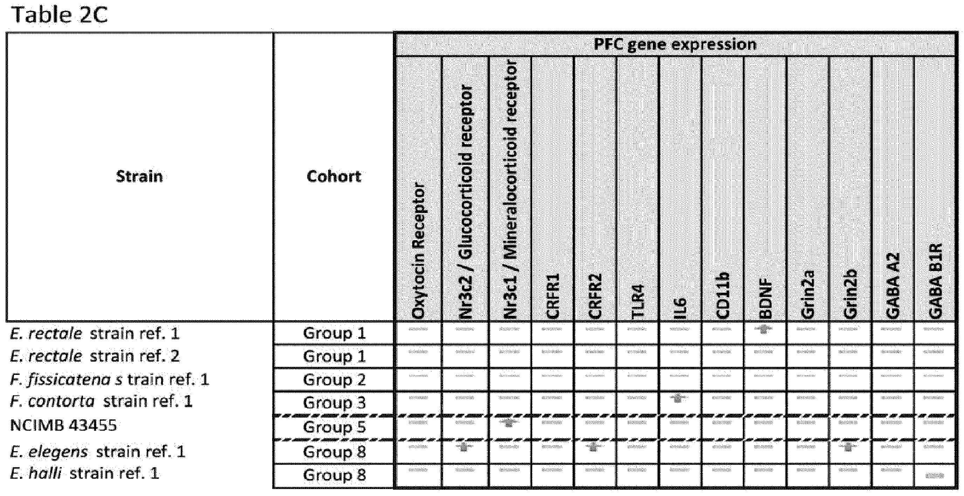

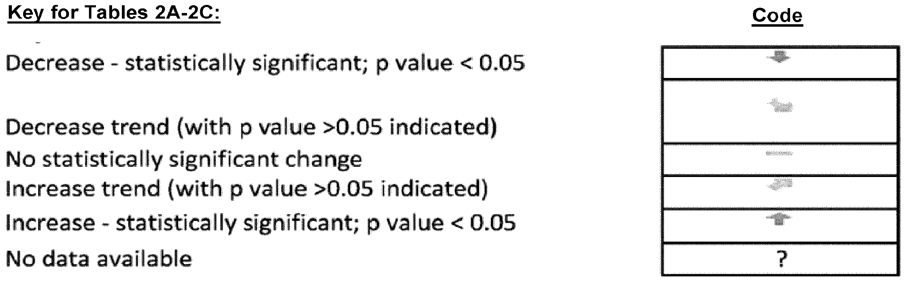

As shown in Figure 20, the expression levels in the amygdala of all of the markers tested were altered in MIA mice administered NCIMB 43457, relative to mice administered vehicle only. Similar, Figure 19 shows that the expression levels of all of the genes tested (apart from GABA B1R and glucocorticoid receptor) were altered in the amygdala in BtBR mice administered NCIMB 43457.

The results indicate that *Anaerostipes* strains may be useful in the treatment or prevention of disorders or conditions that may benefit from the modulation in the levels of expression of these proteins in the brain, e.g. CNS diseases and disorders.

### Example 15 - Effect of Eubacterium and Faecalicatena on gene expression in the brain

### Summary

This study investigated the effect of *Eubacterium* and *Faecalicatena* on expression of certain genes of interest in the brain. mRNA levels for markers for the oxytociergic system (oxytocin receptor and vasopressin receptor), endocrine system (mineralocorticoid receptor (Nr3cl); glucocorticoid receptor (Nr3c2); corticosterone releasing factor (CRF) and receptors (CRFR1 and CRFR2); Brain derived neurotrophic factor (BDNF)), immune system (IL-6, TNFα, TLR-4); and neurotransmitter systems (NMDA receptor 2A (Grin2A); NMDA receptor 2B (Grin2B); GABA A receptor subunit A2 (GABA A2); GABA B receptor subunit B1 (GABA B1R); serotonin 2C) were assessed in the amygdala, hippocampus and prefrontal cortex (PFC), which are key brain regions of the limbic system involved in emotional response.

### Materials and Methods

### Bacterial strains

Five strains of the *Eubacterium* genus (NCIMB 43455, *E. eligens* strain ref. 1, *E. hallii* strain ref. 1, *E*. *rectale strain ref.* 2 and *E. rectale* strain ref. 1) and two strains of the *Faecalicatena* genus (*F*. *fissicatena* strain ref. 1 and *F*. *contorta* strain ref. 1) were investigated in this study.

### Method

Male BALB/c mice received oral gavage (200 µL volume) of 1 X 10⁹ CFU of the relevant bacterial strain for 6 consecutive days. On day 7, the animals were euthanized. The brain was quickly excised, dissected and each brain region was snap-frozen on dry ice and stored at -80 °C for further analysis.

Total RNA was extracted using the mirVana^{™} miRNA Isolation kit (Ambion/Life technologies, Paisley, UK) and DNase treated (Turbo DNA-free, Ambion/Life technologies) according to the manufacturer's recommendations. RNA was quantified using NanoDrop^{™} spectrophotometer (Thermo Fisher Scientific Inc., Wilmington, Delaware, USA) according to the manufacturer's instructions. RNA quality was assessed using the Agilent Bioanalyzer (Agilent, Stockport, UK) according to the manufacturer's procedure and an RNA integrity number (RIN) was calculated. RNA with RIN value >7 was used for subsequent experiments. RNA was reverse transcribed to cDNA using the Applied Biosystems High Capacity cDNA kit (Applied Biosystems, Warrington, UK) according to manufacturer's instructions. Briefly, Multiscribe Reverse Transcriptase (50 U/µL) was added as part of RT master mix, incubated for 25°C for 10 min, 37°C for 2 h, 85°C for 5 min and stored at 4°C. Quantitative PCR was carried out using probes (6 carboxy fluorescein - FAM) designed by Applied Biosystems to mouse specific targeted genes, while using β-actin as an endogenous control. Amplification reactions contained 1 µl cDNA, 5 µl of the 2X PCR Master mix (Roche), and 900 nM of each primer and were brought to a total of 10 µl by the addition of RNase-free water. All reactions were performed in triplicate using 96-well plates on the LightCycler^{®}480 System. Thermal cycling conditions were as recommended by the manufacturer (Roche) for 55 cycles. To check for amplicon contamination, each run contained no template controls in triplicate for each probe used. Cycle threshold (Ct) values were recorded. Data was normalized using β-actin and transformed using the 2-ΔΔCT method and presented as a fold change vs. control group.

### Results

As shown in Tables 1A-C and 2A-C, from the genes tested, an increase in the expression of CRFR1, CRFR2 and Grin2b in the hippocampus; CRFR1 and Nr3c2 in the amygdala; and BDNF in the PFC was observed in mice which have been treated with *E*. *rectale* strain ref. 1, relative to treatment with vehicle only. Mice which have been treated with *E*. *rectale strain ref.* 2 demonstrated an increase in the expression of BDNF, Nr3c2, GABA A2 and GABA BR1 in the amygdala. Additionally, an increase in the expression of IL6 in the amygdala was observed in mice which have been treated with the *F*. *fissicatena* strain ref. 1 strain. Further, a decrease in oxytocin receptor in the hippocampus; an increase in the expression of Nr3c2, CD11b, GABA A2 and GABA BR1 in the amygdala; and IL6 in the PFC was observed in mice which have been treated with *F. conlorta* strain ref. 1. Mice which have been treated with NCIMB 43455 demonstrated an increase in the expression of Grin2a and GABA BR1 in the amygdala, and Nr3cl in the PFC. A decrease in vasopressin receptor in the hippocampus, an increase in the expression of Nr3cl and CRFR2 in the amygdala; and Nr3c2, CRFR2 and Grin2b in the PFC was observed in mice which have been treated with *E. eligens* strain ref. 1. Finally, an increase in the expression of vasopressin receptor, Nr3c1, CRFR2, CD11b , Grin2b and GABA A2 in the amygdala was observed in mice which have been treated with *F*. *contorta* strain ref. 1.

This indicates that *Eubacterium* and *Faecalicatena* may be useful in the treatment or prevention of disorders or conditions that may benefit from the modulation in the levels of expression of these proteins in the brain, e.g. CNS diseases and disorders.

### Example 16 - Effect of Eubacterium on behaviour in models of autism spectrum disorders

### Summary

This study investigated the effect of *Eubacterium* strain NCIMB 43455 on a number of behavioural readouts including stereotype behaviour (grooming and marble burying), anxiety-related behaviour (open field test and elevated plus maze test), social behaviour (3-chamber social interaction test), cognitive performance (novel obj ect recognition) and depression/acute stress (forced swim test) in both an environmental animal model (maternal immune activation (MIA) model) and a genetic animal model (BTBR mouse strain) of autism spectrum disorder.

### Materials and Methods

### BTBR mouse model

BTBR animals were bred in house with brother-sister mating. The male offspring from these animals were separated from their mothers at 3 weeks old and daily administration of the live biotherapeutic or control commenced at 8 weeks of age. Assays for behaviour were initiated when animals were 11 weeks old. A control age-matched C57/B16 group was included as a reference control group.

### MIA mouse model

Female C57/B16 mice (8 weeks old) and age matched males were purchased from Harlan UK. After 1-week habituation these animals were mated. At embryonic day 12.5, females received either an injection of the viral mimetic poly-IC to activate the maternal immune system, or a saline vehicle injection. The male offspring from these animals were separated from their mothers at 3 weeks old and daily administration of the live biotherapeutic or control commenced at 8 weeks of age. Assays for behaviour were initiated when animals were 11 weeks old.

### Experimental design for behaviour assays

As outlined above, dosing with live biotherapeutic commenced when the mice were 8 weeks old. The initial dosing took place for 3 weeks before the behavioural experiments. At week 11 (3 weeks later) the animals began undergoing behaviour testing. There were 10-12 animals per group. Both the MIA and BTBR were run over 2 cohorts each - each animal within each cohort underwent the exact same procedures in the exact same order for the same length of time. Animals were always randomised such that not all one group was run on one individual day.

The behavioural battery occurred in the following order: marble burying and grooming tests at week 4; the elevated plus maze and three chamber tests at week 5; the open field and novel object recognition tests at week 6; and the forced swim test at week 7.

### Stereotype behaviour - Marble burying test

Mice were individually placed in a novel plexiglass cage (35 × 28 × 18.5 cm, L × W × H), filled up with sawdust (5-10 cm) and 20 marbles on top of it (five rows or marbles regularly spaced 2 cm away from the walls and 2 cm apart). Thirty minutes later, the number of marbles buried for more than 2/3 of their surface was counted. A higher number of marbles buried represents a heightened state of stereotype behaviour or higher levels of anxiety (neophobia).

### Stereotype behaviour - Groominz test

Self-grooming was evaluated in a 6.5 cm diameter × 10 cm tall, clear glass beaker covered with a filter top. Experimental animals were brought to the test room to habituate up to 1 hour prior to the test. The test is approximately 20 minutes in duration. The cumulative time spent by the test animal grooming was recorded. Between tests the beakers were cleaned thoroughly for next use. A longer duration spent grooming suggests an increased stereotype behaviour; or higher levels of anxiety in response to a new environment.

### Anxiety - Elevated plus maze

The set up was made of a grey plastic cross-shaped maze 1 m elevated from the floor, comprising two open (fearful) and two closed (safe) arms (50 × 5 × 15 cm walls or 1 cm no wall). Experiments occurred under red light (~5 lux). Mice were individually placed into the center of the maze facing an open arm (to avoid direct entrance into a closed one) and were allowed 5-min free exploration. Experiments were videotaped using a ceiling camera for further parameters analysis using Ethovision software (3.1 version, Noldus, TrackSys, Nottingham, UK). The percentage of time spent, distance moved and the number of entries in each arm were measured, for anxiety behaviour and locomotor activity, respectively (entrance in an arm was defined as all four paws inside the arm). An increase in time or number of entries into an open arm is an index of reduced anxiety.

### Depression - Forced swim test

Mice were individually placed in a clear glass cylinder (24 × 21 cm diameter), containing 15-cm-depth water (25 ± 0.5 °C). Water was changed between successive assays with different animals. The test lasted 6 min and experiments were videotaped using a numeric tripod-fixed camera; data were further scored twice using the videos (Video Media Player software) and averaged by an experimenter blind to conditions. The latency to immobility was scored. The time of immobility (s) was measured for the last 4 min of the test, with immobility being defined as a total absence of movement except slight motions to maintain the head above the water. An increase in immobility means an increase in depressive-like behaviour as the animal has resigned itself to its situation (learned helplessness).

### Social interaction - Three-chambered test

The social testing apparatus was a rectangle, three-chambered box. Each chamber was 20 cm (L) × 40 cm (W) × 22 cm (H). Dividing walls were made with small circular openings (5 cm in diameter) allowing access into each chamber. Two identical wire cup-like cages, with a bottom diameter of 10 cm, 13 cm in height and bars spaced 1.2 cm, allowing nose contact between the bars, but prevented fighting, were placed inside each side chamber in bilaterally symmetric positions. The test has three phases of 10 min each: 1) habitation 2) mouse versus object 3) novel mouse versus familiar mouse. Experiments were videotaped using a ceiling camera for further parameters analysis using Ethovision software (3.1 version, Noldus, TrackSys, Nottingham, UK). For the first phase the test mouse was placed into the middle chamber and allowed to explore the entire box with empty small wire cages inside for a 10-min habituation session. After the habituation period, the test mouse is removed from the testing box for a short interval while an object is placed in one side chamber and an unfamiliar conspecific male mouse (no prior contact with the test subject) in the other side chamber, both enclosed in a wire cup-like cage. During phase two, the test mouse is placed in the middle chamber and allowed to explore the entire box for 10 min. The amount of time spent exploring the object or mouse in each chamber and the number of entries into each chamber were evaluated. The location of the unfamiliar mouse in the left vs right side chamber was systematically alternated between trials. An entry was defined as all four paws in one chamber. During the third phase an object was replaced with an unfamiliar mouse serving as a novel mouse and in the other chamber the mouse used in phase two was kept the same, now serving as familiar mouse. After every trial, all chambers and cup-like wire cages were cleaned with 10% ethanol, dried and ventilated for a few minutes to prevent olfactory cue bias and to ensure proper disinfection. Lack of innate side preference was confirmed during the initial 10 min of habituation to the entire arena. Control animals are naturally interested in a conspecific mouse more than an inanimate object (sociability). In a similar vein, control animals spend more time interacting with a novel unfamiliar mouse then one they have already had interactions with. Some animal models, as used here, have deficits in this social paradigm.

### Cognitive performance - Novel object recognition

Mice were placed in the middle of a grey plastic rectangular box (40 × 32 × 23 cm, L × W × H) under a dim light (60 lux) at the level of the arena, for 10 min. 24 h later, mice were placed in the box with two identical objects for a total time of 10 min (acquisition phase). After 24 h, one of the two identical objects were substituted with a novel object and mice were placed in the middle of the box at the mid-point of the wall opposite the sample objects for a total time of 10 min (retention phase). Animals were acclimatized to the testing room for 30 min prior each experiment. Box and objects were cleaned with alcohol 10% to avoid any cue smell between each trial. Experiments were videotaped using a ceiling camera for further parameter analysis. Directed contacts with the objects, include any contact with mouth, nose or paw or minimal defined distance were counted as an interaction. Control animals will discriminate between an object they have had time to explore and a new object.

### Administration of biotherapeutics

Animals received daily oral gavage of PBS or the live biotherapeutic bacterial strain prepared to 1 X 10⁹ CFU/mL in PBS. Daily administration of live biotherapeutics commenced at 8 weeks of age and dosing continued daily throughout the behavioural paradigm

### Results

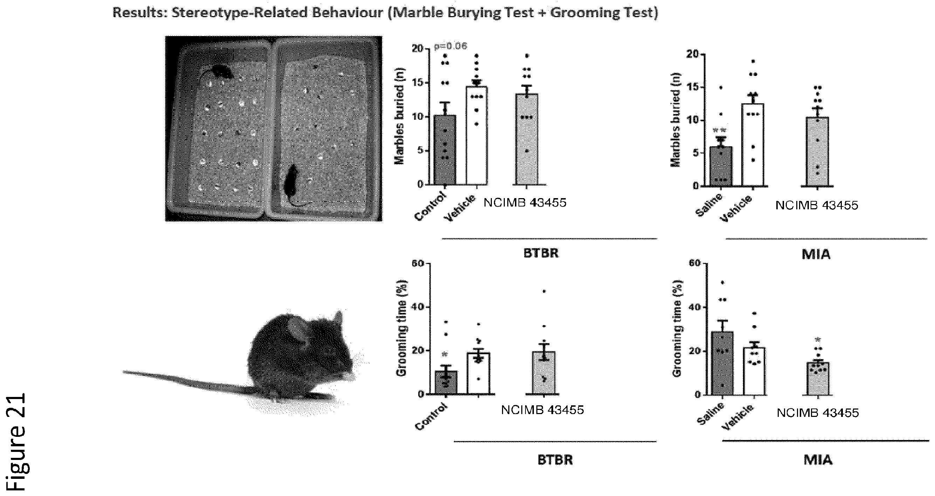

Stereotype behaviour as shown in Figure 21, MIA mice treated with NCIMB 43455 exhibited decreased stereotype-related behaviour, as they had a significantly reduced duration spent grooming. Additionally, both BTBR and MIA mice treated with NCIMB 43455 show reduced stereotype-related behaviour in the marble burying test, as evidenced by the reduction in the number of marbles buried.

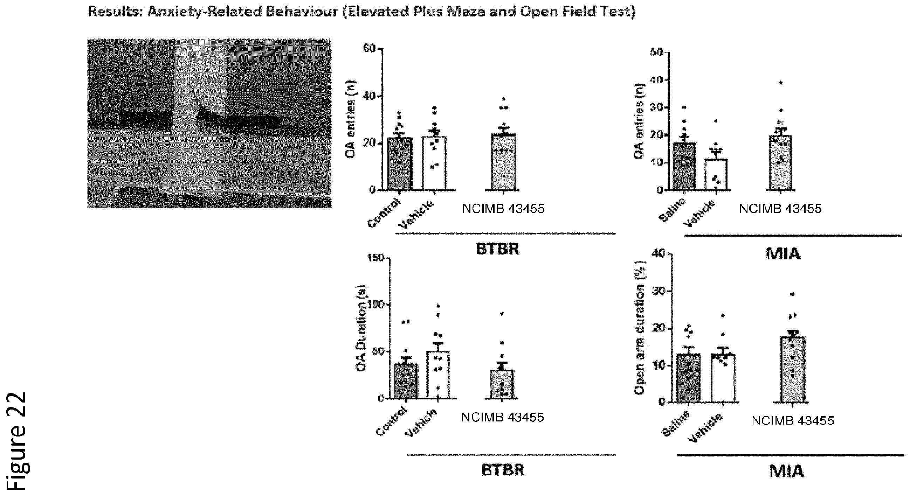

Anxiety-like behaviour, as assessed by the elevated plus maze, was also found to be ameliorated in MIA mice treated with NCIMB 43455. These mice had significantly greater number of entries into the open arm of the maze (Figure 22), and spent a longer duration in the open arms of the maze relative to mice administered vehicle. BTBR mice treated with NCIMB 43455 spent very slightly less time in the open arms relative to control mice.

Cognitive performance, which was assessed by the novel object recognition test, was also found to be increased in both BTBR and MIA mice treated with NCIMB 43455, since these mice spend more time interacting with a novel object over a familiar object.

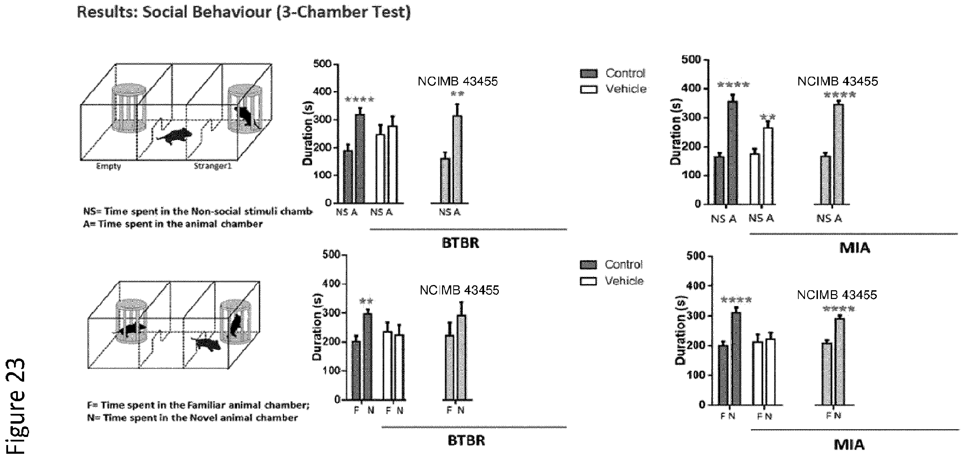

Figure 23 shows that treatment with the bacterial strain also ameliorated deficits in social behaviour, as both MIA and BTBR mice spent significantly more time in the animal chamber than the non-social stimuli chamber compared to the control group. Further, MIA mice treated with NCIMB 43455 also spent a significantly higher duration in a chamber containing a novel conspecific than one housing a familiar conspecific mouse.

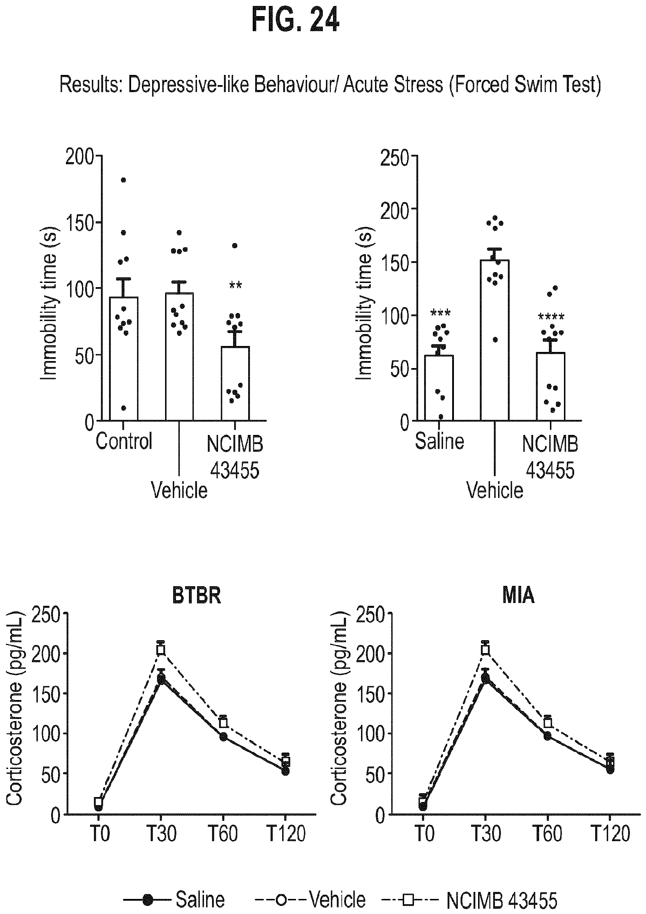

Finally, treatment with NCIMB 43455 also displayed antidepressive-like effects in both a genetic and environmental animal model of autism, since both BTBR and MIA mice spent significantly less time being immobile (Figure 24) when treated with this strain. Together, the results indicate that *Eubacterium* may be useful in therapy, e.g. in the treatment or prevention CNS disorders, such as autism, anxiety and depression.

### Example 17 - Effect of Eubacterium on gut function in animal models of autism

### Summary

This study investigates the effect of *Eubacterium* strain NCIMB 43455 on assays of gut motility and gut permeability in both an environmental animal model (maternal immune activation model) and a genetic animal model (BTBR mouse strain) of autism spectrum disorder.

### Materials and methods

### Mouse models

The BTBR and MIA models were generated and maintained as described in Example 2.

### Administration of biotherapeutics

Animals received daily oral gavage of PBS or the live biotherapeutic bacterial strain prepared to 1 X 10⁹ CFU/mL in PBS. Daily administration of live biotherapeutics commenced at 8 weeks of age.

### Gut function - intestinal motility

Assays for intestinal motility were carried out when mice were 13 weeks old (5 weeks after daily administration of live biotherapeutics was initiated). Mice were single housed for 3h prior to the assay to allow habituation to a new cage. Carmin red dye (100-200 ul of 6% Carmin red in 0.5% methylcellulose per mouse) was given orally by gavage. Each cage was visually inspected every 10 min. The time of the first coloured bolus (red) was recorded. Following the appearance of the first coloured bolus mice were returned to normal housing conditions.

### Gut function - intestinal permeability

Assays for intestinal permeability were carried out on tissue from mice culled at 15 weeks of age (7 weeks after daily administration of live biotherapeutics was initiated). Mice were euthanized by cervical dislocation, and the distal ileum and colon were removed, placed in chilled Krebs solution opened along the mesenteric line and carefully rinsed. Preparations were then placed in Ussing chambers (Harvard Apparatus, Kent, UK, exposed area of 0.12 cm²) as described previously (Hyland and Cox, 2005) with oxygenated (95% O₂, 5% CO₂) Krebs buffer maintained at 37°C. 4 kDa FITC-dextran was added to the mucosal chamber at a final concentration of 2.5 mg/mL; 200 µL samples were collected from the serosal chamber every 30 min for the following 3 h and fluorescence in those samples measured.

### Results

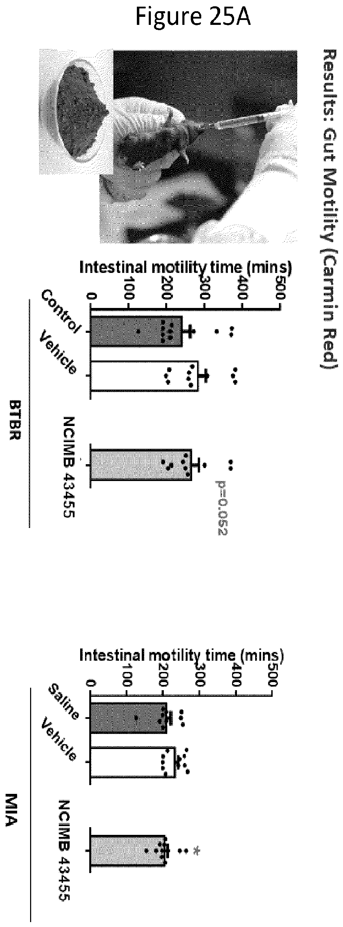

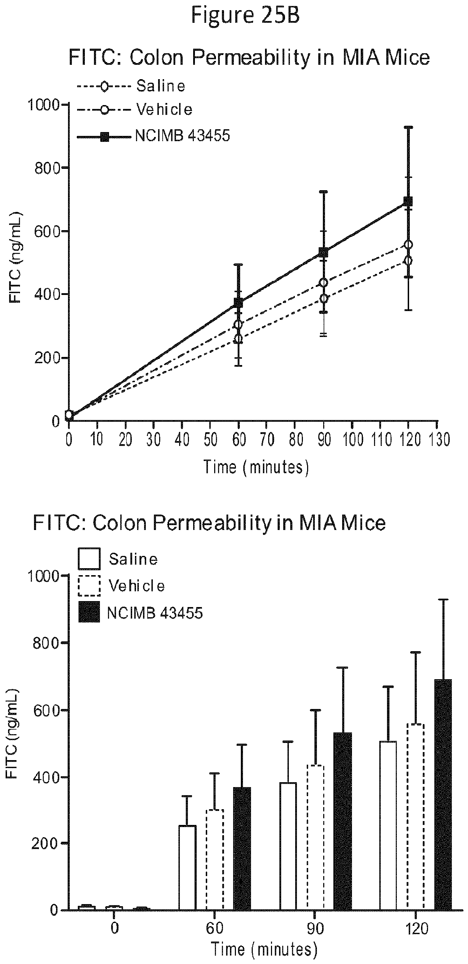

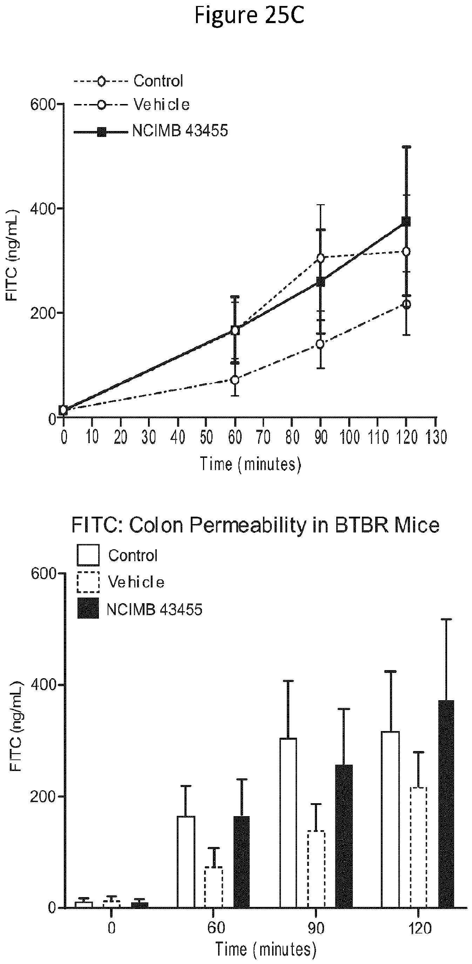

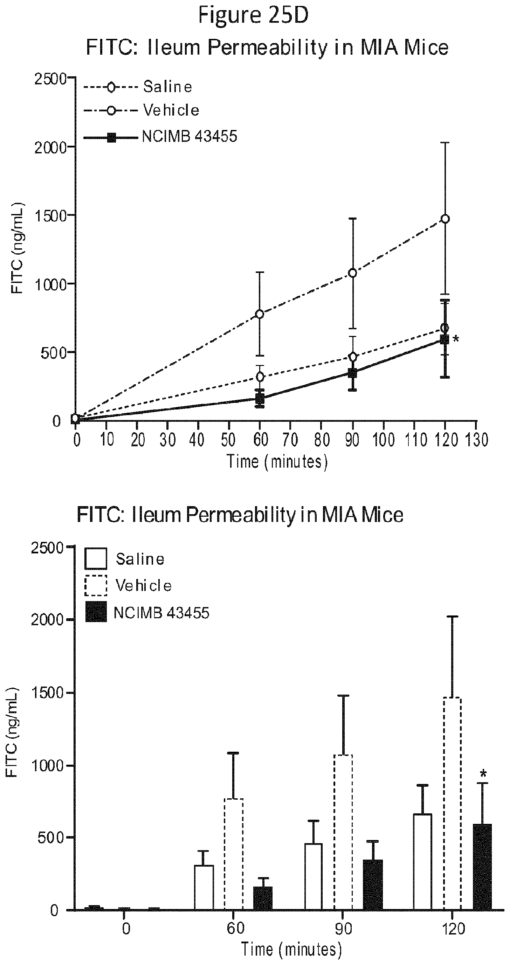

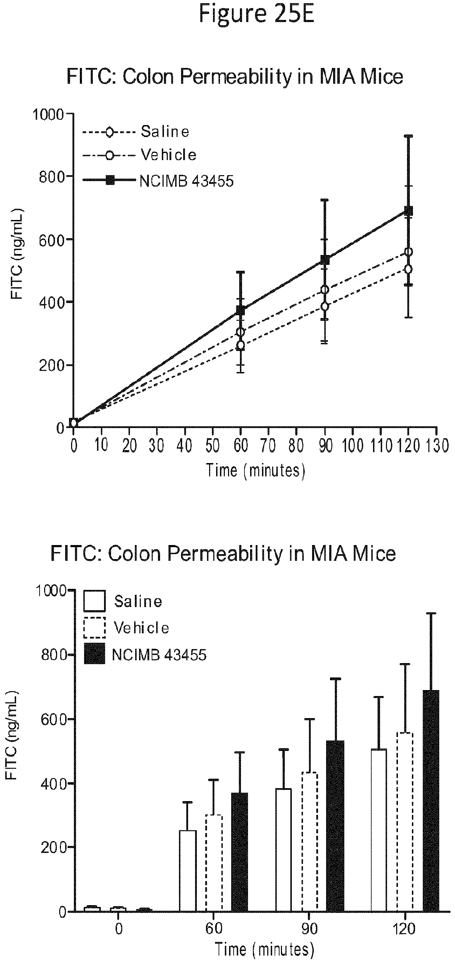

As shown in Fig. 25A, MIA mice treated with NCIMB 43455 have significantly reduced intestinal motility time, which is indicative of increased peristaltic motility in the whole intestine and improved gut function overall. Additionally, NCIMB 43455 treatment also restored deficits in ileal permeability in MIA mice. These results indicate that treatment with *Eubacterium* may be useful in the treatment of impaired gut function associated with autism spectrum disorders, including reduced intestinal motility and/or increased intestinal permeability.

### Example 18 - Effect of Eubacterium on brainstem monoamine levels in models of autism spectrum disorders

### Summary

This study investigated the effect of *Eubacterium* strain NCIMB 43455 on brainstem monoamine levels in BTBR and MIA models of autism spectrum disorder. The brainstem encompasses a number of cell bodies for all the major neurotransmitter systems of the brain including serotonin and noradrenaline. An increase in levels suggest an increase in neurotransmitter release in response to live biotherapeutic administration.

### Materials and Methods

### Mouse models

The BTBR and MIA models were generated and maintained as described in Example 2.

### Method

Animals received daily oral gavage of PBS or the live biotherapeutics prepared to 1 X 10⁹ CFU/mL in PBS, commencing at 8 weeks of age. After a further 7 weeks of dosing with biotherapeutics, the animals were euthanized and neurotransmitter concentration was analysed by HPLC on samples from the brainstem. Briefly, brainstem tissue was sonicated in 500 µl of chilled mobile phase spiked with 4 ng/40 µl of N-Methyl 5-HT (Sigma Chemical Co., UK) as internal standard. The mobile phase contained 0.1 M citric acid, 5.6 mM octane-1-sulphonic acid (Sigma), 0.1 M sodium dihydrogen phosphate, 0.01 mMEDTA (Alkem/Reagecon, Cork) and 9% (v/v) methanol (Alkem/Reagecon), and was adjusted to pH 2.8 using 4 N sodium hydroxide (Alkem/Reagecon). Homogenates were then centrifuged for 15 min at 22,000 g at 4 °C and 40 µl of the supernatant injected onto the HPLC system which consisted of a SCL 10-Avp system controller, LECD 6A electrochemical detector (Shimadzu), aLC-10AS pump, a CTO-10A oven, a SIL-10A autoinjector (with sample cooler maintained at 40 C) and an online Gastorr Degasser (ISS, UK). A reverse-phase column (Kinetex 2.6 u C18 100 × 4.6 mm, Phenomenex) maintained at 30 °C was employed in the separation (Flow rate 0.9 ml/min). The glassy carbon working electrode combined with an Ag/AgCl reference electrode (Shimdazu) operated a +0.8 V and the chromatograms generated were analyzed using Class-VP 5 software (Shimadzu). The neurotransmitters were identified by their characteristic retention times as determined by standard injections, which run at regular intervals during the sample analysis. The ratios of peak heights of analyte versus internal standard were measured and compared with standard injection. Results were expressed as ng of neurotransmitter per g fresh weight of tissue.

### Results

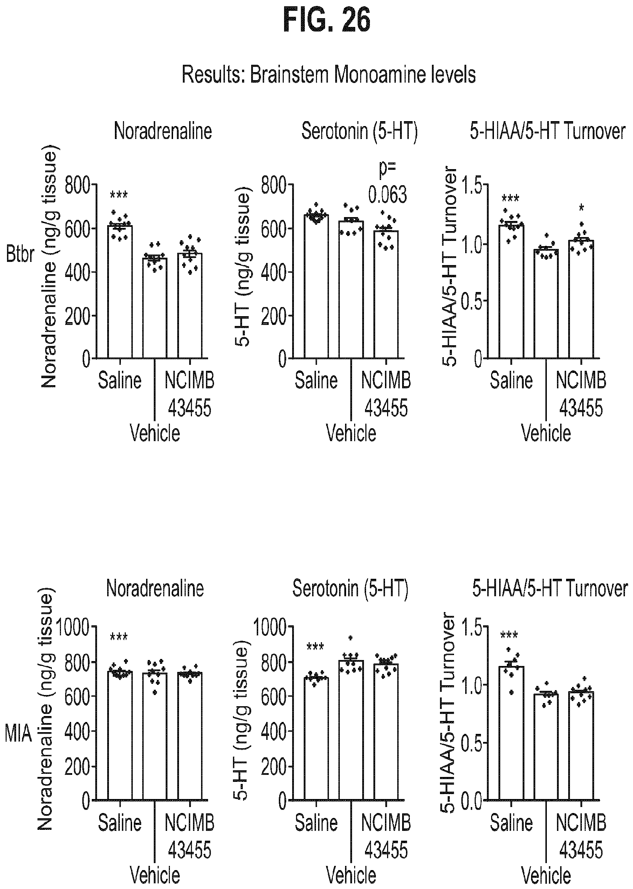

As shown in Figure 26, mice administered NCIMB 43455 have altered levels of certain monoamines in the brainstem. BTBR mice had significantly reduced serotonin levels compared with animals administered vehicle, and there was a reduction in serotonin levels in MIA mice as well. Additionally, the ratio of 5-HIAA/5-HT is significantly increased in MIA mice treated with NCIMB 43455, indicative of increased serotonin turnover and serotonergic activity [111]. This suggests that *Eubacterium* is useful in the treatment or prevention of disorders associated with dysregulation of these neurotransmitter systems, e.g. CNS disorders such as autism, sudden infant death syndrome (SIDS) and major depressive disorder (MDD) [111], [112].

### Example 19 - Effect of Eubacterium on gene expression in the amygdala in models of autism spectrum disorders

### Summary

This study investigated the effect of *Eubacterium* strain NCIMB 43455 on the expression of certain genes of interest in the brain of BTBR and MIA mice. mRNA levels for markers for the oxytocinergic system (oxytocin receptor and vasopressin receptor), endocrine system (mineralocorticoid (Nr3cl); glucocorticoid receptor (Nr3c2); corticosterone releasing factor (CRF) and receptors (CRF1R and CRF2R); Brain derived neurotrophic factor (BDNF)), immune system (Il-6, TNF-α, TLR-4); and neurotransmitter systems (NMDA receptor 2A (Grin2A); NMDA receptor 2B (Grin2B); GABAA receptor subunit A2; GABAB receptor subunit B1; serotonin 2C) were assessed in the amygdala.

### Materials and Methods

### Mouse models

The BTBR and MIA models were generated and maintained as described in Example 2.

### Method

Animals received daily oral gavage of PBS or the live biotherapeutics prepared to 1 X 10⁹ CFU/mL in PBS commencing at 8 weeks of age. After a further 7 weeks of dosing with biotherapeutics, the animals were euthanized. The brain was quickly excised, dissected and each brain region was snap-frozen on dry ice and stored at -80 °C for further analysis.

mRNA expression was quantified as described in Example 1.

### Results

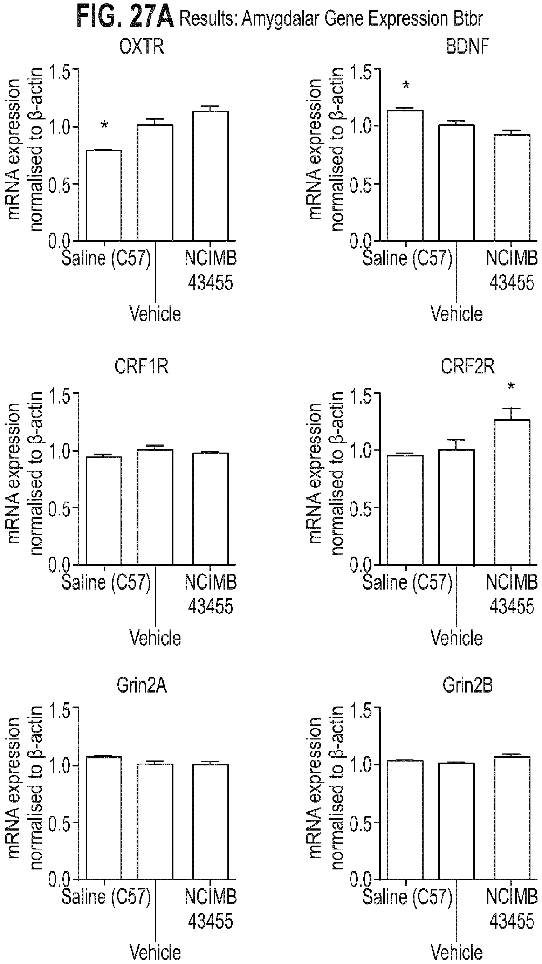

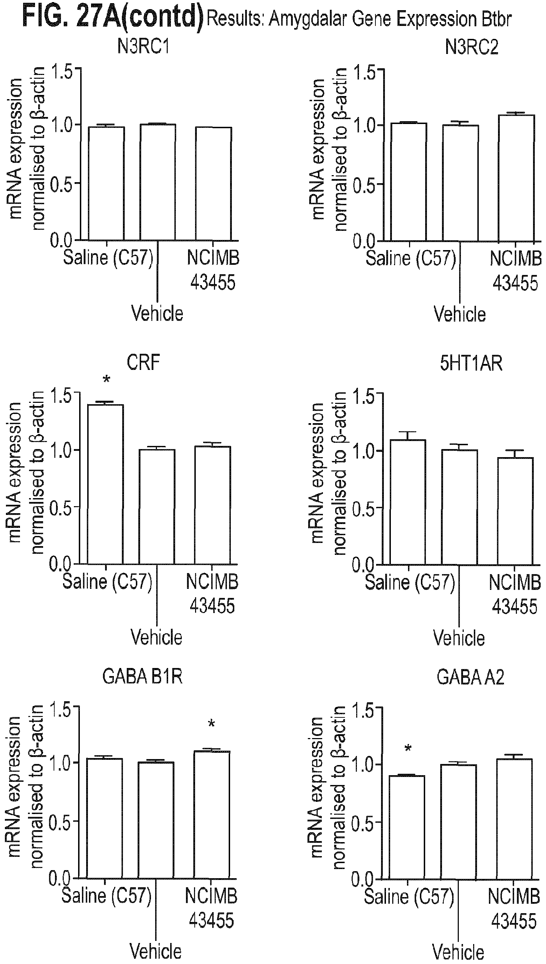

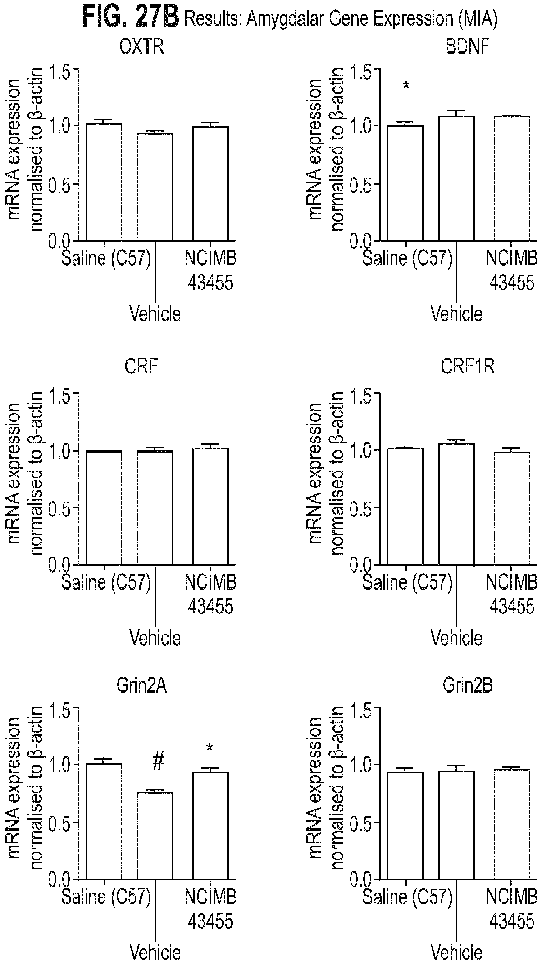

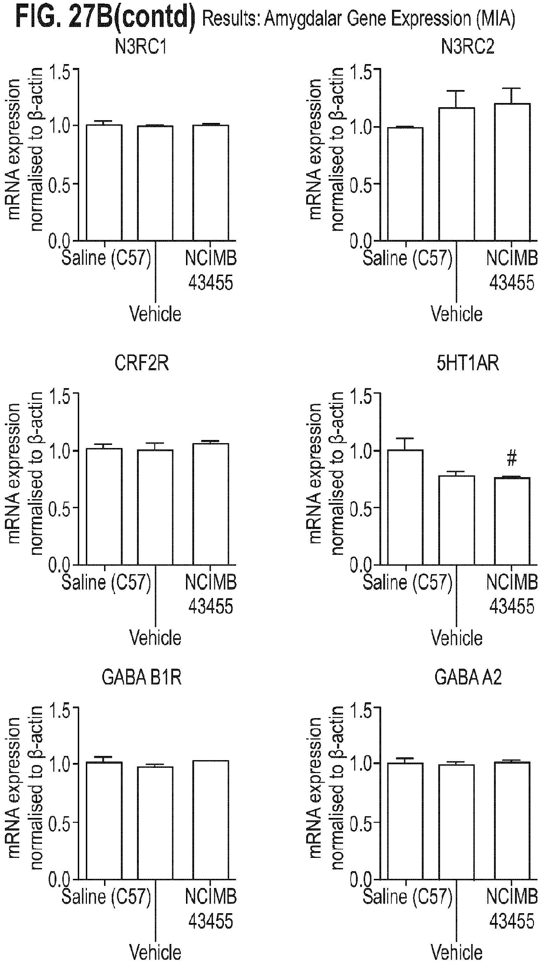

As shown in Figure 27A and B, BTBR mice administered NCIMB 43455 exhibited elevated levels of CRF2R in the amygdala. Treatment with the strains was also associated with a significant increase in the levels of expression Grin2a and increased 5HT1AR expression in the amygdala of MIA mice, relative to mice administered vehicle. The results indicate that *Eubacterium* is useful in the treatment or prevention of disorders or conditions that may benefit from the modulation in the levels of expression of these proteins in the brain, e.g. CNS diseases and disorders.

### Example 20 - Effect of Anaerostipes on H3/H4 acetylation in colorectal cancer cells

### Summary

This study investigated the effect of four bacterial strains of the genus *Anaerostipes* on the acetylation of histone proteins H3 and H4 in two colorectal cancer cell lines (HCT116 and HT29) using indirect immunofluorescence.

### Materials and Methods

### Bacterial strains

The following bacterial strains were tested: *Anaerostipes hadrus* NCIMB 43526, *Anaerostipes hadrus* NCIMB 43457, *Anaerostipes caccae* strain ref. 1 and *Anaerostipes hadrus* strain ref. 1.

### Anaerostipes supernatant preparation

The four bacterial strains of *Anaerostipes* listed above were each cultured separately as follows: 100 µL of a Research Cell Bank vial was used to inoculate 10 mL of YCFA+ broth. The culture was incubated overnight in an anaerobic workstation at 37°C. Each overnight culture was used to inoculate five Hungate tubes containing 10 mL of fresh growth medium with a 10% subculture. Culture tubes were incubated until they reached early stationary phase, following which cell-free supernatants (CFS) were collected as follows. Individual culture tubes were combined and the bacterial density (O.D. 600 nm) was recorded. Cell-free supernatants of the two strains were obtained by centrifugation (5000x g, 15 min) and filtration through a 0.45 µm followed by a 0.22 µm filter.

### Treatment and imaging

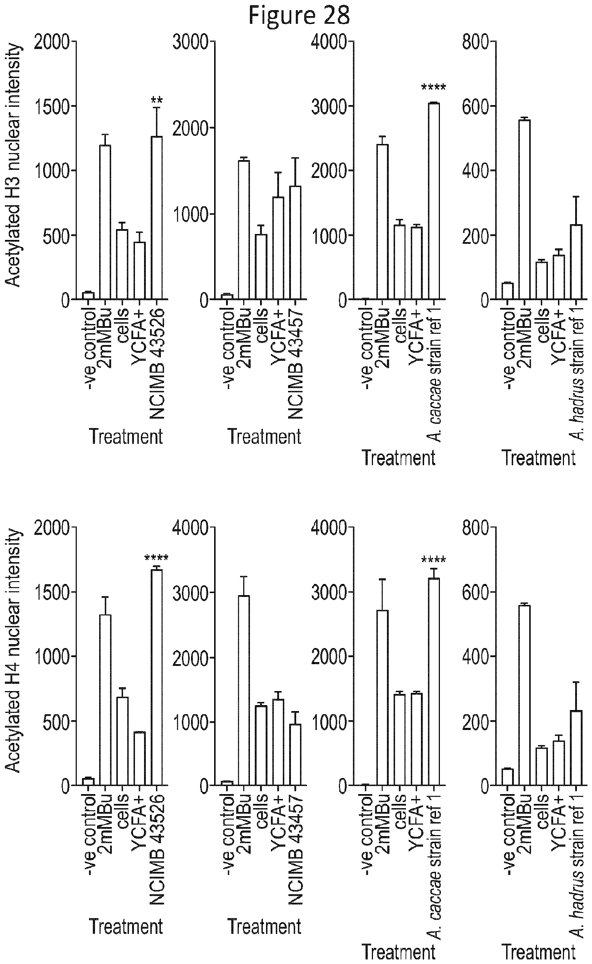

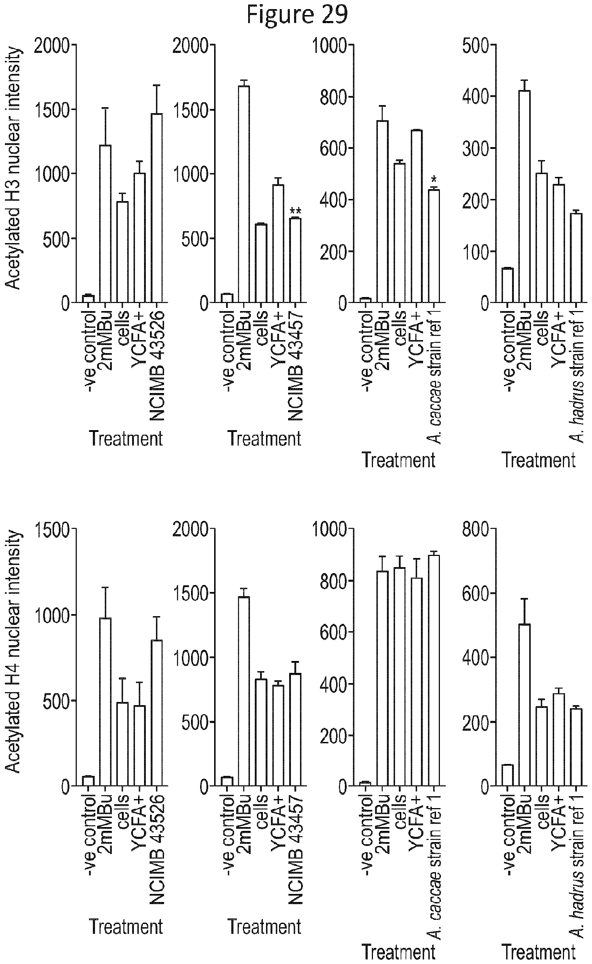

Colorectal cancer cell lines HCT116 and HT29 were seeded in black 96 well plates at a density of 10,000 cells/well overnight. The cells were treated for 24 h with 10% bacterial supernatant from the *indicated Anaerostipes* bacterial strain; or with YCFA+ broth only, 2 mM butyrate or left untreated, as controls. Afterwards, the cells were fixed with 4% paraformaldehyde in PBS (pH 7.3) for 20 min at room temperature (RT). Fixed cells were washed with PBS, and permeabilized with 0.5% Triton X-100 in PBS for 10 min. After washing with PBS, the plates were incubated with blocking buffer (4% BSA/PBS) for 1 h at RT before adding the primary antibody (anti-AcH3 antibody (06-599, Millipore) or anti-AcH4 antibody (06-598, Millipore) both at 1:500, diluted in 1% BSA/PBS for 1 h at 4°C), or 1% BSA/PBS as a negative control, for 12 h at 4°C. They were then washed twice with PBS, followed by incubation with Alexa Flour 488 conjugated anti-rabbit (Molecular Probes Inc) and Alexa Flour 594 (Molecular Probes Inc) conjugated for 1 h at RT. After washing 3x with PBS, the plates were labelled with DAPI and washed with PBS 3x. Plates were viewed using ImageXpress Pico microscope (Molecular Devices) equipped with a 20x objective and filter sets suitable for detection of the fluorochromes used. Stored images were saved as TIFF files. Raw analysis data generated by the PICO analysis module were plotted and analysed using GraphPad Prism 7 software. Representative images were selected to illustrate the differences in acetylation of the histone protein examined. The results for HCT116 cells are shown in Figure 28 and for HT29 cells in Figure 29.

### Results and discussion

As shown in Figures 28 and 29, increases in the levels of acetylated histone proteins H3 and H4 were seen in HCT116 and HT29 cell lines after treatment with supernatants of *Anaerostipes* bacterial strains. In particular, supernatants of *Anaerostipes hadrus* NCIMB 43526, caused increased H3 and H4 acetylation in both colorectal cancer cell lines. Taken together with the direct HDAC inhibition data presented in Example 5 for *Anaerostipes hadrus* strain DSM 3319, this provides further support for the ability of bacterial strains of the genus *Anaerostipes* to inhibit HDAC activity. Therefore, the results indicate that bacterial strains of the genus *Anaerostipes* are useful in the treatment or prevention of disorders or conditions that are mediated by HDAC activity, such as cancer, as explained above (see Example 5).

### Sequences

**SEQ ID NO:**1 - *Anaerostipes hadrus* strain DSM 3319 16S ribosomal RNA, partial sequence 
1 tttgatcctg gctcaggatg aacgctggcg gcgtgcttaa cacatgcaag tcgaacgaaa

61 caccttattt gattttcttc ggaactgaag atttggtgat tgagtggcgg acgggtgagt

121 aacgcgtggg taacctaccc tgtacagggg gataacagtc agaaatgact gctaataccg

181 cataagacca cagcaccgca tggtgcaggg gtaaaaactc cggtggtaca ggatggaccc

241 gcgtctgatt agctggttgg tgaggtaacg gctcaccaag gcgacgatca gtagccggct
 
301 tgagagagtg aacggccaca ttgggactga gacacggccc aaactcctac gggaggcagc
361 agtggggaat attgcacaat gggggaaacc ctgatgcagc gacgccgcgt gagtgaagaa
421 gtatctcggt atgtaaagct ctatcagcag ggaagaaaat gacggtacct gactaagaag
481 ccccggctaa ctacgtgcca gcagccgcgg taatacgtag ggggcaagcg ttatccggaa
541 ttactgggtg taaagggtgc gtaggtggta tggcaagtca gaagtgaaaa cccagggctt
601 aactctggga ctgcttttga aactgtcaga ctggagtgca ggagaggtaa gcggaattcc
661 tagtgtagcg gtgaaatgcg tagatattag gaggaacatc agtggcgaag gcggcttact
721 ggactgaaac tgacactgag gcacgaaagc gtggggagca aacaggatta gataccctgg
781 tagtccacgc cgtaaacgat gaatactagg tgtcggggcc gtagaggctt cggtgccgca
841 gccaacgcag taagtattcc acctggggag tacgttcgca agaatgaaac tcaaaggaat
901 tgacggggac ccgcacaagc ggtggagcat gtggtttaat tcgaagcaac gcgaagaacc
961 ttacctggtc ttgacatcct tctgaccggt ccttaaccgg acctttcctt cgggacagga
1021 gagacaggtg gtgcatggtt gtcgtcagct cgtgtcgtga gatgttgggt taagtcccgc
1081 aacgagcgca acccttatct ttagtagcca gcatataagg tgggcactct agagagactg
1141 ccagggataa cctggaggaa ggtggggacg acgtcaaatc atcatgcccc ttatgaccag
1201 ggctacacac gtgctacaat ggcgtaaaca gagggaagca gcctcgtgag agtgagcaaa
1261 tcccaaaaat aacgtctcag ttcggattgt agtctgcaac tcgactacat gaagctggaa
1321 tcgctagtaa tcgcgaatca gaatgtcgcg gtgaatacgt tcccgggcct tgtacacacc
1381 gcccgtcaca ccatgggagt cagtaacgcc cgaagtcagt gacccaaccg taaggaggga
1441 gctgccgaag gcgggaccga taactggggt gaagtcgtaa caaggtagcc gtatcggaag
1501 gtgcggctgg atcacctcct t
**SEQ ID NO:2** - *Anaerostipes hadrus* strain 5/1/63FAA 16S ribosomal RNA gene 
1 gatgaacgct ggcggcgtgc ttaacacatg caagtcgaac gaaacacctt atttgatttt
61 cttcggaact gaagatttgg tgattgagtg gcggacgggt gagtaacgcg tgggtaacct
121 gccctgtaca gggggataac agtcagaaat gactgctaat accgcataag accacagcac
181 cgcatggtgc aggggtaaaa actccggtgg tacaggatgg acccgcgtct gattagctgg
241 ttggtgaggt aacggctcac caaggcgacg atcagtagcc ggcttgagag agtgaacggc
301 cacattggga ctgagacacg gcccaaactc ctacgggagg cagcagtggg gaatattgca
361 caatggggga aaccctgatg cagcgacgcc gcgtgagtga agaagtatct cggtatgtaa
421 agctctatca gcagggaaga aaatgacggt acctgactaa gaagccccgg ctaactacgt
481 gccagcagcc gcggtaatac gtagggggca agcgttatcc ggaattactg ggtgtaaagg
541 gtgcgtaggt ggtatggcaa gtcagaagtg aaaacccagg gcttaactct gggactgctt
601 ttgaaactgt cagactggag tgcaggagag gtaagcggaa ttcctagtgt agcggtgaaa
661 tgcgtagata ttaggaggaa catcagtggc gaaggcggct tactggactg aaactgacac
721 tgaggcacga aagcgtgggg agcaaacagg attagatacc ctggtagtcc acgccgtaaa
781 cgatgaatac taggtgtcgg ggccgtagag gcttcggtgc cgcagccaac gcagtaagta
841 ttccacctgg ggagtacgtt cgcaagaatg aaactcaaag gaattgacgg ggacccgcac
901 aagcggtgga gcatgtggtt taattcgaag caacgcgaag aaccttacct ggtcttgaca
961 tccttctgac cggtccttaa ccggaccttt ccttcgggac aggagagaca ggtggtgcat
1021 ggttgtcgtc agctcgtgtc gtgagatgtt gggttaagtc ccgcaacgag cgcaacccct
1081 atctttagta gccagcatat aaggtgggca ctctagagag actgccaggg ataacctgga
1141 ggaaggtggg gacgacgtca aatcatcatg ccccttatga ccagggctac acacgtgcta
 
1201 caatggcgta aacagaggga agcagcctcg tgagagtgag caaatcccaa aaataacgtc
1261 tcagttcgga ttgtagtctg caactcgact acatgaagct ggaatcgcta gtaatcgcga
1321 atcagaatgt cgcggtgaat acgttcccgg gtcttgtaca caccgcccgt cacaccatgg
1381 gagtcagtaa cgcccgaagt cagtgaccca accgtaagga gggagctgcc gaag
SEQ ID NO:3 *- Anaerostipes butyraticus* strain 35-7 16S ribosomal RNA gene, partial sequence 
1 ggctcaggat gaacgctggc ggcgtgctta acacatgcaa gtcgaacgaa gcttgatccc
61 ggatcccttc ggggtgacgg gtgatatgac tgagtggcgg acgggtgagt aacgcgtggg
121 caacctgcct catacagggg gataacagtt agaaatgact gctaagaccg cataagacca
181 cagcaccgca tggtggagtg gtaaaagctc cggtggtatg agatgggccc gcgtctgatt
241 agctggttgg tgaggtaacg gctctaccaa ggcaacgatc agtagccggc ttgagagagt
301 gaacggccac attgggactg agacacggcc caaactccta cgggaggcag cagtggggaa
361 tattgcacaa tggggaaacc cctgatgcag cgacgccgcg tgagtgaaga agtatttcgg
421 tatgtaaagc tctatcagca gggaagaaaa tggacggtac ctgactaaga agccccggct
481 aactacgtgc cagcagccgc ggtaatacgt agggggcaag cgttatccgg aattactggg
541 tgtaaagggt gcgtaggtgg cagggcaagt cagaagtgaa aacccggggc tcaactccgg
601 gactgctttt gaaactgtcc agctggagtg caggagaggt aagcggaatt cctagtgtag
661 cggtgaaatg cgtagatatt aggaggaaca tcagtggcga aggcggctta ctggactgaa
721 actgacactg aggcacgaaa gcgtggggag caaacaggat tagataccct ggtagtccac
781 gccgtaaacg atgaatacta ggtgtcgggg ccgtagaggc ttcggtgccg cagcaaacgc
841 attaagtatt ccacctgggg agtacgttcg caagaatgaa actcaaagga attgacgggg
901 acccgcacaa gcggtggagc atgtggttta attcgcaagc aacgcgaaga accttacctg
961 gtcttgacat ccccttgacc gctccttaat cggagttttc cttcgggaca agggtgacag
1021 gtggtgcatg gttgtcgtca gctcgtgtcg tgagatgttg ggttaagtcc cgcaacgagc
1081 gcaaccccta tctttagtag ccagcacggg aaaggtgggc actctagaga gactgccagg
1141 gagaacctgg aggaaggtgg gggacgacgt caaatcatca tgccccttat gaccagggct
1201 acacacgtgc tacaatggcg taaacaaagg gaagcgaccc tgtgaaggca agcaaatccc
1261 aaaaataacg tcacagttcg gattgtagtc tgcaactcga ctacatgaag ctggaatcgc
1321 tagtaatcgc gaatcagaat gtcgcggtga atacgttccc gggtcttgta cacaccgccc
1381 g
SEQ ID NO:4 *- Anaerostipes rhamnosivorans* strain 1y-2 16S ribosomal RNA gene, partial sequence 
1 gcgcttaata catgtcaagt cgaacgaagc atttaggatt gaagttttcg gatggatttc
61 ctatatgact gagtggcgga cgggtgagta acgcgtgggg aacctgccct atacaggggg
121 ataacagctg gaaacggctg ctaataccgc ataagcgcac agaatcgcat gattcagtgt
181 gaaaagccct ggcagtatag gatggtcccg cgtctgatta gctggttggt gaggtaacgg
241 ctcaccaagg cgacgatcag tagccggctt gagagagtga acggccacat tgggactgag
301 acacggccca aactcctacg ggaggcagca gtggggaata ttgcacaatg ggggtaaacc
361 ctgatgcagc gacgccgcgt gagtgaagaa gtatttcggt atgtaaagct ctatcagcag
421 ggaagaaaac agacggtacc tgactaagaa gccccggcta actacgtgcc agcagccgcg
481 gtaatacgta gggggcaagc gttatccgga attactgggt gtaaagggtg cgtaggtggc
541 atggtaagtc agaagtgaaa gcccggggct taaccccggg actgcttttg aaactgtcat
601 gctggagtgc aggagaggta agcggaattc ctagtgtagc ggtgaaatgc gtagatatta
661 ggaggaacac cagtggcgaa ggcggcttac tggactgtca ctgacactga tgcacgaaag
 
721 cgtggggagc aaacaggatt agataccctg gtagtccacg ccgtaaacga tgaatactag
781 gtgtcggggc cgtagaggct tcggtgccgc agcaaacgca gtaagtattc cacctgggga
841 gtacgttcgc aagaatgaaa ctcaaaggaa ttgacgggga cccgcacaag cggtggagca
901 tgtggtttaa ttcgaagcaa cgcgaagaac cttacctggt cttgacatcc caatgaccga
961 accttaaccg gttttttctt tcgagacatt ggagacaggt ggtgcatggt tgtcgtcagc
1021 tcgtgtcgtg agatgttggg ttaagtcccg caacgagcgc aacccctatc tttagtagcc
1081 agcatttaag gtgggcactc tagagagact gccagggata acctggagga aggtggggac
1141 gacgtcaaat catcatgccc cttatggcca gggctacaca cgtgctacaa tggcgtaaac
1201 aaagggaagc gaagtcgtga ggcgaagcaa atcccagaaa taacgtctca gttcggattg
1261 tagtctgcaa ctcgactaca tgaagctgga atcgctagta atcgtgaatc agaatgtcac
1321 ggtgaatacg ttcccgggtc ttgtacacac cgcccgtcac accatgggag tcagtaacgc
1381 ccgaagtcag tgacccaacc gcaaggaggg agctgccgaa ggtgggaccg ataactgggg
1441 tgaagtcgta acaagg
SEQ ID NO:5 *- Anaerostipes caccae* strain L1-92 16S ribosomal RNA gene 
1 gcgcttaata catgtcaagt cgaacgaagc atttaggatt gaagttttcg gatggatttc
61 ctatatgact gagtggcgga cgggtgagta acgcgtgggg aacctgccct atacaggggg
121 ataacagctg gaaacggctg ctaataccgc ataagcgcac agaatcgcat gattcagtgt
181 gaaaagccct ggcagtatag gatggtcccg cgtctgatta gctggttggt gaggtaacgg
241 ctcaccaagg cgacgatcag tagccggctt gagagagtga acggccacat tgggactgag
301 acacggccca aactcctacg ggaggcagca gtggggaata ttgcacaatg ggggtaaacc
361 ctgatgcagc gacgccgcgt gagtgaagaa gtatttcggt atgtaaagct ctatcagcag
421 ggaagaaaac agacggtacc tgactaagaa gccccggcta actacgtgcc agcagccgcg
481 gtaatacgta gggggcaagc gttatccgga attactgggt gtaaagggtg cgtaggtggc
541 atggtaagtc agaagtgaaa gcccggggct taaccccggg actgcttttg aaactgtcat
601 gctggagtgc aggagaggta agcggaattc ctagtgtagc ggtgaaatgc gtagatatta
661 ggaggaacac cagtggcgaa ggcggcttac tggactgtca ctgacactga tgcacgaaag
721 cgtggggagc aaacaggatt agataccctg gtagtccacg ccgtaaacga tgaatactag
781 gtgtcggggc cgtagaggct tcggtgccgc agcaaacgca gtaagtattc cacctgggga
841 gtacgttcgc aagaatgaaa ctcaaaggaa ttgacgggga cccgcacaag cggtggagca
901 tgtggtttaa ttcgaagcaa cgcgaagaac cttacctggt cttgacatcc caatgaccga
961 accttaaccg gttttttctt tcgagacatt ggagacaggt ggtgcatggt tgtcgtcagc
1021 tcgtgtcgtg agatgttggg ttaagtcccg caacgagcgc aacccctatc tttagtagcc
1081 agcatttaag gtgggcactc tagagagact gccagggata acctggagga aggtggggac
1141 gacgtcaaat catcatgccc cttatggcca gggctacaca cgtgctacaa tggcgtaaac
1201 aaagggaagc gaagtcgtga ggcgaagcaa atcccagaaa taacgtctca gttcggattg
1261 tagtctgcaa ctcgactaca tgaagctgga atcgctagta atcgtgaatc agaatgtcac
1321 ggtgaatacg ttcccgggtc ttgtacacac cgcccgtcac accatgggag tcagtaacgc
1381 ccgaagtcag tgacccaacc gcaaggaggg agctgccgaa ggtgggaccg ataactgggg
1441 tgaagtcgta acaagg
SEQ ID NO: 6 *- Anaerostipes hadrus* NCIMB 43457 16S ribosomal RNA 
TTACGGTTGGGTCACTGACTTCGGGCGTTACTGACTCCCATGGTGTGACGGGCGGTGTGTACAAGACCCGGGAACGT
ATTCACCGCGACATTCTGATTCGCGATTACTAGCGATTCCAGCTTCATGTAGTCGAGTTGCAGACTACAATCCGAAC
 
TGAGACGTTATTTTTGGATTTGCTCGACCTCGCGGTTCTGCCTCCCTTTGTTTACGCCATTGTAGCACGTGTGTAG
CCCTGCTCATAAGGGGCATGATGATTTGACGTCATCCCCACCTTCCTCCAGGTTATCCCTGGCAGTCTCTCTAGAGT
GCCCGGCCAGACCGCTGGCTACTAAAGATAGGGGTTGCGCTCGTTGCGGGACTTAACCCAACATCTCACGACACGAG
CTGACGACAACCATGCACCACCTGTCATCCCTGTCCCGAAGGAAAGGCAACATTACTTGCCGGTCAGGGAGATGTCA
AGAGCAGGTAAGGTTCTTCGCGTTGCTTCGAATTAAACCACATGCTCCACCGCTTGTGCGGGTCCCCGTCAATTCCT
TTGAGTTTCATTCTTGCGAACGTACTCCCCAGGTGGACTACTTATTGCGTTTGCTGCGGCACCGAACAGCTTTGCTG
CCCGACACCTAGTAGTCATCGTTTACGGCGTGGACTACCAGGGTATCTAATCCTGTTTGCTCCCCACGCTTTCGAGC
CTCAACGTCAGTTACCGTCCAGTAAGCCGCCTTCGCCACTGGTGTTCCTCCTAATATCTACGCATTTCACCGCTACA
CTAGGAATTCCGCTTACCTCTCCGGTACTCTAGATTGACAGTTTCCAATGCAGTCCCGGGGTTGAGCCCCGGGTTTT
CACATCAGACTTGCCACTCCGTCTACGCTCCCTTTACACCCAGTAAATCCGGATAACGCTTGCACCATACGTATTAC
CGCGGCTGCTGGCACGTATTTAGCCGGTGCTTCTTAGTCAGGTACCGTCATTTTCTTCCCTGCTGATAGAGCTTTAC
ATACCGAAATACTTCATCGCTCACGSGGYGTCGSTGCATCAGGGTTTCCCCCATTGSACAATWTTCCCCACTGCTGC
CTCCTGTAGGAGTGGGGGCCGTGTCTCAGTCACAATG
> **SEQ ID NO:** 7 - *Anaerostipes hadrus* NCIMB 43526 16S ribosomal RNA 
TTCGGCAGCTCCCTCCTTACGGTTGGGTCACTGACTTCGGGCGTTACTGACTCCCATGGTGTGACGGGCGGTGTGTA
CAAGACCCGGGAACGTATTCACCGCGACATTCTGATTCGCGATTACTAGCGATTCCAGCTTCATGTAGTCGAGTTGC
AGACTACAATCCGAACTGAGACGTTATTTTTGGGATTTGCTCACTCTCACGAGGCTGCTTCCCTCTGTTTACGCCAT
TGTAGCACGTGTGTAGCCCTGGTCATAAGGGGCATGATGATTTGACGTCGTCCCCACCTTCCTCCAGGTTATCCCTG
GCAGTCTCTCTAGAGTGCCCACCTKAWATGCTGGCTACTAAAGATAGGGGTTGCGCTCGTTGCGGGACTTAACCCAA
CATCTCACGACACGAGCTGACGACAACCATGCACCACCTGTCWCTCCTGTCCCGAAGGAAAAGTCCGGTTAAGGACC
GGTCAGAAGGATGTCAAGACCAGGTAAGGTTCTTCGCGTTGCTTCGAATTAAACCACATGCTCCACCGCTTGTGCGG
GTCCCCGTCAATTCCTTTGAGTTTCATTCTTGCGAACGTACTCCCCAGGTGGAATACTTACTGCGTTGGCTGCGGCA
CCGAAGCCTCTACGGCCCCGACACCTAGTATTCATCGTTTACGGCGTGGACTACCAGGGTATCTAATCCTGTTTGCT
CCCCACGCTTTCGTGCCTCAGTGTCAGTTTCAGTCCAGTAAGCCGCCTTCGCCACTGATGTTCCTCCTAATATCTAC
GCATTTCACCGCTACACTAGGAATTCCGCTTACCTCTCCTGCACTCYAGTCTGACAGTTTCAAAAGCAGTCCCAGAG
TTAAGCCCTGGGTTTTCACTTCTGACTTGCCATACCACCTACGCACCCTTTACACCCAGTAATTCCGGATAACGCTT
GCCCCCTACGTATTACCGCGGCTGCTGGCACGTAGTTAGCCGGGGCTTCTTAGTCAGGTACCGTCATTTTCTTCCCT
GCTGATAGAGCTTTACATACCGAGATACTTCTTCACTCACGCGGCGTCGCTGCATCAGGGTTTCCCCCATTGTGCAA
TATTCCCCACTGCTGCCTCCCGTAGGAGTTTGGGCCGTGTCTCAGTCCCAATGTGGCCGTTCACTCTCTCAAGCCGG
CTACTGATCGTCGCCTTGGTGAGCCGTTACCTCACCAACCAGCTAATCAGACGCGGGTCCATCCTGTACCACCGGAG
TTTTTACCCCTGCACCATGCGGTGCTGTGGTCTTATGCGGTATTAGCAGTCATTTCTGACTGTTATCCCCCTGTACA
GGGCAGGTTACCCACGCGTTACTCACCCGTCCGCCACTCAATCACCAAATCTTCAGTTCCGAAGAAAATCAAATAAG
GTGTTTCGTTCGACTTGCAT
**SEQ ID NO:** 8 (consensus 16S ribosomal RNA sequence from the *Eubacterium callanderi* strain deposited under accession number NCIMB 43455) 
CTGCTGAGTCCTTGCGGTTCTCTCACAGGCTTCGGGTGTTGCCAACTCTCGTGGTGTGAC
GGGCGGTGTGTACAAGACCCGGAACGCATTCACCGCGGCATTCTGATCCGCGATTACT
AGCAACTCCAACTTCATGCAGGCGAGTTGCAGCCTGCAATCCGAACTGGGATCTGTTTT
AAGGGATTTGCTTCACCTCGCGGCTTCGCGGCCCTCTGTTCAGACCATTGTAGCACGTGT
GTAGCCCAGGTCATAAGGGGCATGATGATTTGACGTCATCCCCACCTTCCTCCGTATTGT
CTACGGCAGTCCCTCTAGAGTGCCCAACTGAATGCTGGCAACTAAAGGCAGGGGTTGCG
 
CTCGTTGCGGGACTTAACCCAACATCTCACGACACGAGCTGACGACAACCATGCACCAC
CTGTCTCTCTGTTCCCGAAGGAAACTTCCTATCTCTAGGCTCGTCAGAGGATGTCAAGAC
CTGGTAAGGTTCTTCGCGTTGCTTCGAATTAAACCACATGCTCCGCTGCTTGTGCGGGTC
CCCGTCAATTCCTTTGAGTTTCAACCTTGCGGTCGTACTCCCCAGGCGGAATGCTTATTG
TGTTAACTGCGGCACTGAGTTTCCCCAACACCTAGCATTCATCGTTTACGGCGTGGACTA
CCAGGGTATCTAATCCTGTTCGCTCCCCACGCTTTCGCACCTCAGCGTCAGTATTTGTCC
AGCAAGCCGCCTTCGCCACTGGTGTTCCTCCTAATATCTACGCATTTCACCGCTACACTA
GGAATTCCACTTGCCTCTCCAATACTCAAGTCTACCAGTTTCCAATGCACTTCACCGGTT
GAGCCGGTACCTTTCACATCAGACTTAATAGACCGCCTACGCGCCCTTTACGCCCAGTCA
TTCCGGACAACGCTTGTCCCCTACGTATTACCGCGGCTGCTGGCACGTAGTTAGCCGGG
ACTTCCTCATTGGGTACCGTCATGTTTCTTCCCCAATAACAGAGCTTTACGATCCGAAA
ACCTTCTTCACTCACGCGGTATTGCTGCGTCAGGGTTGCCCCCATTGCGCAATATTCCCC
ACTGCTGCCTCCCGTAGGAGTCTGGACCGTGTCTCAGTTCCAGTGTGACCGTTCGCCCTC
TCAGACCGGTTACCCATCGTCGCCTTGGTGGGCTGTTATCTCACCAACTAGCTAATGGGA
CGCGGGTCCATCCTATGGCACCGGAGTTTCATGATCTTGCCATGCGACAAAACCATAA
TATAAGGCTTTACTCCCAGTTTCCCGAGGCTATTCCTTTCCATAGGGCAGGTTACCCACG
CGTTACTCACCCGTTCGCCACTTTCCAGTTCTAATTTCACCCGAAGGATCATTCAAAACC
TTCTCGTTCGACTGCATG
**SEQ ID NO:** 9 (consensus 16S ribosomal RNA sequence from *Eubacterium eligens* strain *E. eligens* strain ref. 1) 
TTCGGCGGCTCCTTCTTTCGTTAGGTCACCGACTTCGGGCATTTTCGACTCCCATGGTGT
GACGGGCGGTGTGTACAAGACCCGGAACGTATTCACCGCAGCATTCTGATCTGCGATT
ACTAGCGATTCCAGCTTCATGTAGTCGAGTTGCAGACTACAATCCGAACTGAGACGTTA
TTTTTGTGATTTGCTTGGCCTCACGACTTCGCTTCACTTTGTTACGCCATTGTAGCACGT
GTGTAGCCCAAGTCATAAGGGGCATGATGATTTGACGTCATCCCCACCTTCCTCCAGGTT
ATCCCTGGCAGTCTCCCTAGAGTGCCCATCTTACTGCTGGCTACTAAGGATAGGGGTTGC
GCTCGTTGCGGGACTTAACCCAACATCTCACGACACGAGCTGACGACAACCATGCACCA
CCTGTCACCACTGTCCCGAAGGAAAGGACACATTACTGTCCGGTCAGTGGGATGTCAAG
ACTTGGTAAGGTTCTTCGCGTTGCTTCGAATTAAACCACATGCTCCACCGCTTGTGCGGG
TCCCCGTCAATTCCTTTGAGTTTCATTCTTGCGAACGTACTCCCCAGGTGGAATACTTATT
GCGTTTGCTGCGGCACCGAAGCCCTTATGGCCCCGACACCTAGTATTCATCGTTTACGG
CGTGGACTACCAGGGTATCTAATCCTGTTTGCTCCCCACGCTTTCGAGCCTCAGTGTCAG
TTACAGTCCAGTGAGCCGCCTTCGCCACTGGTGTTCCTCCTAATATCTACGCATTTCACC
GCTACACTAGGAATTCCACTCACCCCTCCTGCACTCTAGCCTTACAGTTTCAAAAGCAGT
TCCGGGGTTGAGCCCCGGATTTCACTTCTGACTTGCATGGCCACCTACACTCCCTTTAC
ACCCAGTAAATCCGGATAACGCTTGCTCCATACGTATTACCGCGGCTGCTGGCACGTATT
TAGCCGGAGCTTCTTAGTCAGGTACCGTCACTATCTTCCCTGCTGATAGAGCTTTACATA
 
ACGAATTACTTCTTCACTCACGCGGCGTCGCTGCATCAGAGTTTCCTCCATTGTGCAATA
TTCCCCACTGCTGCCTCCCGTAGGAGTCTGGGCCGTGTCTCAGTCCCAATGTGGCCGGTC
ACCCTCTCAGGTCGGCTACTGATCGTCGCCTTGGTGGGCTGTTATCTCACCAACTAGCTA
ATCAGACGCGGGTCCATCTTATACCACCGGAGTTTTTCACACCATGTCATGCAACATTGT
GCGCTTATGCGGTATTACCAGCCGTTTCCAGCTGCTATCCCCCAGTACAAGGCAGGTTAC
CCACGCGTTACTCACCCGTCCGCCACTCAGTCATAAGCAACTTCA
**SEQ ID NO:** 10 (consensus 16S ribosomal RNA sequence *from Eubacterium rectale* strain *E*. *rectale* strain ref. 2) 
GCTCCTTCCTTTCGGTTAGGTCACTGGCTTCGGGCATTCCCAACTCCCATGGTGTGACGG
GCGGTGTGTACAAGACCCGGGAACGTATTCACCGCAGCATTCTGATCTGCGATTACTAG
CGATTCCAGCTTCGTGTAGTCGGGTTGCAGACTACAGTCCGAACTGAGACGTTATTTTG
AGATTTGCTCGGCTTCACAGCTTTGCTTCCCTTTGTTTACGCCATTGTAGCACGTGTGTAG
CCCAAGTCATAAGGGGCATGATGATTTGACGTCATCCCCGCCTTCCTCCAGGTTATCCCT
GGCAGTCTCTCTAGAGTGCCCGGCCGAACCGCTGGCTACTAAAGATAAGGGTTGCGCTC
GTTGCGGGACTTAACCCAACATCTCACGACACGAGCTGACGACAACCATGCACCACCTG
TCACTCCTGCTCCGAAGAGAAGGTACGGTTAAGTACCGGTCAGAAGGATGTCAAGACTT
GGTAAGGTTCTTCGCGTTGCTTCGAATTAAACCACATGCTCCACCGCTTGTGCGGGTCCC
CGTCAATTCCTTTGAGTTTCATTCTTGCGAACGTACTCCCCAGGTGGAATACTTACTGCG
TTTGCGACGGCACCGAGAAGCAATGCTTCCCAACACCTAGTATTCATCGTTTACGGCGT
GGACTACCAGGGTATCTAATCCTGTTTGCTCCCCACGCTTTCGAGCCTCAGCGTCAGTTA
TCGTCCAGTAAGCCGCCTTCGCCACTGGTGTTCCTCCTAATATCTACGCATTTCACCGCT
ACACTAGGAATTCCGCTTACCCCTCCGACACTCTAGTACGACAGTTTCCAATGCAGTACC
GGGGTTGAGCCCCGGGCTTTCACATCAGACTTGCCGCACCGCCTGCGCTCCCTTTACACC
CAGTAAATCCGGATAACGCTTGCACCATACGTATTACCGCGGCTGCTGGCACGTATTTA
GCCGGTGCTTCTTAGTCAGGTACCGTCATTATCTTCCCTGCTGATAGAGCTTTACATACC
GAAATACTTCTTCGCTCACGCGGCGTCGCTGCATCAGGCTTTCGCCCATTGTGCAATATT
CCCCACTGCTGCCTCCCGTAGGAGTTTGGGCCGTGTCTCAGTCCCAATGTGGCCGGTCAC
CCTCTCAGGTCGGCTATGGATCGTCGCCTTGGTGGGCCGTTACCTCACCAACTAGCTAAT
CCAACGCGGGTCCATCTTATACCACCGGAGTTTTTCACACTGCATCATGCGATGCTGTGC
GCTTATGCGGTATTAGCAGCCGTTTCCAACTGTTATCCCCCTGTACAAGGCAGGTTACCC
ACGCGTTACTCACCCGTCCGCCACTCAGTCACAAAATAATCAGTCCCGAAGGAAATCAA
ATAAAGTGCTTCGTCGACTGCA
**SEQ ID NO:** 11 (consensus 16S ribosomal RNA sequence from *Eubacterium rectale* strain *E. rectale* strain ref. 1) GCTCCTTCCTTTCGGTTAGGTCACTGGCTTCGGGCATTCCCAACTCCCATGGTGTGACGG 
GCGGTGTGTACAAGACCCGGGAACGTATTCACCGCAGCATTCTGATCTGCGATTACTAG
CGATTCCAGCTTCGTGTAGTCGGGTTGCAGACTACAGTCCGAACTGAGACGTATTTTTG
AGATTTGCTCGGCTTCACAGCTTTGCTTCCCTTTGTTACGCCATTGTAGCACGTGTGTAG
CCCAAGTCATAAGGGGCATGATGATTTGACGTCATCCCCGCCTTCCTCCAGGTTATCCCT
GGCAGTCTCTCTAGAGTGCCCGGCCGAACCGCTGGCTACTAAAGATAAGGGTTGCGCTC
GTTGCGGGACTTAACCCAACATCTCACGACACGAGCTGACGACAACCATGCACCACCTG
TCACTCCTGCTCCGAAGAGAAGGTACGGTTAAGTACCGGTCAGAAGGATGTCAAGACTT
GGTAAGGTTCTTCGCGTTGCTTCGAATTAAACCACATGCTCCACCGCTTGTGCGGGTCCC
CGTCAATTCCTTTGAGTTTCATTCTTGCGAACGTACTCCCCAGGTGGAATACTTACTGCG
TTTGCGACGGCACCGAGAAGCAATGCTTCCCAACACCTAGTATTCATCGTTTACGGCGT
GGACTACCAGGGTATCTAATCCTGTTTGCTCCCCACGCTTTCGAGCCTCAGCGTCAGTTA
TCGTCCAGTAAGCCGCCTTCGCCACTGGTGTTCCTCCTAATATCTACGCATTTCACCGCT
ACACTAGGAATTCCGCTTACCCCTCCGACACTCTAGTACGACAGTTTCCAATGCAGTACC
GGGGTTGAGCCCCGGGCTTTCACATCAGACTTGCCGCACCGCCTGCGCTCCCTTTACACC
CAGTAAATCCGGATAACGCTTGCACCATACGTATTACCGCGGCTGCTGGCACGTATTTA
GCCGGTGCTTCTTAGTCAGGTACCGTCATTATCTTCCCTGCTGATAGAGCTTTACATACC
GAAATACTTCTTCGCTCACGCGGCGTCGCTGCATCAGGCTTTCGCCCATTGTGCAATATT
CCCCACTGCTGCCTCCCGTAGGAGTTTGGGCCGTGTCTCAGTCCCAATGTGGCCGGTCAC
CCTCTCAGGTCGGCTATGGATCGTCGCCTTGGTGGGCCGTTACCTCACCAACTAGCTAAT
CCAACGCGGGTCCATCTTATACCACCGGAGTTTTTCACACTGCATCATGCGATGCTGTGC
GCTTATGCGGTATTAGCAGCCGTTTCCAACTGTTATCCCCCTGTACAAGGCAGGTTACCC
ACGCGTTACTCACCCGTCCGCCACTCAGTCACAAAATAATCAGTCCCGAAGGAAATCAA
ATAAAGTGCTTCGTTCGACTGCA
**SEQ ID NO:** 12 (consensus 16S ribosomal RNA sequence from *Faecalicatena fissicatena* strain *F*. *fissicatena* strain ref. 1) 
GGCAGCTCCCTCCTTACGGTTGGGTCACTGACTTCGGGCGTTACCAACTCCCATGGTGTG
ACGGGCGGTGTGTACAAGACCCGGGAACGTATTCACCGCGACATTCTGATTCGCGATTA
CTAGCGATTCCAGCTTCATGTAGTCGAGTTGCAGACTACAATCCGAACTGAGACGTTATT
TTTGGGATTTGCTCAACCTCGCGGTATTGCCTCCCTTTGTTTACGCCATTGTAGCACGTGT
GTAGCCCTGCTCATAAGGGGCATGATGATTTGACGTCATCCCCACCTTCCTCCAGGTTAT
CCCTGGCAGTCTCTCTAGAGTGCCCGGCCAAACCGCTGGCTACTAAAGATAAGGGTTGC
GCTCGTTGCGGGACTTAACCCAACATCTCACGACACGAGCTGACGACAACCATGCACCA
CCTGTCTCCACTGCCCCGAAGGAAGGCGCCATTACACGCCGGTCAGTGGGATGTCAAG
AGCAGGTAAGGTTCTTCGCGTTGCTTCGAATTAAACCACATGCTCCACCGCTTGTGCGGG
TCCCCGTCAATTCCTTTGAGTTTCATTCTTGCGAACGTACTCCCCAGGTGGAATACTTATT
GCGTTTGCTGCGGCACCGAATGGCTTTGCCACCCGACACCTAGTATTCATCGTTTACGGC
GTGGACTACCAGGGTATCTAATCCTGTTTGCTCCCCACGCTTTCGAGCCTCAACGTCAGT
 
GATCGTCCAGTAAGCCGCCTTCGCCACTGGTGTTCCTCCTAATATCTACGCATTTCACCG
CTACACTAGGAATTCCACTTACCTCTCCGACACTCTAGTTACATAGTTTCCAATGCAGTC
CCGGGGTTGAGCCCCGGGTTTCACATCAGACTTACATAACCGTCTACGCTCCCTTTACA
CCCAGTAAATCCGGATAACGCTTGCCCCCTACGTATTACCGCGGCTGCTGGCACGTAGTT
AGCCGGGGCTTCTTAGTCAGGTACCGTCATTTTCTTCCCTGCTGATAGAAGTTTACATAC
CGAAATACTTCATCCTTCACGCGGCGTCGCTGCATCAGGGTTTCCCCCATTGTGCAATAT
TCCCCACTGCTGCCTCCCGTAGGAGTTTGGGCCGTGTCTCAGTCCCAATGTGGCCGGTCA
CCCTCTCAGGTCGGCTACTGATCGTCGCCTTGGTAAGCCGTTACCTTACCAACTAGCTAA
TCAGACGCGGGTCCATCTCATACCACCGGAGTTTTTCCCACTGTACCATGCGGTACCGTG
GTCTTATGCGGTATTAGCAGTCATTTCTAACTGTTATCCCCCTGTATGAGGCAGGTTACC
CACGCGTTACTCACCCGTCCGCCGCTCAGTCGCAAAACTCTTCAATCCGAAGAAATCAA
AGTAAAGCGCTCCGCTCGACTTGC
**SEQ ID NO:** 13 (consensus 16S ribosomal RNA sequence from *Faecalicatena contorta* strain *F*. *conlorta* strain ref. 1) 
TGCAGTCGAGCGAAGCAGCTTTACTTAGATTTCTTCGGATTGAAAGAGTTTTGCGACTGA
GCGGCGGACGGGTGAGTAACGCGTGGGTAACCTGCCTCATACAGGGGGATAACAGTTA
GAAATGACTGCTAATACCGCATAAGACCACGGTACCGCATGGTACAGTGGGAAAAACT
CCGGTGGTATGAGATGGACCCGCGTCTGATTAGCTGGTTGGTAAGGTAACGGCTTACCA
AGGCGACGATCAGTAGCCGACCTGAGAGGGTGACCGGCCACATTGGGACTGAGACACG
GCCCAAACTCCTACGGGAGGCAGCAGTGGGGAATATTGCACAATGGGGGAAACCCTGA
TGCAGCGACGCCGCGTGAAGGATGAAGTATTTCGGTATGTAAACTTCTATCAGCAGGGA
AGAAAATGACGGTACCTGACTAAGAAGCCCCGGCTAACTACGTGCCAGCAGCCGCGGT
AATACGTAGGGGGCAAGCGTTATCCGGATTTACTGGGTGTAAAGGGAGCGTAGACGGTT
ATGTAAGTCTGATGTGAAAACCCGGGGCTCAACCCCGGGACTGCATTGGAAACTATGTA
ACTAGAGTGTCGGAGAGGTAAGTGGAATTCCTAGTGTAGCGGTGAAATGCGTAGATATT
AGGAGGAACACCAGTGGCGAAGGCGGCTTACTGGACGATGACTGACGTTGAGGCTCGA
AAGCGTGGGGAGCAAACAGGATTAGATACCCTGGTAGTCCACGCCGTAAACGATGAAT
ACTAGGTGTCGGGTGGCAAAGCCATTCGGTGCCGCAGCAAACGCAATAAGTATTCCACC
TGGGGAGTACGTTCGCAAGAATGAAACTCAAAGGAATTGACGGGGACCCGCACAAGCG
GTGGAGCATGTGGTTTAATTCGAAGCAACGCGAAGAACCTTACCTGCTCTTGACATCCC
CCTGACCGGCGCGTAATGGTGCCTTTCCTTCGGGACAGGGGAGACAGGTGGTGCATGGT
TGTCGTCAGCTCGTGTCGTGAGATGTTGGGTTAAGTCCCGCAACGAGCGCAACCCTTATC
TTTAGTAGCCAGCGGTATGGCCGGGCACTCTAGAGAGACTGCCAGGGATAACCTGGAGG
AAGGTGGGGATGACGTCAAATCATCATGCCCCTTATGAGCAGGGCTACACACGTGCTAC
AATGGCGTAAACAAAGGGAGGCGAAGCCGCGAGGTGGAGCAAATCCCAAAAATAACGT
CTCAGTTCGGATTGTAGTCTGCAACTCGACTACATGAAGCTGGAATCGCTAGTAATCGC
GAATCAGAATGTCGCGGTGAATACGTTCCCGGGTCTTGTACACACCGCCCGTCACACCA
TGGGAGTTGGTAACGCCCGAAGTCAGTGACCCAACCGCAAGGAGGGAGCTGCCGAAG
**SEQ ID NO:** 14 (consensus 16S ribosomal RNA sequence from *Eubacterium hallii* strain ref. 1) 
AGGTTGGGTCACTGGSTTCNGGCATTTYCNANTCCCATGGTGTGRCSGGCGGTGTGKACA
ANAMCCGGGAACGTATTYACCGSGACATTYYGRWTYGNGATTACTAGCGATTCCAGCT
TCGTGTAGTCGGGTTGCAGAATACAGTCCGAACTGGACGGCCTTTTGTGGTTTGSTCC
CCCTCGCGGGTTCGCCTCACTCTGTGACCGCCATTGTAGCACGTGTGTCGCCCAGATCAT
AAGGGGCATGATGATTTGACGTCGTCCCCACCTTCCTCCAGGTTATCCCTGGCAGTCTCT
CCAGAGTGCCCAGCCTTACCTGCTGGCTACTGAAGATAGGGGTTGCGCTCGTTGCGGGA
CTTAACCCAACATCTCACGACACGAGCTGACGACAACCATGCACCACCTGTCTCTTCTGT
CCCGAAGGAAAGCACCGATTAAGGTGCGGTCAGAAGGATGTCAAGACCTGGTAAGGTT
CTTCGCGTTGCTTCGAATTAAACCACATGCTCCACCGCTTGTGCGGGTCCCCGTCAATTC
CTTTGAGTTTCATTCTTGCGAACGTACTCCCCAGGTGGAATACTTACTGCGTTTGCGGCG
GCACCGAAGCCTATACGGCCCCGACACCTAGTATTCATCGTTTACGGCGTGGACTACCA
GGGTATCTAATCCTGTTTGCTCCCCACGCTTTCGTGCCTCAGTGTCAGTAACAGTCCAGC
AGGCCGCCTTCGCCACTGGTGTTCCTCCTAATATCTACGCATTTCACCGCTACACTAGGA
ATTCCGCCTGCCTCTCCTGTACTCTAGCCGAGCAGTTTCAAATGCAGCTCCGGGGTTGAG
CCCCGGCCTTTCACATCTGACTTGCACTGCCACCTACGCACCCTTTACACCCAGTAAATC
CGGATAACGCTTGCTCCATACGTATTACCGCGGCTGCTGGCACGTATTTAGCCGGAGCTT
CTTAGTCAGGTACCGTCATTATCTTCCCTGCTGATAGAGCTTTACATACCGAAATACTTC
TTCACTCACGCGGCGTTGCTGCATCAGGGTTTCCCCCATTGTGCAATATTCCCCACTGCT
GCCTCCCGTAGGAGTTTGGACCGTGTCTCAGTTCCAATGTGGCCGTTCATCCTCTCAGAC
CGGCTACTGATCGTTGCCTTGGTAGGCCGTTACCCTGCCAACCAGCTAATCAGACGCGG
GCCCATCCTGTACCACCGGAGTTTTTCACACWAGGAGATGTCTCCTCGTGCGCTTATGC
GGTATTAGCAGCCGTTTCCAGCTGTTATCCCCCTGTACAGGGCAGGTTACCCACGCGTTA
CTCACCCGTCCGCCACTCAGTCACCA
**SEQ ID NO:** 15 (consensus 16S ribosomal RNA sequence from *Anaerostipes caccae* strain ref. 1) 
CGGCAGCTCCCTCCTTGCGGTTGGGTCACTGACTTCGGGCGTTACTGACTCCCATGGTGT
GACGGGCGGTGTGTACAAGACCCGGGAACGTATTCACCGTGACATTCTGATTCACGATT
ACTAGCGATTCCAGCTTCATGTAGTCGAGTTGCAGACTACAATCCGAACTGAGACGTTA
TTTCTGGGATTTGCTTCGCCTCACGACTTCGCTTCCCTTTGTTACGCCATTGTAGCACGT
GTGTAGCCCTGGCCATAAGGGGCATGATGATTTGACGTCGTCCCCACCTTCCTCCAGGTT
ATCCCTGGCAGTCTCTCTAGAGTGCCCATCCGTAATGCTGGCTACTAAAGATAGGGGTT
GCGCTCGTTGCGGGACTTAACCCAACATCTCACGACACGAGCTGACGACAACCATGCAC
CACCTGTCTCCAATGTCTCGAAAGAAAAACCGGTTAAGGTTCGGTCATTGGGATGTCA
AGACCAGGTAAGGTTCTTCGCGTTGCTTCGAATTAAACCACATGCTCCACCGCTTGTGCG
GGTCCCCGTCAATTCCTTTGAGTTTCATTCTTGCGAACGTACTCCCCAGGTGGAATACTT
ACTGCGTTTGCTGCGGCACCGAAGCCTCTACGGCCCCGACACCTAGTATTCATCGTTTAC
GGCGTGGACTACCAGGGTATCTAATCCTGTTTGCTCCCCACGCTTTCGTGCATCAGTGTC
AGTGACAGTCCAGTAAGCCGCCTTCGCCACTGGTGTTCCTCCTAATATCTACGCATTTCA
CCGCTACACTAGGAATTCCGCTTACCTCTCCTGCACTCCAGCATGACAGTTTCAAAAGCA
GTCCCGGGGTTAAGCCCCGGGCTTTCACTTCTGACTTACCATGCCACCTACGCACCCTTT
ACACCCAGTAATTCCGGATAACGCTTGCCCCCTACGTATTACCGCGGCTGCTGGCACGT
AGTTAGCCGGGGCTTCTTAGTCAGGTACCGTCTGTTTCTTCCCTGCTGATAGAGCTTTA
CATACCGAAATACTTCTTCACTCACGCGGCGTCGCTGCATCAGGGTTTCCCCCATTGTGC
 
AATATTCCCCACTGCTGCCTCCCGTAGGAGTTTGGGCCGTGTCTCAGTCCCAATGTGGCC
GTTCACTCTCTCAAGCCGGCTACTGATCGTCGCCTTGGTGAGCCGTTACCTCACCAACCA
GCTAATCAGACGCGGACCATCCTATACTGCCAGGGCTTTTCACACTGAATCATGCGAT
TCTGTGCGCTTATGCGGTATTAGCAGCCGTTTCCAGCTGTTATCCCCCTGTATAGGGCAG
GTTCCCCACGCGTTACTCACCCGTCCGCCACTCAGTCATATAGGAAATCCATCCGAAAA
CTTCAATCCTAAATGCTTCGTTCGACTGCA
**SEQ ID NO:** 16: Anaerostipes rhamnosivorans strain 1y-2 from Netherlands 16S ribosomal RNA gene, partial sequence (DSM 26241)

1 tattttggat tgaagttttc ggatggatct ccttaatgac tgagtggcgg acgggtgagt
61 aacgcgtggg gaacctgccc tatacagggg gataacagct ggaaacggct gctaataccg
121 cataagcgca cagaatcgca tgattcagtg tgaaaagccc tggcagtata ggatggtccc
181 gcgtctgatt agctggttgg cggggtaacg gcccaccaag gcgacgatca gtagccggct
241 tgagagagtg gacggccaca ttgggactga gacacggccc aaactcctac gggaggcagc
301 agtggggaat attgcacaat gggggaaacc ctgatgcagc gacgccgcgt gagtgaagaa
361 gtatttcggt atgtaaagct ctatcagcag ggaagaaata agacggtacc tgactaagaa
421 gccccggcta actacgtgcc agcagccgcg gtaatacgta gggggcaagc gttatccgga
481 attactgggt gtaaagggtg cgtaggtggc atgataagtc agaagtgaaa gcccgggct
541 taaccccggg actgcttttg aaactgtaat gctagagtgc aggagaggta agcggaattc
601 ctagtgtagc ggtgaaatgc gtagatatta ggaggaacac cagtggcgaa ggcggcttac
661 tggactgtca ctgacactga ggcacgaaag cgtggggagc aaacaggatt agataccctg
721 gtagtcnacg ccgtaaacga tgaatactag gtgtcggggc cgtagaggct tcggtgccgc
781 agcaaacgca gtaagtattc cacctgggga gtacgttcgc aagaatgaaa ctcaaaggaa
841 ttgacgggga cccgcacaag cggtggagca tgtggtttaa ttcgaagcaa cgcgaagaac
901 cttacctggt cttgacatcc ttctgaccgg ttnnnaaccg aacctttcct tcgggacaga
961 agtgacaggt ggtgcatggt tgtcgtcagc tcgtgtcgtg agatgttggg ttaagtcccg
1021 caacgagcgc aacccctatc tttagtagcc agcatataag gtgggcactc tagagagact
1081 gccagggata acctggagga aggtggggac gacgtcaaat catcatgccc cttatggcca
1141 gggctacaca cgtgctacaa tggcgtaaac aaagggaagc gaccccgcga gggcaagcaa
1201 atcccagaaa taacgtctca gttcggattg tagtctgcaa ctcgactaca tgaagctgga
1261 atcgctagta atcgtgaatc agaatgtcac ggtgaatacg ttcccgggtc ttgtacacac
1321 cgcccgtcac accatgggag tcagtaacgc ccgaagtcag tgacccaacc gcaaggagga
1381 ga

### REFERENCES

[1] Spor et al. (2011) Nat Rev Microbiol. 9(4):279-90.
[2] Eckburg et al. (2005) Science. 10;308(5728):1635-8.
[3] Macpherson et al. (2001) Microbes Infect. 3(12):1021-35
[4] Macpherson et al. (2002) Cell MolLife Sci. 59(12):2088-96.
[5] Mazmanian et al. (2005) Cell 15;122(1):107-18.
[6] Frank et al. (2007) PNAS 104(34):13780-5.
[7] Scanlan et al. (2006) J Clin Microbiol. 44(11):3980-8.
[8] Kang et al. (2010) Inflamm Bowel Dis. 16(12):2034-42.
[9] Machiels et al. (2013) Gut. 63(8):1275-83.
[10] WO 2013/050792
[11] WO 03/046580
[12] WO 2013/008039
[13] WO 2014/167338
[14] Goldin and Gorbach (2008) Clin Infect Dis. 46 Suppl 2:S96-100.
[15] Azad et al. (2013) BMJ. 347:f6471.
[16] Frank et al. (2007) PNAS 104(34):13780-5.
[17] Scanlan el al. (2006) J Clin Microbiol. 44(11):3980-8.
[18] Kang et al. (2010) Inflamm Bowel Dis. 16(12):2034-42.
[19] Machiels et al. (2013) Gut. 63(8):1275-83.
[20] Mayer et al (2014) The Journal of Neuroscience 34(46):15490 -15496
[21] Cryan and Dinan (2015) Neuropsychopharmacology, 40: 241-2.
[22] Zhou and Foster (2015) Neuropsychiatric Disease and Treatment 11: 715-723.
[23] Wang and Kasper (2014) Brain Behav Immun. 38: 1-12.
[24] WO 2013/050792
[25] WO 03/046580
[26] WO 2013/008039
[27] WO 2014/167338
[28] Goldin and Gorbach (2008) Clin Infect Dis. 46 Suppl 2:S96-100.
[29] Azad et al. (2013) BMJ. 347:f6471.
[30] Bravo et al. (2011) Proc Natl Acad Sci USA, 108: 16050-5.
[31] Kantak et al. (2014) Behav Pharmacol. 25: 71-9.
[32] Savignac et al. (2014) Neurogastroenterol Motil. 26: 1615-27.
[33] WO 2016/069795
[34] US 2016/120920
[35] US 2017/246220
[36] WO 2004/085628
[37] WO 2018/187272
[38] US 5443826
[39] WO 2019/089643
[40] US 2019/070225
[41] de Theije et al. (2014) Brain Behav Immun. 37: 197-206.
[42] Meyza and Blanchard (2017) Neurosci Biobehav Rev.
[43] de Theije et al. (2014) Brain Behav Immun. 37: 197-206.
[44] Hsiao et al. (2013) Cell, 155: 1451-63.
[45] Meyza and Blanchard (2017) Neurosci Biobehav Rev.
[46] Moore et al, (1976) Int J Syst Evol Microbiol; 26(2):238-252
[47] Masco et al. (2003) Systematic and Applied Microbiology, 26:557-563.
[48] Srůtková et al. (2011) J. Microbiol. Methods, 87(1):10-6.
[49] Srůtková et al. (2011) J. Microbiol. Methods, 87(1):10-6.
[50] Varga et al. (2010) Toxins (Basel); 2(7): 1718-1750
[51] Sakamoto et al, (2017) Int J Syst Evol Microbiol; 67:1219-1227
[52] Taylor et al. (1972) J Gen Microbiol; 71: 457-463
[53] Holdeman et al. (1971) Int J Syst Bacteriol; 21, 4: 304-306
[54] Chun et al (2007). IntJ Syst Evol Microbiol 57: 2259-2261.
[55] Masco et al. (2003) Systematic and Applied Microbiology, 26:557-563.
[56] Srůtková et al. (2011) J. Microbiol. Methods, 87(1):10-6.
[57] Wang et al. (2016) J Neurogastroenterol Motil 22: 589-605.
[58] Cook et al (2014) Hum Mol Genet 23:104-106
[59] Simoes-Pires et al (2013) Mol Neurodegen 8: 7
[60] Li and Zhou (2016) Neuroscience 324: 131-139.
[61] Vorstman et al. (2009) Neuropsychopharmacology; 34(3): 739-746.
[62] Carlson (2012) Pharmacol. Biochem. Behav.; 100(4): 850-854.
[63] Kang and Kim (2015) Front. Mol. Neurosci.; 8(17) 1-9.
[64] Zheng et al. (2016) Scientific Reports; 6 (31241) 1-8.
[65] Tang, et al. (2017) J Am Heart Assoc, 6(10).
[66] Wang et al. (2015) PNAS 112(9):2583-2858
[67] Psaty et al. (2003) JAMA, 289(19):2534-44
[68] Lancct. (1995) 346(8991-8992):1647-53
[69] Leoni et al. (2005) Mol. Med., 11(1-12):1-15.
[70] Glauben, et al. (2006) Journal of Immunology, 176(8): 5015-5022
[71] Gasche et al 200 Inflammatory Bowel Diseases 6: 8-15
[72] Fahy (2009) Proc Am Thorac Soc 6.256-259
[73] Reddy et al (2008) J Clin. Invest. 118:2562-73
[74] Leng et al (2006) Exp. Hematol. 34:776-787
[75] Tao et al (2007) Nat. Med. 13:1299-1307
[76] Johansson and Ekman (2004) Blood 104:5075
[77] Song et al (2005) APMIS 113: 264-268
[78] Zimmerman et al (2007) Cancer Res 67: 9074-54
[79] Zhang et al (2005) Breast Cancer Res Treat 94: 11-16
[80] Abbas and Gupta (2008) Epigenetics 3: 300-309
[81] Minamiya et al (2011) Lung Cancer 74: 300-4
[82] Jung et al (2012) J Cell Biochem 113: 2167-2177
[83] Quint et al 2011 Virchows Arch 459: 129-139
[84] Miyamoto-Shinohara et al. (2008) J. Gen. Appl. Microbiol, 54, 9-24.
[85] Cryopreservation and Freeze-Drying Protocols, ed. by Day and McLellan, Humana Press.
[86] Leslie et al. (1995) Appl. Environ. Microbiol. 61, 3592-3597.
[87] Mitropoulou et al. (2013) J Nutr Metab. (2013) 716861.
[88] Kailasapathy et al. (2002) Curr Issues Intest Microbiol. 3(2):39-48.
[89] Handbook of Pharmaceutical Excipients, 2nd Edition, (1994), Edited by A Wade and PJ Weller
[90] Remington's Pharmaceutical Sciences, Mack Publishing Co. (A. R. Gennaro edit. 1985)
[91] US 2016/0067188
[92] Handbook of Microbiological Media, Fourth Edition (2010) Ronald Atlas, CRC Press.
[93] Maintaining Cultures for Biotechnology and Industry (1996) Jennie C. Hunter-Cevera, Academic Press
[94] Strobel (2009) Methods Mol Biol. 581:247-61.
[95] Gennaro (2000) Remington: The Science and Practice of Pharmacy. 20th edition, ISBN: 0683306472.
[96] Molecular Biology Techniques: An Intensive Laboratory Course, (Ream et al., eds., 1998, Academic Press).
[97] Methods In Enzymology (S. Colowick and N. Kaplan, cds., Academic Press, Inc.)
[98] Handbook of Experimental Immunology, Vols. I-IV (D.M. Weir and C.C. Blackwell, eds, 1986, Blackwell Scientific Publications)
[99] Sambrook et al. (2001) Molecular Cloning: A Laboratory Manual, 3rd edition (Cold Spring Harbor Laboratory Press).
[100] Handbook ofSurface and Colloidal Chemistry (Birdi, K.S. ed., CRC Press, 1997)
[101] Ausubcl et al. (cds) (2002) Short protocols in molecular biology, 5th edition (Current Protocols).
[102] PCR (Introduction to Biotechniques Series), 2nd ed. (Newton & Graham eds., 1997, Springer Verlag)
[103] Current Protocols inMolecular Biology (F.M. Ausubel et al., eds., 1987) Supplement 30
[104] Smith & Waterman (1981) Adv. Appl. Math. 2: 482-489
[105] Johnsen et al. Gas chromatography-mass spectrometry data processing made easy. J Chromatogr A. 2017;1503:57-64*.*
[106] Yuille et al. (2018) Human gut bacteria as potent class I histone deacetylase inhibitors in vitro through production of butyric acid and valeric acid. PLoS One. 2018 Jul 27;13(7):
[107] Ettore et αl., poster presented at Neurodegenerative Diseases: New Insights and Therapeutic Opportunities, Keystone Colorado, June 16-20 2019
[108] PCT/EP2019/062236
[109] PCT/EP2019/062238
[110] Hyland & Cox (2005) Br J Pharmacol. 146(5):712-22.
[111] Barton et al. (2008) Archives of General Psychiatry, 65, no. 1: 38-46
[112] Duncan et a/., JAMA 303, no. 5: 430-37

Further numbered embodiments of the invention are provided below:
1. A composition comprising a bacterial strain of the genus *Anaerostipes,* for use in therapy.
2. A composition comprising a bacterial strain which has at least 95%, at least 96%, at least 97%, at least 98%, at least 99%, at least 99.5% or at least 99.95% identity to the sequence of SEQ ID NO: 1, SEQ ID NO: 2, SEQ ID NO: 3, SEQ ID NO: 4, SEQ ID NO: 5, SEQ ID NO: 6 SEQ ID NO: 7, SEQ ID NO: 15 or SEQ ID NO:16 for use in therapy.
3. The composition for use according to any preceding embodiment, wherein the bacterial strain has at least 95%, at least 96%, at least 97%, at least 98%, at least 99%, at least 99.5% or at least 99.95% identity to the sequence of SEQ ID NO: 1, SEQ ID NO: 6 or SEQ ID NO:7.
4. The composition for use according any proceeding embodiment, for use in the treatment or prevention of a disease or condition mediated by histone deacetylase (HDAC) activity.
5. The composition for use according to any preceding embodiment, for use in the treatment or prevention of a disease or condition mediated by Class I HDAC activity.
6. The composition for use according to any preceding embodiment, for use in a method of selectively inhibiting Class I HDAC activity in the treatment of a condition mediated by Class I HDAC activity.
7. The composition for use according to any preceding embodiment, wherein the composition is for use in selectively inhibiting HDAC1, HDAC2 or HDAC3 in a disease or condition mediated by HDAC1, HDAC2 or HDAC3 activity.
8. The composition for use according to any preceding embodiment, for use in a patient with elevated HDAC activity.
9. The composition for use according to any preceding embodiment, for use in the treatment or prevention of a disease or condition selected from the list consisting of: a neurodegenerative disease, such as Alzheimer's disease, Huntington's disease or Parkinson's disease; brain injury, such as stroke; a behavioural or psychiatric disorder, such as attention deficit hyperactivity disorder, obsessive compulsive disorder, anxiety disorder, biopolar disorder, or post-traumatic stress disorder; an inflammatory or autoimmune disease, such as asthma, arthritis, psoriasis, multiple sclerosis, diabetes, allograft rejection, graft-versus-host disease, or an inflammatory bowel disease, such as Crohn's disease; or cancer, such as prostate cancer, colorectal cancer, breast cancer, lung cancer, liver cancer or gastric cancer.
10. The composition for use according to embodiment 9, for use in the treatment or prevention or cancer, such as prostate cancer, colorectal cancer, breast cancer, lung cancer, liver cancer or gastric cancer.
11. The composition for use according to any of embodiments 1-9, for use in a method of treating or preventing a central nervous system disorder or condition.
12. The composition for use according to embodiment 11, for use in a method of treating or preventing a disorder or condition selected from the group consisting of autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's disease; Parkinson's disease; chronic pain; motor neuron disease; Huntington's disease; Guillain-Barre syndrome and meningitis.
13. A composition comprising a bacterial strain of the genus *Anaerostipes,* for use in the treatment or prevention of a disease or condition selected from the list consisting of: a neurodegenerative disease, such as Alzheimer's disease, Huntington's disease or Parkinson's disease, brain injury, such as stroke, an inflammatory or autoimmune disease, such as asthma, arthritis, psoriasis, multiple sclerosis, diabetes, allograft rejection, graft-versus-host disease, or an inflammatory bowel disease, such as Crohn's disease or ulcerative colitis; or cancer, such as prostate cancer, colorectal cancer, breast cancer, lung cancer, liver cancer or gastric cancer.
14. A composition comprising a bacterial strain of the genus *Anaerostipes,* for use for use in a method of treating or preventing a central nervous system disorder or condition, such as autism spectrum disorders (ASDs); child developmental disorder; obsessive compulsive disorder (OCD); major depressive disorder; depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's disease; Parkinson's disease; chronic pain; motor neuron disease; Huntington's disease; Guillain-Barre syndrome and meningitis.
15. The composition for use of any preceding embodiment, wherein the bacterial strain is of the species *Anaerostipes hadrus,* for example the *Anaerostipes hadrus* deposited as DSM 3319.
16. The composition for use of any preceding embodiment, wherein the bacterial strain has a 16s rRNA gene sequence that is at least 95%, 96%, 97%, 98%, 99%, 99.5% or 99.9% identical to SEQ ID NO:1 , SEQ ID NO: 6 or SEQ ID NO: 7 or wherein the bacterial strain has the 16s rRNA gene sequence represented by SEQ ID NO:1 SEQ ID NO: 6 or SEQ ID NO: 7..
17. The composition for use of any preceding embodiment, wherein the composition is for oral administration.
18. The composition for use of any preceding embodiment, wherein the bacterial strain is lyophilised.
19. A food product comprising the composition of any preceding embodiment, for the use of any preceding embodiment.
20. A composition comprising a bacterial strain of the genus *Eubacterium* or *Faecalicatena,* for use in a method of treating or preventing a central nervous system disorder or condition.
21. The composition for use of embodiment 20, wherein the composition is for use in a method of treating or preventing a neurodevelopmental disorder or a neuropsychiatric condition.
22. The composition for use of embodiment 20 or 21, wherein the composition is for use in a method of treating or preventing a disorder or condition selected from the group consisting of autism spectrum disorders (ASDs); child developmental disorder; obsessive compulsive disorder (OCD); major depressive disorder (MDD); depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; chronic pain; motor neuron disease; Huntington's disease; Guillain-Barre syndrome and meningitis.
23. The composition for use of embodiment 22, wherein the composition is for use in a method of treating or preventing autism spectrum disorder.
24. The composition for use of embodiment 22, wherein the composition is for use in a method of treating or preventing obsessive compulsive disorder.
25. The composition for use of embodiment 22, wherein the composition is for use in a method of treating or preventing major depressive disorder.
26. The composition for use of embodiment 20 or 21, wherein the composition is for use in a method of treating or preventing anxiety disorders, optionally wherein the anxiety disorder is generalised anxiety disorder (GAD); specific phobia; social anxiety disorder; separation anxiety disorder; agoraphobia; panic disorder and/or selective mutism.
27. The composition for use of any one of embodiments 20-26, wherein the composition is for use in preventing, reducing or alleviating stereotyped, repetitive, compulsive or anxious behaviour.
28. The composition for use of embodiment 20 wherein the composition is for use in a method of treating or preventing neurocognitive disorders, optionally wherein the neurocognitive disorder is vascular dementia; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.
29. The composition for use of any one of embodiments 20-28, wherein the bacterial strain has a 16s rRNA gene sequence that is at least 95%, 96%, 97%, 98%, 99%, 99.5% or 99.9% identical to SEQ ID NO: 8, 9, 10, 11, 12, 13 or 14.
30. The composition for use of embodiment 29, wherein the bacterial strain has a 16s rRNA gene sequence that is at least 95%, 96%, 97%, 98%, 99%, 99.5% or 99.9% identical to SEQ ID NO: 8, or wherein the bacterial strain has the 16s rRNA gene sequence represented by SEQ ID NO: 8.
31. The composition for use of any one of embodiments 20-30, wherein the bacterial strain is of the species *Eubacterium callanderi, Eubacterium limosum, Eubacterium eligens, Eubacterium rectale, Eubacterium hallii, Faecalicatena fissicatena* or *Faecalicatena contorta.*
32. The composition for use of embodiment 31, wherein the bacterial strain is of the species *Eubacterium callanderi.*
33. A cell of the bacterial strain deposited under accession number NCIMB 43455, or a derivative thereof.
34. A cell according to embodiment 33, for use in therapy, optionally wherein the cell is for use in a method according to any of embodiments 20-28.

## Claims

1. A composition comprising a bacterial strain of the species Anaerostipes hadrus and a pharmaceutically acceptable excipient, diluent or carrier for use in a method of treating or preventing a central nervous system disorder or condition.

2. The composition for use of any preceding claim wherein the composition is for use in a method of treating or preventing a neurodevelopmental disorder or a neuropsychiatric condition.

3. The composition for use of any preceding claim, wherein the composition is for use in a method of treating or preventing a disorder or condition selected from the group consisting of autism spectrum disorders (ASDs); child developmental disorder; obsessive compulsive disorder (OCD); major depressive disorder (MDD); depression; seasonal affective disorder; anxiety disorders; chronic fatigue syndrome (myalgic encephalomyelitis); stress disorder; post-traumatic stress disorder; schizophrenia spectrum disorders; schizophrenia; bipolar disorder; psychosis; mood disorder; dementia; Alzheimer's; Parkinson's disease; chronic pain; motor neuron disease; Huntington's disease; Guillain-Barre syndrome and meningitis.

4. The composition for use of Claim 3, wherein the composition is for use in a method of treating or preventing:
- autism spectrum disorder.
- obsessive compulsive disorder and/or
- major depressive disorder.

5. The composition for use of Claim 1 or Claim 2, wherein the composition is for use in a method of treating or preventing anxiety disorders, optionally wherein the anxiety disorder is generalised anxiety disorder (GAD); specific phobia; social anxiety disorder; separation anxiety disorder; agoraphobia; panic disorder and/or selective mutism.

6. The composition for use of any preceding claim, wherein the composition is for use in preventing, reducing or alleviating stereotyped, repetitive, compulsive or anxious behaviour.

7. The composition for use of Claim 1, wherein the composition is for use in a method of treating or preventing neurocognitive disorders, optionally wherein the neurocognitive disorder is vascular dementia; mixed form Alzheimer's disease and vascular dementia; Lewy body disease; frontotemporal dementia; Parkinson's dementia; Creutzfeldt-Jakob disease; Huntington's disease; and Wernicke-Korsakoff syndrome.

8. The composition for use of any preceding claim, wherein the bacterial strain has a 16s rRNA gene sequence that is at least 95%, 96%, 97%, 98%, 99%, 99.5% or 99.9% identical to SEQ ID NO: 6, 7 or 1, or wherein the bacterial strain has the 16s rRNA gene sequence represented by SEQ ID NO: 6, 7 or 1, optionally wherein the bacterial strain is deposited under accession number NCIMB 43457, NCIMB 43526 or DSM 3319.

9. The composition for use of any preceding claim, wherein the composition does not comprise both of the bacterial strains deposited under accession numbers Anaerostipes caccae DSM 14662 and Anaerostipes hadrus DSM 3319 / ATCC 29173.

10. The composition for use of any preceding claim, wherein the composition comprises fewer than 40, 30, 20 or 10 different bacterial strains.

11. The composition for use of any preceding claim, wherein the composition is for oral administration and/or wherein the bacterial strain is lyophilised.

12. A food product or vaccine composition comprising the composition of any preceding claim, for the use of any preceding claim.

13. A cell of the bacterial strain deposited under accession number NCIMB 43457.

14. A cell of the bacterial strain deposited under accession number NCIMB 43526.

15. A cell according to Claim 13 or Claim 14, for use in therapy, optionally wherein the cell is for use in a method according to any of Claims 1 to 7.
